(19) Europäisches Patentamt
European Patent Office
Office européen des brevets

(11) EP 4 512 804 A1

(12) EUROPEAN PATENT APPLICATION
published in accordance with Art. 153(4) EPC

(43) Date of publication:
**26.02.2025 Bulletin 2025/09**

(21) Application number: **23791296.9**

(22) Date of filing: **19.04.2023**

(51) International Patent Classification (IPC):
***C07D 403/14*** *(2006.01)* ***A61K 31/506*** *(2006.01)*
***A61P 19/02*** *(2006.01)* ***A61P 17/00*** *(2006.01)*

(52) Cooperative Patent Classification (CPC):
**A61K 31/4725; A61K 31/4985; A61K 31/506; A61K 31/517; A61K 31/519; A61K 31/53; A61K 31/675; A61P 1/16; A61P 1/18; A61P 3/00; A61P 3/10; A61P 7/02; A61P 9/00; A61P 9/10; A61P 13/12;** (Cont.)

(86) International application number:
**PCT/CN2023/089314**

(87) International publication number:
**WO 2023/202642 (26.10.2023 Gazette 2023/43)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **20.04.2022 CN 202210420382**

(71) Applicant: **Shanghai Zhimeng Biopharma, Inc.**
**Shanghai 201210 (CN)**

(72) Inventors:
- **LIANG, Bo**
  **Shanghai 201210 (CN)**
- **HUANG, Mengwei**
  **Shanghai 201210 (CN)**
- **LIU, Gang**
  **Shanghai 201210 (CN)**
- **JIANG, Zhaojian**
  **Shanghai 201210 (CN)**
- **CHEN, Huanming**
  **Shanghai 201210 (CN)**

(74) Representative: **Santoro, Sofia et al**
**Società Italiana Brevetti S.p.A.**
**Via Giosuè Carducci, 8**
**20123 Milano (IT)**

# (54) RHO-ASSOCIATED PROTEIN KINASE INHIBITOR AND PREPARATION AND USE THEREOF

(57) The present invention relates to a Rho-associated protein kinase inhibitor and preparation and use thereof. Specifically, the compound of the present invention has a structure represented by formula I, wherein the definitions of each group and each substituent are as described in the description. Also disclosed in the present invention are a pharmaceutical composition related to the compound, a preparation method and use thereof in the manufacture of a medicament for preventing or treating a Rho-associated protein kinase (ROCK)-mediated disease.

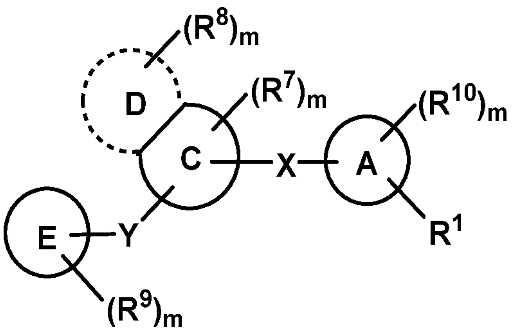

Formula I

EP 4 512 804 A1

Processed by Luminess, 75001 PARIS (FR)

(Cont. next page)

(52) Cooperative Patent Classification (CPC): (Cont.)
**A61P 17/00; A61P 17/06; A61P 19/02; A61P 19/10; A61P 25/00; A61P 27/02; A61P 29/00; A61P 31/10; A61P 31/12; A61P 33/00; A61P 35/00; A61P 37/06; C07D 401/14; C07D 403/14; C07D 409/14; C07D 487/04; C07D 491/056; C07F 9/6558**

## Description

### THCHNICAL FIFLD

The present invention relates to the field of biomedicine and specifically to Rho-associated protein kinase inhibitors and their preparation and application.

### BACKGROUND ART

Rho-associated kinases (ROCKs) are serine/threonine kinases in the AGC kinase family and include two isoforms, ROCK1 and ROCK2. ROCK2 (ROCKα) was first identified as a RhoA-binding protein, and ROCK1 (ROCKβ or p160ROCK) was subsequently found to be a highly homologous RhoA-binding protein to ROCK2. RhoA is a GTPase that activates and promotes the activation of downstream effectors upon binding to GTP. ROCKs have been extensively studied over the past two decades as major effectors of RhoA proteins that achieve their biological functions by phosphorylating downstream effectors (MYPT1, MLC, Moesin, etc.). More than 30 substrates of ROCKs have been identified, which are involved in the regulation of multiple processes such as cytoskeleton dynamics, cell morphology, and cell contraction. One of the most studied and reported substrates is myosin phosphatase target subunit 1 (MYPT1), the myosin-binding subunit of myosin light chain phosphatase (MLCP). ROCKs mediate the phosphorylation of MYPT1 at the Thr697 and Thr855 sites to inhibit the catalytic activity of MLCP, which in turn regulates the phosphorylation of myosin light chain (MLC) and affects the generation of cellular contractility. Studies have shown that the development of fibrosis, cardiovascular, metabolic, and neurological diseases, as well as cancer, are associated with ROCKs-mediated pathways, and thus ROCKs are considered to be important targets for the treatment of these diseases.

ROCK1 and ROCK2 share 64% amino acid sequence similarity and an even higher 92% similarity in the kinase structural domain, which gives the two isoforms some common effector proteins. A variety of small molecule ROCK inhibitors have been reported, most of which have comparable inhibitory activity against both ROCKs isoforms. Only two ROCK inhibitors have been approved for topical clinical use, Fasudil, first approved in Japan and China for the treatment of cerebral vasospasm, and Ripasudil, approved in Japan for the treatment of glaucoma. Since simultaneous inhibition of both subtypes of ROCKs has potential side effects such as decreased blood pressure, there are currently no small-molecule ROCK inhibitors suitable for systemic administration. The development of small molecule inhibitors for specific subtypes will be an important direction in the development of ROCK-targeted drugs.

### SUMMARY OF THE INVENTION

The purpose of the present invention is to provide a compound shown in Formula I and its preparation method and its use as an inhibitor of RHO-associated frizzled helix-containing protein kinase.

In the first aspect of the present invention, it provides a compound shown in Formula I or a pharmaceutically acceptable salt, stereoisomer, polymorph, solvate, N-oxide, isotope labeled compound, metabolite or prodrug thereof:

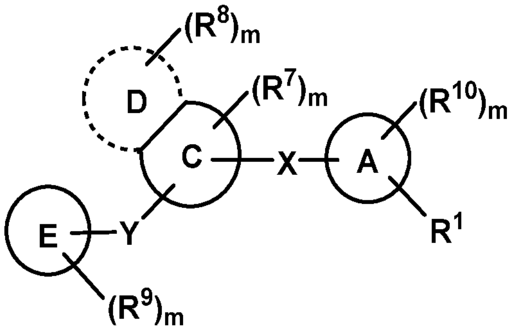

Formula I

wherein,

X is selected from the group consisting of

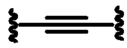, 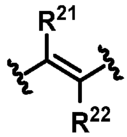, 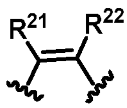;

$R^{21}$, $R^{22}$ are each independently selected from the group consisting of H, $C_{1-6}$ alkyl, $C_{3-10}$ cycloalkyl;
Y is selected from the group consisting of none, C(=O), O, $S(=O)_i$, $CR^{23}R^{24}$, NR;
R is selected from the group consisting of H, $C_{1-6}$ alkyl, $C_{2-6}$ alkenyl, $C_{2-6}$ alkynyl, saturated or partially unsaturated $C_{3-10}$ cycloalkyl, saturated or unsaturated 3-10 membered heterocyclyl containing 1, 2, or 3 heteroatoms selected from N, O or S, $C_{6-10}$ aryl, 5-14 membered heteroaryl containing 1, 2, or 3 heteroatoms selected from N, O or S, $C_{6-12}$ aralkyl, and at most two ring members in the cycloalkyl and heterocyclyl are C(=O);
$R^{23}$, $R^{24}$ are each independently selected from the group consisting of H, halogen, C $_{1-6}$ alkyl, $C_{3-10}$ cycloalkyl;
Ring A is selected from the group consisting of saturated or partially unsaturated $C_{3-10}$ cycloalkyl, saturated or partially unsaturated 3-10-membered heterocyclyl containing 1, 2 or 3 heteroatoms selected from N, O or S, $C_{6-10}$ aryl, 5-14-membered heteroaryl containing 1, 2 or 3 heteroatoms selected from N, O or S, and at most two ring members in the cycloalkyl and heterocyclyl are C(=O);
Ring C is selected from the group consisting of saturated or partially unsaturated $C_{3-10}$ cycloalkyl, saturated or partially unsaturated 3-10-membered heterocyclyl containing 1, 2 or 3 heteroatoms selected from N, O or S, $C_{6-10}$ aryl, and 5-14-membered heteroaryl containing 1, 2 or 3 heteroatoms selected from N, O or S, and at most two ring members in the cycloalkyl and heterocyclyl are C(=O);
Ring D is absent or is selected from the group consisting of saturated or partially unsaturated $C_{3-10}$ cycloalkyl, saturated or partially unsaturated 3-10-membered heterocyclyl containing 1, 2 or 3 heteroatoms selected from N, O or S, $C_{6-10}$ aryl, 5-14-membered heteroaryl containing 1, 2 or 3 heteroatoms selected from N, O or S, and at most two ring members in the cycloalkyl and heterocyclyl are C(=O);
Ring E is selected from the group consisting of

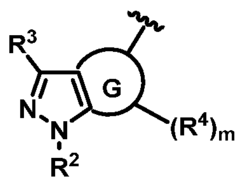,

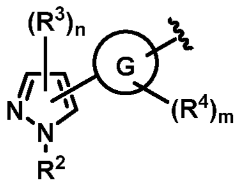, 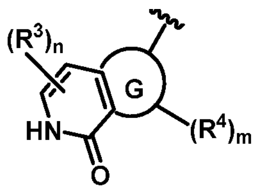, 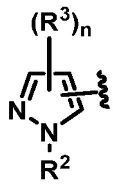;

Ring G is selected from the group consisting of saturated or partially unsaturated $C_{3-10}$ cycloalkyl, saturated or partially unsaturated 3-10-membered heterocyclyl containing 1, 2 or 3 heteroatoms selected from N, O or S, $C_{6-10}$ aryl, and 5-14-membered heteroaryl containing 1, 2 or 3 heteroatoms selected from N, O or S, and at most two ring members in the cycloalkyl and heterocyclyl are C(=O);
$R^1$ is selected from the group consisting of H, halogen, -CN, $-NH_2$ , $C_{1-6}$ alkyl, $C_{6-10}$ aryl, 5-14-membered heteroaryl containing 1, 2 or 3 heteroatoms selected from N, O or S, N-methyltetrahydropyrrolyl, N-methylpiperidinyl, acetyl, -C(=O)-($C_{1-6}$ alkylene)$_n$-$CF_3$ , -($C_{1-6}$ alkylene)$_n$-$CF_3$ , -C(=O) -($C_{1-6}$ alkylene)$_n$-CN, -C(=O)-(saturated or partially unsaturated $C_{3-10}$ cycloalkyl)$_n$, -NH-C(=O)-(saturated or partially unsaturated $C_{3-10}$ cycloalkyl), -C(=O)-(saturated or partially unsaturated 3-10-membered heterocyclyl containing 1, 2 or 3 heteroatoms selected from N, O or S), -C(=O)-$C_{1-6}$ alkylene-(saturated or partially unsaturated 3-10-membered heterocyclyl containing 1, 2 or 3 heteroatoms selected from N, O or S), -C(=O)-(5-14-membered heteroaryl containing 1, 2 or 3 heteroatoms selected from N, O or S), -C(=O)-$C_{1-6}$ alkylene-NH-($C_{1-6}$ alkyl), -C(=O)-$C_{1-6}$ alkylene-N ($C_{1-6}$ alkyl)$_2$, N-methylpiperazine-substituted acetyl, $-S(=O)_2R^{1a}$, $-P(=O)R^{1a}R^{1b}$,

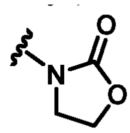,

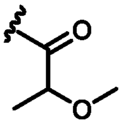, 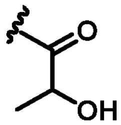, 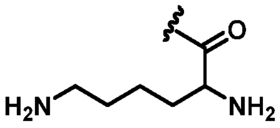, 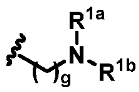, 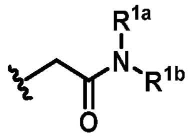, 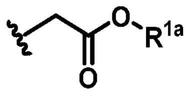,

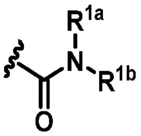, 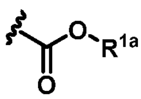, 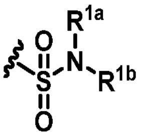, 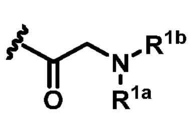, 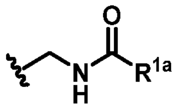;

$R^{1a}$ and $R^{1b}$ are each independently selected from the group consisting of H, halogen, amino, cyano, nitro, $C_{1-6}$ alkyl, $C_{2-6}$ alkenyl, $C_{2-6}$ alkynyl, $C_{3-10}$ cycloalkyl, 3-10-membered heterocyclyl containing 1, 2 or 3 heteroatoms selected from N, O or S, $C_{6-10}$ aryl, 5-14-membered heteroaryl containing 1, 2 or 3 heteroatoms selected from N, O or S, $C_{6-12}$ aralkyl, $-C(=O)R^5$, $-OC(=O)R^5$, $-C(=O)OR^5$, $-OR^5$, $-SR^5$, $-S(=O)R^5$, $-S(=O)_2R^5$, $-S(=O)_2NR^5R^6$, $-NR^5R^6$, $-C(=O)NR^5R^6$, $-NR^5-C(=O)R^6$, $-NR^5-C(=O)OR^6$, $-NR^5-S(=O)_2-R^6$, $-NR^5-C(=O)-NR^5R^6$, $-C_{1-6}$ alkylene-$NR^5R^6$, $-C_{1-6}$ alkylene-$OR^5$, $-O-C_{1-6}$ alkylene $-NR^5R^6$; or $R^{1a}$ and $R^{1b}$ together with the atoms to which they are attached constitute a 3-12-membered heterocyclyl or heteroaryl containing 1, 2 or 3 heteroatoms selected from N, O or S;

$R^3$, $R^4$, $R^7$, $R^8$, $R^9$ and $R^{10}$ are each independently at each occurrence selected from the group consisting of H, halogen, amino, cyano, nitro, $C_{1-6}$ alkyl, deuterated $C_{1-6}$ alkyl, halogenated $C_{1-6}$ alkyl, $C_{2-6}$ alkenyl, $C_{2-6}$ alkynyl, $C_{3-10}$ cycloalkyl, 3-10-membered heterocyclyl containing 1, 2 or 3 heteroatoms selected from N, O or S, $C_{6-10}$ aryl, 5-14-membered heteroaryl containing 1, 2 or 3 heteroatoms selected from N, O or S, $C_{6-12}$ aralkyl, $-C(=O)R^5$, $-OC(=O)R^5$, $-C(=O)OR^5$, $-OR^5$, $-SR^5$, $-S(=O)R^5$, $-S(=O)_2R^5$, $-S(=O)_2NR^5R^6$, $-NR^5R^6$, $-C(=O)NR^5R^6$, $-NR^5-C(=O)R^6$, $-NR^5-C(=O)OR^6$, $-NR^5-S(=O)_2-R^6$, $-NR^5-C(=O)-NR^5R^6$, $-C_{1-6}$ alkylene-$NR^5R^6$, $-C_{1-6}$ alkylene-$OR^5$, $-O-C_{1-6}$ alkylene-$NR^5R^6$, $-C_{1-6}$ alkylene-$O-(P=O)(OH)_2$;

$R^2$ is selected from the group consisting of H, halogen, amino, cyano, nitro, $C_{1-6}$ alkyl, deuterated $C_{1-6}$ alkyl, halogenated $C_{1-6}$ alkyl, $C_{2-6}$ alkenyl, $C_{2-6}$ alkynyl, $C_{3-10}$ cycloalkyl, 3-10-membered heterocyclyl containing 1, 2 or 3 heteroatoms selected from N, O or S, $C_{6-10}$ aryl, 5-14-membered heteroaryl containing 1, 2 or 3 heteroatoms selected from N, O or S, $C_{6-12}$ aralkyl, $-C(=O)R^5$, $-OC(=O)R^5$, $-C(=O)OR^5$, $-OR^5$, $-SR^5$, $-S(=O)R^5$, $-S(=O)_2R^5$, $-S(=O)_2NR^5R^6$, $-NR^5R^6$, $-C(=O)NR^5R^6$, $-NR^5-C(=O)R^6$, $-NR^5-C(=O)OR^6$, $-NR^5-S(=O)_2-R^6$, $-NR^5-C(=O)-NR^5R^6$, $-C_{1-6}$ alkylene-$NR^5R^6$, $-C_{1-6}$ alkylene-$OR^5$, $-O-C_{1-6}$ alkylene-$NR^5R^6$, $-C_{1-6}$ alkylene-$O-(P=O)(OH)_2$, $-C_{1-6}$ alkylene-$O(C=O)R^{11}$, $-C_{1-6}$ alkylene-$O(P=O)(OR^{12})(NR^{13}R^{14})$, $-C_{1-6}$ alkylene-$O(P=O)(OR^{12})(OR^{13})$;

$R^{11}$, $R^{12}$, $R^{13}$, $R^{14}$ are each independently at each occurrence selected from the group consisting of H, $C_{1-6}$ alkyl, $C_{3-6}$ cycloalkyl, 3-10-membered heterocyclyl containing 1, 2 or 3 heteroatoms selected from N, O or S, $C_{6-10}$ aryl, 3-10-membered heteroaryl containing 1, 2 or 3 heteroatoms selected from N, O or S, and cation;

the cation is selected from the group consisting of $Na^+$, $K^+$, $Ca^{2+}$, $Mg^{2+}$, $NH^{4+}$;

the alkyl, alkylene, alkenyl, alkynyl, cycloalkyl, heterocyclyl, aryl, heteroaryl, and aralkyl are each optionally at each occurrence substituted with one or more substituents independently selected from the group consisting of halogen, hydroxyl, oxo, amino, cyano, nitro, $C_{1-6}$ alkyl, $C_{2-6}$ alkenyl, $C_{2-6}$ alkynyl, $C_{3-10}$ cycloalkyl, 3-10-membered heterocyclyl containing 1, 2 or 3 heteroatoms selected from N, O or S, $C_{6-10}$ aryl, 5-14-membered heteroaryl containing 1, 2 or 3 heteroatoms selected from N, O or S, $C_{6-12}$ aralkyl, $=N-OR^5$, $-C(=NH)NH_2$, $-C(=O)R^5$, $-OC(=O)R^5$, $-C(=O)OR^5$, $-OR^5$, $-SR^5$, $-S(=O)R^5$, $-S(=O)_2R^5$, $-S(=O)_2NR^5R^6$, $-NR^5R^6$, $-C(=O)NR^5R^6$, $-NR^5-C(=O)R^6$, $-NR^5-C(=O)OR^6$, $-NR^5-S(=O)_2-R^6$, $-NR^5-C(=O)-NR^5R^6$, $-C_{1-6}$ alkylene-$NR^5R^6$, $-C_{1-6}$ alkylene-$OR^5$, $-O-C_{1-6}$ alkylene-$NR^5R^6$;

$R^5$ and $R^6$ are each independently at each occurrence selected from the group consisting of H, $C_{1-6}$ alkyl, $C_{3-10}$ cycloalkyl, 3-10-membered heterocyclyl containing 1, 2, or 3 heteroatoms selected from N, O, or S, $C_{6-10}$ aryl, 5-14-membered heteroaryl containing 1, 2, or 3 heteroatoms selected from N, O, or S, and $C_{6-12}$ aralkyl;

each m is independently at each occurrence selected from the group consisting of 0, 1, 2, 3;

each n is independently at each occurrence selected from the group consisting of 0, 1, 2;

i is selected from the group consisting of 1, 2;

g is selected from the group consisting of 0, 1, 2, 3, 4.

In another preferred embodiment,

X is selected from the group consisting of

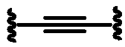,
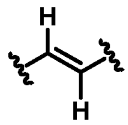,
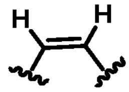,
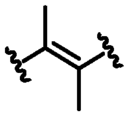,
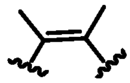;

Y is selected from the group consisting of NH, $NCH_3$.

In another preferred embodiment,

Ring C is

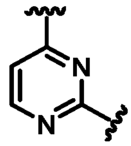;

Ring E is selected from the group consisting of

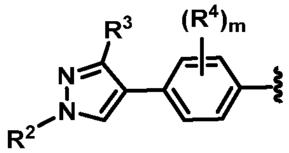,
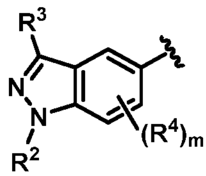,
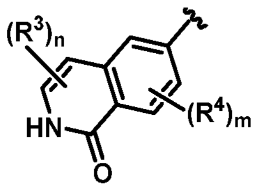.

In another preferred embodiment,

Ring A is selected from the group consisting of saturated or partially unsaturated $C_{3-10}$ cycloalkyl, saturated or partially unsaturated 3-10-membered heterocyclyl containing 1, 2 or 3 heteroatoms selected from N, O or S, $C_{6-10}$ aryl, 5-14-membered heteroaryl containing 1, 2 or 3 heteroatoms selected from N, O or S; and at most two ring members in the cycloalkyl and heterocyclyl are C(=O);

Ring D is absent or is selected from the group consisting of saturated or partially unsaturated C3-10 cycloalkyl, saturated or partially unsaturated 3-10-membered heterocyclyl containing 1, 2 or 3 heteroatoms selected from N, O or S.

In another preferred embodiment,

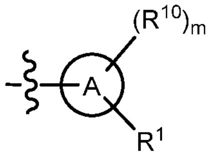

is selected from the group consisting of

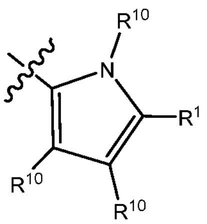 , 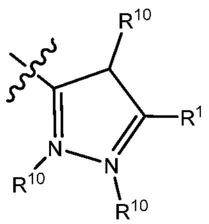 , 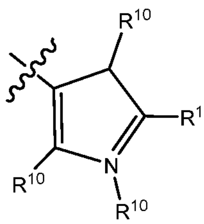 , 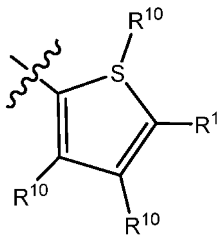 ,

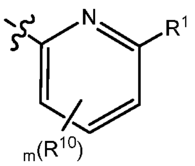 , 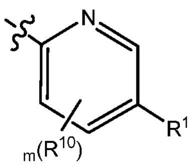 , 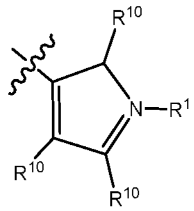 , 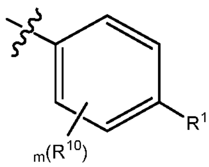 , 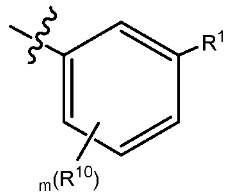 ,

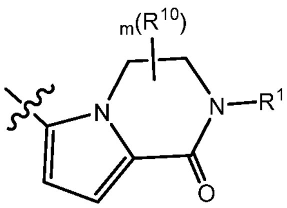 , 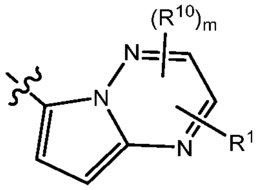 .

In another preferred embodiment, Ring D is absent or selected from the group consisting of

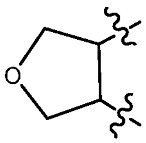 , 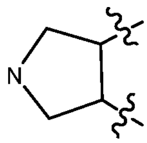 , 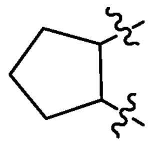 , 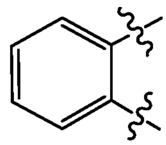 .

In another preferred embodiment, Ring G is selected from the group consisting of

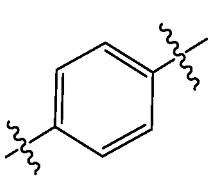 , 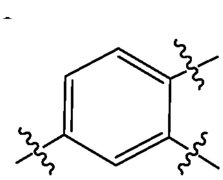 , 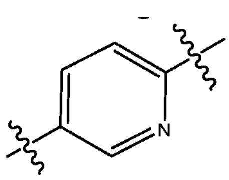 .

In another preferred embodiment, $R^1$ is selected from the group consisting of H, -C(=O)-($C_{1-6}$ alkylene)$_n$-$CF_3$, -($C_{1-6}$ alkylene)$_n$-$CF_3$, -C(=O)-(saturated or partially unsaturated $C_{3-10}$ cycloalkyl)$_n$,

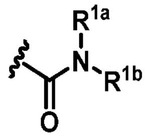

;

$R^{1a}$ and $R^{1b}$ together with the atoms to which they are attached constitute a 3-12-membered heterocyclyl or heteroaryl containing 1, 2 or 3 heteroatoms selected from N, O or S.

In another preferred embodiment, $R^1$ is selected from the group consisting of H,

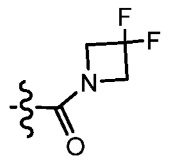 , 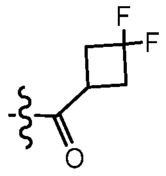 , 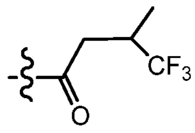 , 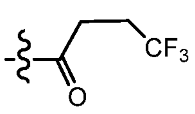 , 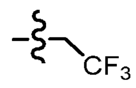 , 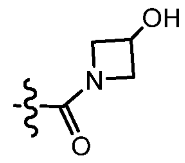 ,

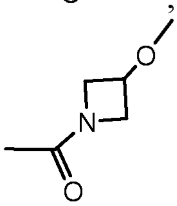 .

In another preferred embodiment, $R^2$ is selected from the group consisting of H, halogen, amino, cyano, nitro, $C_{1-6}$ alkyl, deuterated $C_{1-6}$ alkyl, halogenated $C_{1-6}$ alkyl, $C_{2-6}$ alkenyl, $C_{2-6}$ alkynyl, $C_{3-10}$ cycloalkyl, 3-10-membered heterocyclyl containing 1, 2 or 3 heteroatoms selected from N, O or S, $C_{6-10}$ aryl, 5-14-membered heteroaryl containing 1, 2 or 3 heteroatoms selected from N, O or S, $-C(=O)R^5$, $-OC(=O)R^5$, $-C(=O)OR^5$, $-C_{1-6}$ alkylene-O-$(P=O)(OH)_2$, $-C_{1-6}$ alkylene-$O(C=O)R^{11}$, $-C_{1-6}$ alkylene-$O(P=O)(OR^{12})$ $(NR^{13}R^{14})$, - $C_{1-6}$ alkylene-$O(P=O)(OR^{12})$ $(OR^{13})$;

$R^{11}$, $R^{12}$, $R^{13}$, $R^{14}$ are each independently at each occurrence selected from the group consisting of H, $C_{1-6}$ alkyl, $C_{3-6}$ cycloalkyl, 3-10-membered heterocyclyl containing 1, 2 or 3 heteroatoms selected from N, O or S, $C_{6-10}$ aryl, 3-10-membered heteroaryl containing 1, 2 or 3 heteroatoms selected from N, O or S, and cation;
the cation is selected from the group consisting of $Na^+$, $K^+$, $Ca^{2+}$, $Mg^{2+}$, $NH^{4+}$.

In another preferred embodiment, $R^2$ is selected from the group consisting of H, - $C_{1-6}$ alkylene-$O(C=O)R^{11}$, $-C_{1-6}$ alkylene-$O(P=O)(OR^{12})(NR^{13}R^{14})$, $-C_{1-6}$ alkylene-$O(P=O)(OR^{12})$ $(OR^{13})$;

$R^{11}$, $R^{12}$, $R^{13}$, $R^{14}$ are each independently at each occurrence selected from the group consisting of H, $C_{1-6}$ alkyl, 3-10-membered heterocyclyl containing 1, 2 or 3 heteroatoms selected from N, O or S, $C_{6-10}$ aryl, and cation;
the cation is selected from the group consisting of $Na^+$, $K^+$, $Ca^{2+}$, $Mg^{2+}$, $NH^{4+}$.

In another preferred embodiment, the compound has a structure of any of the following formula:

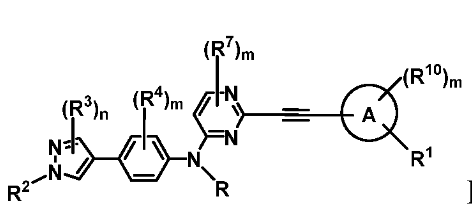

Formula Va

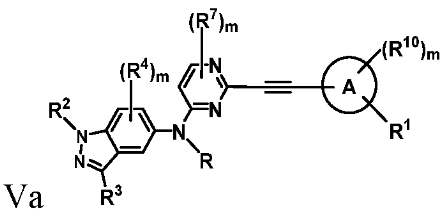

Formula Vb

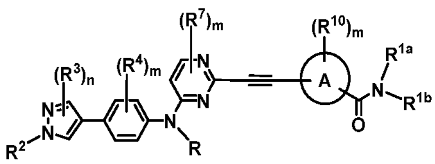

Formula Vc

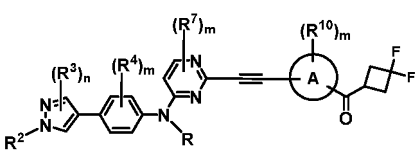

Formula Vd

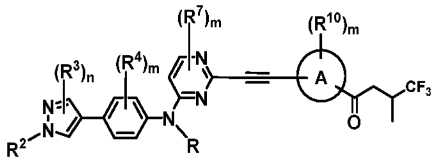 Formula Ve 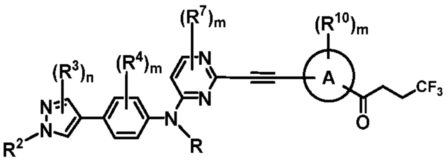 Formula Vf

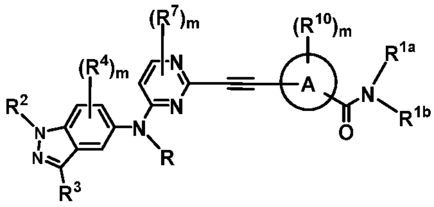 Formula Vg 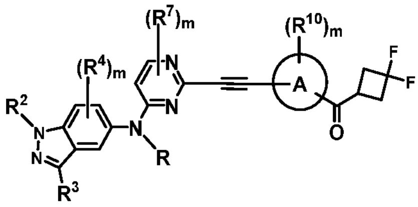 Formula Vh

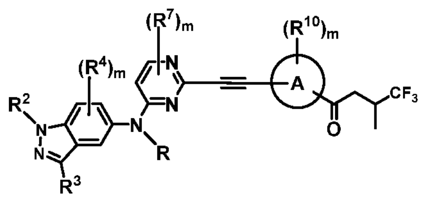 Formula Vi 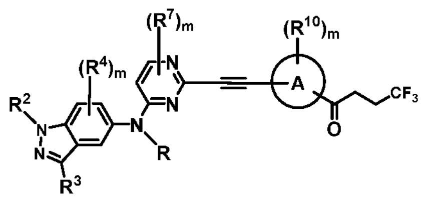 Formula Vj

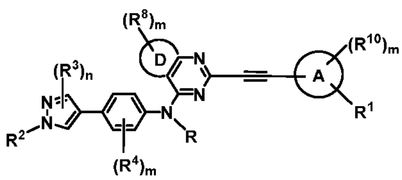 Formula Vk 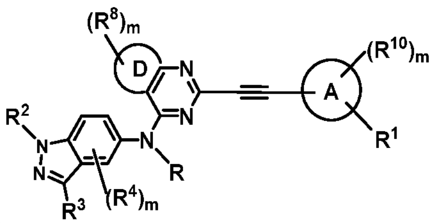 Formula Vl

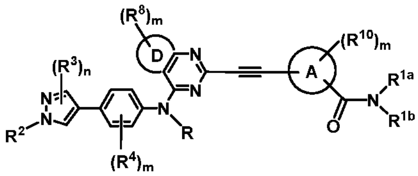 Formula Vm 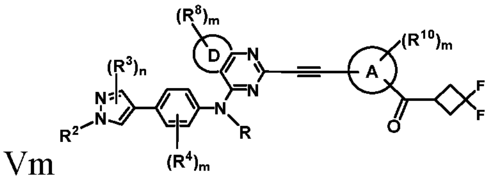 Formula Vn

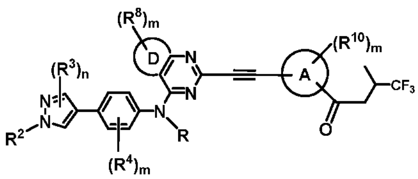 Formula Vp 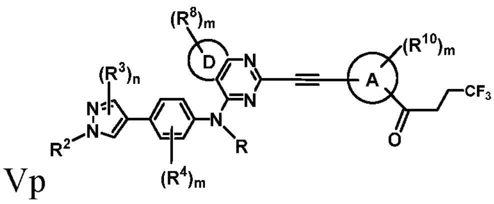 Formula Vq

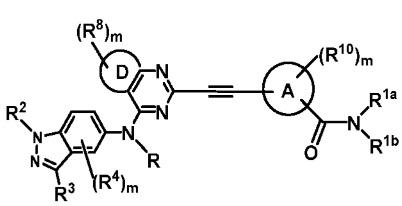 Formula Vr 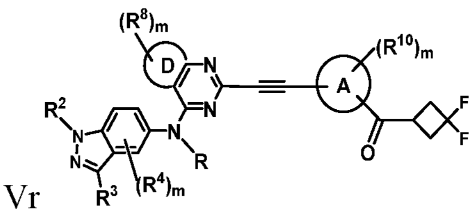 Formula Vs

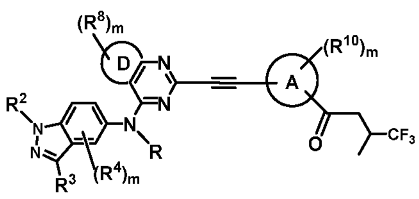 Formula Vt 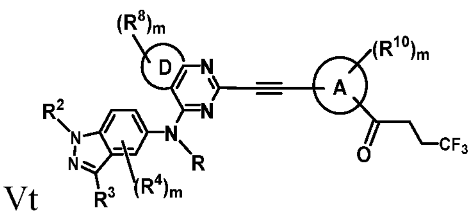 Formula Vu

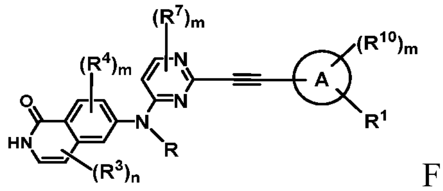 Formula Vw 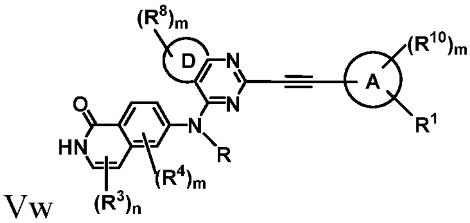 Formula Vx

wherein, each group is as defined above.

In another preferred embodiment, the compound is selected from the group consisting of:

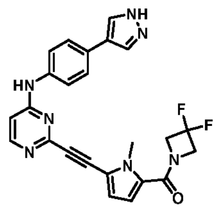
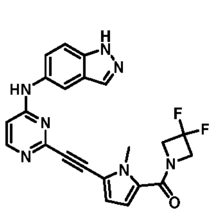
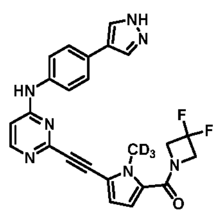
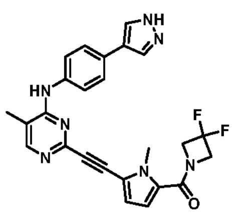
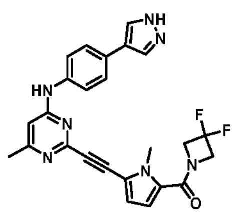
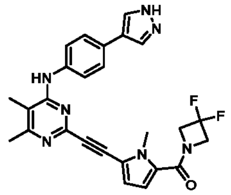
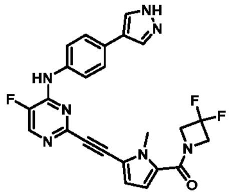
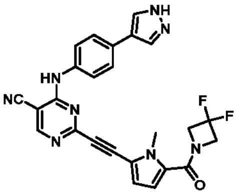
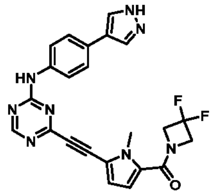
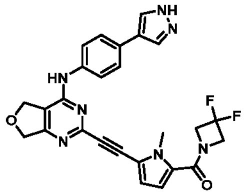
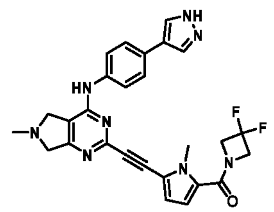
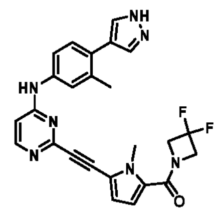
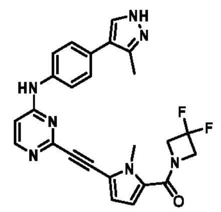
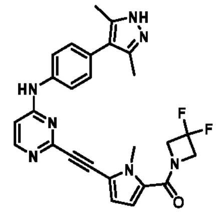
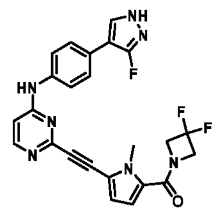
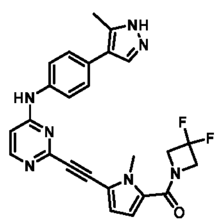
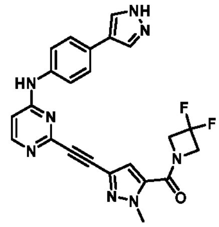
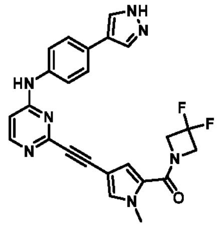
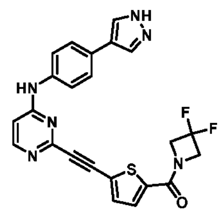
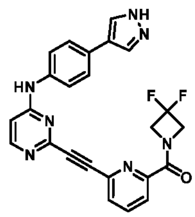
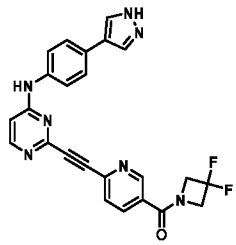
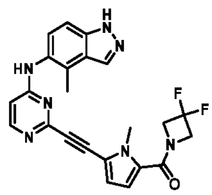
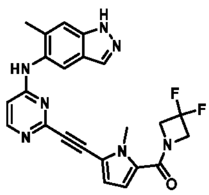
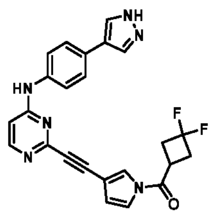
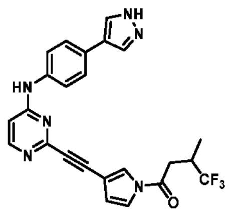
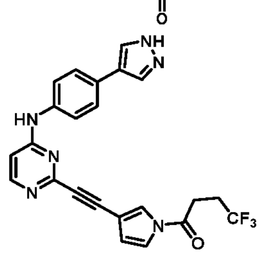
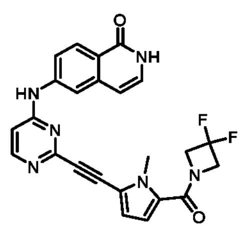

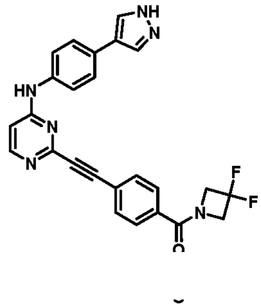 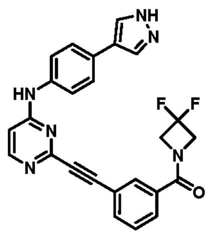 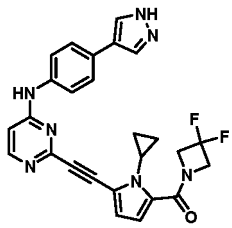 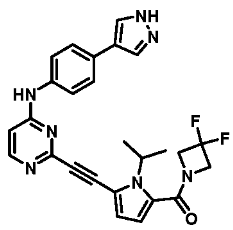 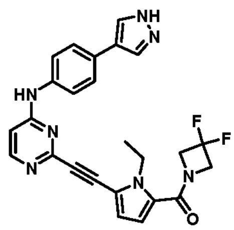

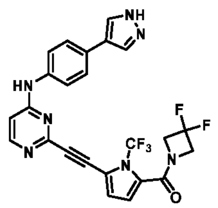 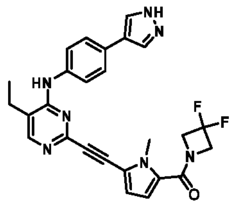 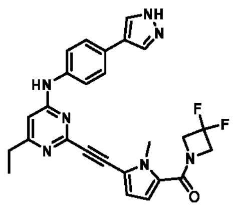 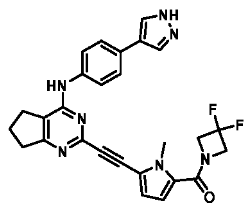 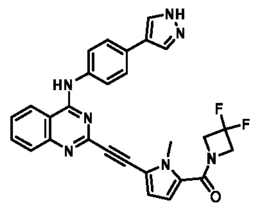

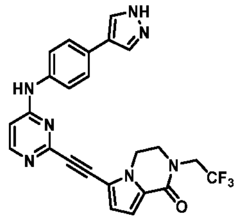 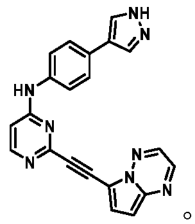 。

In another preferred embodiment, the compound is selected from the group consisting of:

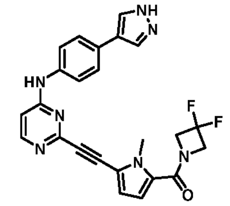
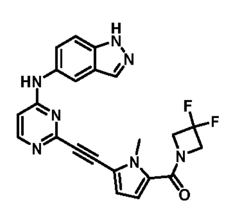
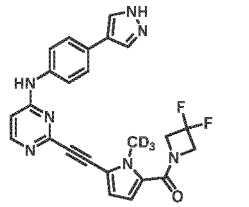
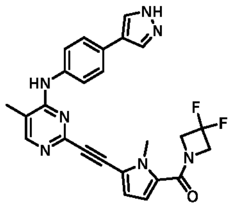
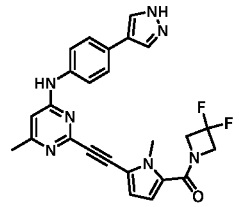
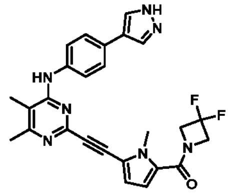
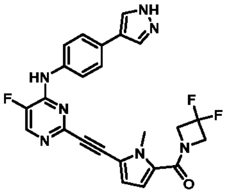
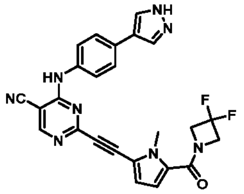
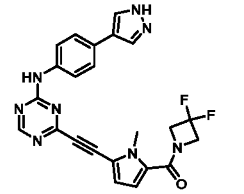
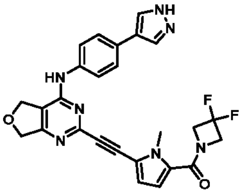
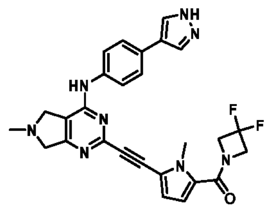
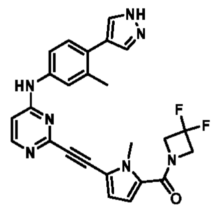
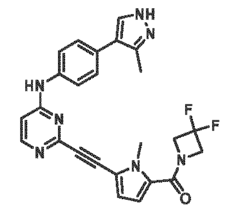
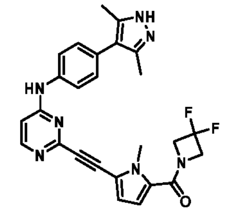
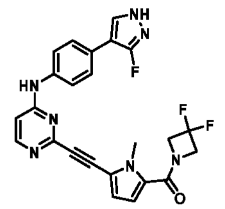
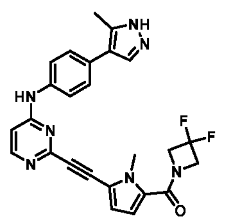
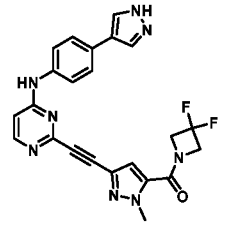
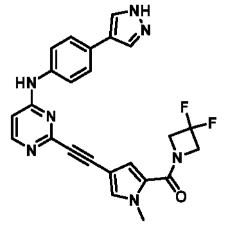
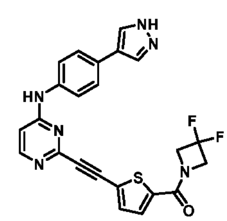
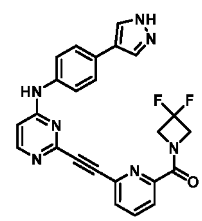
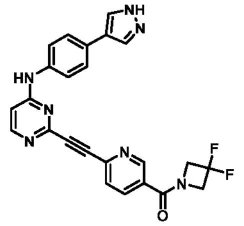
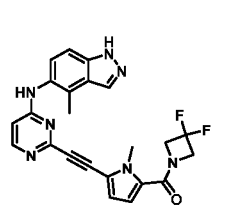
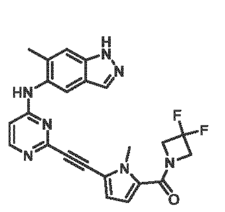
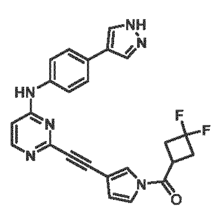
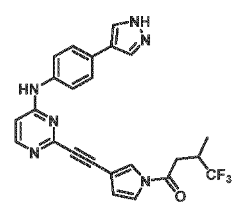
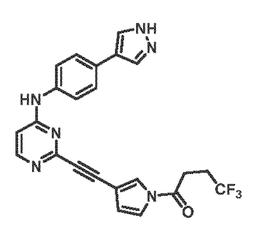
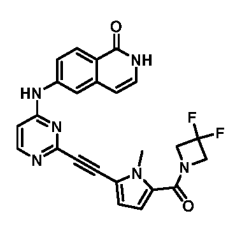
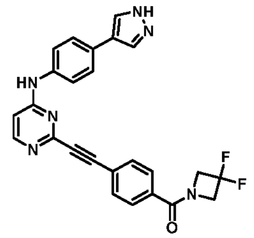
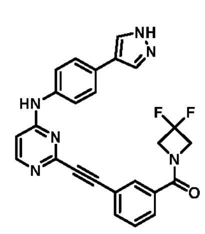
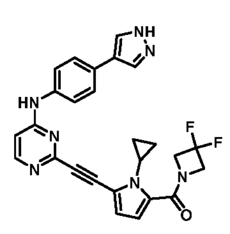
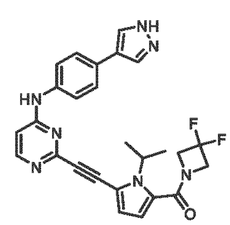
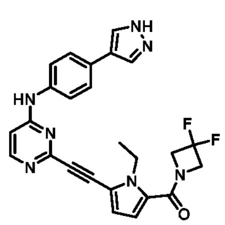
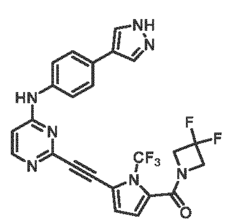
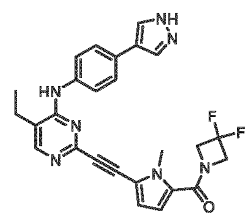
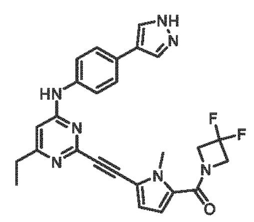

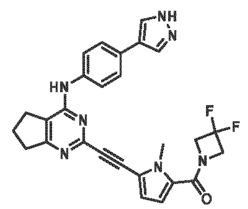
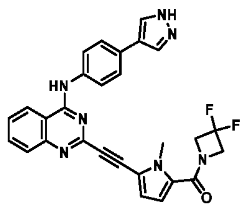
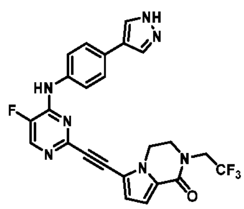
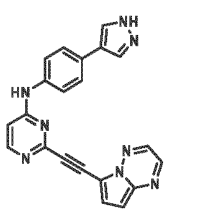
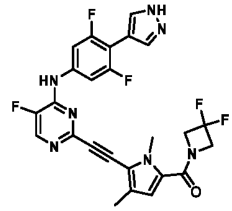
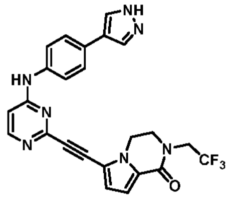
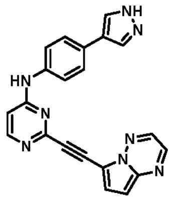
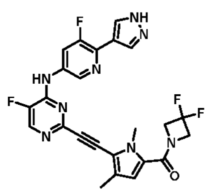
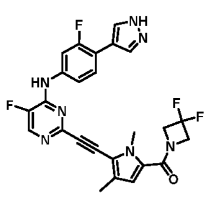
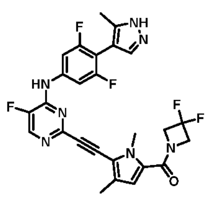
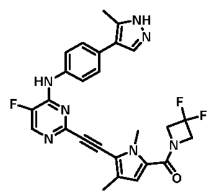
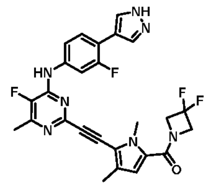
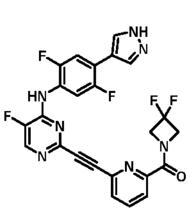
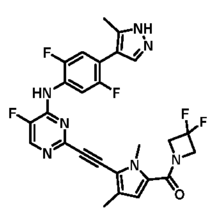
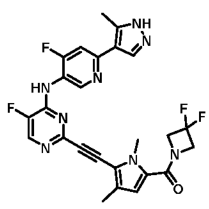
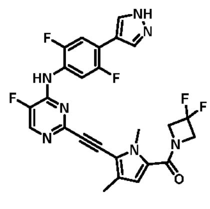
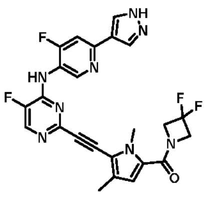
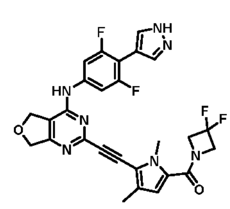
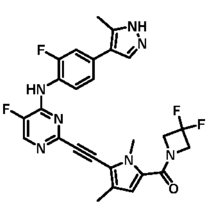
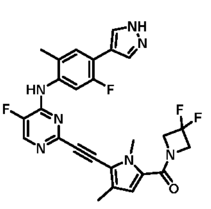
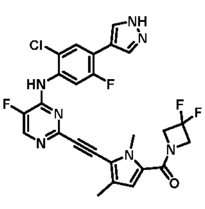
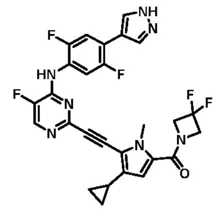
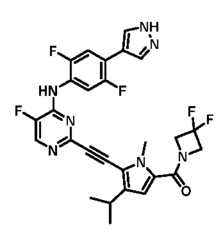
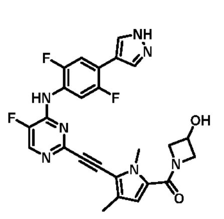
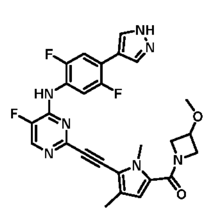
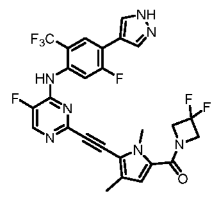
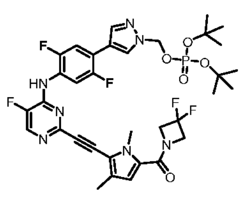
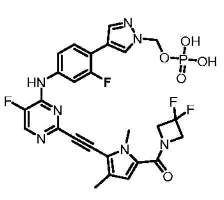
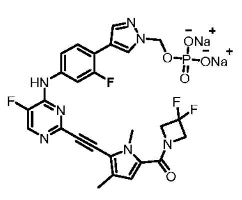
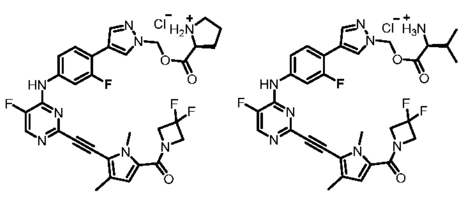

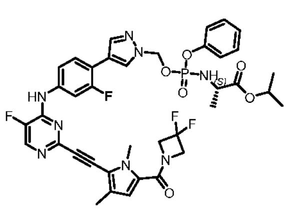
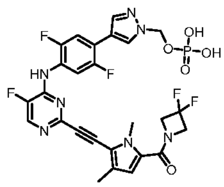
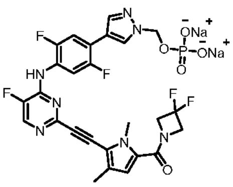
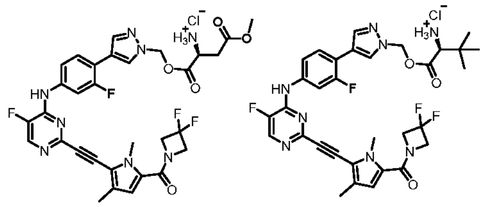

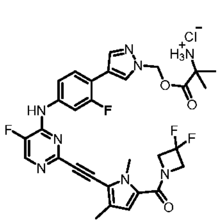
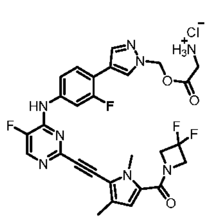
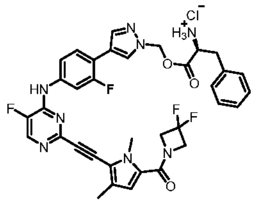

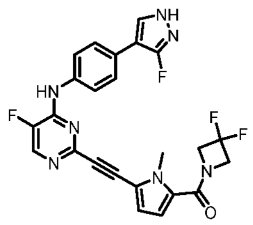
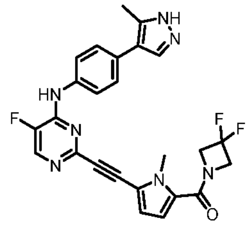
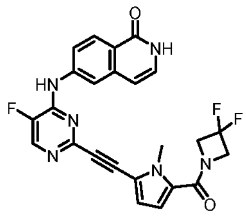
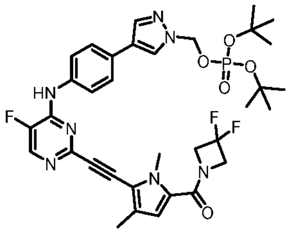

In the second aspect of the present invention, it provides a pharmaceutical composition comprising one or more pharmaceutically acceptable carriers and a prophylactically and/or therapeutically effective amount of one or more of the compounds described in the first aspect of the present invention, or a pharmaceutically acceptable salt, stereoisomer, polymorph, solvate, N-oxide, isotope labeled compound, metabolite or prodrug thereof.

In the third aspect of the present invention, it provides a use of the compound or a pharmaceutically acceptable salt, stereoisomer, polymorph, solvate, N-oxide, isotope labeled compound, metabolite or prodrug thereof of the first aspect of the present invention for the preparation of a drug, the drug is used for the prevention and/or treatment of Rho-associated protein kinase-mediated diseases.

In another preferred embodiment, the Rho-related protein kinases are ROCK1 and/or ROCK2.

In another preferred embodiment, the drug is a selective ROCK2 inhibitor.

In another preferred embodiment, the Rho-associated protein kinase-mediated disease is selected from the group consisting of autoimmune disease, cardiovascular disease, inflammation, central nervous system disease, arterial thrombotic disease, fibrotic disease, neoplastic disease, metabolic syndrome, insulin resistance, hyperinsulinemia, type 2 diabetes mellitus, glucose intolerance, osteoporosis, and ocular disease.

In another preferred embodiment, the autoimmune disease is selected from the group consisting of rheumatoid arthritis, systemic lupus erythematosus (SLE, lupus) psoriasis, Crohn's disease, atopic dermatitis, eczema, or plant-versus-host disease (GVHD).

In another preferred embodiment, the cardiovascular disease is selected from the group consisting of hypertension, atherosclerosis, restenosis, cardiac hypertrophy, cerebral ischemia, cerebral vasospasm, erectile dysfunction.

In another preferred embodiment, the inflammation is selected from the group consisting of asthma, cardiovascular inflammation, ulcerative colitis, renal inflammation.

In another preferred embodiment, the central nervous system disease is selected from the group consisting of neuronal degeneration, spinal cord injury.

In another preferred embodiment, the central nervous system disease is selected from the group consisting of Huntington's disease, Parkinson's disease, Alzheimer's disease, amyotrophic lateral sclerosis (ALS), multiple sclerosis.

In another preferred embodiment, the arterial thrombotic disease is selected from the group consisting of platelet aggregation, leukocyte aggregation.

In another preferred embodiment, the fibrotic disease is selected from the group consisting of hepatic fibrosis, pulmonary fibrosis, renal fibrosis.

In another preferred embodiment, the neoplastic disease is selected from the group consisting of lymphoma, carcinoma, leukemia, astrocytoma, soft tissue sarcoma, sarcoma, and blastoma.

In another preferred embodiment, the carcinoma is selected from the group consisting of squamous cell carcinoma, small cell lung carcinoma, pituitary gland carcinoma, esophageal carcinoma, non-small cell lung carcinoma,

lung adenocarcinoma, squamous lung carcinoma, peritoneal carcinoma, hepatocellular carcinoma, gastrointestinal carcinoma, pancreatic carcinoma, glioblastoma, cervical carcinoma, ovarian carcinoma, bladder carcinoma, hepatocellular carcinoma, breast carcinoma, colon carcinoma, colorectal carcinoma, endometrial carcinoma, uterine carcinoma, salivary gland carcinoma, renal carcinoma, prostatic carcinoma, vulval carcinoma, thyroid cancer, brain cancer, testicular cancer, bile duct cancer, gallbladder cancer, stomach cancer, melanoma, head and neck cancer.

In another preferred embodiment, the ocular disease is selected from the group consisting of high intraocular pressure, age-related macular degeneration (AMD), choroidal neovascularization (CNV), diabetic macular edema (DME), iris neovascularization, uveitis, glaucoma, and retinopathy of prematurity (ROP).

In another preferred embodiment, the glaucoma is selected from the group consisting of primary open-angle glaucoma, acute angle-closure glaucoma, pigmentary glaucoma, congenital glaucoma, normal tension glaucoma, secondary glaucoma, and neovascular glaucoma.

In another preferred embodiment, the Rho-associated protein kinase-mediated disease is selected from the group consisting of lupus nephritis, atherosclerosis, rheumatoid arthritis (RA), hemangioma, angiofibroma, pulmonary fibrosis, hepatic fibrosis, psoriasis, corneal transplant rejection, insulin-dependent diabetes mellitus, multiple sclerosis, myasthenia gravis, Crohn's disease, autoimmune nephritis, primary biliary cirrhosis, Acute pancreatitis, allograft rejection, allergic inflammation, contact dermatitis, delayed hypersensitivity reaction, inflammatory bowel disease, infectious shock, osteoporosis, osteoarthritis, neuronal inflammation, Osler-Weber syndrome, restenosis, fungal infections, parasitic infections, viral infections.

It should be understood that in the present invention, any of the technical features specifically described above and below (such as in the Examples) can be combined with each other, so as to constitute new or preferred technical solutions. Due to space limitations, it will not redundantly be described one by one herein.

## DETAILED DESCRIPTION OF THE INVENTION

After long-term and in-depth research, through structural optimization, the present inventors unexpectedly prepared a novel class of compounds shown in Formula I with excellent ROCK2 inhibitory activity, which have excellent ROCK2 inhibitory activity, better selectivity for ROCK2, more excellent pharmacokinetics (e.g., in vivo exposure, tissue targeting, half-life, metabolic stability, etc.) , and better in vivo efficacy and safety. On above basis, the inventors have completed the present invention.

## TERMS

In the present invention, unless otherwise indicated, the terms used have the ordinary meaning known to the skilled person in the art.

In the present invention, the term "halogen" refers to F, Cl, Br or I.

In the present invention, the term "$C_{1-6}$ alkyl" refers to straight-chain or branched alkyl groups comprising 1-6 carbon atoms, for example methyl, ethyl, propyl, iso-propyl, butyl, iso-butyl, tert-butyl, neo-pentyl, ter-pentyl, or the like.

In the present invention, the term "$C_{2-6}$ alkenyl" refers to a straight or branched alkenyl group with 2-6 carbon atoms containing a double bond, and non-limitingly includes vinyl, propenyl, butenyl, iso-butenyl, pentenyl, hexenyl and the like.

In the present invention, the term "$C_{2-6}$ alkynyl" refers to a straight or branched alkynyl group with 2-6 carbon atoms containing a triple bond, and non-limitingly includes ethynyl, propynyl, butynyl, iso-butynyl, pentynyl and hexynyl, etc.

In the present invention, the term "$C_{3-6}$ cycloalkyl" includes a group selected from the group consisting of $C_{3-6}$ cycloalkyl, $C_{3-6}$ cycloalkenyl, $C_{3-6}$ cycloalkynyl. The term "$C_{3-10}$ cycloalkyl" has a similar meaning.

In the present invention, the term "$C_{3-6}$ cycloalkyl" refers to a cyclic alkyl group with 3-6 carbon atoms on the ring, and non-limitingly includes cyclopropyl, cyclobutyl, cyclopentyl, cyclohexyl, and the like. The term "$C_{3-10}$ cycloalkyl" has a similar meaning.

In the present invention, the term "$C_{1-6}$ alkoxy" refers to straight-chain or branched alkoxy groups with 1-6 carbon atoms, and non-limitingly includes methoxy, ethoxy, propoxy, iso-propoxy and butoxy. Preferably $C_{1-4}$ alkoxy.

In the present invention, the term "heterocyclyl" refers to a 4-8 membered heterocvclvl containing 1, 2 or 3 heteroatoms selected from N, O, S, including but not limited to the following groups:

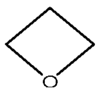, 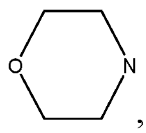, 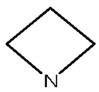, 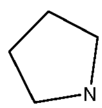, 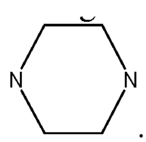.

In the present invention, the terms "aryl ring" or "aryl" have the same meaning, preferably "$C_{6-10}$ aryl". The term "$C_{6-10}$ aryl" refers to an aromatic ring with 6-10 carbon atoms without heteroatoms on the ring, such as phenyl, naphthyl, etc.

In the present invention, the term "aromatic heterocycle" or "heteroaryl" has the same meaning and refers to a heteroaromatic group containing one to more heteroatoms. For example, "5-14-membered heteroaryl" refers to an aromatic heterocyclic ring containing 1 to 3 heteroatoms selected from oxygen, sulfur, and nitrogen and 2 to 13 carbon atoms. Non-limiting examples are furanyl, thienyl, pyridinyl, pyrazolyl, pyrrolyl, N-alkylpyrrolyl, pyrimidinyl, pyrazinyl, imidazolyl, tetrazolyl, etc. The heteroaryl ring may be fused on an aryl, heterocyclyl or cycloalkyl ring, wherein the ring attached to the parent structure is a heteroaryl ring. The heteroaryl group may be optionally substituted or unsubstituted.

In the present invention, the term "$C_{6-12}$ aralkyl" refers to a group with the structure $C_{6-12}$ aryl-$C_{1-6}$ alkylene-.

In the present invention, the term "deuterated" means substituted with deuterium.

In the present invention, the term "substituted" refers to one or more hydrogen atoms on a particular group are substituted by a particular substituent. The particular substituent is a substituent described above accordingly, or a substituent in the embodiments. Unless indicated specifically, a substituted group may have a substituent group selected from a particular group at any substitutable site of the group, and the substituent group can be identical or different at each site. It should be understood by the person skilled in the art that the combinations of substituents expected in the present invention are those that are stable or chemically realizable. For example (but not limited to), the substituents are halogen, hydroxyl, carboxy (-COOH), C1-C6 alkyl, C2-C6 alkenyl, C2-C6 alkynyl, C3-C8 cycloalkyl, 3-12 membered heterocyclyl, aryl, heteroaryl, C1-C8 aldehyde, C2-C10 acyl, C2-C10 ester, amino, C1-C6 alkoxy, C1-C10 sulfonyl, etc.

In the present invention, the terms 1-6 refer to 1, 2, 3, 4, 5 or 6. Other similar terms each independently have a similar meaning. The term "more" refers to 2-6, such as 2, 3, 4, 5 or 6.

It should be understood that when a certain group is present at a number of different sites in a compound, its definition at each site is independent and may be the same or different. That is, the term "selected from the group consisting of" has the same meaning as the term "each independently selected from the group consisting of'.

Compounds and Preparation Method thereof

The present invention provides a compound shown in Formula I or a pharmaceutically acceptable salt, stereoisomer, polymorph, solvate, N-oxide, isotope labeled compound, metabolite or prodrug thereof,

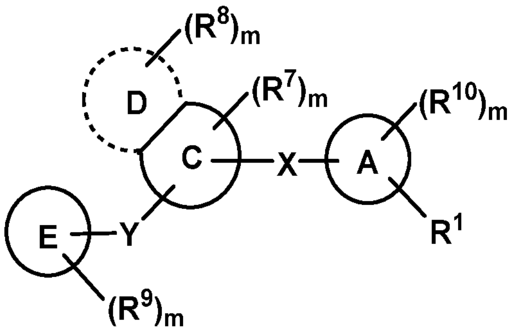

Formula I

wherein, each of the groups is as defined above.

In another preferred embodiment, any one of X, Y, ring A, ring C, ring D, ring E, $R^1$, $R^7$, $R^8$, $R^9$, $R^{10}$, m in the compound, respectively, is independently a corresponding group in the specific compound described in the present invention.

As used herein, the term "pharmaceutically acceptable salt" refers to a salt formed by a compound of the present invention with an acid or base suitable for use as a medicine. Pharmaceutically acceptable salts include inorganic salts and organic salts. A preferred class of salts is the salts of the compounds of the invention formed with acids. Suitable acids for forming salts include, but are not limited to inorganic acids such as hydrochloric acid, hydrobromic acid, hydrofluoric acid, sulfuric acid, nitric acid, phosphoric acid; organic acids such as formic acid, acetic acid, trifluoroacetic acid, propionic acid, oxalic acid, malonic acid, succinic acid, fumaric acid, maleic acid, lactic acid, malic acid, tartaric acid, citric acid, picric acid, benzoic acid, methylsulfonic acid, ethanesulfonic acid, p-toluenesulfonic acid, benzenesulfonic acid, naphthalenesulfonic acid; and amino acids such as proline, phenylalanine, aspartic acid and glutamic acid.

Another preferred class of salts are salts of the compounds of the invention formed with bases, such as alkali metal salts (such as sodium or potassium salts), alkaline earth metal salts (such as magnesium or calcium salts), ammonium salts (such as lower grades alkanol ammonium salts and other pharmaceutically acceptable amine salts), such as methylamine salt, ethylamine salt, propylamine salt, dimethylamine salt, trimethylamine salt, diethylamine salt,

triethylamine salt, tert-butylamine salt, ethylenediamine salt, hydroxyethylamine salt, dihydroxyethylamine salt, trishydroxyethylamine salt, and an amine salt formed from morpholine, piperazine, and lysine, respectively.

The methods described below for the preparation of the compounds of the structure of Formula I of the present invention do not constitute any limitation of the present invention. The compounds of the present invention may also be conveniently made by optionally combining various synthetic methods described in this specification or known in the art, and such combinations may be readily carried out by one skilled in the field to which the present invention belongs.

It should be understood that the raw materials and reagents used in the processes for the preparation of the compounds of the present invention are commercially available without special indication.

Pharmaceutical Compositions and Administration Methods

The pharmaceutical composition of the present invention comprises a safe and effective amount of a compound of the present invention or a pharmacologically acceptable salt thereof, and a pharmacologically acceptable excipient or carrier. In which, "safe and effective amount" is meant that the amount of the compound is sufficient to significantly improve the condition without causing serious side effects. Generally, the pharmaceutical composition contains 1-2000 mg of the compound of the present invention/dose, more preferably, 5-1000 mg of the compound of the present invention/dose. Preferably, the "dose" is a capsule or tablet.

"Pharmaceutically acceptable carrier" means one or more compatible solid or liquid fillers or gelatinous materials which are suitable for human use and should be of sufficient purity and sufficiently low toxicity. "Compatibility" means that each component in the composition can be admixed with the compounds of the present invention and with each other without significantly reducing the efficacy of the compounds. Some examples of pharmaceutically acceptable carriers include cellulose and the derivatives thereof (such as sodium carboxymethyl cellulose, sodium ethyl cellulose, cellulose acetate, etc.), gelatin, talc, solid lubricants (such as stearic acid, magnesium stearate), calcium sulfate, vegetable oils (such as soybean oil, sesame oil, peanut oil, olive oil, etc.), polyols (such as propylene glycol, glycerol, mannitol, sorbitol, etc.), emulsifiers (such as Tween®), wetting agent (such as sodium dodecyl sulfate), coloring agents, flavoring agents, stabilizers, antioxidants, preservatives, pyrogen-free water, etc..

The pharmaceutical composition is an injection, a capsule, a tablet, a pill, a powder, or a granule.

The administration mode of the compound or pharmaceutical composition of the present invention is not particularly limited, and representative administration modes include, but are not limited to, oral, intratumoral, rectal, parenteral (intravenous, intramuscular or subcutaneous) and topical administration.

Solid dosage forms for oral administration include capsules, tablets, pills, powders and granules. In these solid dosage forms, the active ingredient is mixed with at least one conventional inert excipient (or carrier), such as sodium citrate or dicalcium phosphate, or mixed with any of the following components: (a) fillers or compatibilizer, such as starch, lactose, sucrose, glucose, mannitol and silicic acid; (b) binders, such as hydroxymethyl cellulose, alginate, gelatin, polyvinylpyrrolidone, sucrose and arabic gum; (c) humectants, such as glycerol; (d) disintegrating agents such as agar, calcium carbonate, potato starch or tapioca starch, alginic acid, certain composite silicates, and sodium carbonate; (e) retarding solvents, such as wax, (f) absorption accelerators, such as quaternary ammonium compound; (g) wetting agents, such as cetyl alcohol and glyceryl monostearate; (h) adsorbents, such as kaolin; and (i) lubricants, such as talc, calcium stearate, magnesium stearate, solid polyethylene glycol, sodium dodecyl sulfate or mixture thereof. In capsules, tablets and pills, the dosage forms may also contain buffering agents.

Solid dosage forms such as tablets, dragees, capsules, pills and granules can be prepared with coatings and shells such as enteric coatings and other materials known in the art. They may contain opacifying agents and the release of the active compound or compound in such compositions may be released in a portion of the digestive tract in a delayed manner. Examples of embedding components that can be employed are polymeric materials and waxy materials. If necessary, the active compound may also be in microencapsulated form with one or more of the above-mentioned excipients.

Liquid dosage forms for oral administration include pharmaceutically acceptable emulsions, solutions, suspensions, syrups or tinctures. In addition to the active compound, the liquid dosage form may contain inert diluents conventionally used in the art, such as water or other solvents, solubilizers and emulsifiers, such as ethanol, isopropanol, ethyl carbonate, ethyl acetate, propylene glycol, 1,3-butanediol, dimethylformamide and oils, especially cottonseed oil, peanut oil, corn germ oil, olive oil, castor oil and sesame oil or mixtures of these substances.

In addition to these inert diluents, the compositions may contain adjuvants such as wetting agents, emulsifying and suspending agents, sweetening agents, flavoring agents and spices.

In addition to the active compound, the suspension may contain suspending agent, such as ethoxylated isooctadecanol, polyoxyethylene sorbitol and dehydrated sorbitan ester, microcrystalline cellulose, aluminum methoxide and agar, or the mixture thereof etc.

The compositions for parenteral injection may comprise physiologically acceptable sterile aqueous or anhydrous solutions, dispersions, suspensions or emulsions, and sterile powders which can be re-dissolved into sterile injectable

solutions or dispersions. Suitable aqueous and non-aqueous carriers, diluents, solvents or excipients include water, ethanol, polyols and any suitable mixtures thereof.

Dosage forms for the compounds of the invention for topical administration include ointments, powders, patches, sprays and inhalants. The active ingredient is mixed under sterile conditions with a physiologically acceptable carrier and any preservatives, buffers, or propellants which may be required if necessary.

The compounds of the invention can be administered alone or in combination with other pharmaceutically acceptable compounds.

The treatment method of the present invention can be administered alone or in combination with other treatment means or therapeutic drugs.

When the pharmaceutical composition is used, a safe and effective amount of the compound of the present invention is administered to a mammal(such as a human) in need of treatment, wherein the dosage at the time of administration is the pharmaceutically effective dosage, for people having a body weight of 60kg, the daily dose is usually 1~2000mg, preferably 5~1000mg. Of course, specific doses should also consider factors such as the administration route, the health of the patient, etc., which are within the skill of the skilled physician.

Compared with the prior art, the present invention has the following main advantages:

(1) The compounds have superior pharmacokinetic properties, such as in vivo exposure, tissue targeting, half-life, and metabolic stability;
(2) The compounds have better ROCK2 inhibitory activity, better selectivity for ROCK2, and better selectivity for other human kinases;
(3) The compounds have a superior safety, superior in vivo efficacy;
(4) The compounds are expected to be used for the prevention and/or treatment of diseases associated with the regulation of Rho-associated protein kinases (ROCK1 and/or ROCK2).

The present invention will be further illustrated below with reference to the specific examples. It should be understood that these examples are only to illustrate the invention but not to limit the scope of the invention. Experimental methods in which the specific conditions are not specified in the following examples are usually in accordance with conventional conditions such as the conditions described in Sambrook et al., Molecular Cloning: Laboratory Manual (New York: Cold Spring Harbor Laboratory Press, 1989), or in accordance with the conditions recommended by the manufacturer. Unless indicated otherwise, percentage and parts are calculated by weight.

Unless otherwise defined, all professional and scientific terminology used in the text have the same meanings as known to the skilled in the art. In addition, any methods and materials similar or equal with the recorded content can apply to the methods of the invention. The method of the preferred embodiment described herein and the material are only for demonstration purposes.

The experimental materials and reagents used in the following examples can be obtained from commercial sources unless otherwise specified.

**Example 1-1 Preparation of Compound A1**

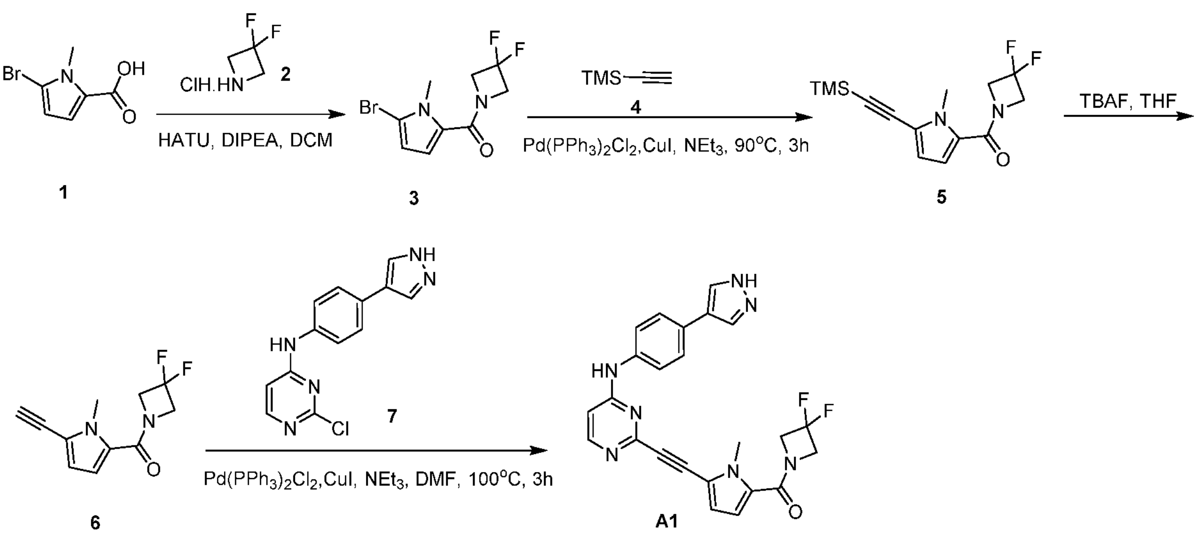


1 2 3 4 5 6 7 A1

Step 1: Compound 3

Compound 1 (2.0 g, 9.8 mmol, 1.0 eq), Compound 2 (1.5 g, 11.8 mmol, 1.2 eq), HATU (5.6 g, 14.7 mmol, 1.5 eq) and diisopropylethylamine (6.3 g, 49.0 mmol, 5.0 eq) were sequentially added to anhydrous dichloromethane (60 mL), and the reaction solution was stirred at 25 °C for 2 hours. The reaction solution was concentrated and the residue was purified by silica gel column chromatography (n-hexane/ethyl acetate = 4/1) to obtain compound 3 (1.6 g, 58% yield) as a colorless oil.
LCMS: $[M+H]^+$ = 279.0

Step 2: Compound 5

Compound 3 (1.0 g, 3.58 mmol, 1.0 eq), compound 4 (7.0 g, 71.60 mmol, 20.0 eq), $Pd(PPh_3)_2Cl_2$ (500 mg, 0.716 mmol, 0.2 eq), and cuprous iodide (136 mg, 0.716 mmol, 0.2 eq) were sequentially added to triethylamine (10 mL). The reaction system was exchanged with nitrogen for three times, heated to 90 °C and stirred for 3 h. The reaction solution was cooled down to 25 °C and concentrated, and the obtained residue was purified by silica gel column chromatography (n-hexane/ethyl acetate = 4/1) to obtain compound 5 (1.0 g, 94% yield).
LCMS: $[M+H]^+$ = 297.2

Step 3: Compound 6

Compound 5 (1.0 g, 3.38 mmol, 1.0 eq) was dissolved in tetrahydrofuran (10 mL), tetrabutylammonium fluoride (0.9 g, 3.38 mmol, 1.0 eq) was added, and the resulting solution was stirred for 0.5 h at room temperature. The residue obtained after concentration of the reaction solution was purified by silica gel column chromatography (n-hexane/ethyl acetate=4/1) to obtain compound 6 (0.65 g, 87%).
LCMS: $[M+H]^+$ =225.1

Step 4: Compound A1

Compound 7 (50 mg, 0.184 mmol, 1.0 eq) was dissolved in anhydrous N,N-dimethylformamide (2 mL), bis(triphenylphosphine)palladium dichloride (13 mg, 0.0184 mmol, 0.1 eq), cuprous iodide (4 mg, 0.0184 mmol, 0.1 eq) and triethylamine (372 mg, 3.68 mmol, 20.0 eq) were added, and the reaction system was exchanged three times with nitrogen. The reaction solution was warmed up to 100 °C under nitrogen atmosphere and compound 6 (103 mg, 0.460 mmol, 2.5 eq) in N,N-dimethylformamide (1 mL) was slowly added dropwise to the reaction solution, and stirring was continued at 100 °C for 2 hours after the end of dropwise addition. The reaction solution was cooled to room temperature and concentrated, and the residue was purified by Pre-HPLC to obtain compound A1 (8 mg).

LCMS: $[M+H]^+$ = 460.2
$^1$H NMR (400 MHz, DMSO-$d_6$) δ 12.79 (s, 1H), 9.78 (s, 1H), 8.26 (d, *J* = 6.0 Hz, 1H), 8.02 (s, 2H), 7.60 (s, 4H), 6.75 (d, *J* = 6.0 Hz, 1H), 6.73 - 6.68 (m, 2H), 4.64 (s, 4H), 3.95 (s, 3H).

**Example 1-2 Preparation of Compound A2**

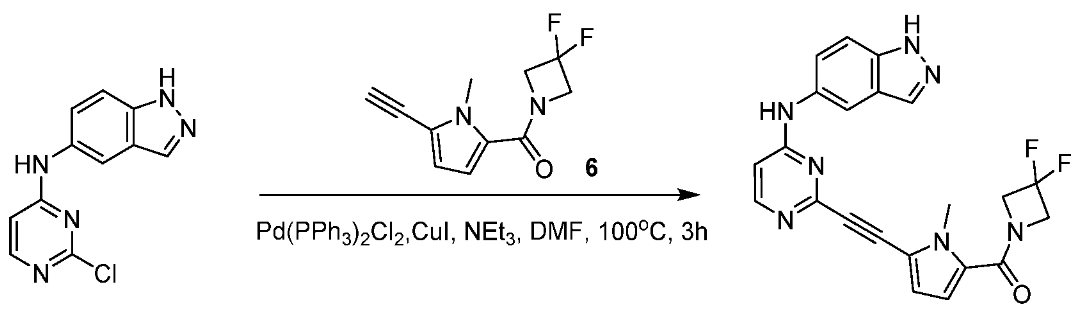


8 A2

Step 1: Compound A2

Compound 8 (100 mg, 0.408 mmol, 1.0 eq) was dissolved in anhydrous N,N-dimethylformamide (2 mL), bis(triphenylphosphine)palladium dichloride (29 mg, 0.0408 mmol, 0.1 eq), cuprous iodide (8 mg, 0.0408 mmol, 0.1 eq) and triethylamine (826 mg, 8.16 mmol, 20.0 eq) were added, and the reaction system was exchanged three times with nitrogen. The reaction solution was warmed to 100 °C under nitrogen atmosphere and compound 6 (228 mg, 1.02 mmol, 2.5 eq) in N,N-dimethylformamide (1 mL)was slowly added dropwise to the reaction solution, and continued to stir at 100 °C for 2 hours after the end of the dropwise addition. The reaction solution was cooled to room temperature and concentrated, and the residue was purified by Pre-HPLC to obtain compound A2 (20.2 mg).

LCMS: $[M+H]^+$ = 434.2
$^1$H NMR (400 MHz, DMSO-$d_6$) δ 13.03 (s, 1H), 9.76 (s, 1H), 8.23 (d, *J* = 6.0 Hz, 1H), 8.06 (s, 2H), 7.56 (d, *J* = 8.8 Hz, 1H), 7.40 (d, *J* = 8.8 Hz, 1H), 6.74 - 6.67 (m, 3H), 4.65 (s, 4H), 3.95 (s, 3H).

**Example 1-3 Preparation of Compound A4**

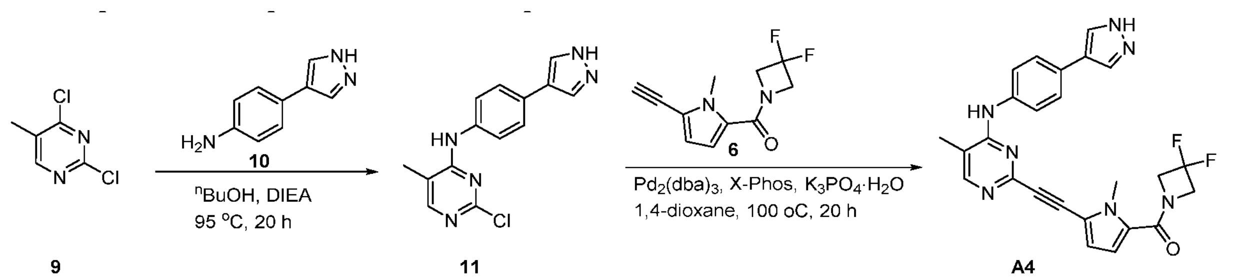


9 11 A4

Step 1: Compound 11

Compound 9 (370 mg, 2.27 mmol, 1.2 eq) and compound 10 (301 mg, 1.89 mmol, 1.0 eq) were dissolved in n-butanol (10 mL) and diisopropylethylamine (733 mg, 5.67 mmol, 3.0 eq) was added. The reaction mixture was stirred at 95 °C for 20 h. After LCMS detection showed that the reaction was completed, the reaction solution was cooled to room temperature and concentrated under reduced pressure. The residue was purified using silica gel column chromatography (dichloromethane/ethyl acetate = 1/1) to obtain compound 11 (250 mg, 39%) as a yellow solid.
LCMS: $[M+H]^+$ = 286.0

Step 2: Compound A4

Compound 11 (250 mg, 0.875 mmol, 1.0 eq) and compound 6 (196 mg, 0.875 mmol, 1.0 eq) were dissolved in dioxane (30 mL), potassium phosphate monohydrate (705 mg, 3.06 mmol, 3.5 eq) was added, and finally $Pd_2(dba)_3$ (80 mg, 0.086 mmol, 0.10 eq) and X-Phos (83 mg, 0.175 mmol, 0.2 eq) were added. The reaction mixture was stirred at 100 °C for 20 h under nitrogen protection. The reaction solution was cooled to room temperature and concentrated under reduced pressure. The residue was purified using silica gel column chromatography (dichloromethane/ethyl acetate = 5/1 to 1/1, then dichloromethane/methanol = 40:1) to obtain compound A4 (64.1 mg, 15 %) as a yellow solid.

LCMS: $[M+H]^+$ = 502.2
$^1$H NMR (400 MHz, DMSO-$d_6$) δ 12.90 (br s, 1H), 8.63 (s, 1H), 8.14-8.07 (m, 2H), 7.92 (br s, 1H), 7.69 (d, *J* = 8.8 Hz, 2H), 7.60 (d, *J* = 8.8 Hz, 2H), 6.69 (d, *J* = 4.0 Hz, 1H), 6.66 (d, *J* = 4.0 Hz, 1H), 4.63 (br s, 4H), 3.92 (s, 3H), 2.23 (s, 3H).

**Example 1-4 Preparation of Compound A5**

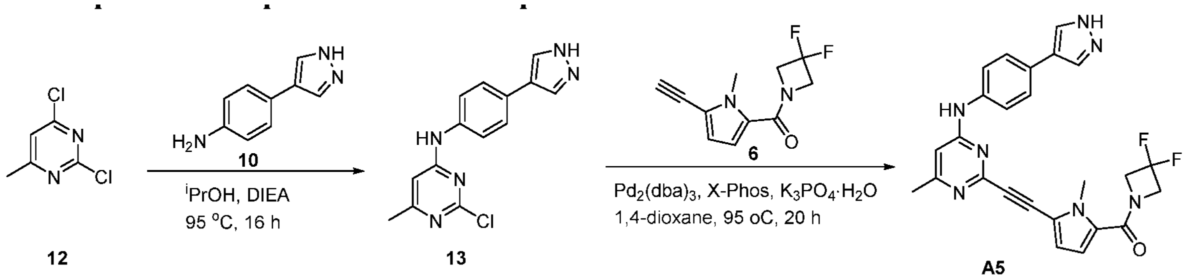


12 13 A5

Step 1: Compound 13

Compound 12 (370 mg, 2.27 mmol, 1.2 eq) and compound 10 (301 mg, 1.89 mmol, 1.0 eq) were dissolved in isopropanol (5 mL) and diisopropylethylamine (733 mg, 5.67 mmol, 3.0 eq) was added. The reaction mixture was stirred at 95 °C for 16 h. After LCMS detection showed that the reaction was completed, the reaction solution was cooled to room temperature and concentrated under reduced pressure. The residue was purified using silica gel column chromatography (dichloromethane/ethyl acetate = 1/1) to obtain compound 13 (150 mg, 23%) as a yellow solid.
LCMS: $[M+H]^+$ = 286.0

Step 2: Compound A5

Compound 13 (150 mg, 0.525 mmol, 1.0 eq) and compound 6 (117 mg, 0.875 mmol, 1.0 eq) were dissolved in dioxane (15 mL), potassium phosphate monohydrate (423 mg, 3.06 mmol, 3.5 eq) was added, and finally $Pd_2(dba)_3$ (48 mg, 0.053 mmol, 0.10 eq) and X-Phos (50 mg, 0.105 mmol, 0.2 eq) were added. The reaction mixture was stirred at 95 °C for 20 h under nitrogen protection. The reaction solution was cooled to room temperature and concentrated under reduced pressure. The residue was purified by silica gel column chromatography (dichloromethane/ethyl acetate = 5/1 to 0/1) to obtain the crude product. The crude was nurified by silica gel column chromatography (dichloromethane/methanol = 150/1 to 40/1) to obtain compound A5 (64.1 mg, 16 %) as a yellow solid.

LCMS: $[M+H]^+$ = 474.3
$^1$H NMR (400 MHz, DMSO-$d_6$) δ 12.90 (br s, 1H), 9.65 (s, 1H), 8.13 (br s, 1H), 7.89 (br s, 1H), 7.63-7.53 (m, 4H), 6.72-6.69 (m, 2H), 6.60 (s, 1H), 4.64 (br s, 4H), 3.94 (s, 3H), 2.31 (s, 3H).

**Example 1-5 Preparation of Compound A6**

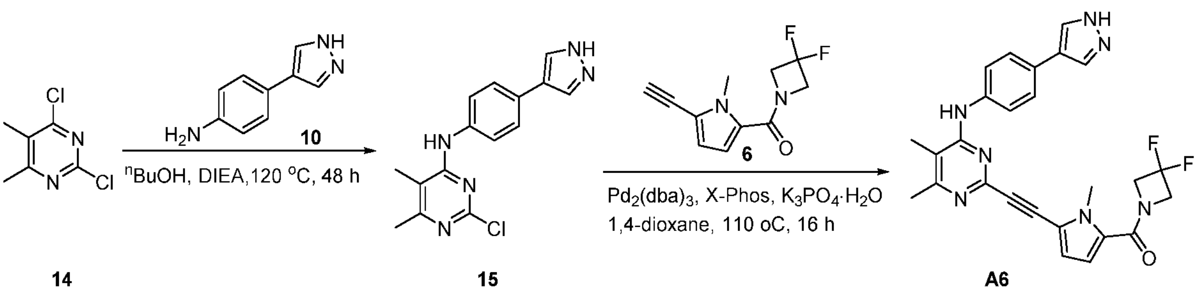


14 15 A6

Step 1: Compound 15

Compound 14 (1.3 g, 7.34 mmol, 2.4 eq) and compound 10 (487 mg, 3.06 mmol, 1.0 eq) were dissolved in n-butanol (25 mL) and diisopropylethylamine (1.19 g, 9.18 mmol, 3.0 eq) was added. The reaction mixture was stirred at 120 °C for 48 h. After LCMS detection showed that the reaction was completed, the reaction solution was cooled to room temperature and concentrated under reduced pressure. The residue was purified using silica gel column chromatography (petroleum ether/ethyl acetate = 1/1) to obtain compound 15 (300 mg, 32%) as a yellow solid.
LCMS: $[M+H]^+$ = 300.0

Step 2: Compound A6

Compound 3 (300 mg, 1.0 mmol, 1.0 eq) and compound 4 (224 mg, 1.0 mmol, 1.0 eq) were dissolved in dioxane (30 mL), potassium phosphate monohydrate (807 mg, 3.5 mmol, 3.5 eq) was added and $Pd_2(dba)_3$ (92 mg, 0.1 mmol, 0.10 eq) and X-Phos (95 mg, 0.2 mmol, 0.2 eq) were finally added. The reaction mixture was stirred at 110 °C for 16 h under nitrogen protection. The reaction solution was cooled to room temperature and concentrated under reduced pressure. The residue was purified using silica gel column chromatography (dichloromethane/ethyl acetate = 10/1 to 1/1) to obtain the crude product (200 mg). The crude was pulped with dichloromethane/ethyl acetate/petroleum ether (20 mL/20 mL/10 mL) for 5 h, filtered and the solvent was removed under low pressure to obtain compound A6 (158.1 mg, 32 %) as a yellow solid.

LCMS: $[M+H]^+$ = 488.2
$^1$H NMR (400 MHz, DMSO-$d_6$) δ 12.89 (br s, 1H), 8.51 (s, 1H), 8.14 (br s, 1H), 7.90 (br s, 1H), 7.63 (d, *J* = 8.8 Hz, 2H), 7.57 (d, *J* = 8.8 Hz, 2H), 6.69-6.64 (m, 2H), 4.63 (br s, 4H), 3.91 (s, 3H), 2.37 (s, 3H), 2.21 (s, 3H).

**Example 1-6 Preparation of Compound A7**

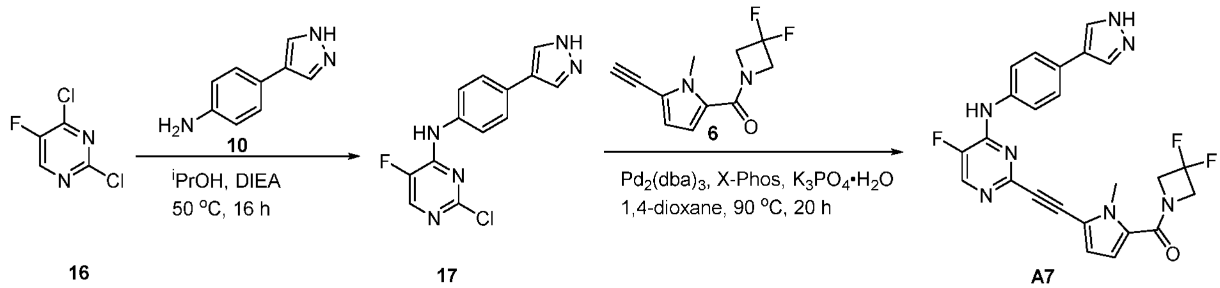


16 17 A7

Step 1: Compound 17

Compound 16 (370 mg, 2.22 mmol, 1.2 eq) and compound 10 (294 mg, 1.85 mmol, 1.0 eq) were dissolved in isopropanol (30 mL) and diisopropylethylamine (716 mg, 5.54 mmol, 2.5 eq) was added. The reaction mixture was stirred at 50 °C for 16 h. After LCMS detection showed that the reaction was completed, the reaction solution was cooled to room temperature and concentrated under reduced pressure. The residue was purified by silica gel column chromatography (dichloromethane/ethyl acetate = 1/1) to obtain compound 17 (500 mg, 93%) as a yellow solid.
LCMS: $[M+H]^+$ = 290.0

Step 2: Compound A7

Compound 17 (250 mg, 0.86 mmol, 1.0 eq) and compound 6 (193 mg, 1.0 mmol, 1.0 eq) were dissolved in dioxane (20 mL), potassium phosphate monohydrate (695 mg, 3.02 mmol, 3.5 eq) was added, and $Pd_2(dba)_3$ (87 mg, 0.095 mmol, 0.10 eq) and X-Phos (68 mg, 0.143 mmol, 0.15 eq) were finally added, and the reaction mixture was stirred at 90 °C for 20 h under nitrogen protection. The reaction solution was cooled to room temperature, diluted with dichloromethane/methanol (50 mL, 10:1), the reaction solution was passed through diatomaceous earth, and the filtrate was concentrated under reduced pressure to obtain the residue, which was purified using silica gel column chromatography (dichloromethane/methanol = 5/1 to 1/1) to obtain the crude product. The crude was pulped with dichloromethane/dioxane/methanol (20 mL/5 mL/1 mL) for 5 hours, then concentrated under reduced pressure at low temperature (30 °C) to remove the dichloromethane and methanol, the resulting mixture was filtered, and the solvent was removed from the solid under low pressure to obtain compound A7 (59 mg, 15 %) as a white solid.

LCMS: $[M+H]^+$ = 478.1
$^1$H NMR (400 MHz, DMSO-$d_6$) δ 12.92 (br s, 1H), 9.77 (s, 1H), 8.39 (s, 1H), 8.16 (br s, 1H), 7.91 (br s, 1H), 7.74 (d, *J* = 8.8 Hz, 2H), 7.62 (d, *J* = 8.8 Hz, 2H), 6.71-6.58 (m, 2H), 4.62 (br s, 4H), 3.93 (s, 3H).

**Example 1-7 Preparation of Compound A8**

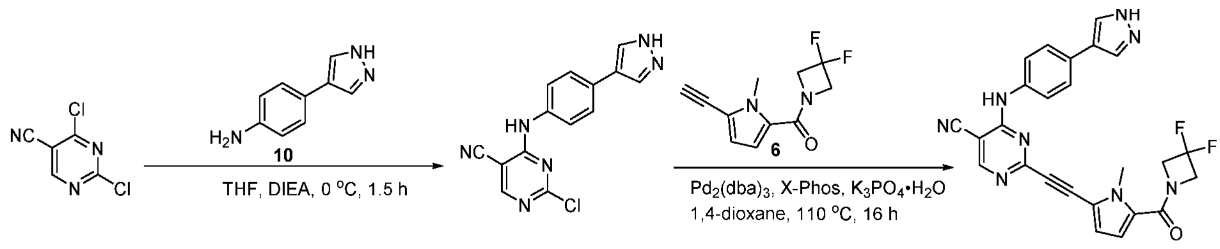


18 19 A8

Step 1: Compound 19

Compound 18 (323 mg, 1.86 mmol, 1.0 eq) was dissolved in tetrahydrofuran (8 mL), then diisopropylethylamine (600 mg, 4.65 mmol, 3.0 eq) was added, followed by compound 10 (400 mg, 1.55 mmol, 1.0 eq) under ice bath conditions. After the addition, the reaction solution was stirred in an ice bath for 1.5 hours. After the end of the reaction, the reaction solution was diluted with ethyl acetate (100 mL), washed with saturated saline, dried over anhydrous sodium sulfate and concentrated. The concentrated crude was purified by silica gel column chromatography (PE/EA=3/1) to obtain compound 19 (280 mg, 46%) as a yellow solid.
LCMS: $[M+H]^+$ = 297.7

Step 2: Compound A8

Compound 19 (202 mg, 0.68 mmol, 1.0 eq) was dissolved in dioxane (10 mL) and then compound 6 (183 mg, 0.82 mmol, 1.2 eq), potassium phosphate monohydrate (548 mg, 2.38 mmol, 3.5 eq), X-Phos (65 mg, 0.14 mmol, 0.2 eq) and $Pd_{2(}dba)_3$ (62 mg, 0.07 mmol. 0.1 eq) were successively added. Nitrogen was replaced after dosing and the reaction solution was stirred at 110 °C for 16 h under nitrogen atmosphere. The reaction solution was cooled to room temperature and concentrated. The residue was purified by silica gel column chromatography (dichloromethane/methanol=15/1) to obtain the crude product (65 mg). The crude was purified by prep-HPLC (mobile phase: 0.1% formic acid/acetonitrile/-water) to obtain compound A8 (8.3 mg, 2%) as a yellow solid.

LCMS: $[M+H]^+$ = 485.2
$^1H$ NMR (400 MHz, DMSO-$d_6$) δ 12.81 (br s, 1H), 10.04 (s, 1H), 8.79 (s, 1H), 8.20-7.90 (m, 2H), 7.63 (d, *J* = 8.4 Hz, 2H), 7.53 (d, *J* = 8.4 Hz, 2H), 6.77 (d, *J* = 4.0 Hz, 1H), 6.71 (d, *J* = 4.4 Hz, 1H), 4.63 (br s, 4H), 3.90 (s, 3H).

**Example 1-8 Preparation of Compound A9**

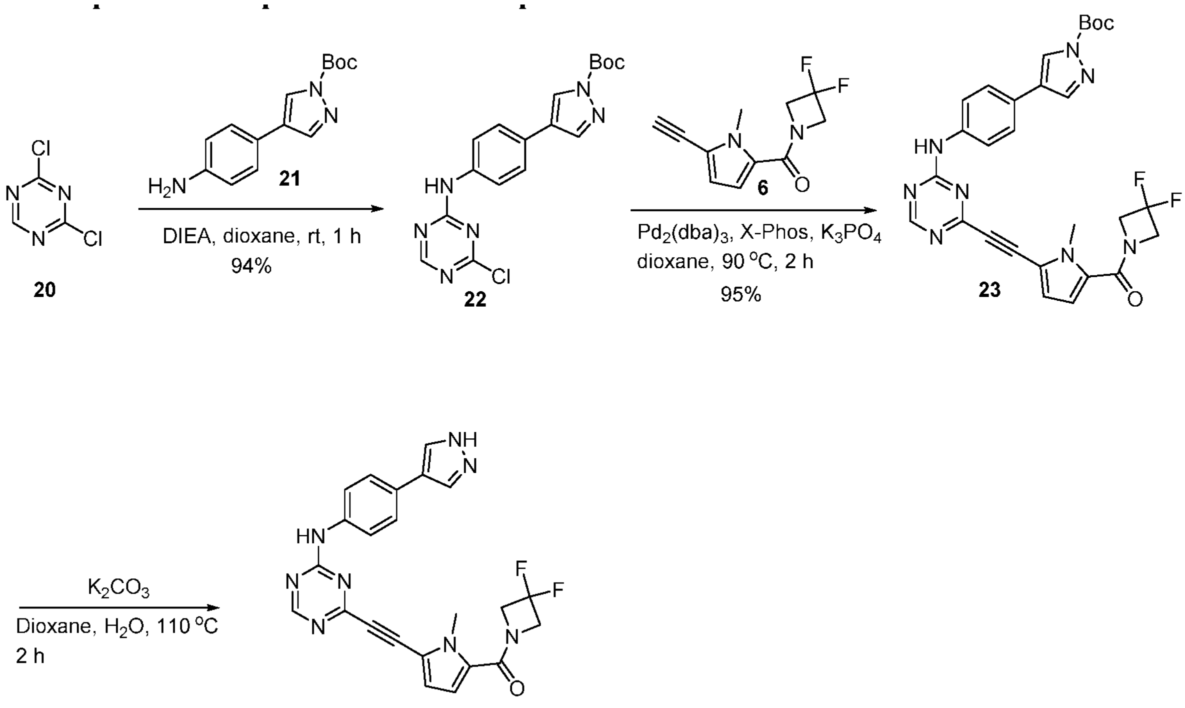


A9

Step 1: Compound 22

Compound 20 (150 mg, 1.0 mmol, 1.0 eq) was dissolved in dioxane (3 mL), then compound 21 (159 mg, 1.0 mmol, 1.0 eq) was added, then diisopropylethylamine (142 mg, 1.1 mmol, 1.1 eq) was added, the reaction solution was stirred for 1 h at 25 °C, and the reaction product was slowly added to water, and extracted twice with ethyl acetate, the combined organic phases were washed with saturated brine, dried over anhydrous sodium sulfate and concentrated, and the residue was purified by silica gel column chromatography (petroleum ether/ethyl acetate = 1/1) to obtain compound 22 (350 mg, 94%) as a white solid.
LCMS: $[M+H]^+$ = 373.1

Step 2: Compound 23

Compound 22 (50 mg, 0.13 mmol, 1.0 eq) was dissolved in dioxane (3 mL), then compound 6 (30 mg, 0.13 mmol, 1.0 eq), potassium phosphate monohydrate (90 mg, 0.39 mmol, 3.0 eq), X-Phos (12 mg, 0.026 mmol, 0.2 eq) and $Pd_2(dba)_3$ (12 mg, 0.013 mmol, 0.1 eq) were added, the reaction solution was stirred at 90 °C for 2 h under nitrogen protection after dosing. The reaction solution was cooled to room temperature, spin-dried and the residue was purified by silica gel column chromatography (DCM/MeOH=10/1) to obtain compound 23 (70 mg, 95%) as a pale yellow solid.
LCMS: $[M+H]^+$ =561.2

Step 3: Compound A9

Compound 23 (140 mg, 0.25 mmol, 1.0 eq) was dissolved in a solvent mixture of dioxane and water (3 mL/1 mL), and then potassium carbonate (103.5 mg, 0.75 mmol, 3.0 eq) was added at 0 °C and the reaction was carried out at 110 °C for 2 hours. After the reaction was completed, the reaction solution was concentrated to obtain the crude product, and the crude product was purified by prep-HPLC (mobile phase 0.1% FA/acetonitrile/water) to obtain compound A9 (9.6 mg, 8%) as a white solid.

LCMS: $[M+H]^+$ = 461.2
$^1$H NMR (400 MHz, DMSO-$d_6$) δ 12.88 (s, 1H), 10.50 (s, 1H), 8.70 (s, 1H), 8.11 (s, 1H), 7.88 (s, 1H), 7.65 (d, *J* = 8.4 Hz, 2H), 7.57 (d, *J* = 7.6 Hz, 2H), 6.79 (d, *J*= 4.4 Hz, 1H), 6.71 (d, *J* = 4.4 Hz, 1H), 4.66 (br s, 4H), 3.91 (s, 3H).

**Example 1-9 Preparation of Compound A10**

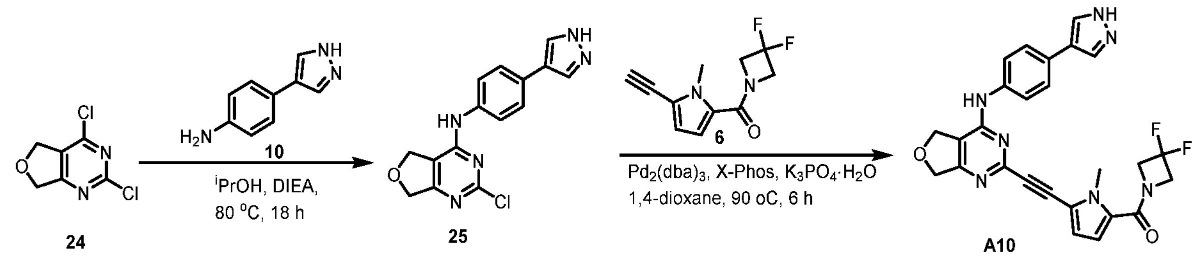


24 25 A10

Step 1: Compound 25

Compound 24 (500 mg, 2.62 mmol, 1.0 eq) and compound 10 (420 mg, 2.62 mmol, 1.0 eq) were dissolved in isopropanol (24 mL), diisopropylethylamine (3380 mg, 26.20 mmol, 10.0 eq) was added, and the reaction mixture was stirred at 80 °C for 18 h. After LCMS detection showed that the reaction was completed, the reaction solution was cooled to room temperature and concentrated to 10 mL under reduced pressure, the reaction suspension was filtered and the filter cake was washed with isopropanol, the resulting wet product was dried to obtain compound 25 (800 mg, 97%) as a yellow solid.
LCMS: $[M+H]^+$ = 314.0

Step 2: Compound A10

Compound 25 (300 mg, 0.956 mmol, 1.0 eq) and compound 6 (214 mg, 0.956 mmol, 1.0 eq) were dissolved in dioxane (10 mL), potassium phosphate monohydrate (660 mg, 2.868 mmol, 3.0 eq) was added and $Pd_2(dba)_3$ (87 mg, 0.095 mmol, 0.10 eq) and X-Phos (68 mg, 0.143 mmol, 0.15 eq) were finally added. The reaction mixture was stirred at 90 °C for 6 h under nitrogen protection. The reaction solution was cooled to room temperature, diluted with dichloromethane/methanol (50 mL, 10:1), the reaction solution was passed through diatomaceous earth and the filtrate was concentrated under reduced pressure to obtain the residue which was purified by silica gel column chromatography (dichloromethane/methanol = 93/7) to obtain compound A10 (66.3 mg, 14 %) as a yellow solid.

LCMS: $[M+H]^+$ = 502.2
$^1$H NMR (400 MHz, DMSO-$d_6$) δ 9.34 (s, 1H), 8.04 (s, 2H), 7.66 (d, *J* = 8.8 Hz, 2H), 7.61 (d, *J* = 8.8 Hz, 2H), 6.76-6.64 (m, 2H), 5.00 (s, 2H), 4.88 (s, 2H), 4.62 (br s, 4H), 3.94 (s, 3H).

**Example 1-10 Preparation of Compound A11**

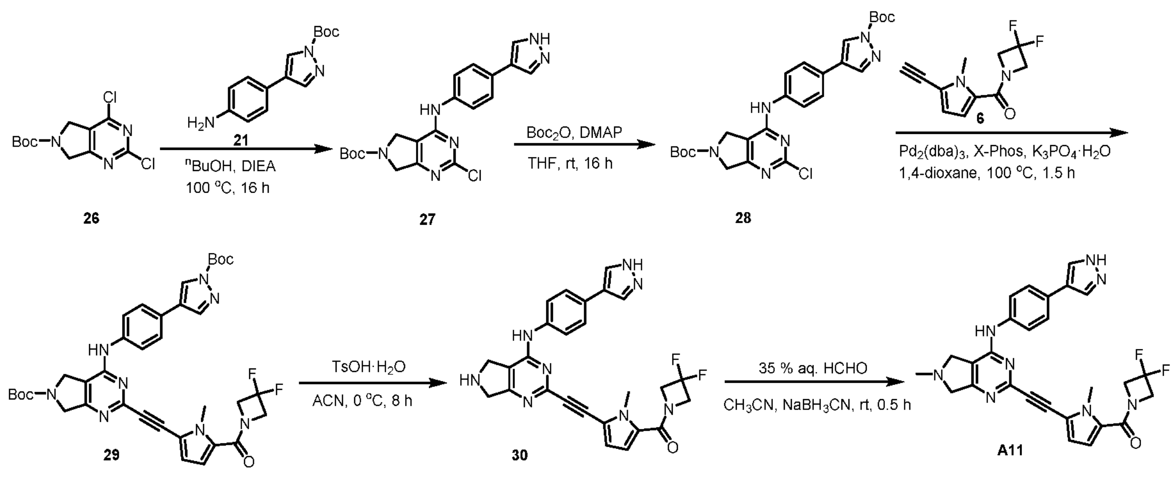


26 27 28 29 30 A11

Step 1: Compound 27

Compound 26 (600 mg, 2.068 mmol, 1.0 eq) and compound 21 (536 mg, 2.068 mmol, 1.0 eq) were dissolved in n-butanol (20 mL), diisopropylethylamine (1.3 g, 10.340 mmol, 5.0 eq) was added to the reaction solution, and the reaction solution was stirred for 16 h at 100 °C under nitrogen atmosphere. After the end of the reaction, the reaction was cooled, concentrated and the residue was purified using silica gel column chromatography (dichloromethane/methanol = 15:1) to obtain compound 27 (600 mg, 70%) as a pale yellow solid.
LCMS: $[M+H]^+$ = 413.2

Step 2: Compound 28

Compound 27 (300 mg, 0.727 mmol, 1.0 eq) and 4-dimethylaminopyridine (9 mg, 0.073 mmol, 0.1 eq) were dissolved in tetrahydrofuran (15 mL), and di-tert-butyl dicarbonate (158 mg, 0.727 mmol, 1.0 eq) was added to the reaction solution, and the reaction was stirred for 16 h at room temperature. After the end of the reaction, the reaction solution was concentrated, the residue was purified using silica gel column chromatography (petroleum ether/ethyl acetate = 1:1) to obtain compound 28 (350 mg, 94%) as a yellow solid.
LCMS: $[M+H]^+$ = 513.3

Step 3: Compound 29

Compound 28 (350 mg, 0.682 mmol, 1.0 eq), compound 5 (152 mg, 0.682 mmol, 1.0 eq) and potassium phosphate monohydrate (470 mg, 2.046 mmol, 3.0 eq) were dissolved in dioxane (8 mL) followed by addition of $Pd_2(dba)_3$ (62 mg, 0.068 mmol, 0.1 eq) and X-Phos (65 mg, 0.136 mmol, 0.2 eq) into the reaction solution. The reaction solution was stirred under nitrogen atmosphere at 100 °C for 1.5 h. After the end of the reaction, the reaction solution was cooled, passed through diatomaceous earth and washed with ethyl acetate and the filtrate was concentrated to obtain the crude product which was purified using silica gel column chromatography (petroleum ether/ ethyl acetate = 1:3) to obtain compound 29 (360 mg, 75%) as a yellow solid.
LCMS: $[M+H]^+$ = 701.2

Step 4: Compound 30

Compound 29 (360 mg, 0.513 mmol, 1.0 eq) was dissolved in acetonitrile (10 mL), p-toluenesulfonic acid monohydrate (975 mg, 5.130 mmol, 10.0 eq) was added to the reaction solution, the reaction solution was stirred for 8 h at 0°C. After the end of the reaction, N-methylimidazole was added to the system to neutralise the excess p-toluenesulphonic acid, and the resulting reaction mixture was concentrated to obtain the crude product which was purified by prep-HPLC (mobile phase: 0.1% formic acid/acetonitrile/water) to give compound 30 (100 mg) as a yellow solid, and the rest of the crude product was used directly in the next step of the reaction.

LCMS: $[M+H]^+$ = 487.2
$^1$H NMR (400 MHz, DMSO-$d_6$) δ 9.06 (s, 1H), 8.26 (s, 1H), 7.68 (d, *J* = 8.4 Hz, 2H), 7.59 (d, *J* = 8.4 Hz, 2H), 6.70 (d, *J* = 4.0 Hz, 1H), 6.68 (s, 1H), 6.55 (s, 1H), 4.56-4.47 (m, 4H), 4.06 (s, 2H), 3.97 (s, 2H), 3.93 (s, 3H).

Step 5: Compound A11

Compound 30 (100 mg) was dissolved in acetonitrile and dichloromethane (8 mL/6 mL), 37% aqueous formaldehyde (72 mg, 0.891 mmol) was added to the reaction solution and the reaction mixture was stirred at room temperature for 5 min, followed by the addition of sodium cyanoborohydride (126 mg, 0.594 mmol, 2.0 eq) to the reaction solution. The reaction solution was stirred at room temperature for half an hour, after the reaction was completed, the reaction solution was concentrated directly under reduced pressure and the residue was purified by prep-HPLC (mobile phase: 0.1% formic acid/acetonitrile/water) to obtain compound A11 (53.5 mg,) as a yellow solid.

LCMS: $[M+H]^+$ = 515.2
$^1$H NMR (400 MHz, DMSO-$d_6$) δ12.89 (br s, 1H), 9.37 (s, 1H), 8.03 (br s, 2H), 7.66-7.60 (m, 4H), 6.71 (s, 2H), 4.64 (br s, 4H), 4.26 (s, 4H), 3.93 (s, 3H), 2.29 (s, 3H).

Example 1-11 Preparation of Compound A12

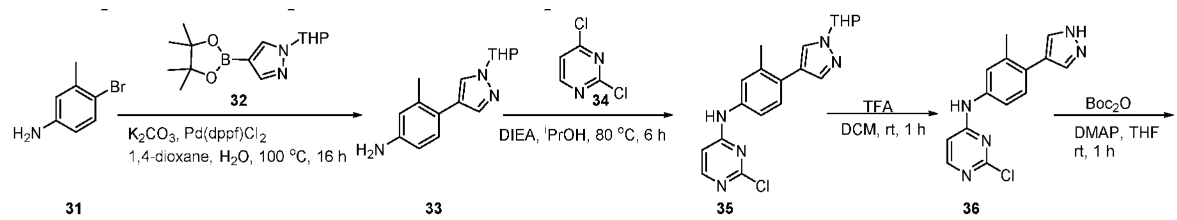


31 33 35 36

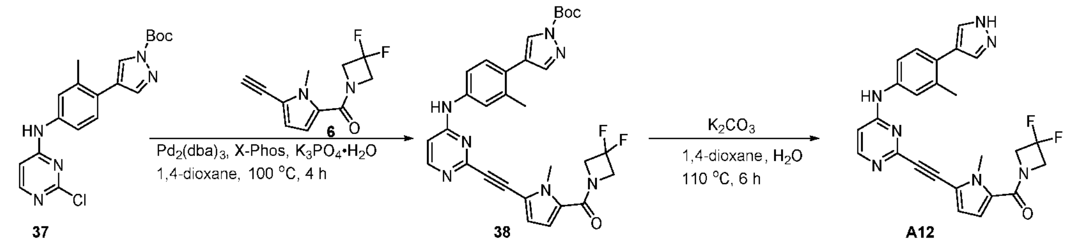


37 38 A12

Step 1: Compound 33

Compound 31 (1.0 g, 5.37 mmol, 1.1 eq) was dissolved in a solvent mixture of dioxane/water (10/1, 20 mL/2 mL) followed by rapid addition of Compound 32 (1.4 g, 4.89 mmol, 1.0 eq), potassium carbonate (2.0 g, 14.49 mmol, 3.0 eq), and Pd(dppf)$Cl_2$ (355 mg, 0.15 mmol, 0.1 eq). After replacing the air with nitrogen three times, the reaction solution was stirred at 100 °C for 16 h under nitrogen atmosphere. The reaction solution was cooled to room temperature and concentrated. The residue was purified by silica gel column chromatography (petroleum ether/ethyl acetate = 1/1) to obtain compound 33 (750 mg, 58%) as a yellow solid.
LCMS: $[M+H]^+$ = 258.1

Step 2: Compound 35

Compound 33 (690 mg, 2.69 mmol, 1.0 eq) was dissolved in isopropanol (10 mL), then compound 34 (1.2 g, 8.06 mmol, 3.0 eq) and diisopropylethylamine (1.4 g, 10.74 mmol, 4.0 eq) were added. The reaction solution was stirred at 80 °C for 6 hours. The reaction solution was cooled to room temperature, diluted with ethyl acetate (100 mL), and then sequentially washed with water, washed with saturated brine, dried over anhydrous sodium sulfate and concentrated. The residue was purified by silica gel column chromatography (petroleum ether/ethyl acetate=1/2) to obtain compound 35 (800 mg, 81%) as a yellow solid.
LCMS: $[M+H]^+$ = 370.1

Step 3: Compound 36

Compound 35 (750 mg, 2.03 mmol, 1.0 eq) was dissolved in dichloromethane (30 mL) and then TFA (30 mL) was added. The reaction solution was stirred at 25 °C for 1 hour. The reaction solution was spin-dried, the residue was dissolved with ethyl acetate, the pH was adjusted to 7 with aqueous sodium carbonate, and the aqueous phase was extracted with ethyl acetate (40 mL × 3). The combined organic phases were washed with saturated brine, dried over anhydrous sodium sulfate and concentrated to obtain compound 36 (750 mg, 100%) as a yellow solid.
LCMS: $[M+H]^+$ = 286.2

Step 4: Compound 37

Compound 36 (400 mg, 1.4 mmol, 1.0 eq) was dissolved in tetrahydrofuran (5 mL) followed by the addition of $Boc_2O$ (327 mg. 2.1 mmol, 1.5 eq) and DMAP (34 mg. 0.28 mmol, 0.2 eq). The reaction solution was stirred at 25 °C for 1 hour. The reaction solution was concentrated and the residue was purified by silica gel column chromatography (petroleum ether/ethyl acetate = 4/1) to obtain compound 37 (350 mg, 65%) as a yellow solid.
LCMS: $[M+H]^+$ = 386.2

Step 5: Compound 38

Compound 37 (200 mg, 0.45 mmol, 1.0 eq) was dissolved in dioxane (5 mL), followed by rapid addition of

Compound 8 (101 mg, 0.45 mmol, 1.0 eq), potassium phosphate monohydrate (363 mg, 1.58 mmol,3.5 eq), X-Phos (86 mg, 0.18 mmol,0.4 eq) and $Pd_2(dba)_3$ (83 mg, 0.09 mmol, 0.2 eq). After displacement with nitrogen for three times, the reaction solution was stirred at 100 °C for 4 h under nitrogen atmosphere. The reaction solution was cooled to room temperature, diluted with ethyl acetate (60 mL), sequentially washed with water, washed with saturated saline, dried over anhydrous sodium sulfate and concentrated. The residue was purified by silica gel column chromatography (petroleum ether/ethyl acetate=1/1) to obtain compound 38 (90 mg, 42%) as a yellow solid.
LCMS: $[M+H]^+$ = 574.3

Step 6: Compound A12

Compound 38 (90 mg, 0.16 mmol, 1.0 eq) was dissolved in a solvent mixture of dioxane/water (10/1,20 mL/2 mL) followed by addition of potassium carbonate (331 mg, 2.4 mmol, 15.0 eq). The reaction solution was stirred at 110 °C for 6 hours. The reaction solution was concentrated and the residue was purified by prep-HPLC (mobile phase: 0.1% formic acid/acetonitrile/water) to obtain compound A12 (25.5 mg, 21%) as a yellow solid.

LCMS: $[M+H]^+$ = 474.3
$^1$H NMR (400 MHz, DMSO-$d_6$) δ 12.93 (br s, 1H), 9.76 (s, 1H), 8.27 (d, *J* = 6.0 Hz, 1H), 7.91-8.26 (m, 2H), 7.53 (s, 1H), 7.47 (d, *J* = 8.8 Hz, 1H), 7.38 (d, *J* = 8.4 Hz, 1H), 6.77 (d, *J* = 6.0 Hz, 1H), 6.73-6.70 (m, 2H), 4.66 (br s, 4H), 3.96 (s, 3H), 2.38 (s, 3H).

**Example 1-12 Preparation of Compound A14**

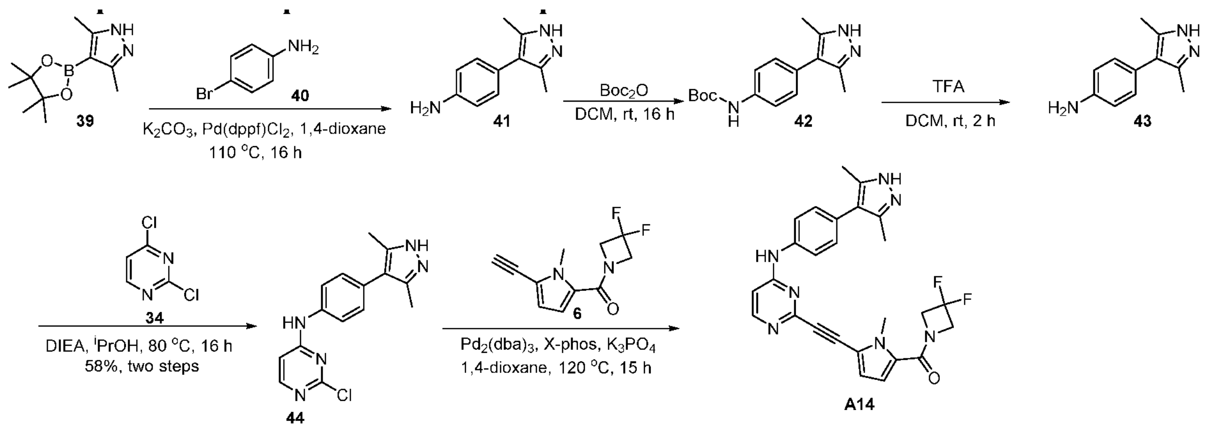


Step 1: Compound 41

Compound 39 (830 mg, 3.74 mmol, 1.0 eq) was dissolved in dioxane (5 mL), followed by the sequential addition of Compound 40 (707 mg, 4.11 mmol, 1.1 eq), potassium carbonate (2.58 g, 18.6 mmol, 5.0 eq) and $Pd(dppf)Cl_2$ (272 mg, 0.37 mmol, 0.1 eq). After the addition, the reaction solution was warmed to 110 °C and stirred for 16 hours. The reaction solution was directly spin-dried to obtain crude compound 41 which was used in the next step of the reaction without purification.
LCMS: $[M+H]^+$ = 188.2

Step 2: Compound 42

Crude compound 41 was dissolved in DCM (50 mL), then $Boc_2O$ (872 mg, 4.0 mmol, 1.0 eq) was added and stirred at room temperature for 16 hours. The reaction solution was extracted by ethyl acetate/water, washed with saturated brine, dried over anhydrous sodium sulfate, and the residue was purified by silica gel column chromatography (PE/EA = 1:1) to obtain Compound 42 (160 mg, two steps: 15%) as a colorless oil.
LCMS: $[M+H]^+$ =288.2

Step 3: Compound 43

Compound 42 (160 mg, 0.56 mmol, 1.0 eq) was dissolved in dichloromethane (3 mL), and then trifluoroacetic acid (3 mL) was added, the reaction solution was stirred at 25 °C for 2 hours. The reaction solution was spin-dried directly to obtain crude compound 43.
LCMS: $[M+H]^+$ = 188.2

Step 4: Compound 44

The crude compound 44 was dissolved in isopropanol (3 mL), and compound 34 (83 mg, 0.56 mmol, 1.0 eq) and diisopropylethylamine (1.45 g, 11.2 mmol, 20.0 eq) were added sequentially, and the reaction solution was stirred at 80 °C for 16 hours. The reaction solution was extracted with ethyl acetate/water, washed with saturated brine, dried over anhydrous sodium sulfate, and the residue was purified by silica gel column chromatography (PE/EA = 1:1) to obtain compound 44 (98 mg, 58% in two steps) as a yellow solid.
LCMS: $[M+H]^+$ =300.1

Step 5: Compound A14

Compound 44 (88 mg, 0.29 mmol, 1.0 eq) was dissolved in dioxane (10 ml), and then compound 6 (66 mg, 0.29 mmol, 1.0 eq), potassium phosphate (369 mg, 1.74 mmol, 6.0 eq) and $Pd_2(dba)_3$ (26 mg, 0.029 mmol, 6.0 eq), and X-Phos (13.8 mg, 0.029 mmol, 0.1 eq) were added in sequence. After addition, the reaction solution was warmed to 120 °C and stirred for 15 h. The reaction solution was concentrated and the residue ontained after concentration was purified by prep-HPLC (mobile phase 0.1% formic acid/acetonitrile/water) to obtain compound A14 (10.5 mg, 7%) as a grey solid.

LCMS: $[M+H]^+$ =488.2
$^1$H NMR (400 MHz, DMSO-$d_6$) δ 12.22 (br s, 1H), 9.82 (s, 1H), 8.27 (d, *J* = 6.0 Hz, 1H), 7.65 (d, *J* = 8.4 Hz, 2H), 7.28 (d, *J* = 8.4 Hz, 2H), 6.79 (d, *J* = 6.0 Hz, 1H), 6.71 (s, 2H), 4.65 (br s, 4H), 3.94 (s, 3H), 2.20 (s, 6H).

**Example 1-13 Preparation of Compound A15**

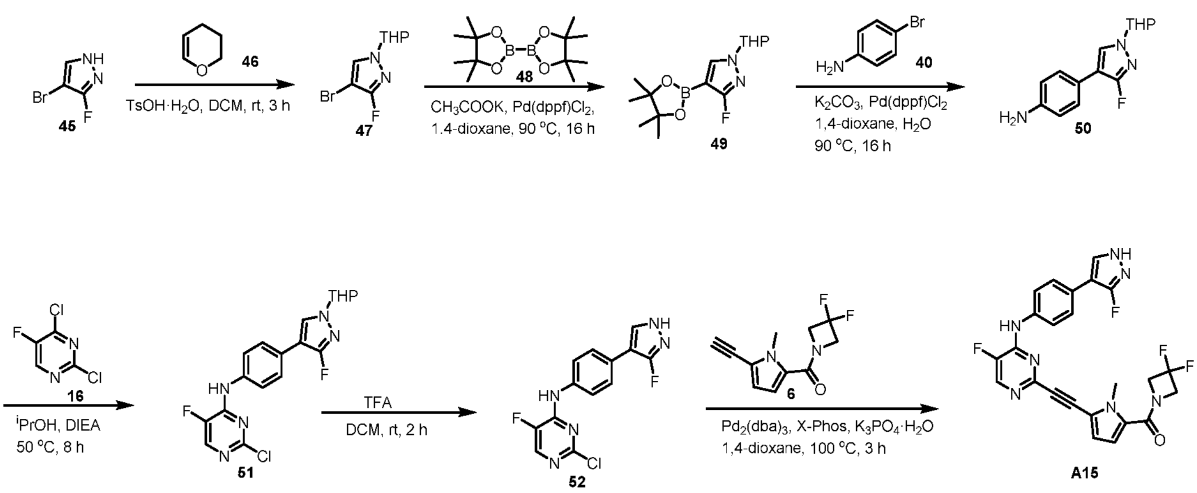


Step 1: Compound 47

Compound 45 (100 mg, 0.606 mmol, 1.0 eq) and p-toluenesulfonic acid monohydrate (17 mg, 0.091 mmol, 0.15 eq) were dissolved in dichloromethane (4 mL) followed by addition of compound 46 (152 mg, 1.818 mmol, 3.0 eq). After the addition, the reaction solution was allowed to react under nitrogen atmosphere at 25 °C for 3 h. After LC-MS detection showed that the reaction was completed, the solvent was removed by distillation under reduced pressure and the residue was purified by silica gel column chromatography (petroleum ether/dichloromethane=10/1) to obtain compound 47 (140 mg, 93%) as a yellow oily liquid.
LCMS: $[M+Na]^+$ = 271.2

Step 2. Compound 49

Compound 47 (130 mg, 0.522 mmol, 1.0 eq), Compound 48 (265 mg, 1.044 mmol, 2.0 eq), Pd(dppf)$Cl_2$ (38 mg, 0.052 mmol, 0.1 eq), and Potassium Acetate (153 mg, 1.566 mmol, 3.0 eq) were added to 1, 4-dioxane (4 mL). After the addition, nitrogen was replaced and the reaction solution was reacted under nitrogen atmosphere at 90 °C for 16 h. After LC-MS detection showed that the reaction was completed, the reaction mixture was passed through diatomaceous earth and the diatomaceous earth was washed with ethyl acetate, and the filtrate obtained was directly distilled under reduced pressure to remove the solvent, and the crude compound 49 was directly used in the next step of the reaction.
LCMS: $[M+Na]^+$ = 319.2

Step 3: Compound 50

The crude compound 49 from the previous step was dissolved with dioxane/water (4 mL/1 mL), then compound 6 (179 mg, 1.044 mmol, 2.0 eq), Pd(dppf)$Cl_2$ (76 mg, 0.104 mmol, 0.2 eq), potassium carbonate (360 mg, 2.610 mmol, 5.0 eq) were added. After dosing, nitrogen was replaced and the reaction solution was reacted under nitrogen atmosphere at 90 °C for 16 h. After LC-MS detection showed that the reaction was completed, the reaction mixture was passed through diatomaceous earth and the diatomaceous earth was rinsed with ethyl acetate, and the filtrate obtained was directly distilled under reduced pressure to remove the solvent, and the residue was eluted by silica gel column chromatography (petroleum ether/ethyl acetate=1/1) to obtain Compound 50 (100 mg, 73%) as a yellowish solid.
LCMS: $[M+Na]^+$ = 262.2

Step 4: Compound 51

Compound 50 (100 mg, 0.383 mmol, 1.0 eq) and compound 16 (96 mg, 0.575 mmol, 1.5 eq) were dissolved in isopropanol (4 mL) followed by addition of diisopropylethylamine (247 mg, 1.915 mmol, 5.0 eq). After the addition, the reaction solution was allowed to react under nitrogen atmosphere at 50 °C for 8 h. After LC-MS detection showed that the reaction was completed, the solvent was removed by distillation under reduced pressure and the residue was eluted by silica gel column chromatography (petroleum ether/dichloromethane=1/1) to obtain compound 51 (90 mg, 40%) as a yellow solid.
LCMS: $[M+H]^+$ = 392.2

Step 5. Compound 52

Compound 51 (250 mg, 0.638 mmol, 1.0 eq) was dissolved in dichloromethane (3 mL) followed by addition of trifluoroacetic acid (3 mL) to the solution. After the addition was completed, the reaction solution was reacted at 25 °C for 2 hours. The solvent was removed by distillation under reduced pressure and the residue was adjusted to pH (9-10) with saturated sodium carbonate solution, the solution was filtered and the solid was collected to obtain compound 52 (130 mg, 66%) as an offwhite solid.
LCMS: $[M+H]^+$ = 308.0

Step 6: Compound A15

Compound 52 (100 mg, 0.325 mmol, 1.0 eq), compound 6 (73 mg, 0.325 mmol, 1.0 eq), $Pd_2(dba)_3$ (45 mg, 0.049 mmol, 0.15 eq), X-Phos (31 mg, 0.065 mmol, 0.2 eq), and potassium phosphate monohydrate (224 mg, 0.975 mmol, 3.0 eq) were added to 1, 4-dioxane (4 mL). After the addition, nitrogen was replaced and the reaction solution was reacted under nitrogen atmosphere at 100 °C for 3 h. After LC-MS detection showed that the reaction was completed, the reaction solution was cooled down to room temperature, then the reaction mixture was passed through diatomaceous earth and the diatomaceous earth was rinsed with dichloromethane, and the organic phase obtained was distilled under reduced pressure to remove the solvent, and the residue was eluted by silica gel column chromatography (dichloromethane/-methanol=13/1) to obtain the crude product which was purified using prep -HPLC (mobile phase: 0.1% formic acid/-acetonitrile/water) to obtain compound A15 (2.0 mg, 1%) as a yellow solid.

LCMS: $[M+H]^+$ = 496.1
$^1$H NMR (400 MHz, DMSO-$d_6$) δ 12.57 (s, 0.5H), 9.82 (s, 1H), 8.41 (d, *J* = 3.6 Hz, 1H), 8.14 (s, 1H), 7.79 (d, *J* = 8.4 Hz, 2H), 7.57 (d, *J* = 8.4 Hz, 2H), 6.70 (s, 2H), 4.67 (br s, 4H), 3.93 (s, 3H).

**Example 1-14 Preparation of Compound A16**

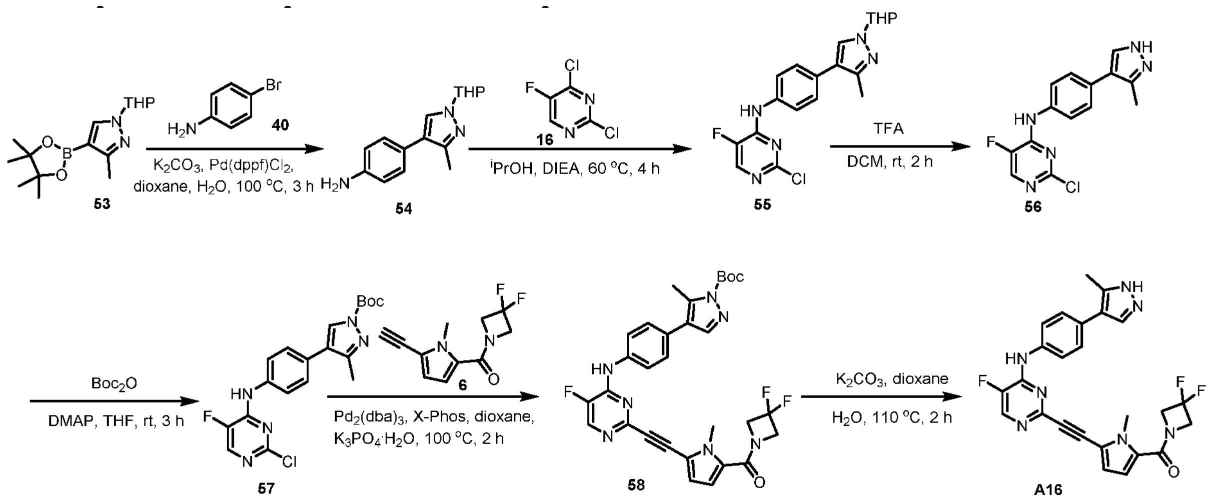


Step 1: Compound 54

Compound 53 (1.08 g, 3.69 mmol, 1.2 eq) and Compound 40 (524 mg, 3.07 mmol, 1.0 eq) were dissolved in 1,4 dioxane (12 mL), and then potassium carbonate (1.27 g, 9.21 mmol, 3.0 eq), Pd(dppf)$Cl_2$ (336 mg, 0.46 mmol, 0.15 eq) and water (3 mL) were added. The reaction solution was stirred at 100 °C under nitrogen atmosphere for 3 hours. The reaction solution was cooled to room temperature, diluted with ethyl acetate, passed through diatomaceous earth, and the filtrate was concentrated to obtain the residue which was purified by rapid column chromatography on silica gel (petroleum ether/ethyl acetate=4/1) to obtain compound 54 (330 mg, 41%) as a yellow solid.
LCMS: $[M+H]^+$ = 258.2

Step 2: Compound 55

Compound 54 (330 mg, 1.28 mmol, 1.0 eq) and compound 16 (638 mg, 3.84 mmol, 3.0 eq) were dissolved in isopropanol (8 mL) and then diisopropylethylamine (495 mg, 3.84 mmol, 3.0 eq) was added. The reaction solution was stirred under nitrogen atmosphere at 60 °C for 4 h. After LC-MS detection showed that the reaction was completed, the reaction solution was concentrated to remove the solvent to obtain the residue which was purified by rapid column chromatography on silica gel (petroleum ether/ethyl acetate = 65/35) to obtain compound 55 (350 mg, 70%) as a yellow solid.
LCMS: $[M+H]^+$ = 388.1

Step 3: Compound 56

Compound 55 (730 mg, 1.88 mmol, 1.0 eq) was dissolved in dichloromethane (4 mL) and then trifluoroacetic acid (22 mL) was added. The reaction solution was stirred at room temperature for 2 hours. The reaction solution was concentrated to obtain a residue which was diluted with ethyl acetate. Then the pH was adjusted to alkaline with saturated aqueous sodium carbonate solution, and the solution was extracted with ethyl acetate. The combined organic phases were dried over anhydrous sodium sulfate, and concentrated to obtain the crude compound 56 (569 mg, 100%) as a yellow solid.
LCMS: $[M+H]^+$ = 304.1

Step 4: Compound 57

Compound 56 (569 mg, 1.87 mmol, 1.0 eq) was added to tetrahydrofuran (5 mL) followed by DMAP (45 mg, 0.37 mmol, 0.2 eq) and $Boc_2O$ (325 mg, 1.49 mmol, 0.8 eq) sequentially. The reaction solution was stirred at room temperature for 3 hours. The reaction solution was diluted with ethyl acetate and then washed sequentially with water and saturated saline, dried over anhydrous sodium sulfate and concentrated. The residue was purified by rapid column chromatography on silica gel (petroleum ether/ethyl acetate = 80/20) to obtain compound 57 (470 mg, 78%) as a white solid.

LCMS: [M+H] $^+$ = 404.1

Step 5: Compound 58

Compound 57 (200 mg, 0.49 mmol, 1.0 eq) was dissolved in dioxane (8 mL) followed by the addition of compound 6 (163 mg, 0.73 mmol, 1.5 eq), potassium phosphate (338 mg, 1.47 mmol, 3.0 eq), X-Phos (42 mg, 0.09 mmol, 0.2 eq), and $Pd_2(dba)_3$ (64 mg, 0.07 mmol, 0.15 eq), and the reaction solution was stirred at 100 °C for 2 h under nitrogen atmosphere after dosing. The reaction solution was cooled to room temperature, diluted with ethyl acetate, passed through diatomaceous earth, and the filtrate was concentrated to obtain the crude product, and the crude compound 58 was directly put into the next step of the reaction.
LCMS: [M+H] $^+$ =592.2

Step 6: Compound A16

Crude compound 58 (110 mg, 0.18 mmol, 1.0 eq) was dissolved in a solvent mixture of dioxane (4 mL) and water (4 mL), then potassium carbonate (248 mg, 1.8 mmol, 10.0 eq) was added. The reaction solution was stirred at 110 °C for 2 hours. The reaction solution was diluted with ethyl acetate, then washed sequentially with water and saturated saline, dried over anhydrous sodium sulfate and concentrated. The residue was purified by prep-HPLC (mobile phase: 0.1% formic acid/acetonitrile/water) to obtain compound A16 (77.5 mg, 87%) as a white solid.

LCMS: [M+H] $^+$ =492.1
$^1$H NMR (400 MHz, DMSO-$d_6$) δ 12.63-12.55 (m, 1H), 9.79 (s, 1H), 8.40 (d, $J$ = 3.6 Hz, 1H), 7.77-7.75 (m, 2H), 7.67 (br. s, 1H), 7.46-7.44 (m, 2H), 6.70-6.68 (m, 2H), 4.62 (br. s, 4H), 3.92 (s, 3H), 2.39 (s, 3H).

**Example 1-15 Preparation of Compound A17**

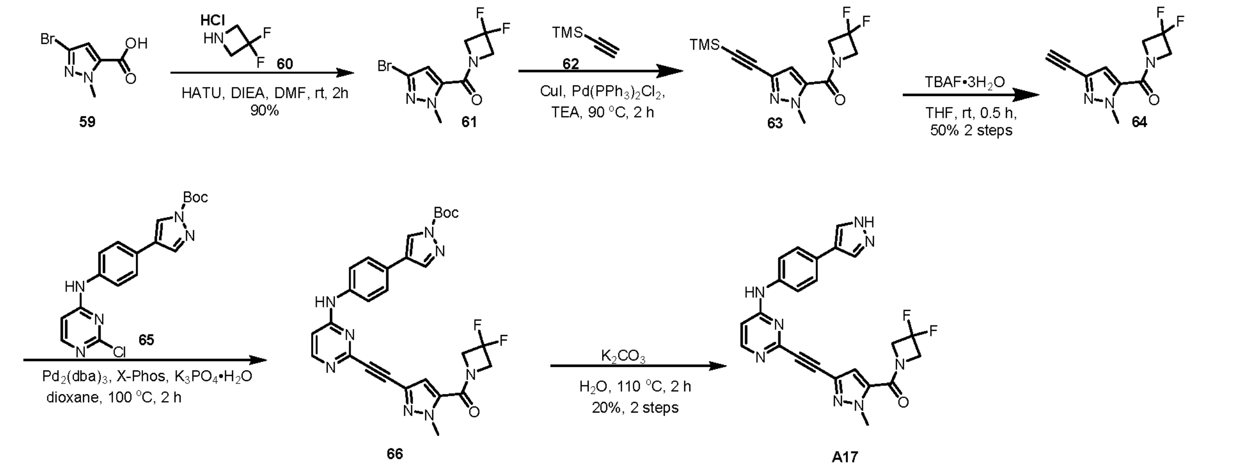


Step 1: Compound 61

Compound 59 (500 mg, 2.45 mmol, 1.0 eq), compound 60 (476 mg, 3.68 mmol, 1.5 eq), HATU (1.68 g, 4.41 mmol, 1.8 eq) and diisopropylethylamine (1.26 g, 9.8 mmol, 4.0 eq) were dissolved in DMF (20 mL) and stirred at room temperature for 2 hours. After completion of the reaction, the reaction solution was diluted with water and extracted three times with ethyl acetate. The organic phases were combined, washed with saturated saline, dried over anhydrous sodium sulfate and concentrated. The obtained residue was purified by column chromatography (PE:EA=83:17) to obtain compound 61 (664 mg, 90%) as a white solid.
LCMS: [M+H] $^+$ = 280.1

Step 2: Compound 63

Compound 61 (600 mg, 2.15 mmol, 1.0 eq), Compound 62 (632 mg, 6.45 mmol, 3.0 eq), CuI (123 mg, 0.645 mmol, 0.3 eq) and Pd(PPh3)$_2$Cl$_2$ (151 mg, 0.215 mmol, 0.1 eq) were dissolved in triethylamine (10 mL). The reaction was

protected with nitrogen and stirred at 90 °C for 2 hours. After completion of the reaction, the reaction solution was diluted with water and extracted three times with ethyl acetate. The organic phases were combined, washed with saturated brine, dried over anhydrous sodium sulfate and concentrated to obtain the crude compound 63 (1.05 g, 100%) as a brown liquid.
LCMS: $[M+H]^+ = 298.1$

Step 3: Compound 64

Compound 63 (1.05 g, 2.15 mmol, 1.0 eq) and tetrabutylammonium fluoride trihydrate (845 mg, 3.22 mmol, 1.5 eq) were dissolved in tetrahydrofuran (10 mL). The reaction solution was stirred at room temperature for half an hour. After completion of the reaction, the reaction solution was diluted with water and extracted three times with ethyl acetate. The organic phases were combined, washed with saturated saline, dried over anhydrous sodium sulfate and concentrated. The residue was purified by column chromatography (PE:EA=90:10) to obtain compound 64 (220 mg, 50% two-step) as a white solid.
LCMS: $[M+H]^+ = 226.0$

Step 4: Compound 66

Compound 64 (220 mg, 0.978 mmol, 1.0 eq) , Compound 65 (365 mg, 0.978 mmol, 1.0 eq) , $Pd_2(dba)_3$ (90 mg, 0.0978 mmol, 0.1 eq), X-Phos (187 mg, 0.391 mmol, 0.4 eq) and Potassium phosphate monohydrate ( 675 mg, 2.93 mmol, 3.0 eq) were dissolved in dioxane (10 mL). The reaction solution was protected with nitrogen and stirred at 100 °C for 2 h. After LCMS detection showed that the reaction was completed, the reaction solution was cooled and the solution of crude compound 66 was used directly in the next step.
LCMS: $[M+H]^+ = 561.1$

Step 4: Compound A17

Potassium carbonate (195 mg, 1.41 mmol, 3.0 eq) and water (1 mL) were added directly to the reaction solution of crude compound 66 from the previous step and stirred at 110 °C for 2 hours. After completion of the reaction, the reaction solution was filtered and the filtrate was concentrated. The residue was purified by prep-HPLC (mobile phase: 0.1% formic acid/acetonitrile/water) to obtain compound A17 (70 mg, 20% two steps) as a white solid.

LCMS: $[M+H]^+ = 461.1$
$^1$H NMR (400 MHz, DMSO-$d_6$) δ 9.95 (s, 1H), 8.28 (d, *J* = 6.0 Hz, 1H), 8.04 (s, 2H), 7.65-7.58 (m, 4H), 7.20 (s, 1H), 6.79 (d, *J* = 6.0 Hz, 1H), 4.89 (t, *J* = 12.0 Hz, 2H), 4.49 (t, *J* = 12.0 Hz, 2H), 4.07 (s, 3H).

**Example 1-16 Preparation of Compound A18**

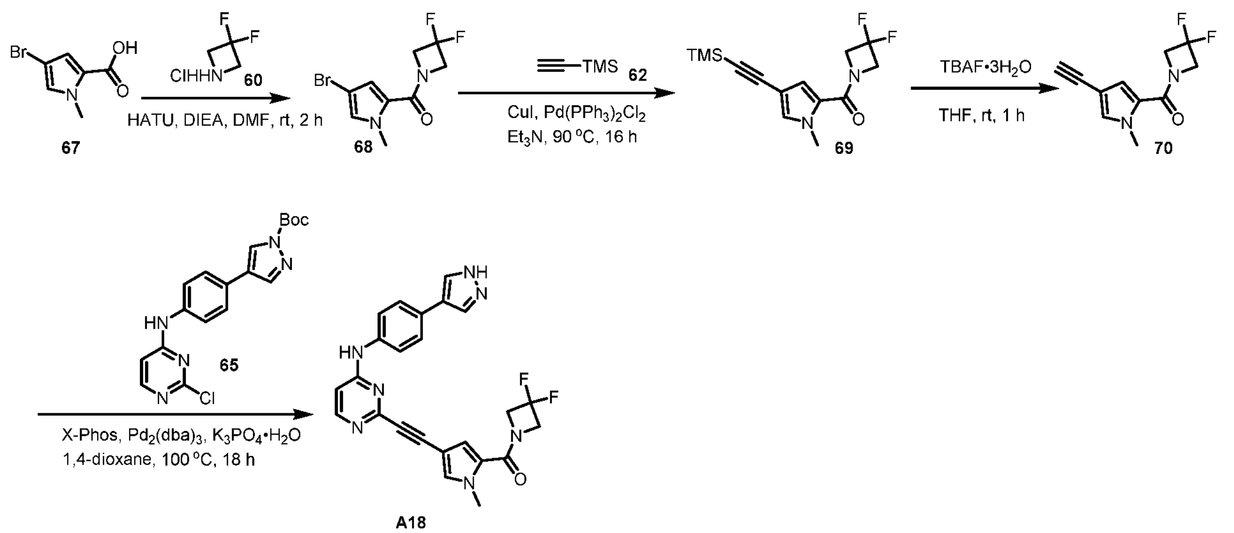

Step 1: Compound 68

Compound 67 (2.9 g, 14.21 mmol, 1.0 eq) was dissolved in DMF (30 mL), HATU (6.49 g, 17.06 mmol, 1.2 eq) was added, and stirred for 10 min, then compound 60 (1.84 g, 14.21 mmol, 1.0 eq) and diisopropylethylamine (5.51 g, 42.64 mmol, 3.0 eq) were added, and the reaction mixture was continued to be stirred at room temperature for 2 h. After LCMS determined that the reaction was completed, the reaction solution was diluted with ethyl acetate, washed with water, washed with brine, dried over anhydrous sodium sulfate and concentrated. The residue was purified by silica gel column chromatography (EA:PE = 1:10) to obtain compound 68 (2.8 g, 70 %) as a white solid.
LCMS: [M+H] = 279.0

Step 2: Compound 69

Compound 68 (2.7 g, 9.67 mmol, 1.0 eq) was dissolved in triethylamine (30 mL), and cuprous iodide (553 mg, 2.9 mmol, 0.3 eq) and $Pd(PPh3)_2Cl_2$ (679 mg, 0.967 mmol, 0.1 eq) and compound 62 (2.85 g, 29.02 mmol, 3.0 eq) were added. The reaction mixture was stirred under nitrogen atmosphere at 90 °C for 16 h. After LCMS determined that the reaction was completed, water (100 mL) was added to the reaction solution, and then extracted three times with ethyl acetate (150 mL ). The combined organic phases were washed with saturated brine, dried over anhydrous sodium sulfate, and concentrated to obtain the crude compound 69 (595 mg, 80%) as a black solid which was used directly in the next step.
LCMS: [M+H] + = 297.1

Step 2: Compound 70

Compound 69 (2.2 g, 7.65 mmol, 1.0 eq), the crude product from the previous step, was dissolved in THF (10 mL), and tetrabutylammonium fluoride trihydrate (4.8 g, 15.3 mmol, 2.0 eq) was added. The mixture was stirred at room temperature for 1 h. After LCMS detection showed that the reaction was completed, water (100 mL) was added to the reaction solution, and then extracted three times with ethyl acetate (150 mL). The combined organic phases were washed with saturated brine, dried over anhydrous sodium sulfate and concentrated. The residue was purified by silica gel column chromatography (EA:PE = 1:9) to obtain compound 70 (1.2 g, 70 % yield in two steps) as a yellow solid.
LCMS: $[M+H]^+$ = 225.1

Step 3: Compound A18

Compound 70 (150 mg, 0.67 mmol, 1.0 eq) and Compound 65 (250 mg, 0.67 mmol, 1.0 eq) were dissolved in dioxane (5 mL), X-Phos (47 mg, 0.1 mmol, 0.15 eq) was added along with potassium phosphate monohydrate (462 mg, 2.01 mmol, 3.0 eq), and $Pd_2(dba)_3$ (61 mg, 0.06 mmol, 0.1 eq) was finally added. The reaction mixture was stirred under nitrogen protection at 100 °C for 18 h. After LCMS detection showed that the reaction was completed, the reaction mixture was filtered through diatomaceous earth, the filtrate was washed with ethyl acetate, and the filtrate was concentrated to obtain the residue. The residue was purified by prep-HPLC (mobile phase: 0.1% HCOOH/acetonitrile/water) to obtain compound A18 (36 mg, 11 %) as a yellow solid.

LCMS: $[M+H]^+$ = 460.2
$^1$H NMR (400 MHz, DMSO-$d_6$) δ 12.88 (br s, 1H), 9.66 (s, 1H), 8.22 (d, *J* = 6.0 Hz, 1H), 8.09 (br s, 1H), 7.93 (br s, 1H), 7.59 (s, 4H), 7.56 (d, *J* = 1.6 Hz, 1H), 6.92 (d, *J* = 1.6 Hz, 1H), 6.70 (d, *J* = 6.0 Hz, 1H), 4.66 (br s, 4H), 3.85 (s, 3H).

**Example 1-17 Preparation of Compound A19**

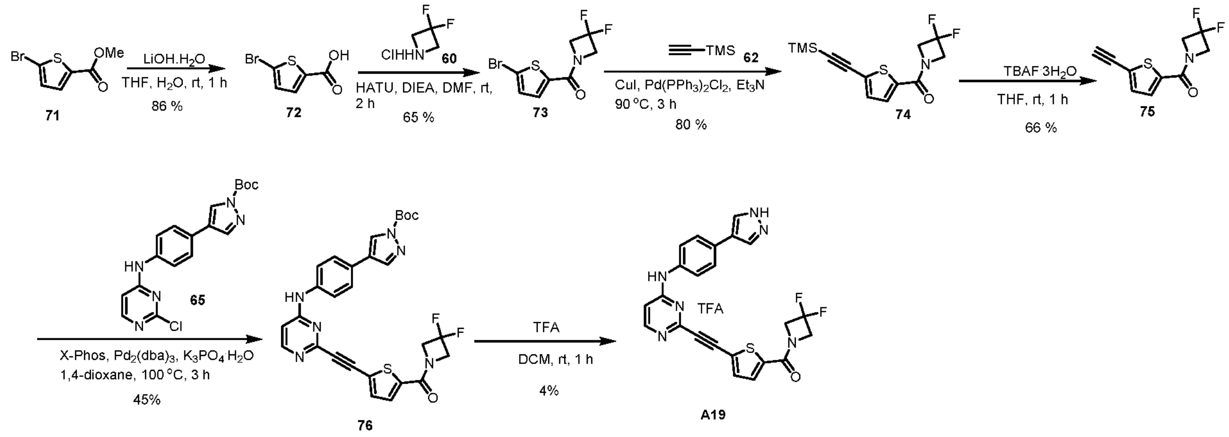


71 72 60 73 74 75 65 76 A19

Step 1: Compound 72

Compound 71 (2.0 g, 9.0 mmol, 1.0 eq) was dissolved in THF (16 mL), lithium hydroxide monohydrate (2.1 g, 90.0 mmol, 10.0 eq) and water (10 mL) were added, and the reaction mixture was stirred for 1 h at 25 °C. After LCMS detection showed that the reaction was completed, water (80 mL) was added to the reaction solution, and the reaction solution was extracted with ethyl acetate (50 mL × 3), the aqueous phase was collected, the pH was adjusted to acidic=3 by adding dioxane hydrochloride solution (5 mL) to the aqueous phase, ethyl acetate was added to extract, the organic phase was washed with saturated brine, dried over anhydrous sodium sulfate, and then concentrated to obtain the crude compound 72 (1.6 g, 86%) as a yellow solid.
LCMS: [M+H] + = 206.9

Step 2: Compound 73

Compound 72 (800 mg, 3.8 mmol, 1.0 eq) was dissolved in DMF (10 mL) at room temperature, HATU (2.88 g, 7.6 mmol, 2.0 eq) was added, and stirred for 10 min, then compound 3 (600 mg, 4.6 mmol, 1.2 eq) was added along with diisopropylethylamine (2.4 g, 19.0 mmol, 5.0 eq), the reaction mixture was continued to be stirred at 25°C for 2 hours. After LCMS detection showed that the reaction was completed, water (60 mL) was added to the reaction solution, and the reaction solution was extracted with ethyl acetate (40 mL×3), the combined organic phases were washed with saturated brine, dried over anhydrous sodium sulfate, and concentrated to give the residue which was purified using silica gel column chromatography (0-10 % PE/EA) to obtain compound 73 (700 mg, 65%) as a white solid.
LCMS: $[M+H]^+ = 282.0$

Step 3: Compound 74

Compound 73 (700 mg, 2.49 mmol, 1.0 eq) was dissolved in triethylamine (15 mL), and cuprous iodide (93 mg, 0.49 mmol, 0.2 eq) and $Pd(PPh3)_2Cl_2$ (168 mg, 0.24 mmol, 0.1 eq) and compound 62 (732 mg, 7.47 mmol, 3.0 eq) were added, the reaction mixture was stirred under nitrogen atmosphere at 90 °C for 3 h. After LCMS detection showed that the reaction was completed, water (100 mL) was added to the reaction solution, the reaction solution was then extracted with ethyl acetate (80 mL × 3), and the combined organic phases were washed with saturated brine, dried over anhydrous sodium sulfate, and then concentrated to obtain the crude Compound 74 (595 mg, 80%) as a white solid.
LCMS: $[M+H]^+ = 300.1$

Step 4: Compound 75

Crude compound 74 (595 mg, 1.98 mmol, 1.0 eq) was dissolved in THF (8 mL), tetrabutylammonium fluoride trihydrate (1.2 g, 3.96 mmol, 2.0 eq) was added, and the mixture was stirred and reacted at 25 °C for 1 h. After LCMS determined that the reaction was completed, water (50 mL) was added to the reaction solution, and then the reaction solution was extracted with ethyl acetate (30 mL × 3). The combined organic phases were washed with saturated brine, dried over anhydrous sodium sulfate, and concentrated to obtain the residue which was purified using silica gel column chromatography (0-30 % PE/EA) to obtain compound 75 (300 mg, 66 %) as a yellow solid.
LCMS: [M+H] = 228.1

Step 6: Compound 76

Compound 75 (107 mg, 0.47 mmol, 1.0 eq) and compound 65 (170 mg, 0.47 mmol, 1.0 eq) were dissolved in dioxane (10 mL), X-Phos (33 mg, 0.07 mmol, 0.15 eq) was added along with potassium phosphate monohydrate (324 mg, 1.41 mmol, 3.0 eq). Finally, $Pd_2(dba)_3$ (36 mg, 0.04 mmol, 0.1 eq) was added, and the reaction mixture was stirred under nitrogen protection at 100 °C for 3 h. After LCMS detection showed that the reaction was completed, water (30 mL) was added to the reaction solution, and then the reaction solution was extracted with ethyl acetate (40 mL×3), and the combined organic phases were washed with saturated brine, and dried over anhydrous sodium sulphate and concentrated to obtain the residue, the resulting residue was purified using silica gel column chromatography (0-70 % PE/EA) to obtain compound 76 (119 mg, 45 %) as a yellow solid.
LCMS: $[M+H]^+$ = 563.2

Step 7: Compound A19

Compound 76 (110 mg, 0.19 mmol, 1.0 eq) was dissolved in dichloromethane (6 mL), trifluoroacetic acid (4 mL) was added, and the reaction mixture was stirred at room temperature for 1 h. After LCMS detection showed that the reaction was completed, and the reaction mixture was concentrated under reduced pressure to obtain the residue which was dissolved in DMF (4 mL) and filtered. The filtrate was purified by prep-HPLC (mobile phase: 0.1% HCOOH/acetonitrile/water) to obtain compound A19 (3.5 mg, 4%) as a yellow solid.

LCMS: $[M+H]^+$ = 463.1
$^1H$ NMR (400 MHz, DMSO-$d_6$) δ 12.91 (s, 1H), 9.82 (s, 1H), 8.28 (d, *J* = 6.0 Hz, 1H), 8.14 (s, 1H), 7.89 (s, 1H), 7.69-7.47 (m, 6H), 6.78 (d, *J* = 6.4 Hz, 1H), 4.98 (s, 2H), 4.55 (s, 2H).

**Example 1-18 Preparation of Compound A20**

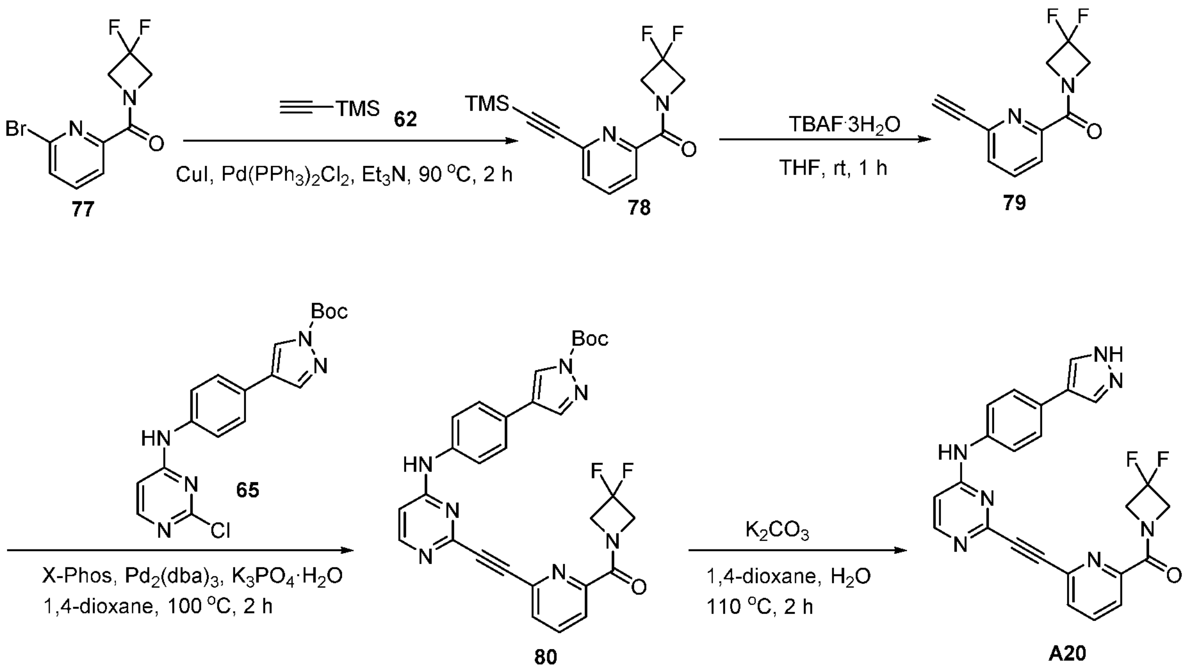

77 78 79 80 A20

Step 1: Compound 78

Compound 77 (3.7 g, 13.4 mmol, 1.0 eq) was dissolved in triethylamine (30 mL), and cuprous iodide (508 mg, 2.68 mmol, 0.2 eq) and $Pd(PPh_3)_2Cl_2$ (940 mg, 1.34 mmol, 0.1 eq) were added along with compound 62 (6.5 g, 67 mmol, 5.0 eq). The reaction mixture was stirred under nitrogen atmosphere at 90 °C for 2 h. After LCMS detection showed that the reaction was completed, water (120 mL) was added to the reaction solution, the reaction solution was then extracted with ethyl acetate (30 mL×3), the organic phases were combined and washed with saturated brine, dried over anhydrous

sodium sulfate and concentrated to obtain the crude compound 78 (1.97 g, 49 %).
LCMS: [M+H] + = 295.1

Step 2: Compound 79

Compound 78 (1.97 g, 6.69 mmol, 1.0 eq) was dissolved in THF (10 mL), tetrabutylammonium fluoride trihydrate (4.22 g, 13.38 mmol, 2.0 eq) was added, and the reaction solution was stirred at room temperature for 1 h. After LCMS detection showed that the reaction was completed, water (60 mL) was added to the reaction solution, and then the reaction solution was extracted with ethyl acetate (20 mL × 3). The organic phases were combined and washed with saturated brine, dried over anhydrous sodium sulfate and concentrated to obtain the residue which was purified by silica gel column chromatography (0-12 % EA/PE) to obtain compound 79 (900 mg, 60 %) as a yellow solid.
LCMS: [M+H] + = 223.1

Step 3: Compound 80

Compound 79 (165 mg, 0.8 mmol, 1.0 eq) and compound 65 (300 mg, 0.8 mmol, 1.0 eq) were dissolved in dioxane (8 mL), X-Phos (57 mg, 0.12 mmol, 0.15 eq) and potassium phosphate monohydrate (552 mg, 2.4 mmol, 3.0 eq) were added, and $Pd_2(dba)_3$ (73 mg, 0.08 mmol, 0.1 eq) was finally added. The reaction mixture was stirred under nitrogen protection at 100 °C for 2 h. After LCMS detection showed that the reaction was completed, water (40 mL) was added to the reaction solution, and then the reaction solution was extracted with ethyl acetate (30 mL×3), and the organic phases were combined and washed with saturated brine, dried over anhydrous sodium sulphate and concentrated to obtain the residue, the resulting residue was purified using silica gel column chromatography (50 %-70 % EA/PE) to obtain compound 80 (95 mg, 21%) as a yellow solid.
LCMS: [M+H] + = 558.2

Step 4: Compound A20

Compound 80 (95.0 mg, 0.17 mmol, 1.0 eq) was dissolved in dioxane (5 mL), potassium carbonate (187.0 mg, 1.36 mmol, 8.0 eq) and water (4 mL) were added, and the reaction mixture was stirred at 110 °C for 2 h. After LCMS detection showed that the reaction was completed, and the reaction mixture was concentrated under reduced pressure to obtain the residue, the residue was dissolved in DMF (4 mL) and filtered. The filtrate was purified by prep-HPLC (mobile phase: 0.1% HCOOH/acetonitrile/water) to obtain compound A20 (23.5 mg, 30%) as a yellow solid.

LCMS: [M+H] + = 458.1
$^1$H NMR (400 MHz, DMSO-$d_6$) δ 12.88 (br s, 1H), 9.83 (s, 1H), 8.30 (d, *J* = 6.0 Hz, 1H), 8.14-8.04 (m, 3H), 7.93-7.91 (m, 2H), 7.64-7.58 (m, 4H), 6.82 (d, *J* = 6.0 Hz, 1H), 5.05 (t, *J* = 12.4 Hz, 2H), 4.54 (t, *J* = 12.4 Hz, 2H).

**Example 1-19 Preparation of Compound A21**

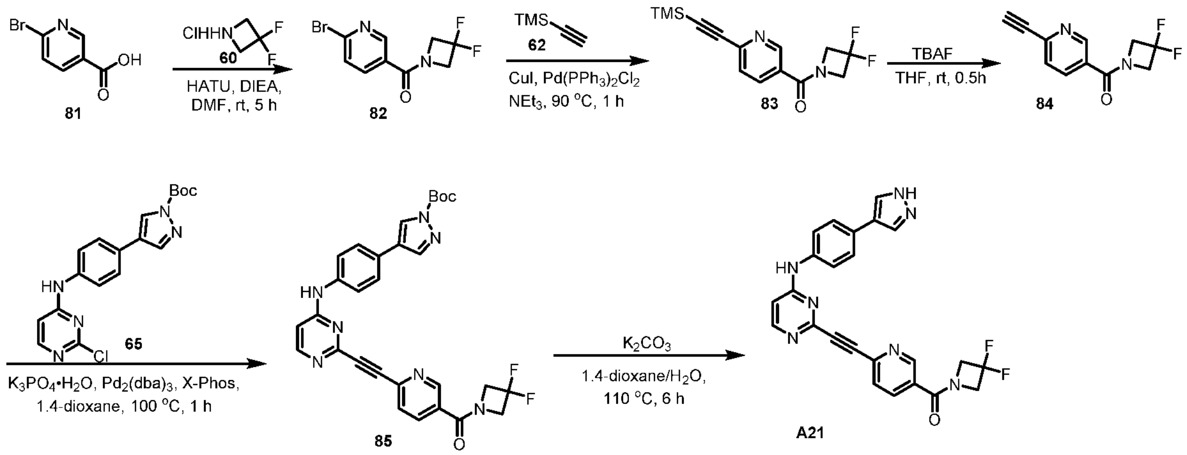

Step 1: Compound 82

Compound 81 (2.0 g, 9.9 mmol, 1.0 eq) was dissolved in DMF (20 mL), HATU (5.65 g, 14.9 mmol, 1.5 eq) was added, and stirred for 10 min, then Compound 60 (1.28 g, 9.9 mmol, 1.0 eq) was added along with diisopropylethylamine (5.12 g, 39.60 mmol, 4.0 eq). The reaction mixture was continued to be stirred at room temperature for 5 h. After LCMS detection showed that the reaction was completed, the reaction was quenched by the addition of water (60 mL) to the reaction solution, and then extracted with ethyl acetate (50 mL × 3). The organic phases were combined and washed with saturated saline, dried over anhydrous sodium sulfate and concentrated to obtain the residue. The residue was purified by silica gel column chromatography (PE/EA=4/1) to obtain compound 82 (1.7 g, 62%) as a white solid.
LCMS: $[M+H]^+$ = 277.0

Step 2: Compound 83

Compound 82 (1.0 g, 3.6 mmol, 1.0 eq) was dissolved in triethylamine (10 mL), and cuprous iodide (137 mg, 0.72 mmol, 0.2 eq) and $Pd(PPh3)_2Cl_2$ (253 mg, 0.36 mmol, 0.1 eq) were added along with compound 62 (1.0 g, 10.8 mmol, 3.0 eq). The reaction mixture was stirred under nitrogen atmosphere at 90 °C for 1 h. After LCMS detection showed that the reaction was completed, the reaction solution was concentrated, and the residue was purified by silica gel column chromatography ( EA/PE=1/3) to obtain compound 83 (0.9 g, 85 %).
LCMS: $[M+H]^+$ = 295.1

Step 3: Compound 84

Compound 83 (0.9 g, 3.06 mmol, 1.0 eq) was dissolved in tetrahydrofuran (20 mL) and tetrabutylammonium fluoride trihydrate (1.1 g, 3.67 mmol, 1.2 eq) was added. The mixture was stirred at 25 °C for 0.5 h. After LCMS determined that the reaction was completed, the reaction solution was concentrated, and the residue was purified by silica gel column chromatography ( EA/PE=1/2) to obtain compound 84 (300 mg, 44 %) as a yellow solid.
LCMS: $[M+H]^+$ = 223.1

Step 4: Compound 85

Compound 84 (200 mg, 0.45 mmol, 1.0 eq) and Compound 65 (150 mg, 0.41 mmol, 0.9 eq) were dissolved in dioxane (5 mL), X-Phos (43 mg, 0.09 mmol, 0.2 eq) and potassium phosphate monohydrate (311 mg, 1.35 mmol, 3.0 eq) were added, and $Pd_2(dba)_3$ (41 mg, 0.05 mmol, 0.1 eq) was finally added. The reaction mixture was stirred under nitrogen protection at 100 °C for 1 h. After LCMS determined that the reaction was completed, water (40 mL) was added to the reaction solution, the reaction solution was then extracted with ethyl acetate (30 mL×3). The combined organic phases were washed with saturated brine, dried over anhydrous sodium sulfate and concentrated to obtain a residue. The residue was purified by column (silica gel, DCM/MeOH=20/1) to obtain compound 85 (200 mg, 80%).
LCMS: $[M+H]^+$ = 558.2

Step 5: Compound A21

Compound 85 (190.0 mg, 0.34 mmol, 1.0 eq) was dissolved in dioxane (2 mL), potassium carbonate (141.0 mg, 1.02 mmol, 3.0 eq) and water (0.4 mL) were added. The reaction mixture was stirred at 110 °C for 6 h. After LCMS showed that the reaction was completed, the reaction mixture was concentrated under reduced pressure to obtain the residue. The residue was dissolved in DMF (4 mL) and filtered, and the filtrate was purified by prep-HPLC (mobile phase: 0.1% HCOOH/acetonitrile/water) to obtain compound A21 (5.5 mg, 4%) as a yellow solid.

LCMS: $[M+H]^+$ = 458.1
$^1$H NMR (400 MHz, DMSO-$d_6$) δ 12.89 (br s, 1H), 9.86 (s, 1H), 8.93 (d, *J* = 2.0 Hz, 1H), 8.31 (d, *J* = 6.0 Hz, 1H), 8.21-8.16 (m, 1H), 8.13-7.88 (m, 2H), 7.85 (d, *J* = 8.0 Hz, 1H), 7.66-7.58 (m, 4H), 6.82 (d, *J* = 6.0 Hz, 1H), 4.89 (br s, 2H), 4.54 (br s, 2H).

**Example 1-20 Preparation of Compound A22**

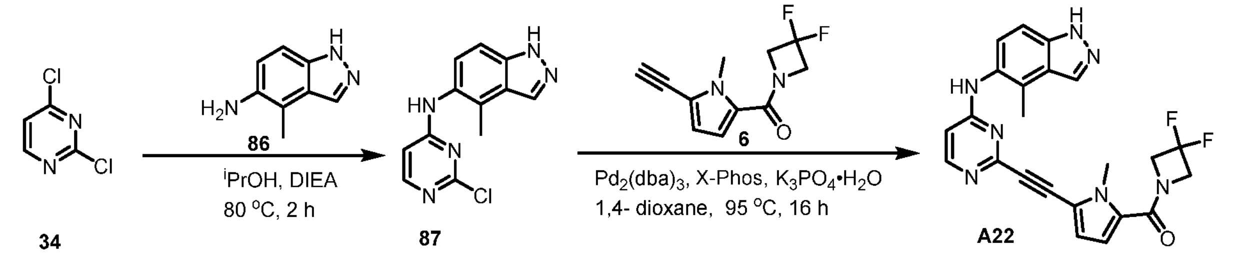


34 87 A22

Step 1: Compound 87

Compound 34 (500 mg, 3.356 mmol, 1.0 eq) and compound 86 (468 mg, 3.188 mmol, 1.0 eq) were dissolved in isopropanol (10 mL), and diisopropylethylamine (2.2 mg, 16.780 mmol, 5.0 eq) was added, and the reaction mixture was stirred at 80 °C for 2 h. After LCMS detection showed that the reaction was completed, the reaction solution was cooled to room temperature, and the reaction solution was concentrated under reduced pressure to obtain the residue, the residue was purified by silica gel column chromatography (petroleum ether/ethyl acetate = 1/2) to obtain compound 87 (520 mg, 60%) as a yellow solid.
LCMS: $[M+H]^+$ = 260.1

Step 2: Compound A22

Compound 87 (150 mg, 0.578 mmol, 1.0 eq) and compound 6 (116 mg, 0.520 mmol, 0.9 eq) were dissolved in dioxane (10 mL), potassium phosphate monohydrate (399 mg, 1.734 mmol, 3.0 eq) was added, and $Pd_2(dba)_3$ (53 mg, 0.058 mmol, 0.10 eq) and X-Phos (41 mg, 0.087 mmol, 0.15 eq) were finally added, and the reaction mixture was stirred at 95 °C for 16 h under nitrogen protection. The reaction solution was cooled to room temperature, diluted with dichloromethane/methanol (50 mL, 10:1), the reaction solution was passed through diatomaceous earth, and the filtrate was concentrated under reduced pressure to obtain the residue, the residue was purified by silica gel column chromatography (dichloromethane/methanol = 93/7) to obtain compound A22 (59.0 mg, 23 %) as a yellow solid.

LCMS: $[M+H]^+$ = 448.2
$^1H$ NMR (400 MHz, DMSO-$d_6$) δ 13.10 (s, 1H), 9.33 (s, 1H), 8.23-8.08 (m, 2H), 7.40 (d, *J* = 8.8 Hz, 1H), 7.21 (d, *J* = 8.4 Hz, 1H), 6.69 (d, *J* = 4.4 Hz, 1H), 6.65 (d, *J* = 4.4 Hz, 1H), 4.61 (br s, 4H), 3.89 (s, 3H), 2.40 (s, 3H).

**Example 1-21 Preparation of Compound A27**

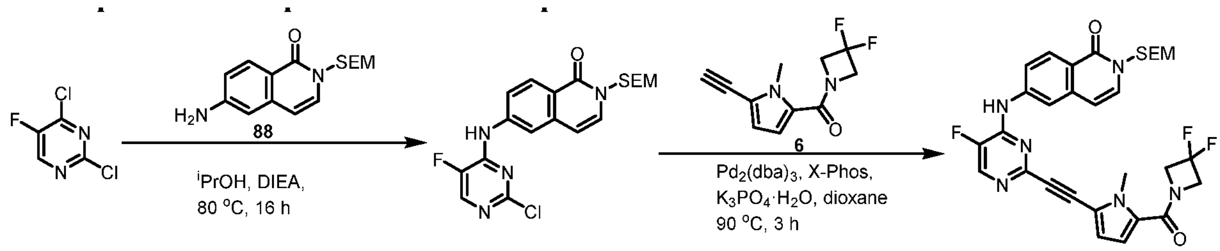


16 89 90

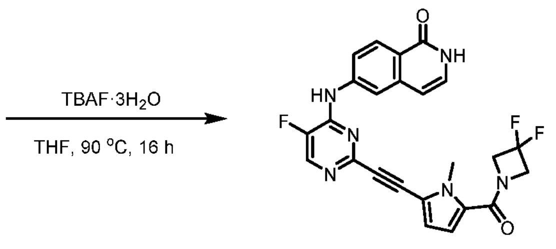


A27

Step 1: Compound 89

Compound 16 (193.15 mg, 1.15 mmol, 1.2 eq), Compound 88 (280 mg, 0.97 mmol, 1.0 eq) and diisopropylethylamine (373.5 mg, 2.89 mmol, 3.0 eq) were added to isopropanol (5 mL). The reaction solution was allowed to react at 80 °C for 16 hours. The reaction solution was cooled down, and the solvent was removed under reduced pressure. The residue was purified by silica gel column chromatography (PE/EA=2/1) to obtain compound 89 (280 mg, 69%) as a pale yellow solid.
LCMS: $[M+H]^+$= 421.1

Step 2: Compound 90

Compound 89 (230 mg, 0.546 mmol, 1.0 eq) was dissolved in dioxane (30 mL), followed by the addition of Compound 6 (124.7 mg, 0.546 mmol, 1.0 eq), potassium phosphate monohydrate (440.36 mg, 1.912 mmol, 3.5 eq), X-Phos (109.3 mg, 0.109 mmol, 0.2 eq) and $Pd_2(dba)_3$ (50.04 mg, 0.0546 mmol, 0.1 eq). After the addition, the reaction solution was reacted at 90 °C for 3 h under nitrogen atmosphere. The reaction solution was cooled to room temperature and the solvent was removed by distillation under reduced pressure. The residue was purified by silica gel column chromatography (PE/EA=1/1) to obtain compound 90 (200 mg, 60%) as a pale yellow solid.
LCMS: [M+H] + =609.2

Step 3: Compound A27

Compound 90 (190 mg, 0.312 mmol, 1.0 eq) was dissolved in tetrahydrofuran (15 mL) and then tetrabutylammonium fluoride trihydrate (820 mg, 3.125 mmol, 10.0 eq) was added. The reaction solution was reacted at 90 °C for 18 hours. After completion of the reaction, the reaction solution was concentrated to remove the tetrahydrofuran. The residue was diluted with ethyl acetate and washed three times with water, dried over anhydrous sodium sulfate and concentrated to obtain the crude. The crude was purified by prep-HPLC (mobile phase: 0.1% FA/acetonitrile/water) to obtain compound A27 (46 mg, 30.8%) as a yellow solid.

LCMS: $[M+H]^+$ =479.1
$^1$H NMR (400 MHz, DMSO-$d_6$) δ 11.13 (s, 1H), 10.07 (s, 1H), 8.49 (d, $J$ = 8.4 Hz, 1H), 8.22-8.11 (m, 2H), 7.83 (d, $J$ = 8.8 Hz, 1H), 7.16 (t, $J$ = 4.0 H, 1H), 6.74-6.71 (m, 2H), 6.44 (d, $J$ = 8.0 Hz 1H), 4.763 (br s, 4H), 3.96 (s, 3H).

**Example 1-22 Preparation of Compound A28**

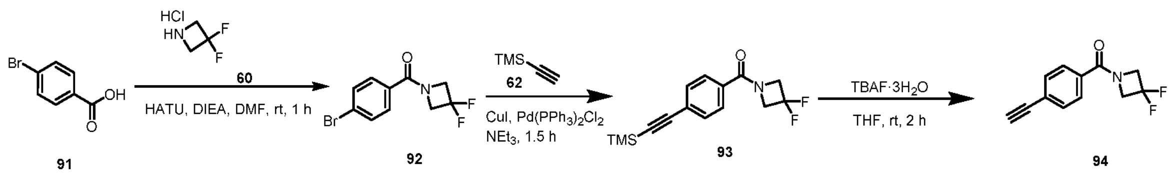

91 92 93 94

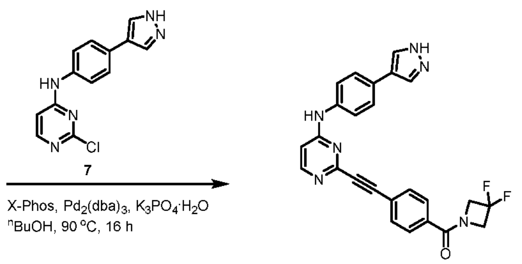

A28

Step 1: Compound 92

Compound 91 (1.0 g, 4.975 mmol, 1.0 eq) was dissolved in DMF (10 mL), HATU (2.27 g, 5.97 mmol, 1.2 eq) was added, and stirred for 10 min, then compound 60 (963 mg, 7.462 mmol, 1.5 eq) was added with diisopropylethylamine (1.9

g, 14.925 mmol, 3.0 eq), and the reaction mixture was continued to be stirred at 25 °C for 1 h. After LCMS determined that the reaction was completed, water (30 mL) was added to the reaction solution, and the suspension was filtered and dried to obtain compound 92 (1.1 g, 80 %) as a white solid.
LCMS: [M+H] = 296.1

Step 2: Compound 93

Compound 92 (1.0 g, 3.610 mmol, 1.0 eq) was dissolved in triethylamine (20 mL), cuprous iodide (137 mg, 0.722 mmol, 0.2 eq), $Pd(PPh3)_2Cl_2$ (253 mg, 0.361 mmol, 0.1 eq) and compound 4 (1.0 g, 10.830 mmol, 3.0 eq) were added, the reaction mixture was stirred at 90 °C for 1.5 h under nitrogen atmosphere. After LCMS detection showed that the reaction was completed, water (50 mL) was added to the reaction solution, the reaction solution was then extracted with ethyl acetate (60 mL×3). The combined organic phases were washed with saturated brine, dried over anhydrous sodium sulfate and concentrated. The residue obtained was purified by silica gel column chromatography (silica gel, 0-15 % EA/PE) to obtain Compound 93 (900 mg, 84 %) as a yellow solid.
LCMS: $[M+H]^+$ = 294.1

Step 3: Compound 94

Compound 93 (850 mg, 2.897 mmol, 1.0 eq) was dissolved in tetrahydrofuran (20 mL), tetrabutylammonium fluoride trihydrate (970 mg, 3.476 mmol, 1.2 eq) was added, and the mixture was stirred at room temperature for 2 h. After LCMS detection showed that the reaction was completed, and then water (30 mL) was added to the reaction solution, and then extracted with ethyl acetate (50 mL × 3). The combined organic phases were washed with saturated saline, dried over anhydrous sodium sulfate and concentrated to obtain the residue, the residue was purified using silica gel column chromatography (silica gel, 0-30 % EA/PE) to obtain compound 94 (380 mg, 60 %) as a yellow solid.
LCMS: $[M+H]^+$ = 222.1

Step 4: Compound A28

Compound 94 (300 mg, 1.357 mmol, 1.0 eq) and compound 7 (441 mg, 1.629 mmol, 1.2 eq) were dissolved in n-butanol (20 mL), X-Phos (97 mg, 0.203 mmol, 0.15 eq) was added with potassium phosphate monohydrate (863 mg, 4.071 mmol, 3.0 eq), and $Pd_2(dba)_3$ (124 mg, 0.136 mmol, 0.1 eq) was finally added, and the reaction mixture was stirred at 90 °C for 16 h under nitrogen protection. After LCMS detection showed that the reaction was completed, the reaction solution was concentrated directly, and the residue obtained was purified using silica gel column chromatography (silica gel, 0-7% MeOH/DCM) to obtain the crude product, the crude product was purified by prep-HPLC (mobile phase: 0.1% formic acid/acetonitrile/water) to obtain compound A28 (8.5 mg, 1 %) as a yellow solid.

LCMS: $[M+H]^+$ = 457.2
$^1$H NMR (400 MHz, DMSO-$d_6$) δ 9.84 (s, 1H), 8.29 (d, *J* = 5.6 Hz, 1H), 8.02 (s, 2H), 7.78 (d, *J* = 8.4 Hz, 2H), 7.73 (d, *J* = 8.4 Hz, 2H), 7.61 (s, 4H), 6.79 (d, *J* = 6.0 Hz, 1H), 4.82 (br s, 2H), 4.50 (br s, 2H).

**Example 1-23 Preparation of Compound A29**

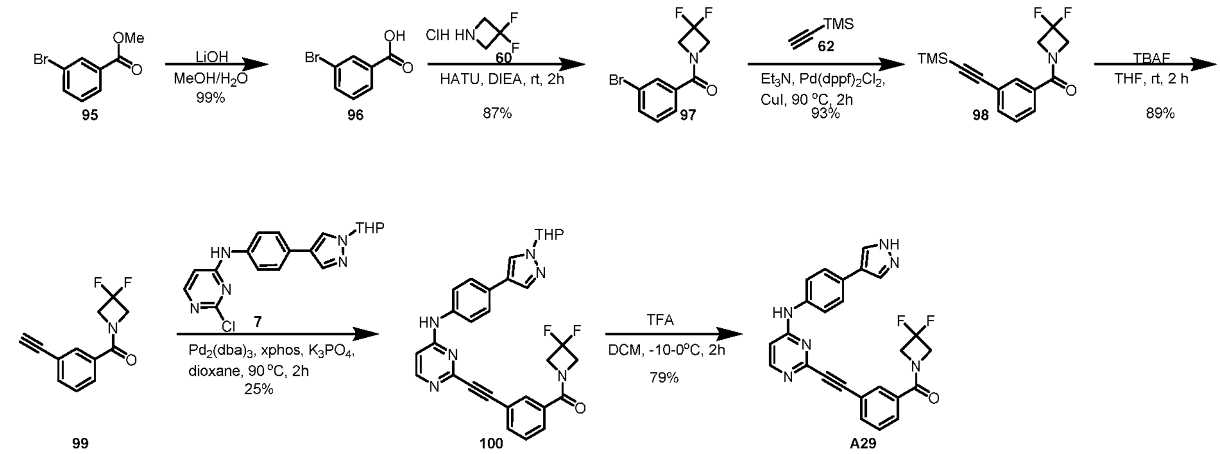


Step 1: Compound 96

Compound 95 (200 mg, 0.93 mmol, 1.0 eq) was dissolved in methanol/water (2 mL/1mL), and then lithium hydroxide (158 mg, 3.74 mmol, 4.0 eq) was added, and the reaction solution was reacted for 1 hour at room temperature. The reaction solution was spin-dried to obtain compound 96 (190 mg, 99%) as a white solid.
LCMS: $[M+H]^+$ = 201.1

Step 2: Compound 97

Compound 96 (200 mg, 1.0 mmol, 1.0 eq) was dissolved in DMF (3 mL), then compound 60 (154 mg, 1.2 mmol, 1.2 eq) and HATU (575 mg, 1.5 mmol, 1.5 eq) were added, and the reaction solution was stirred at room temperature for 0.5 h. Diisopropylethylamine (387 mg, 3.0 mmol, 3.0 eq) was added, the reaction solution was stirred at room temperature for 1.5 h. The reaction product was slowly added to water, and extracted with ethyl acetate twice, the combined organic phases were washed with saturated brine, dried over anhydrous sodium sulfate and concentrated, the residue was purified by silica gel column chromatography (petroleum ether/ethyl acetate =15/1) to obtain compound 97 (240 mg, 87%) as a white solid.
LCMS: [M+H] = 276.0

Step 3: Compound 98

Compound 97 (150 mg, 0.55 mmol, 1.0 eq) was dissolved in triethylamine (3 mL), and then compound 5 (640 mg, 6.5 mmol, 12 eq), $Pd(dppf)Cl_2$ (40 mg, 0.055 mmol, 0.1 eq), and cuprous iodide (21 mg, 0.11 mmol, 0.2 eq) were sequentially added. After the addition was completed, the reaction solution was warmed to 90 °C and stirred for 2 hours. The reaction solution was extracted with ethyl acetate/water, and the organic phase was purified by silica gel column chromatography (PE/EA=15/1) to obtain compound 98 (150 mg, 62%) as a white solid.
LCMS: $[M+H]^+$ =294.1

Step 4: Compound 99

Compound 98 (150 mg, 0.51 mmol, 1.0 eq) was dissolved in tetrahydrofuran (5 mL), then tetrabutylammonium fluoride trihydrate (200 mg, 0.77 mmol, 1.5 eq) was added, and the reaction solution was reacted at room temperature for 2 hours. The reaction solution was spin-dried and the residue was purified by silica gel column chromatography (PE/EA=15/1) to obtain compound 99 (100 mg, 89%) as a pale yellow solid.
LCMS: $[M+H]^+$ = 222.1

Step 5: Compound 100

Compound 99 (320 mg, 0.90 mmol, 1.0 eq) was dissolved in dioxane (5 mL), then compound 8 (200 mg, 0.90 mmol, 1.0 eq), potassium phosphate (621 mg, 2.7 mmol, 3.0 eq), X-phos (86 mg, 0.18 mmol, 0.2 eq) and $Pd_2(dba)_3$ (23 mg, 0.031 mmol, 0.1 eq) were added, and the reaction solution was stirred at 90 °C for 2 h after the addition. After cooling to room temperature, the reaction solution was spin-dried and the residue was purified by silica gel column chromatography (DCM/MeOH=10/1) to obtain compound 100 (120 mg, 25%) as a light yellow solid.

LCMS: $[M+H]^+$ =541.2

Step 6: Compound A29

Compound 100 (100 mg, 0.18 mmol, 1.0 eq) was dissolved in dichloromethane (3 mL) followed by the addition of trifluoroacetic acid (3 mL) at 0 °C, and the reaction was carried out for 2 h at room temperature. After completion of the reaction, the reaction solution was slowly added to saturated aqueous sodium bicarbonate solution, the mixture was extracted twice with ethyl acetate (100 mL), the organic phases were combined and washed with water, the organic phases were concentrated, the residue was added with ethyl acetate (4 mL) , methanol (1 mL) and dichloromethane (1 mL), and the mixture was stirred at room temperature for 0.5 h. The mixture was filtered to obtain compound A29 (80.3 mg, 79%) as a pale yellow solid.

LCMS: $[M+H]^+$ = 457.2

$^1$H NMR (400 MHz, DMSO-$d_6$) δ 9.79 (s, 1H), 8.36 - 8.22 (m, 2H), 7.94 - 7.87 (m, 2H), 7.85 - 7.75 (m, 2H), 7.66 - 7.56 (m, 5H), 6.79 (d, $J$ = 6.0 Hz, 1H), 5.40 (dd, $J$ = 10.0, 2.4 Hz, 1H), 4.67 (d, $J$ = 127.6 Hz, 4H), 3.94 (d, $J$= 12.0 Hz, 1H), 3.71 - 3.55 (m, 1H), 2.17 - 2.06 (m, 1H), 1.96 (t, $J$ = 9.6 Hz, 2H), 1.68 (d, $J$ = 12.8 Hz, 1H), 1.59 - 1.49 (m, 2H).

**Example 1-24 Preparation of Compound A30**

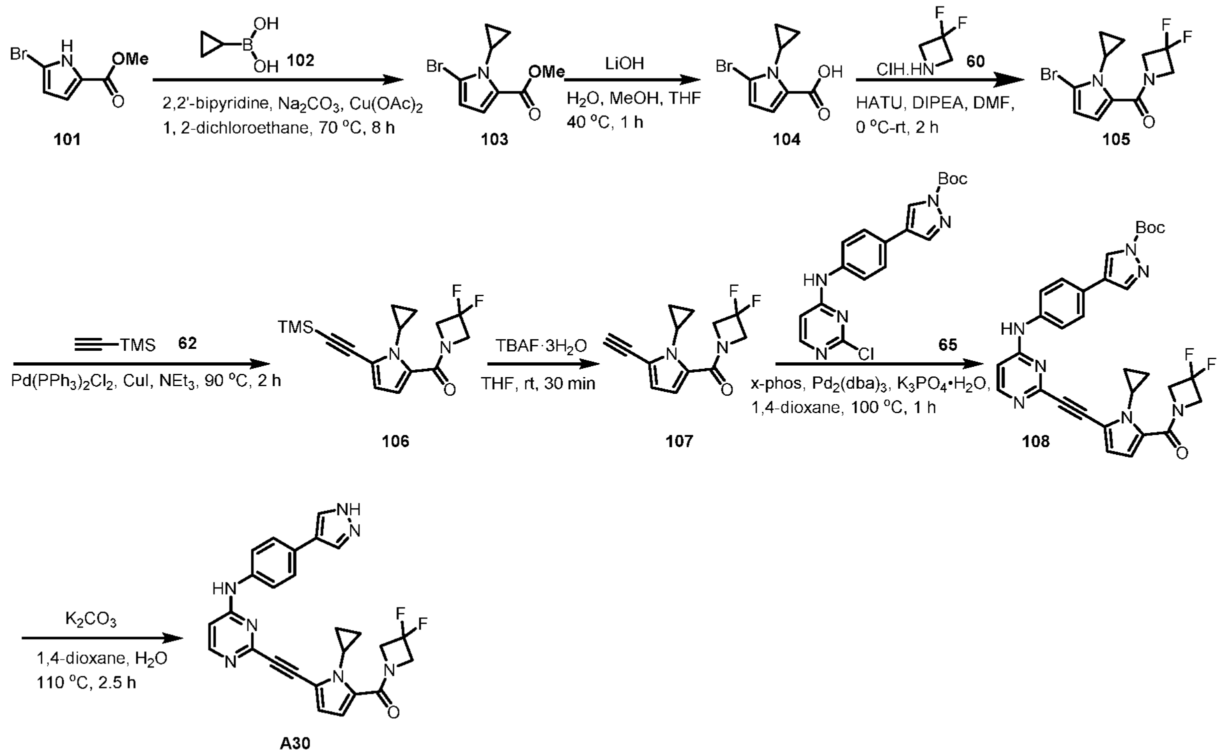


Step 1: Compound 103

Compound 100 (5.0 g, 24.51 mmol, 1.0 eq), Compound 102 (4.21 g, 49.01 mmol, 2.0 eq), and sodium carbonate (4.07 g, 49.01 mmol, 2.0 eq) were dissolved in 1,2-dichloroethane (130 mL), followed by addition of 2,2'-bipyridine (3.83 g, 24.51 mmol, 1.0 eq) and anhydrous copper acetate (4.45 g, 24.51 mmol, 1.0 eq). The reaction solution was displaced with oxygen three times and then stirred at 70 °C for 8 h under oxygen atmosphere. The reaction solution was cooled to room temperature. The reaction solution was diluted with 1,2-dichloroethane, sequentially washed with ammonia, washed with water, washed with saturated saline, dried over anhydrous sodium sulfate and concentrated. The residue was purified by fast silica gel column chromatography (petroleum ether/ethyl acetate=16/1) to obtain compound 103 (5.54 g, 92%) as a yellow oil.

LCMS: $[M+H]^+$ = 244.0

Step 2: Compound 104

Compound 103 (5.54 g, 27.70 mmol, 1.0 eq) was dissolved in a solvent mixture of tetrahydrofuran/methanol/-water (1/1/2, 60 mL) and then lithium hydroxide (2.72 g, 113.48 mmol, 5.0 eq) was added. The reaction solution was reacted at 40 °C for 1 hour. After the end of the reaction, the reaction solution was distilled under reduced pressure to remove most of the methanol and tetrahydrofuran. The pH was adjusted to 5.0 by adding saturated aqueous sodium citrate solution dropwise to the residual solution, and extracted with ethyl acetate (200 mL × 3) . The combined organic phases were washed with saturated brine, dried over anhydrous sodium sulfate and concentrated to obtain compound 104 (5.05 g, 96% ) as a white solid.
LCMS: $[M+H]^+$ = 230.0

Step 3: Compound 105

Compound 104 (5.05 g, 24.08 mmol, 1.0 eq), Compound 60 (3.74 g, 28.90 mmol, 1.2 eq) and HATU (10.99 g, 28.90 mmol, 1.2 eq) were dissolved in DMF (80 mL) and the reaction solution was cooled down to 0 °C, diisopropylethylamine (12.45 g, 96.32 mmol, 4.0 eq) was added slowly. The reaction solution was reacted at room temperature for 2 hours. After the reaction was completed, water (200 mL) was added to the reaction solution, and extracted with ethyl acetate (150 mL × 3) . The combined organic phases were washed with saturated saline, dried over anhydrous sodium sulfate and concentrated. The residue was purified by silica gel column chromatography (petroleum ether/ethyl acetate = 15/1) to obtain compound 105 (6.34 g, 94%) as a white solid.
LCMS: $[M+H]^+$ = 305.0

Step 4: Compound 106

Compound 105 (1.0 g, 3.28 mmol, 1.0 eq) was dissolved in triethylamine (14.5 mL), then compound 62 (966 mg, 9.83 mmol, 3.0 eq), bis(triphenylphosphine)palladium dichloride (230 mg, 0.328 mmol, 0.1 eq), and cuprous iodide (187 mg, 0.983 mmol, 0.3 eq) were added, and the nitrogen was displaced three times. The reaction solution was stirred at 90 °C for 2 h under nitrogen atmosphere. The reaction solutions were combined, diluted with ethyl acetate and filtered. The filtrate was sequentially washed with water and saturated saline, dried over anhydrous sodium sulfate and concentrated. The residue was purified by fast silica gel column chromatography (petroleum ether/ethyl acetate = 13/1) to obtain compound 106 (625 mg, 59%) as a tan solid.
LCMS: $[M+H]^+$ = 323.2

Step 5: Compound 107

Compound 106 (330 mg, 1.02 mmol, 1.0 eq) was dissolved in tetrahydrofuran (8 mL) and then tetrabutylammonium fluoride trihydrate (484 mg, 1.54 mmol, 1.5 eq) was added. The reaction solution was stirred at 25 °C for 30 min. The reaction solution was concentrated. The residue was purified by fast silica gel column chromatography (petroleum ether/ethyl acetate = 19/1) to obtain compound 107 (240 mg, 93%) as a yellow solid.
LCMS: [M+H] = 251.1

Step 6: Compound 108

Compound 107 (200 mg, 0.799 mmol, 1.0 eq), Compound 65 (357 mg, 0.959 mmol, 1.2 eq), and Potassium phosphate monohydrate (552 mg, 2.400 mmol, 3.0 eq) were dissolved in dioxane (10 mL), followed by rapid addition of X-phos (76 mg, 0.160 mmol, 0.2 eq) and $Pd_2(dba)_3$ (73 mg, 0.80 mmol, 0.1 eq). After displacing nitrogen three times, the reaction solution was stirred at 100 °C for 1 h under nitrogen atmosphere. The reaction solution was cooled to room temperature, water (20 mL) was added to the reaction solution, and then extracted with ethyl acetate (20 mL × 3). The combined organic phases were washed with saturated brine, dried over anhydrous sodium sulfate and concentrated. The residue was purified by fast silica gel column chromatography (dichloromethane/methanol = 16/1) to obtain compound 108 (193 mg, 41%) as a yellow solid.
LCMS: $[M+H]^+$ = 586.2

Step 7: Compound A30

Compound 108 (188 mg, 0.321 mmol, 1.0 eq) and potassium carbonate (133 mg, 0.963 mmol, 3.0 eq) were dissolved in a mixture of solvents (dioxane/water = 5/1, 3.6 mL), and the reaction solution was stirred for 2.5 hours at 110 °C. Cooled to room temperature, the reaction solution was concentrated. The residue was purified by prep-HPLC (mobile

phase: 0.1% formic acid/acetonitrile/water) to obtain A30 (83.0 mg, 53% ) as a yellow solid.

LCMS: [M+H] $^+$ = 486.1

$^1$H NMR (400 MHz, DMSO-$d_6$) δ 12.90 (br s, 1H), 9.77 (s, 1H), 8.27 (d, *J* = 6.0 Hz, 1H), 8.01 (br s, 2H), 7.67 (d, *J* = 8.4 Hz, 2H), 7.58 (d, *J* = 8.8 Hz, 2H), 6.74 (d, *J* = 6.0 Hz, 1H), 6.67-6.59 (m, 2H), 4.62 (br s, 4H), 3.52-3.44 (m, 1H), 1.17-1.07 (m, 2H), 1.05-0.96 (m, 2H).

**Example 1-25 Preparation of Compound A32**

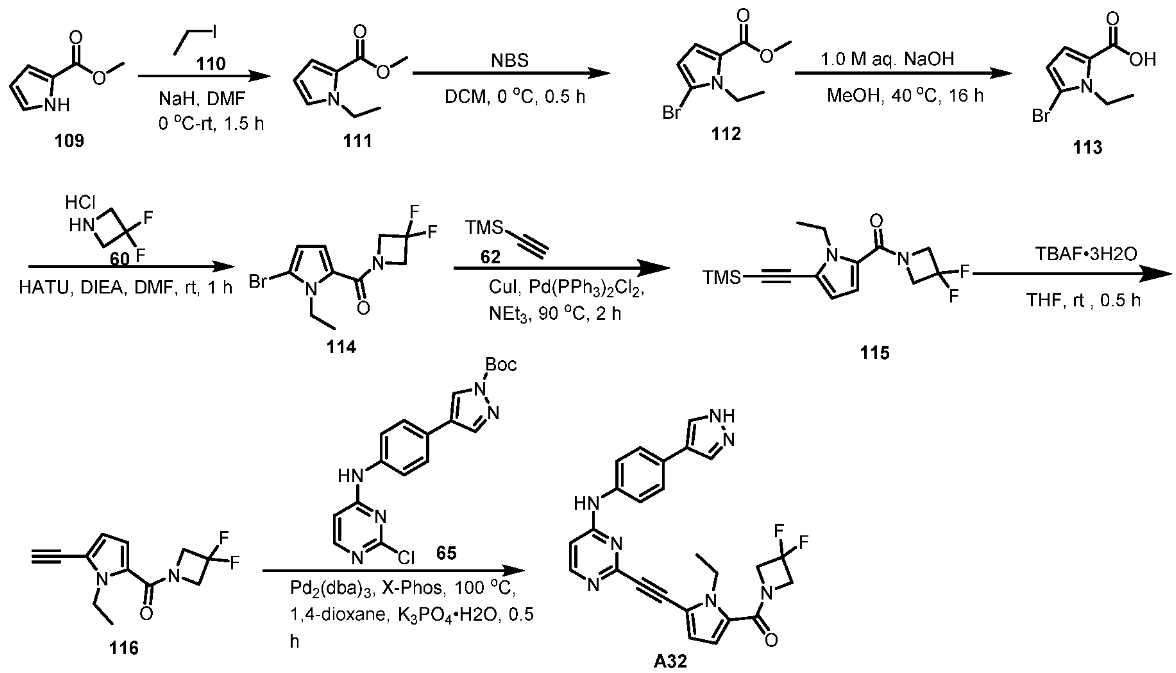


109 111 112 113 114 115 116 A32

Step 1: Compound 111

Compound 109 (1.0 g, 7.99 mmol, 1.0 eq) was dissolved in DMF (20 mL), then NaH (480 mg, 60% in mineral oil, 11.99 mmol, 1.5 eq) was added under ice bath conditions, and the reaction solution was stirred at 25 °C for 20 min before adding compound 110 (2.49 g, 15.98 mmol, 2.0 eq). After the addition, the reaction solution was stirred at room temperature for 1 hour. After the end of the reaction, the reaction solution was poured into ice water, and then extracted with ethyl acetate. The combined organic phases were washed with saturated brine, dried over anhydrous sodium sulfate and concentrated. The residue was purified by silica gel column chromatography (PE/EA=20/1) to obtain compound 111 (1.0 g, 81%) as a brown oily liquid.
LCMS: [M+H] $^+$ = 154.1

Step 2: Compound 112

Compound 111 (9.80 g, 63.98 mmol, 1.0 eq) was dissolved in DCM (10 mL), nitrogen was displaced, and NBS (3.64 g, 63.89 mmol, 1.0 eq) was added after cooling down to 0°C. After the dosing, the reaction solution was stirred under ice bath conditions for 0.5 hours. At the end of the reaction, the reaction solution was poured into water and then extracted with ethyl acetate. The combined organic phases were washed with saturated brine, dried over anhydrous sodium sulfate and concentrated to obtain the crude compound 112 (11.0 g, 74%) as a brown oily liquid.
LCMS: [M+H] $^+$ = 232.0

Step 3: Compound 113

Crude compound 112 (11.0 g, 47.6 mmol, 1.0 eq) was dissolved in methanol (50 mL) and then an aqueous 1.0 M sodium hydroxide solution configured with sodium hydroxide (5.7 g, 142.2 mmol, 3.0 eq) was added. After addition, the

reaction solution was stirred at 40 °C for 16 hours. The reaction solution was cooled to room temperature and concentrated. The resulting aqueous solution was adjusted to pH about 4-5 with 1.0 M hydrochloric acid solution and extracted with ethyl acetate. The combined organic phases were washed with saturated brine, dried over anhydrous sodium sulfate and concentrated. The residue was purified by silica gel column chromatography (PE/EA=30/1) to obtain compound 113 (2.6 g, 25%) as a white solid.
LCMS: $[M+H]^+$ = 218.0

Step 4: Compound 114

Compound 113 (850 mg, 3.86 mmol, 1.0 eq) was dissolved in DMF (5 mL) and then HATU ( 1.93 g, 5.07 mmol, 1.3 eq) was added, and the reaction solution was stirred at room temperature for 10 min. Diisopropylethylamine (1.51 g, 11.69 mmol, 3.0 eq) and Compound 60 (505 mg, 3.90 mmol, 1.0 eq) were sequentially added to the reaction solution. The reaction solution was stirred at room temperature for 1 hour. The reaction solution was diluted with ethyl acetate, washed with water and saturated saline, dried over anhydrous sodium sulfate and concentrated. The residue was purified by silica gel column chromatography (PE/EA=30/1) to obtain compound 114 (1.0 g, 88%) as a yellow oily liquid.
LCMS: $[M+H]^+$ = 293.1

Step 5: Compound 115

Compound 114 (600 mg, 2.04 mmol, 1.0 eq) was dissolved in DMF (5 mL), and then CuI ( 78 mg, 0.41 mmol, 0.2 eq), $Pd(PPh3)_2Cl_2$ ( 144 mg, 0.20 mmol, 0.1 eq) and compound 62 (603 mg, 12.2 mmol, 6.0 eq) were sequentially added at room temperature. Nitrogen was replaced and the reaction solution was stirred at 90 °C for 2 h under nitrogen atmosphere. The reaction solution was cooled to room temperature, filtered and the filtrate was concentrated. The residue was diluted with ethyl acetate, washed with water and saturated saline, dried over anhydrous sodium sulfate and concentrated to obtain the crude product. The crude was purified by silica gel column chromatography (PE/EA=20/1) to obtain compound 115 (400 mg, 63%) as a black oily liquid.
LCMS: $[M+H]^+$ = 311.2

Step 6: Compound 116

Compound 115 (400 mg, 1.68 mmol, 1.0 eq) was dissolved in tetrahydrofuran (10 mL) and then tetrabutylammonium fluoride trihydrate ( 936 mg, 2.52 mmol, 1.5 eq) was added. After the addition was completed, the reaction solution was stirred at 25 °C for 0.5 h. The reaction solution was diluted with ethyl acetate and washed with water and saturated saline. The combined organic phases were dried over anhydrous sodium sulfate and concentrated. The residue was purified by silica gel column chromatography (PE/EA=20/1) to obtain compound 116 (270 mg, 68%) as a red solid.
LCMS: $[M+H]^+$ =239.1

Step 7: Compound A32

Compound 116 ( 280 mg , 0.75 mmol, 1.0 eq) was dissolved in dioxane (10 mL) and then compound 10 (197 mg, 0.83 mmol, 1.1 eq), potassium phosphate monohydrate (607 mg, 2.64 mmol, 3.5 eq), X-Phos (72 mg, 0.15 mmol, 0.2 eq) and $Pd_2(dba)_3$ (69 mg, 0.08 mmol, 0.1 eq) were added in order. After displacing nitrogen three times, the reaction solution was stirred at 110 °C for 5 hours. The reaction solution was cooled to room temperature and concentrated. The residue was diluted with ethyl acetate (30 mL) and then washed with water and saturated brine, dried over anhydrous sodium sulfate and concentrated to obtain the crude product. The crude was purified by prep-HPLC (mobile phase: 0.1% formic acid/acetonitrile/water) to obtain compound A32 (18.5 mg, 5%) as a yellow solid.

LCMS: $[M+H]^+$ = 474.0
$^1$H NMR (400 MHz, DMSO-$d_6$) δ 12.86 (br s, 1H), 9.79 (s, 1H), 8.25 (d, *J* = 6.0 Hz, 1H), 8.15- 7.85 (m, 2H), 7.66-7.54 (m, 4H), 6.74 (d, *J* = 6.0 Hz, 1H), 6.72-6.68 (m, 2H), 4.65 (br s, 4H), 4.49- 4.40 (m, 2H), 1.37 (t, *J* = 6.8 Hz, 3H).

**Example 1-26 Preparation of Compound A37**

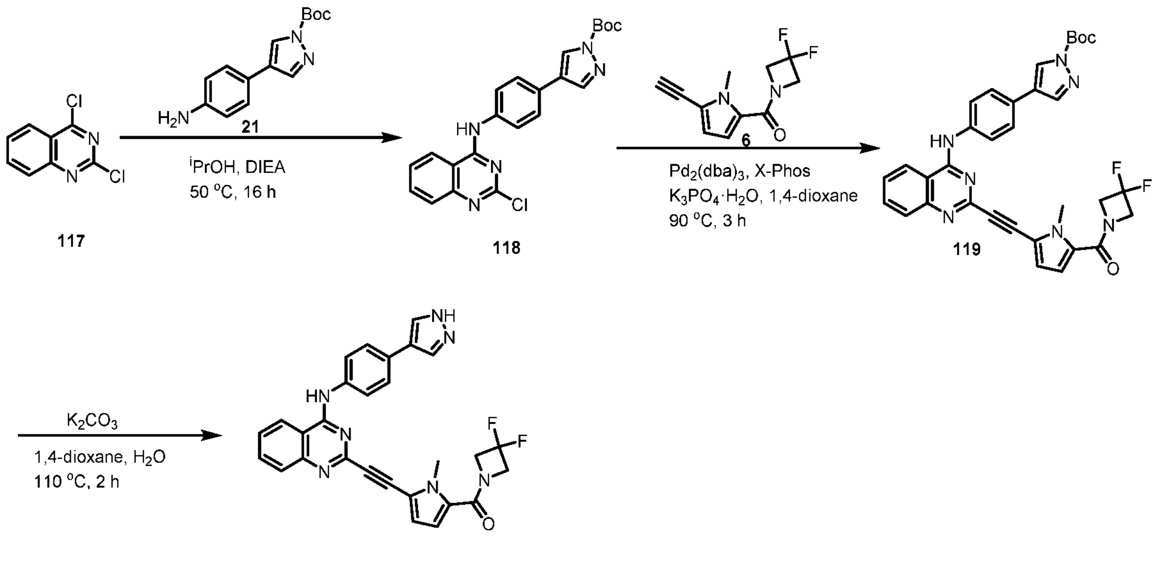

117
118
119
A37

Step 1: Compound 118

Compound 117 (400 mg, 2.01 mmol, 1.2 eq), Compound 21 (434.28 mg, 1.67 mmol, 1.0 eq) and diisopropylethylamine (649.37 mg, 5.01 mmol, 3.0 eq) were added to isopropanol (20 mL). The reaction solution was stirred at 50 °C for 16 hours. The reaction solution was cooled down and depressurized to remove the solution. The reaction solution was purified by silica gel column chromatography (PE/EA=2/1) to obtain compound 118 (538 mg, 76%) as a pale yellow solid.
LCMS: $[M+H]^+$= 422.0

Step 2: Compound 119

Compound 117 (200 mg, 0.47 mmol, 1.0 eq) was dissolved in dioxane (6 mL), then compound 21 (106 mg, 0.47 mmol, 1.0 eq), potassium phosphate monohydrate (382 mg, 1.7 mmol, 3.5 eq), X-Phos (45 mg, 0.094 mmol, 0.2 eq) and $Pd_2(dba)_3$ (43 mg, 0.047 mmol, 0.1 eq) were added, and the reaction solution was reacted at 90 °C for 3 h under nitrogen atmosphere after dosing. The reaction solution was cooled to room temperature and the solvent was removed by distillation under reduced pressure, and the yellowish solid crude product 119 obtained was used directly in the next step of the reaction.
LCMS: $[M+H]^+$ =610.2

Step 3: Compound A37

The crude compound 119 (289 mg, 474 mmol, 1.0 eq) was dissolved in a mixed solvent of dioxane/water (5 mL/1 mL), and then potassium carbonate (654 mg, 4.74 mmol, 10.0 eq) was added at 25 °C, and the reaction solution was reacted at 110 °C for 2 hours. After completion of the reaction, the reaction solution was concentrated to obtain the crude product, and the crude product was purified by prep-HPLC (mobile phase: 0.1% FA/acetonitrile/water) to obtain compound A37 (58 mg, 24%) as a yellow solid.
LCMS: $[M+H]^+$ =510.2
$^1$H NMR (400 MHz, DMSO-$d_6$) δ 12.93 (s, 1H), 9.93 (s, 1H), 8.57 (d, *J* = 8.4 Hz, 1H), 8.17 (br s, 1H), 7.98 (br s, 1H), 7.91-7.86 (m, 3H), 7.80 (d, *J* = 8.4 Hz, 1H), 7.68-7.66 (m, 3H), 6.71 (s, 2H), 4.62 (br s, 4H), 3.97 (s, 3H).

**Example 1-27 Preparation of Compound A38**

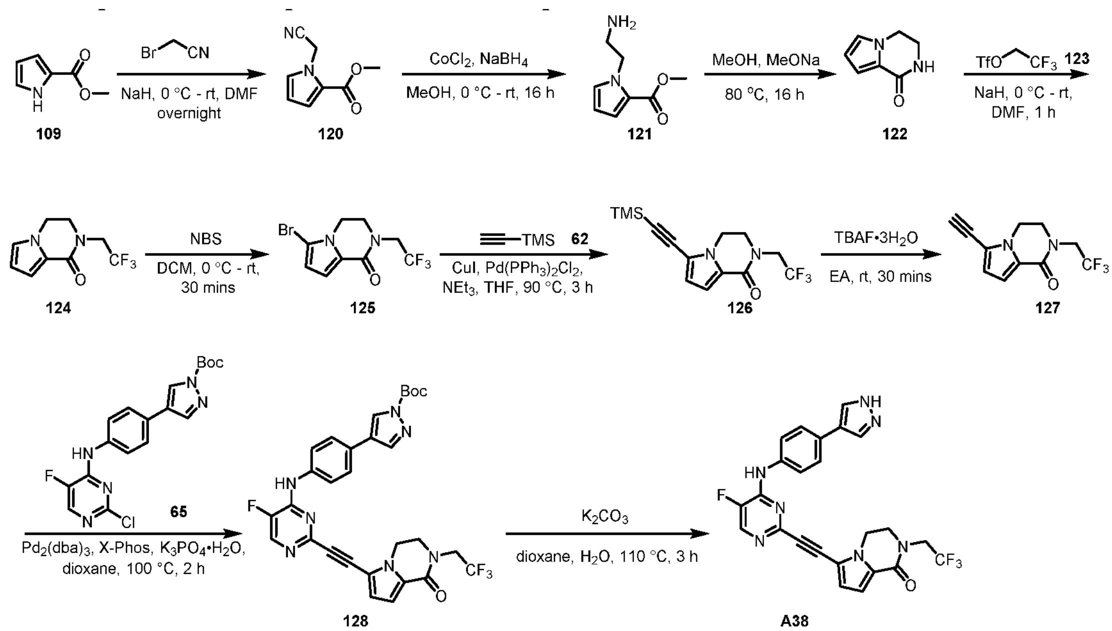


Step 1: Compound 120

Compound 109 (20.0 g, 159.84 mmol, 1.0 eq) was dissolved in anhydrous DMF (100 mL) and then NaH (9.59 g, 60% in mineral oil, 239.76 mmol, 1.5 eq) was added under ice bath conditions. The reaction solution was stirred under ice bath for 30 min, then compound bromoacetonitrile (38.34 g, 319.68 mmol, 2.0 eq) was added. The reaction solution was stirred at 25°C overnight. After the end of the reaction, the reaction solution was quenched by adding to saturated aqueous ammonium chloride solution, and then water (200 mL) was added to obtain a mixture. The mixture was extracted with ethyl acetate (300 mL × 3). The organic phases were combined and washed with saturated saline, dried over anhydrous sodium sulfate and concentrated. The residue was purified by silica gel column chromatography (petroleum ether/ethyl acetate = 10/1) to obtain compound 120 (20.3 g, 77%) as a white solid.
LCMS: $[M+H]^+$ = 165.1

Step 2: Compound 121

Compound 120 (20.3 g, 123.66 mmol, 1.0 eq) was dissolved in methanol (250 mL), and cobalt dichloride (9.63 g, 74.19 mmol, 0.6 eq) was added. The reaction solution was cooled down with an ice bath for 10 min, and then sodium borohydride (28.07 g, 741.94 mmol, 6.0 eq) was slowly added to the reaction solution while maintaining the ice bath condition. The reaction solution was stirred at 25 °C for 16 h. After LCMS detection showed that the reaction was completed, the reaction solution of crude compound 121 was used directly in the next step of the reaction.
LCMS: $[M+H]^+$ = 169.1

Step 3: Compound 122

The reaction solution of crude compound 121 (123.66 mmol, 1.0 eq) was added to sodium methanol (10.02 g, 185.50 mmol, 1.5 eq). The reaction solution was stirred at 80 °C overnight. The reaction solution was cooled to room temperature, filtered, the filter cake was washed with methanol and the filtrate was concentrated in vacuum. The residue was purified by silica gel column chromatography (petroleum ether/ethyl acetate = 10/1-3/1, ethyl acetate ) to obtain compound 122 (13.5 g, 80%, two steps) as a pale pink solid.
LCMS: $[M+H]^+$ = 137.2

Step 4: Compound 124

Compound 122 (3.0 g, 22.03 mmol, 1.0 eq) was dissolved in anhydrous DMF (40 mL) and then NaH (1.76 g, 60% in mineral oil, 44.07 mmol, 2.0 eq) was added under ice bath conditions. The reaction solution was stirred under ice bath for 30 min, then compound 123 (7.67 g, 33.05 mmol, 1.5 eq) was added. The reaction solution was stirred at 25°C for 30 min. After the end of the reaction, the reaction solution was quenched with saturated aqueous ammonium chloride solution and water (60 mL) was added to obtain a mixture. The mixture was extracted with ethyl acetate (90 mL × 3). The combined

organic phases were washed with saturated saline, dried over anhydrous sodium sulfate and concentrated. The crude product was purified by silica gel column chromatography (petroleum ether/ethyl acetate = 10/1 - 2/1) to obtain compound 124 (989 mg, 20%) as a white solid.
LCMS: $[M+H]^+ = 219.1$

Step 5: Compound 125

Compound 124 (950 mg, 4.35 mmol, 1.0 eq) was dissolved in dichloromethane (10 mL), and then cooled down to 0 °C, and kept at 0 °C, then NBS (697 mg, 3.92 mmol, 0.9 eq) was added. The reaction solution was reacted at 0 °C for 30 min. After the end of the reaction, the reaction solution was evaporated to dryness and the residue was purified by fast silica gel column chromatography (petroleum ether/ethyl acetate = 4/1 ) to obtain compound 125 (956 mg, 73%) as a white solid.
LCMS: $[M+H]^+ = 297.0$

Step 6: Compound 126

Compound 125 (920 mg, 3.10 mmol, 1.0 eq) was dissolved in a mixed solvent (triethylamine/tetrahydrofuran = 1/1/, 12 mL), followed by the addition of Compound 62 (3.04 g, 31.0 mmol, 10.0 eq), bis(triphenylphosphine)palladium dichloride (217 mg, 0.310 mmol, 0.1 eq) and cuprous iodide ( 118 mg, 0.620 mmol, 0.2 eq). After displacing nitrogen three times, the reaction solution was stirred at 90 °C for 3 h under nitrogen atmosphere. The reaction solution was cooled, diluted with ethyl acetate and filtered. The filtrate was sequentially washed with ammonia, washed with saturated brine, dried over anhydrous sodium sulfate, and concentrated to obtain the crude product 126 (4.5 g). The crude product 126 was used directly in the next reaction.
LCMS: $[M+H]^+ = 315.1$

Step 7: Compound 127

The crude product 126 (4.5 g, 3.10 mmol, 1.0 eq) obtained in the previous step was dissolved in ethyl acetate (20 mL) and then tetrabutylammonium fluoride trihydrate (1.40 g, 4.43 mmol, 1.4 eq) was added. The reaction solution was stirred at room temperature for 30 min. The reaction solution was concentrated and the residue was purified by fast silica gel column chromatography (petroleum ether/ethyl acetate = 5/1) to obtain compound 127 (711 mg, 92%, two steps) as a yellow solid.
LCMS: $[M+H]^+ = 243.1$

Step 8: Compound 128

Compound 127 (100 mg, 0.413 mmol, 1.0 eq), Compound 65 (161 mg, 0.413 mmol, 1.0 eq), and Potassium phosphate monohydrate (285 mg, 1.239 mmol, 3.0 eq) were added to dioxane (5 mL), followed by rapid addition of X-phos (39 mg, 0.083 mmol, 0.2 eq) and $Pd_2dba_3$ (38 mg, 0.041 mmol, 0.1 eq). After replacing nitrogen three times, the reaction mixture was stirred at 100 °C for 2 h under nitrogen atmosphere. The reaction solution was cooled to room temperature and concentrated. The residue was purified by fast silica gel column chromatography (petroleum ether/ethyl acetate = 9/11) to obtain compound 128 (163 mg, 66%) as a brown solid.
LCMS: $[M+H]^+ = 596.2$

Step 9: Compound A38

Compound 128 (160 mg, 0.269 mmol, 1.0 eq) and potassium carbonate (111 mg, 0.807 mmol, 3.0 eq) were dissolved in a mixture of solvents (dioxane/water = 5/1, 6 mL). The reaction solution was stirred at 110 °C for 3 hours. Cooled to room temperature, the reaction solution was concentrated. The residue was purified by pre-HPLC (mobile phase: 0.1% formic acid/acetonitrile/water) to obtain A38 (62.5 mg, 46% ) as a white solid.

LCMS: $[M+H]^+ = 496.1$
$^1H$ NMR (400 MHz, DMSO-$d_6$) δ 12.92 (br. s, 1H), 9.78 (s, 1H), 8.40 (d, *J* = 3.6 Hz, 1H), 8.15 (br. s, 1H), 7.91 (br. s, 1H), 7.74 (d, *J* = 8.8 Hz, 2H), 7.62 (d, *J* = 8.8 Hz, 2H), 6.82 (d, *J* = 4.0 Hz, 1H), 6.74 (d, *J* = 4.0 Hz, 1H), 4.38-4.25 (m, 4H), 3.96-3.85 (m, 2H).

**Example 1-28 Preparation of Compound A40**

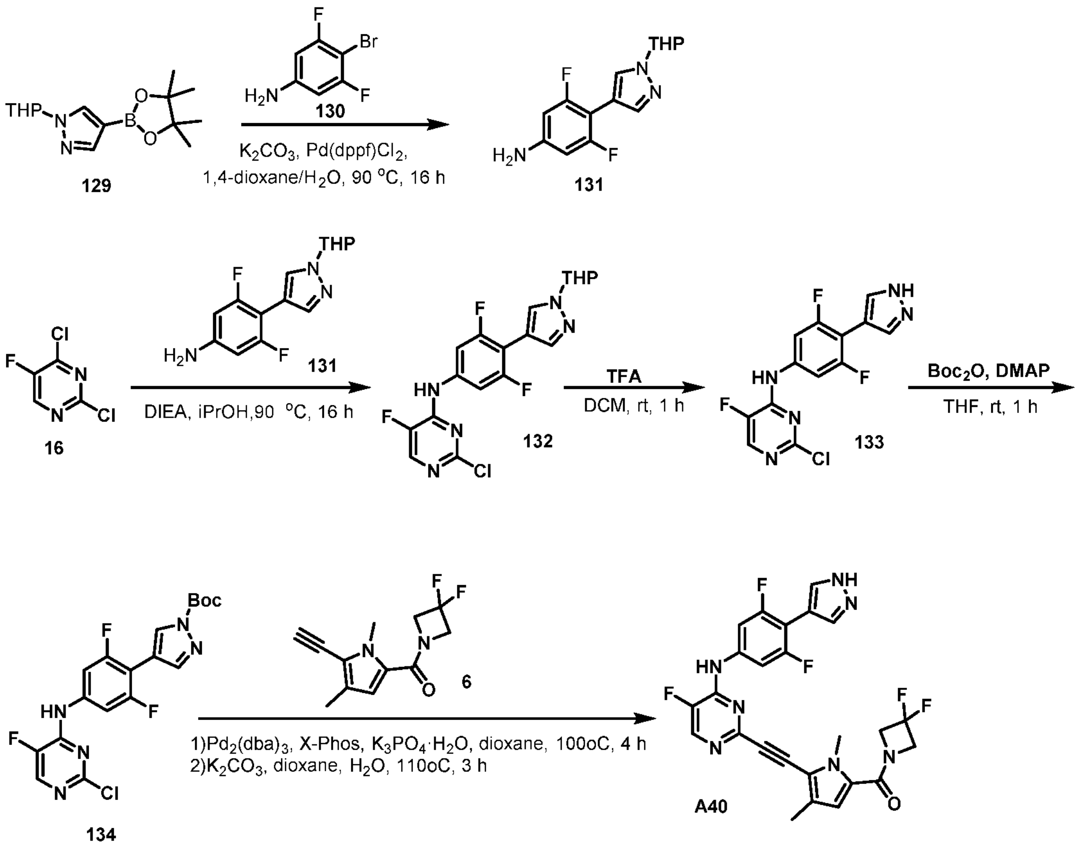


Step 1: Compound 131

Compound 129 (4.0 g, 14.38 mmol, 1.5 eq), Compound 130 (2.0 g, 9.58 mmol, 1.0 eq), Pd(dppf)$Cl_2$ (1.39 g, 1.92 mmol, 0.2 eq), and Potassium Carbonate (4.0 g, 28.76 mmol, 3.0 eq) were added to dioxane/water (10/ 1, 100 mL). The reaction solution was allowed to react at 90 °C for 16 h under nitrogen atmosphere. The reaction solution was cooled down and the solvent was removed by depressurization. The residue was purified by silica gel column chromatography (PE/EA=4/1) to obtain Compound 131 (3.35 g, 76%) as a pale yellow solid.
LCMS: [M+H] = 280.1

Step 2: Compound 132

Compound 16 (1.2 g, 7.2 mmol, 5.0 eq) was dissolved in isopropanol (6 mL), followed by the addition of Compound 131 (400 mg, 1.44 mmol, 1.0 eq) and diisopropylethylamine (743 mg, 5.8 mmol, 4.0 eq). The reaction solution was stirred at 90 °C for 16 hours. The reaction solution was cooled to room temperature and concentrated. The residue was purified by silica gel column chromatography (petroleum ether/ethyl acetate=3/1) to obtain compound 132 (430 mg, 73 %) as a yellow solid.
LCMS: $[M+H]^+$ = 410.1

Step 3: Compound 133

Compound 132 (430 mg, 1.05 mmol, 1.0 eq) was dissolved in dichloromethane (5 mL) and then trifluoroacetic acid (5 mL) was added. The reaction solution was stirred at room temperature for 1 hour. The reaction solution was concentrated and the pH was adjusted to 7 with aqueous sodium carbonate, then extracted with ethyl acetate (50 mL×3). The combined organic phases were washed with saturated brine, dried over anhydrous sodium sulfate and concentrated to obtain compound 133 (340 mg, 100 %) as a yellow solid.
LCMS: $[M+H]^+$ = 326.1

Step 4: Compound 134

Compound 133 (340 mg, 1.04 mmol, 1.0 eq) was dissolved in tetrahydrofuran (6 mL), then $Boc_2O$ (228 mg, 1.04

mmol, 1.0 eq) and DMAP (26 mg, 0.21 mmol, 0.2 eq ) were added. The reaction solution was stirred at 25°C for 1 hour. The reaction solution was concentrated and the residue was purified by silica gel column chromatography (petroleum ether/ethyl acetate = 4/1) to obtain compound 134 (280 mg, 63 %) as a white solid.
LCMS: $[M+H]^+$ = 426.1

Step 5: Compound A40

Compound 134 (125 mg, 0.29 mmol, 1.0 eq) was dissolved in dioxane (5 mL), and then compound 6 (70 mg, 0.29 mmol, 1.0 eq), potassium phosphate monohydrate (237 mg, 1.02 mmol, 3.5 eq) , X-Phos (28 mg, 0.058 mmol, 0.2 eq) and $Pd_2(dba)_3$ (27 mg, 0.029 mmol, 0.1 eq) were added. After three air exchanges with nitrogen, the reaction solution was stirred at 100 °C for 4 h under nitrogen atmosphere. After cooling to room temperature, potassium carbonate (121 mg, 0.87 mmol, 3.0 eq) and water (1 mL) were added to the reaction solution. The reaction solution was stirred at 110 °C for 3 hours. The reaction solution was concentrated and the residue was purified by pre-HPLC (mobile phase: 0.1% formic acid/acetonitrile/water) to obtain compound A40 (30.3 mg, 20 %) as a yellow solid.

LCMS: $[M+H]^+$ = 528.1
$^1H$ NMR (400 MHz, DMSO-$d_6$) δ 13.20 (br. s, 1H), 10.13 (s, 1H), 8.50 (d, *J* = 3.6 Hz, 1H), 8.10 (br. s, 1H), 7.87 (br. s, 1H), 7.84 - 7.76 (m, 2H), 6.59 (s, 1H), 4.62 (br. s, 4H), 3.91 (s, 3H), 2.19 (s, 3H).

**Example 1-29 Preparation of Compound A41**

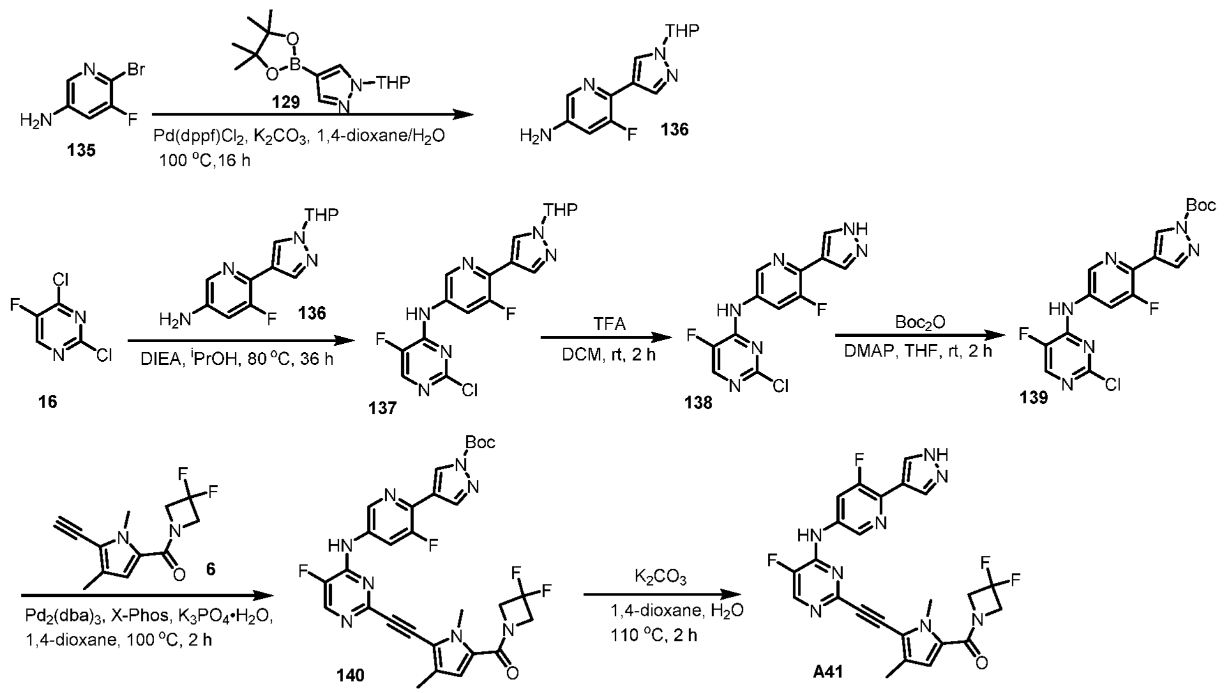


Step 1: Compound 136

Compound 135 ( 2.0 g , 10.47 mmol, 1.0 eq) was dissolved in a solvent mixture of dioxane (40 mL) and water (6 mL), and then compound 129 (3.5 g, 12.57 mmol, 1.2 eq), potassium carbonate (4.3 g, 31.41 mmol, 3.0 eq), and Pd(dppf) $Cl_2$ ( 760 mg, 1.047 mmol, 0.1 eq) were added in order. Nitrogen was replaced, and the reaction solution was stirred at 100 °C for 16 h. The reaction solution was cooled to room temperature and concentrated. The residue was purified by silica gel column chromatography (PE/EA=1/1) to obtain compound 136 (2.0 g, 73%) as a brown solid.
LCMS: $[M+H]^+$ = 263.2

Step 2: Compound 137

Compound 16 (1.1 g, 6.7 mmol, 5.0 eq) and Compound 136 (350 mg, 1.3 mmol, 1.0 eq) were dissolved in

isopropanol ( 10 mL), and then diisopropylethylamine (503 mg, 3.9 mmol, 3.0 eq) was added. The reaction solution was stirred at 80 °C for 36 hours. The reaction solution was cooled to room temperature and concentrated. The residue was purified by silica gel column chromatography (PE/EA=1/1) to obtain compound 137 (400 mg, 78%) as a white solid.
LCMS: [M+H] + = 393.1

Step 3: Compound 138

Compound 137 (400 mg, 1.0 mmol, 1.0 eq) was dissolved in dichloromethane (5 mL) and then trifluoroacetic acid (5 mL) was added. The reaction solution was stirred at 25°C for 2 hours. The reaction solution was concentrated and the residue was diluted with dichloromethane and the pH was adjusted to be alkaline with saturated aqueous sodium carbonate. The resulting reaction mixture was filtered and the solid was collected. The solid was lyophilized to obtain compound 138 (300 mg, 95%) as a white solid.
LCMS: $[M+H]^+$ = 309.1

Step 4: Compound 139

Compound 138 (300 mg, 0.97 mmol, 1.0 eq) was dissolved in tetrahydrofuran (10 mL) and then DMAP ( 24 mg, 0.19 mmol, 0.2 eq) and $Boc_2O$ (211 mg, 0.97 mmol, 1.0 eq) were added sequentially. The reaction solution was stirred at room temperature for 2 hours. The reaction solution was concentrated and the residue was purified by silica gel column chromatography (PE/EA=2/1) to obtain compound 5 (280 mg, 71%) as a white solid.
LCMS: $[M-55]^+$ = 409.1

Step 5: Compound 140

Compound 139 ( 120 mg , 0.29 mmol, 1.0 eq) was dissolved in dioxane (8 mL), followed by the sequential addition of compound 6 (70 mg, 0.29 mmol, 1.0 eq), potassium phosphate monohydrate (200 mg, 0.87 mmol, 3.0 eq), X-Phos (29 mg, 0.06 mmol, 0.2 eq) ) and $Pd_2(dba)_3$ (28 mg, 0.03 mmol, 0.1 eq). Nitrogen was replaced and the reaction solution was stirred at 100 °C for 2 h. The reaction solution was cooled to room temperature and concentrated. The crude 140 was used directly in the next step of the reaction.
LCMS: $[M+H]^+$ = 611.2

Step 6: Compound A41

Crude compound 140 was dissolved in a solvent mixture of dioxane (5 mL) and water (2 mL) followed by addition of potassium carbonate (120 mg, 0.87 mmol, 3.0 eq). The reaction solution was stirred at 110 °C for 2 hours. The reaction solution was cooled to room temperature and concentrated. The residue was purified by prep-HPLC (mobile phase: 0.1% formic acid/acetonitrile/water) to obtain compound A41 (22.6 mg, two-step yield: 15%) as a white solid.

LCMS: $[M+H]^+$ = 510.2
$^1H$ NMR (400 MHz, DMSO-$d_6$) δ 13.17 (br s, 1H), 10.22 (s, 1H), 8.79-8.72 (m, 1H), 8.48 (d, *J* = 3.6 Hz, 1H), 8.40 (d, *J* = 13.6 Hz, 1H), 8.21 (s, 1H), 8.02 (s, 1H), 6.57 (s, 1H), 4.61 (br s, 4H), 3.88 (s, 3H), 2.17 (s, 3H).

**Example 1-30 Preparation of Compound A42**

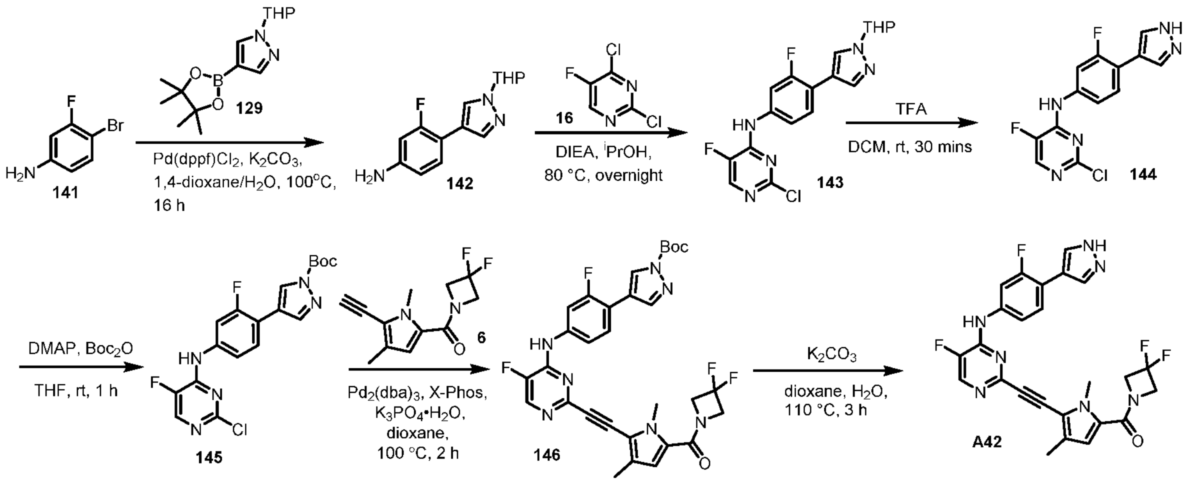


Step 1: Compound 142

Compound 141 (2.0 g, 9.58 mmol, 1.0 eq), Compound 129 (4.39 g, 14.38 mmol, 1.5 eq) and Pd(dppf)$Cl_2$ (1.39 g, 1.91 mmol, 0.2 eq), Potassium Carbonate (4.0 g, 29.0 mmol, 3.0 eq) were added to dioxane/water (10/1, 100 mL). The reaction solution was stirred at 100 °C for 16 h under nitrogen atmosphere. The reaction solution was cooled down and the solution was removed by depressurization. The residue was purified by silica gel column chromatography (PE/EA=4/1) to obtain compound 142 (3.35 g, 76%) as a pale yellow solid.
LCMS: $[M+H]^+$= 262.13

Step 2: Compound 143

Compound 142 (300 mg, 1.15 mmol, 1.0 eq), compound 16 (958 mg, 5.75 mmol, 5.0 eq) and diisopropylethylamine (445 mg, 3.45 mmol, 3.0 eq) were dissolved in isopropanol (15 mL). The reaction solution was stirred at 80 °C overnight. The reaction solution was cooled to room temperature and the solvent was removed under pressure. The residue was purified by rapid silica gel column chromatography (PE/EA=18/7) to obtain compound 143 (388 mg, 86%) as a yellow solid.
LCMS: $[M+H]^+$ = 392.1

Step 3: Compound 144

Compound 143 (384 mg, 0.980 mmol, 1.0 eq) was dissolved in dichloromethane (3 mL) and then trifluoroacetic acid (8 mL) was added. The reaction solution was stirred at 25 °C for 30 min. The reaction solution was concentrated in vacuum, water (10 mL) was added, the pH was adjusted to 8 by addition of saturated aqueous sodium carbonate, and extracted with ethyl acetate (20 mL × 3). The organic phases were combined and washed with saturated brine, dried over anhydrous sodium sulfate and concentrated to obtain compound 144 (302 mg, 100%) as a yellow solid.
LCMS: $[M+H]^+$ = 308.1

Step 4: Compound 145

Compound 144 (302 mg, 0.980 mmol, 1.0 eq) was dissolved in tetrahydrofuran (10 mL), then 4-dimethylaminopyridine (12 mg, 0.098 mmol, 0.1 eq) and di-tert-butyl dicarbonate (214 mg, 0.980 mmol, 1.0 eq) were added, and the reaction solution was stirred for 1 hour at room temperature. After completion of the reaction, the reaction solution was concentrated and the residue was purified by fast silica gel column chromatography (PE/EA = 3/1) to obtain compound 145 (362 mg, 90%) as a pale yellow solid.
LCMS: $[M+H]^+$ = 408.1

Step 5: Compound 146

Compound 145 (120 mg, 0.294 mmol, 1.0 eq), Compound 6 (70 mg, 0.294 mmol, 1.0 eq) and potassium phosphate monohydrate (203 mg, 0.882 mmol, 3.0 eq) were dissolved in dioxane (5 mL) followed by rapid addition of X-phos (28 mg, 0.059 mmol, 0.2 eq) and $Pd_2(dba)_3$ (27 mg, 0.029 mmol, 0.1 eq). After three nitrogen replacements, the

reaction solution was stirred at 100 °C for 2 h under nitrogen atmosphere. The reaction solution was concentrated and the residue was purified by fast silica gel column chromatography (PE/EA = 2/3) to obtain compound 146 (125 mg, 69%) as a tan solid.
LCMS: [M+H] $^+$ = 610.2

Step 6: Compound A42

Compound 146 (120 mg, 0.197 mmol, 1.0 eq) and potassium carbonate (136 mg, 0.985 mmol, 5.0 eq) were dissolved in a mixture of solvents (dioxane/water = 2/1, 4.5 mL). The reaction solution was stirred at 110 °C for 3 hours. Cooled to room temperature and the reaction solution was concentrated. The residue was purified by pre-HPLC (0.1% formic acid/acetonitrile/water) to obtain A42 (25.0 mg, 24%) as a white solid.

LCMS: [M+H] $^+$ = 510.2
$^1$H NMR (400 MHz, DMSO-$d_6$) δ 13.05 (br. s, 1H), 9.96 (s, 1H), 8.45 (d, *J* = 3.6 Hz, 1H), 8.12 (br. s, 1H), 7.99 (dd, *J* = 14.0, 2.0 Hz, 1H), 7.92 (br. s, 1H), 7.72 (t, *J* = 8.8 Hz, 1H), 7.61 (dd, *J* = 8.4, 2.0 Hz, 1H), 6.59 (s, 1H), 4.61 (br. s, 4H), 3.91 (s, 3H), 2.19 (s, 3H).

**Example 1-31 Preparation of Compound A43**

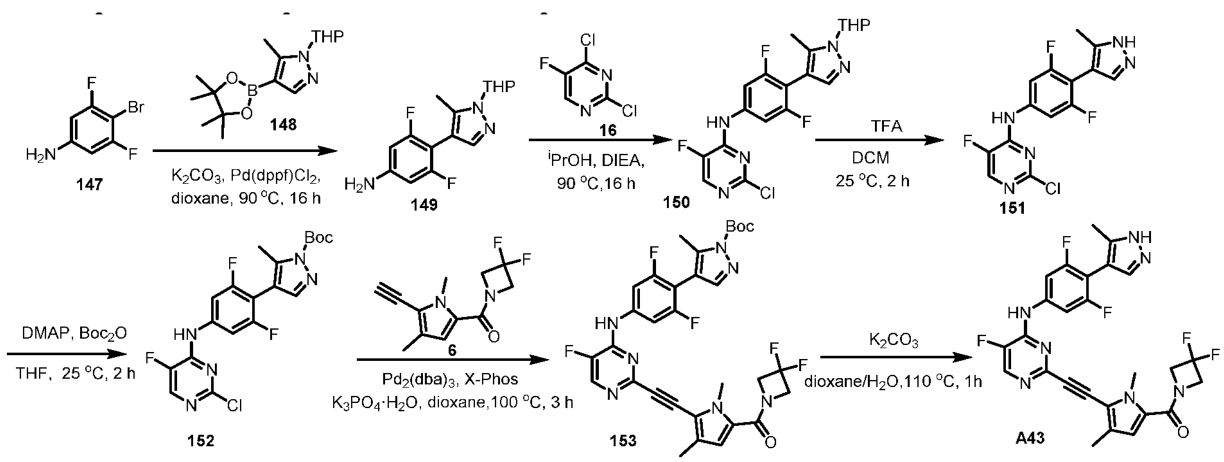


Step 1: Compound 149

Compound 147 (400 mg, 1.92 mmol, 1.0 eq), Compound 148 (660 mg, 2.30 mmol, 1.2 eq) and Pd(dppf)$Cl_2$ (139.38 mg, 0.192 mmol, 0.1 eq) , Potassium Carbonate (3.0 g, 4.78 mmol, 3.0 eq) were added to Dioxane/water (5/1, 10 mL). The reaction solution was allowed to react at 90 °C for 16 h under nitrogen atmosphere. The reaction solution was cooled down and depressurized to remove the solution. The obtained was purified by silica gel column chromatography (PE/EA=4/1) to obtain compound 149 (500 mg, 88 %) as a pale yellow solid.
LCMS: [M+H] + =294.1

Step 2: Compound 150

Compound 149 (500 mg, 1.70 mmol, 1.0 eq) and compound 16 (370 mg, 2.22 mmol, 1.3 eq) were dissolved in isopropanol (10 mL) followed by addition of diisopropylethylamine (659.12 mg, 5.1 mmol, 3.0 eq). After addition, nitrogen was replaced and the reaction solution was allowed to react at 90 °C for 16 h under nitrogen atmosphere. The reaction solution was cooled to room temperature, and the solvent was removed by decompression distillation. The residue was purified by silica gel column chromatography (ethyl acetate/petroleum ether=2/1) to obtain compound 150 (400 mg, 55.4 %) as a yellow solid.
LCMS: [M+H] $^+$ =424.1

Step 3: Compound 151

Compound 150 (280 mg, 0.662 mmol, 1.0 eq) was dissolved in dichloromethane (5 mL) followed by addition of trifluoroacetic acid (5 mL) to the solution. After the addition was completed, the reaction solution was reacted at 25 °C for 2 hours. The reaction solution was distilled under reduced pressure to remove the solvent. The residue was dissolved with a small amount of dichloromethane and the pH was adjusted to 9-10 with saturated sodium carbonate solution. The reaction mixture was filtered and the solid was collected. The solid was dried over rotary evaporation to obtain compound 151 (200 mg, 86.3 %) as a white solid which was used directly in the next step.
LCMS: $[M+H]^+$ =339.1

Step 4: Compound 152

Compound 151 (150 mg, 0.442 mmol, 1.0 eq) was dissolved in tetrahydrofuran (25 mL), followed by addition of $Boc_2O$ (67.60 mg, 0.309 mmol, 0.7 eq) and DMAP (5.40 mg, 0.044 mmol, 0.1 eq) to the solution. After the addition was completed, the reaction solution was reacted at room temperature for 2 hours. The reaction solution was distilled under reduced pressure to remove the solvent and the residue was purified by silica gel column chromatography (ethyl acetate/petroleum ether=1/1) to obtain compound 152 (80 mg, 41 %) as a white solid.
LCMS: $[M+H]^+$ =440.1

Step 5: Compound 153

Compound 152 (70 mg, 0.159 mmol, 1.0 eq), Compound 6 (37.92 mg, 0.159 mmol, 1.0 eq), $Pd_2(dba)_3$ (29.15 mg, 0.032 mmol, 0.2 eq), X-Phos (15.16 mg, 0.032 mmol, 0.2 eq), potassium phosphate monohydrate (109.98 mg, 0.478 mmol, 3.0 eq) were added to 1, 4-dioxane (5 mL). After the addition was completed, nitrogen was replaced and the reaction solution was reacted at 100 °C for 3 h under nitrogen atmosphere. The reaction solution was distilled under reduced pressure to remove the solvent and the residue was purified by silica gel column chromatography (ethyl acetate/petroleum ether=1/1) to obtain compound 153 (90 mg, 89%) as a white solid.
LCMS: $[M+H]^+$ =642.2

Step 5: Compound A43

Compound 153 (80 mg, 0.125 mmol, 1.0 eq) and potassium carbonate (51.70 mg, 0.374 mmol, 3.0 eq) were added to a mixed solution of 1, 4-dioxane (4 mL) and water (2 mL). After the addition was completed, the reaction solution was reacted at 110 °C for 1 hour. The solvent was removed by distillation under reduced pressure, the residue was dissolved in DMF (4 mL) and filtered, and the filtrate was purified by prep-HPLC (mobile phase: 0.1% HCOOH/acetonitrile/-water) to obtain compound A43 (18 mg, 26.6%) as a white solid.

LCMS: $[M+H]^+$ =542.2
$^1H$ NMR (400 MHz, DMSO-$d_6$) δ 12.84 (br. s, 1H), 10.17 (s, 1H), 8.51 (d, *J* = 3.6 Hz, 1H), 7.78 (d, *J* = 10 Hz, 3H), 6.59 (s, 1H), 4.95-4.32 (br. s, 4H), 3.89 (s, 3H), 2.18 (s, 6H).

**Example 1-32 Preparation of Compound A44**

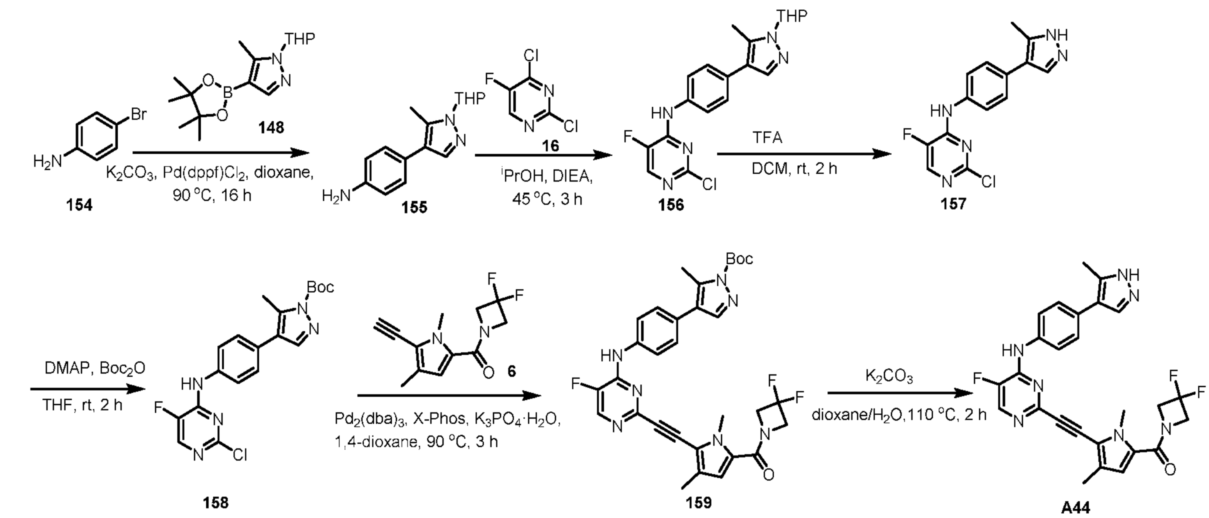


154 155 156 157 158 159 A44

Step 1: Compound 155

Compound 154 (3.4 g, 19.8 mmol, 0.9 eq) and Compound 148 (6.1 g, 20.8 mmol, 1.0 eq) were dissolved in 1,4-dioxane (20 mL), followed by the addition of potassium carbonate (8.6 g, 62.4 mmol, 3.0 eq) and 1,1 '-bis(diphenylphosphino) ferrocene palladium dichloride (456 mg, 0.62 mmol, 0.03 eq). Nitrogen was replaced and the reaction solution was stirred at 90 °C for 16 h under nitrogen atmosphere. The reaction solution was cooled to room temperature, passed through diatomaceous earth and the filtrate was concentrated to obtain the residue. The residue was purified by silica gel column chromatography (PE/EA=4/1) to obtain compound 155 (3.3 g, 64%) as a yellow solid.
LCMS: $[M+H]^+$ = 258.1

Step 2: Compound 156

Compound 155 (3.3 g, 12.8 mmol, 1.0 eq) was dissolved in isopropanol (30 mL), compound 16 (4.2 g, 25.6 mmol, 2.0 eq) was added along with diisopropylethylamine (8.2 g, 64 mmol, 5.0 eq), and the reaction mixture was stirred at 45 °C for 3 h. After LC-MS detection showed that the reaction was completed, the reaction mixture was concentrated to obtain the residue, the residue was purified by silica gel column chromatography (petroleum ether/ethyl acetate=4/1) to obtain compound 156 (2.8 g, 56%) as a yellow solid.
LCMS: $[M+H]^+$ = 388.1

Step 3: Compound 157

Compound 156 (2.8 g, 7.23 mmol, 1.0 eq) was dissolved in dichloromethane (20 mL) and then trifluoroacetic acid (120 mL) was added. The reaction solution was stirred at room temperature for 2 hours. The reaction solution was concentrated to obtain the residue, the residue was adjusted to be alkaline pH with saturated aqueous sodium carbonate, a large amount of solid was precipitated, the solid was collected by filtration and dried to obtain the crude compound 157 (1.7 g) as a yellow solid.
LCMS: $[M+H]^+$ = 304.0

Step 4: Compound 158

Compound 157 (1.7 g, 5.6 mmol, 1.0 eq) was added to tetrahydrofuran (25 mL) followed by DMAP (136 mg, 1.12 mmol, 0.2 eq) and $Boc_2O$ (977 mg, 4.48 mmol, 0.8 eq) sequentially. The reaction solution was stirred at room temperature for 2 hours. The reaction solution was diluted with ethyl acetate and then sequentially washed with water and saturated saline, dried over anhydrous sodium sulfate and concentrated. The residue was purified by silica gel column chromatography (petroleum ether/ethyl acetate=4/1) to obtain compound 158 (2.0 g, 88%) as a white solid.
LCMS: $[M+H]^+$ = 404.0

Step 5: Compound 159

Compound 158 (510 mg, 1.26 mmol, 1.0 eq) was dissolved in dioxane (15 mL), then compound 6 (300 mg, 1.26

mmol, 1.0 eq), potassium phosphate monohydrate (870 mg, 3.78 mmol, 3.0 eq), X-Phos (119 mg, 0.25 mmol, 0.2 eq) and $Pd_2(dba)_3$ (164 mg, 0.18 mmol, 0.15 eq) were added. After the addition, the reaction solution was stirred at 90 °C for 3 h under nitrogen atmosphere. Cooled to room temperature, the reaction solution was diluted with ethyl acetate, passed through diatomaceous earth, the filtrate was concentrated to obtain the residue, the residue was treated to obtain the crude product of compound 159 which was directly used in the next step.
LCMS: $[M+H]^+$ =606.2

Step 6: Compound A44

Crude compound 159 (200 mg, 0.33 mmol, 1.0 eq) was dissolved in a solvent mixture of dioxane (8 mL) and water (3 mL), then potassium carbonate (182 mg, 1.32 mmol, 4.0 eq) was added. The reaction solution was stirred at 110 °C for 2 hours. The reaction solution was diluted with ethyl acetate and then sequentially washed with water and saturated saline, dried over anhydrous sodium sulfate and concentrated to obtain the residue. The residue was purified by pre-HPLC (mobile phase: 0.1% formic acid/acetonitrile/water) to obtain compound A44 (104.5 mg, 62%) as a white solid.

LCMS: $[M+H]^+$ =506.1
$^1H$ NMR (400 MHz, DMSO-$d_6$) δ 12.62 (s, 1H), 9.78 (s, 1H), 8.39 (d, $J$ = 4.0 Hz, 1H), 7.79-7.78 (m, 3H), 7.44 (d, $J$ = 8.4 Hz, 2H), 6.57 (s, 1H), 4.61 (br. s, 4H), 3.88 (s, 3H), 2.37 (s, 3H), 2.16 (s, 3H).

## Example 1-33 Preparation of Compound A45

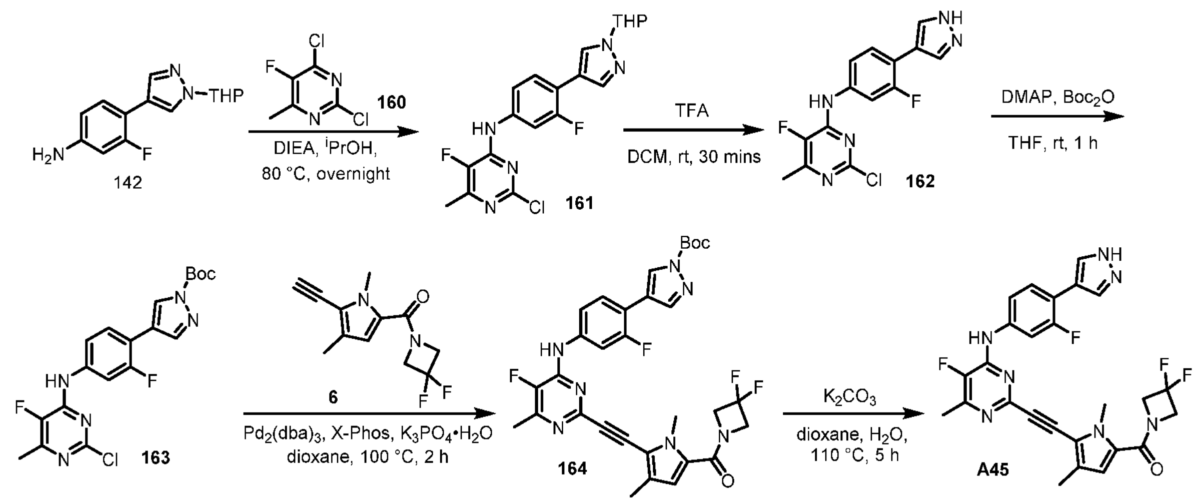


Step 1: Compound 161

Compound 142 (350 mg, 1.34 mmol, 1.0 eq), compound 160 (242 mg, 1.34 mmol, 1.0 eq) and diisopropylethylamine (519 mg, 4.02 mmol, 3.0 eq) were dissolved in isopropanol (10 mL). The reaction solution was stirred at 80 °C overnight. The reaction solution was cooled to room temperature and the solvent was removed under reduced pressure. The residue, crude compound 161, was used directly in the next step of the reaction.
LCMS: $[M+H]^+$ = 406.1

Step 2: Compound 162

The crude compound 161 obtained above was dissolved in dichloromethane (5 mL) and then trifluoroacetic acid (10 mL) was added. The reaction solution was stirred at 25 °C for 30 min. After the reaction solution was concentrated in vacuum, water (15 mL) was added to the residue, the pH was adjusted to 8 by adding saturated aqueous sodium carbonate solution, and extracted with ethyl acetate (20 mL × 3). The combined organic phases were washed with saturated saline, dried over anhydrous sodium sulfate and the concentrated residue was purified by fast silica gel column chromatography (PE/EA = 3/7) to obtain compound 162 (200 mg, 46%, two steps) as a brown solid.
LCMS: $[M+H]^+$ = 322.1

Step 3: Compound 163

Compound 162 (200 mg, 0.621 mmol, 1.0 eq) was dissolved in tetrahydrofuran (10 mL), then 4-dimethylaminopyridine (7 mg, 0.062 mmol, 0.1 eq) and di-tert-butyl dicarbonate (135 mg, 0.621 mmol, 1.0 eq) were added, and the reaction solution was stirred for 1 hour at 25 °C. After completion of the reaction, the reaction solution was concentrated and the residue was purified by fast silica gel column chromatography (PE/EA = 7/3) to obtain compound 163 (166 mg, 63% ) as a white solid.
LCMS: $[M+H]^+$ = 422.1

Step 4: Compound 164

Compound 163 (155 mg, 0.296 mmol, 1.0 eq), Compound 6 (71 mg, 0.296 mmol, 1.0 eq) and Potassium Phosphate Hydrate (204 mg, 0.888 mmol, 3.0 eq) were dissolved in dioxane (5 mL), followed by a rapid addition of X-phos (28 mg, 0.0296 mmol, 0.2 eq) and $Pd_2(dba)_3$ (23 mg, 0.0592 mmol, 0.1 eq). After displacing nitrogen three times, the reaction solution was stirred at 100 °C for 2 h under nitrogen atmosphere. The reaction solution was concentrated and the residue was purified by fast silica gel column chromatography (PE/EA = 2/3) to obtain compound 164 (162 mg, 87%) as a tan solid.
LCMS: $[M+H]^+$ = 624.2

Step 5: Compound A45

Compound 164 (160 mg, 0.256 mmol, 1.0 eq) and potassium carbonate (213 mg, 1.536 mmol, 6.0 eq) were dissolved in a mixture of solvents (dioxane/water = 2/1, 6 mL). The reaction solution was stirred at 110 °C for 5 hours. The reaction solution was cooled to room temperature and concentrated. The residue was purified by pre-HPLC (0.1% formic acid/acetonitrile/water) to obtain A45 (29.7 mg, 21% ) as a yellow solid.

LCMS: $[M+H]^+$ = 524.2
$^1H$ NMR (400 MHz, DMSO-$d_6$) δ 13.04 (br. s, 1H), 9.81 (s, 1H), 8.10 (br. s, 1H), 8.02 - 7.85 (m, 2H), 7.70 (t, *J* = 8.8 Hz, 1H), 7.59 (dd, *J* = 8.8, 2.0 Hz, 1H), 6.58 (s, 1H), 4.62 (br. s, 4H), 3.90 (s, 3H), 2.38 (d, *J* = 2.8 Hz, 3H), 2.18 (s, 3H).

**Example 1-34 Preparation of Compound A46**

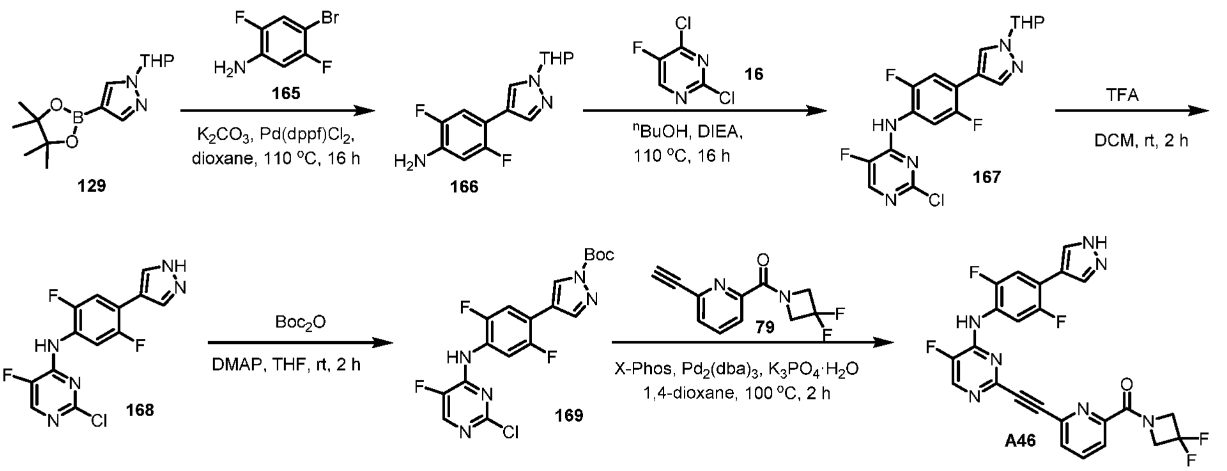


Step 1: Compound 166

Compound 129 (2.01 g, 7.3 mmol, 1.5 eq), Compound 165 (1 g, 4.8 mmol, 1 eq), Pd(dppf)$Cl_2$ (349 mg, 0.48 mmol, 0.1 eq), and Potassium Carbonate (1.99 mg, 14.4 mmol, 3.0 eq) were added to dioxane/water (20 mL/5 mL). After the addition was completed, nitrogen was displaced and the reaction mixture was reacted under nitrogen atmosphere at 90 °C for 16 h. After LC-MS detection showed that the reaction was completed, the reaction mixture was filtered through diatomaceous earth and the solids were washed with ethyl acetate. The filtrate was directly distilled under reduced pressure to remove the solvent and the residue was purified by silica gel column chromatography (petroleum ether/ethyl

acetate=5/1) to obtain compound 166 (1.3 g, 97%) as a pale yellow solid.
LCMS: $[M+H]^+$ = 280.1

Step 2: Compound 167

Compound 166 (600 mg, 2.15 mmol, 1.0 eq) and Compound 16 (3.59 mg, 21.48 mmol, 10 eq) were dissolved in n-butanol (10 mL) followed by addition of diisopropylethylamine (2.78 mg, 21.48 mmol, 10.0 eq). After addition, the reaction solution was reacted under nitrogen atmosphere at 110 °C for 16 hours. The reaction solution was distilled under reduced pressure to remove the solvent and the residue was purified by silica gel column chromatography (petroleum ether/ethyl acetate) to obtain compound 167 (280 mg, 32%) as a pale yellow solid.
LCMS: $[M+H]^+$ = 410.1

Step 3: Compound 168

Compound 167 (278 mg, 0.682 mmol, 1.0 eq) was dissolved in dichloromethane (4 mL) followed by addition of trifluoroacetic acid (3 mL) to the solution. After the addition, the reaction solution was reacted at 25 °C for 2 hours. The reaction solution was concentrated by distillation under reduced pressure. The residue was dissolved in dichloromethane, pH (8-9) was adjusted with saturated sodium carbonate solution, and the aqueous phase was extracted with dichloromethane (10 mL × 3). The organic phases were combined, dried over anhydrous sodium sulfate, concentrated by distillation under reduced pressure, and dried under vacuum to obtain the crude compound 168 (222 mg) as a pale yellow solid.
LCMS: $[M+H]^+$ = 326.0

Step 4: Compound 169

The crude compound 168 was dissolved in tetrahydrofuran (4 mL), followed by the addition of di-tert-butyl dicarbonate (118 mg, 0.541 mmol, 0.8 eq) and 4-dimethylaminopyridine (8 mg, 0.068 mmol, 0.1 eq). After addition, the reaction solution was reacted at room temperature for two hours. After LC-MS detection showed that the reaction was completed, the reaction solution was distilled under reduced pressure to remove the solvent and the residue was purified by rapid column chromatography (silica gel, petroleum ether/ethyl acetate 0-10%) to obtain compound 169 (180 mg, 62%) as a yellow solid.
LCMS: $[M+H]^+$ = 426.1

Step 5: Compound A46

Compound 169 (270 mg, 0.634 mmol, 1.0 eq) was dissolved in dioxane (10 mL) followed by the rapid addition of Compound 79 (142 mg, 0.634 mmol, 1.0 eq), potassium phosphate monohydrate (511 mg, 2.22 mmol, 3.5 eq), X-Phos (60 mg, 0.127 mmol, 0.2 eq) and $Pd_2(dba)_3$ (58 mg, 0.063 mmol, 0.1 eq). After three air changes with nitrogen, the reaction solution was stirred at 100 °C for 4 h under nitrogen atmosphere. The reaction solution was cooled to room temperature and potassium carbonate (525 mg, 3.8 mmol, 6.0 eq) and water (2 mL) were added to the reaction solution. The reaction solution was stirred at 110 °C for 3 hours. The reaction solution was concentrated and the residue was purified by pre-HPLC (mobile phase: 0.1% formic acid/acetonitrile/water) to obtain compound A46 (54.3 mg, 17 %) as a yellow solid.

LCMS: $[M+H]^+$ = 512.1
$^1$H NMR (400 MHz, DMSO-$d_6$) δ 13.17 (br. s, 1H), 9.83 (br. s, 1H), 8.49 (d, *J* = 3.2 Hz, 1H), 8.21 (br. s, 1H), 8.09-7.98 (m, 3H), 7.89-7.86 (m, 1H), 7.79-7.74 (m, 1H), 7.59-7.54 (m, 1H), 4.98 (t, *J* = 12.4 Hz, 2H), 4.51 (t, *J* = 12.4 Hz, 2H).

**Example 1-35 Preparation of Compound A47**

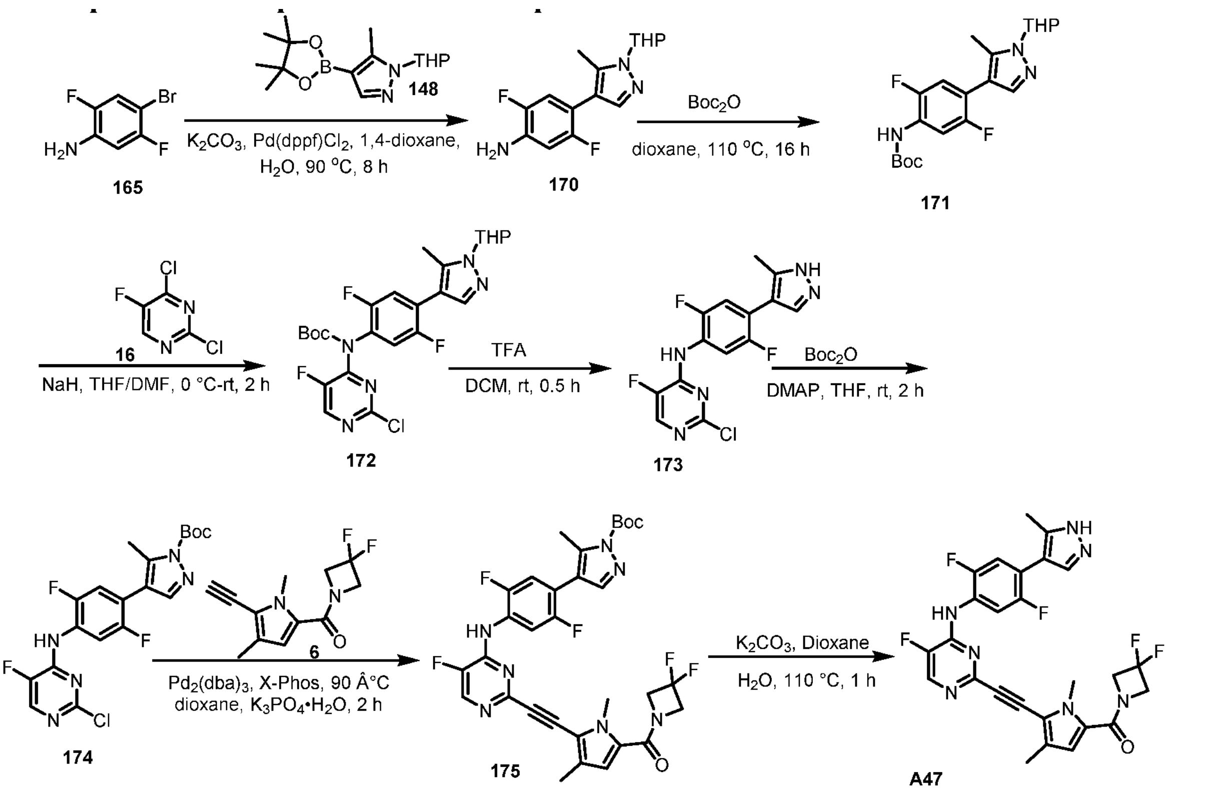


Step 1: Compound 170

Compound 165 (5.0 g, 24.04 mmol, 1.0 eq) and compound 148 (8.4 g, 28.85 mmol, 1.2 eq) were dissolved in a mixed solution of 1,4-dioxane (80 mL) and water (8 mL), followed by the addition of potassium carbonate (9.9 g, 72.12 mmol, 3.0 eq) and 1,1 '-bis(diphenylphosphino)ferrocene palladium dichloride (2.63 g, 3.60 mmol, 0.15 eq). Nitrogen was replaced, under nitrogen atmosphere, and the reaction solution was stirred at 90 °C for 8 h. LCMS detection showed that the completion of the reaction, the reaction solution was cooled to room temperature, passed through diatomaceous earth, and the filtrate was concentrated to obtain the residue. The residue was purified by silica gel column chromatography (PE/EA=12/1) to obtain compound 170 (6.4 g, 90%) as a yellow solid.
LCMS: $[M+H]^+$ = 294.1

Step 2: Compound 171

Compound 170 (6.0 g, 20.4 mmol, 1.0 eq) was added to dioxane (150 mL) followed by $Boc_2O$ (17.8 g, 81.6 mmol, 4.0 eq). The reaction solution was stirred at 110 °C for 16 hours. The reaction solution was concentrated to obtain a residue, the residue was purified by silica gel column chromatography (petroleum ether/ethyl acetate=20/1) to obtain compound 171 (4.4 g, 54%) as a yellow solid.
LCMS: $[M+H]^+$ = 394.1

Step 3: Compound 172

Compound 171 (330 mg, 0.83 mmol, 1.0 eq) was dissolved in tetrahydrofuran (5 mL), NaH (60% in mineral oil, 79 mg, 3.32 mmol, 4.0 eq) was added, and the mixture was stirred at 0 °C for 15 min, and then Compound 16 (413 mg, 2.49 mmol, 3.0 eq) was added and the reaction mixture was stirred at room temperature for 2 h. After LC-MS detection showed that the reaction was completed, the pH was adjusted to weak acidity by adding aqueous ammonium chloride to the reaction mixture, diluted with water, extracted with ethyl acetate, washed with saturated brine, dried over anhydrous sodium sulfate and concentrated to obtain the residue, and the residue was purified by rapid column chromatography on silica gel (petroleum ether/ethyl acetate=80/20) to obtain compound 172 (400 mg, 90%) as a yellow solid.
LCMS: $[M+H]^+$ = 524.1

Step 4: Compound 173

Compound 172 (400 mg, 0.76 mmol, 1.0 eq) was dissolved in dichloromethane (3 mL) and then trifluoroacetic acid (16 mL) was added. The reaction solution was stirred at 25°C for 0.5 hours. The reaction solution was concentrated to obtain the residue, the residue was adjusted to alkaline pH with saturated aqueous sodium carbonate solution, a large amount of solid was precipitated, the solid wsa filtered and collected and dried to obtain the crude compound 173 (227 mg) as a yellow solid.
LCMS: [M+H] + = 340.0

Step 5: Compound 174

Compound 173 (227 mg, 0.67 mmol, 1.0 eq) was added to tetrahydrofuran (5 mL) followed by DMAP (15 mg, 0.13 mmol, 0.2 eq) and $Boc_2O$ (120 mg, 0.54 mmol, 0.8 eq) sequentially. The reaction solution was stirred at room temperature for 2 hours. The reaction solution was diluted with ethyl acetate and then sequentially washed with water and saturated saline, dried over anhydrous sodium sulfate and concentrated. The residue was purified by rapid column chromatography on silica gel (petroleum ether/ethyl acetate = 81/19) to obtain compound 174 (200 mg, 67%) as a white solid.
LCMS: $[M+H]^+$ = 440.1

Step 6: Compound 175

Compound 174 (200 mg, 0.45 mmol, 1.0 eq) was dissolved in dioxane (6 mL), followed by the addition of Compound 6 (128 mg, 0.54 mmol, 1.2 eq), potassium phosphate monohydrate (310 mg, 1.35 mmol, 3.0 eq), X-Phos (42 mg, 0.09 mmol, 0.2 eq) and $Pd_2(dba)_3$ (54 mg, 0.06 mmol, 0.15 eq). The reaction solution was stirred at 90 °C for 2 h under nitrogen atmosphere. Cooled to room temperature, the reaction solution was diluted with ethyl acetate, passed through diatomaceous earth and the filtrate was concentrated to obtain the residue, the residue was purified by rapid column chromatography on silica gel (petroleum ether/ethyl acetate=62/38) to obtain compound 175 (120 mg, 40%) as a yellow solid.
LCMS: $[M+H]^+$ =642.2

Step 7: Compound A47

Compound 175 (120 mg, 0.18 mmol, 1.0 eq) was dissolved in a solvent mixture of dioxane (4 mL) and water (4 mL), then potassium carbonate (248 mg, 1.8 mmol, 10.0 eq) was added. The reaction solution was stirred at 110 °C for 1 hour. The reaction solution was diluted with ethyl acetate and then washed sequentially with water and saturated saline, dried over anhydrous sodium sulfate and concentrated. The residue was purified by prep-HPLC (mobile phase: 0.1% formic acid/acetonitrile/water) to obtain compound A47 (43.2 mg, 44%) as a yellow solid.

LCMS: $[M+H]^+$ =542.2
$^1H$ NMR (400 MHz, DMSO-$d_6$) δ 12.84 (br. s, 1H), 9.78 (s, 1H), 8.45 (d, $J$ = 3.6 Hz, 1H), 7.61-7.58 (m, 2H), 7.41-7.36 (m, 1H), 6.55 (s, 1H), 4.59 (br. s, 4H), 3.83 (s, 3H), 2.32 (s, 3H), 2.11 (s, 3H).

**Example 1-36 Preparation of Compound A48**

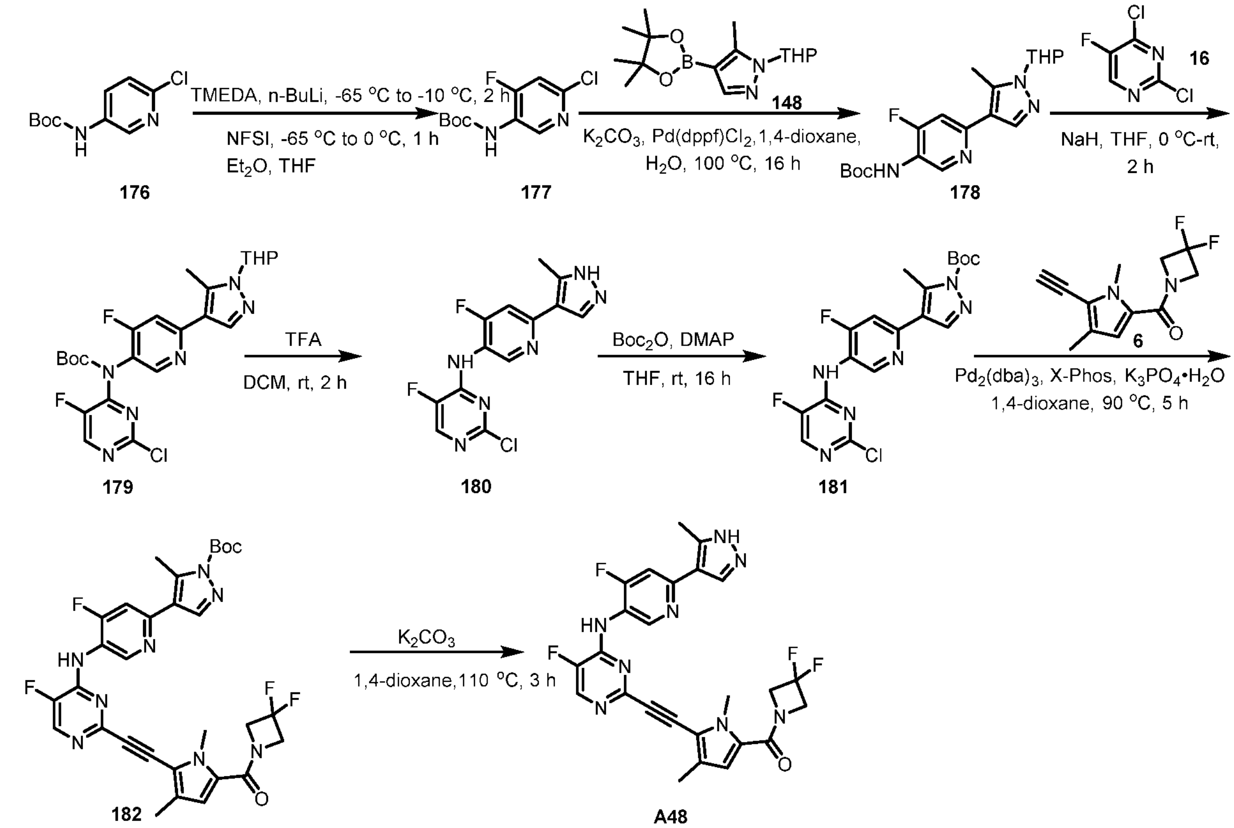


176 177 178 179 180 181 182 A48

Step 1: Compound 177

Compound 176 (2.50 g , 10.93 mmol, 1.0 eq) and 1,2-ethylenediamine (3.05 g , 26.24 mmol, 2.4 eq) were dissolved in ether (70 mL). Nitrogen was replaced and the solution was cooled to -65 °C under nitrogen protection, then a solution of n-butyllithium (2.4 M, 15 mL, 36.0 mmol, 3.29 eq) in n-hexane was slowly added dropwise (about half an hour) between -50 °C and -65 °C. The reaction solution was stirred at -60 °C for ten minutes under nitrogen protection, then slowly warmed to - 15 °C and kept stirring at -10 °C to -25 °C for 2 hours. The reaction solution was cooled down to -60 °C under nitrogen protection, and then a solution of NFSI (11.72 g, 37.17 mmol, 3.4 eq) in tetrahydrofuran (20 mL) was added dropwise slowly between -50 °C to -65 °C. After addition, the reaction solution was slowly warmed to 0 °C under nitrogen protection and stirred at 0 °C for 1 h. After completion of the reaction, the reaction was quenched by adding the reaction solution to saturated aqueous ammonium chloride solution, and the aqueous phase was extracted with ethyl acetate. The combined organic phases were dried over anhydrous sodium sulfate and concentrated. The residue was purified by silica gel column chromatography (PE/EA=1/1) to obtain compound 177 (1.2 g, 44%) as a light yellow solid.
LCMS: $[M+H]^+$ = 247.1

Step 2: Compound 178

Compound 177 (350 mg, 1.42 mmol, 1.0 eq) and compound 148 (456 mg, 1.56 mmol, 1.1 eq) were dissolved in a mixed solution of 1,4-dioxane (15 mL) and water (2 mL), potassium carbonate (686 mg, 4.97 mmol, 3.5 eq) and 1,1 '-bis(diphenylphosphino)ferrocene palladium dichloride (103 mg, 0.14 mmol, 0.1 eq). Nitrogen was replaced and the reaction solution was stirred at 100 °C for 16 h under nitrogen atmosphere. The reaction solution was cooled to room temperature and concentrated. The residue was purified by silica gel column chromatography (PE/EA=3/1) to obtain compound 178 (370 mg, 69%) as a reddish brown solid.
LCMS: $[M+H]^+$ = 377.2

Step 3: Compound 179

Compound 178 (370 mg, 0.98 mmol, 1.0 eq) was dissolved in tetrahydrofuran (10 mL) under nitrogen protection, cooled down to 0 °C, kept at 0 °C and NaH (118 mg, 60% content in mineral oil, 2.95 mmol, 3.0 eq) was added. The reaction solution was stirred at 25 °C for 15 min. Compound 148 (985 mg, 5.90 mmol, 6.0 eq) was then added to the reaction

solution. The reaction solution was stirred at 25°C for two hours. The reaction solution was diluted with ethyl acetate and then washed sequentially with water and saturated saline, dried over anhydrous sodium sulfate and concentrated. The crude was purified by silica gel column chromatography (PE/EA=1/1) to obtain compound 179 (400 mg, 80%) as a reddish brown solid.
LCMS: [M+H] = 507.1

Step 4: Compound 180

Compound 179 (390 mg, 0.77 mmol, 1.0 eq) was dissolved in dichloromethane (3 mL) and then trifluoroacetic acid (3 mL) was added. The reaction solution was stirred at 25°C for 2 hours. The reaction solution was concentrated and the residue was diluted with ethyl acetate. Then the pH was adjusted to be alkaline with saturated aqueous sodium carbonate solution and extracted with ethyl acetate. The combined organic phases were dried over anhydrous sodium sulfate and concentrated to obtain compound 180 (220 mg, 87%) as a yellow solid.
LCMS: $[M+H]^+$ = 323.1

Step 5: Compound 181

Compound 180 (220 mg, 0.68 mmol, 1.0 eq) was added to tetrahydrofuran (8 mL) followed by DMAP ( 8 mg, 0.07 mmol, 0.1 eq) and $Boc_2O$ (149 mg, 0.68 mmol, 1.0 eq) sequentially. The reaction solution was stirred at 25 °C for 16 hours. The reaction solution was concentrated and the residue was purified by silica gel column chromatography (PE/EA=1/1) to obtain compound 181 (220 mg, 76%) as a yellow solid.
LCMS: $[M+H]^+$ = 423.1

Step 6: Compound 182

Compound 181 (200 mg, 0.47 mmol, 1.0 eq) was dissolved in dioxane (10 mL) and then compound 6 (113 mg, 0.47 mmol, 1.0 eq), potassium phosphate monohydrate (327 mg, 1.42 mmol, 3.0 eq), X-Phos (45 mg, 0.09 mmol, 0.2 eq) and $Pd_2(dba)_3$ (43 mg, 0.05 mmol, 0.1 eq) were added. Nitrogen was replaced and the reaction solution was stirred at 90 °C for 5 h under nitrogen atmosphere. The reaction solution was cooled to room temperature and concentrated. The residue was purified by silica gel column chromatography (PE/EA=3/2) to obtain compound 182 (140 mg, 47%) as a reddish brown solid.
LCMS: [M+H] + = 625.2

Step 7: Compound A48

Compound 182 (140 mg, 0.22 mmol, 1.0 eq) was dissolved in a solvent mixture of dioxane (5 mL) and water (2 mL), then potassium carbonate (155 mg, 1.12 mmol, 5.0 eq) was added. The reaction solution was stirred at 110 °C for 3 hours. The reaction solution was cooled to room temperature and concentrated. The residue was purified by prep-HPLC (mobile phase: 0.1% formic acid/acetonitrile/water) to obtain compound A48 (13.3 mg, 11%) as a yellow solid.

LCMS: $[M+H]^+$ = 525.1
$^1H$ NMR (400 MHz, DMSO-$d_6$) δ 12.90-12.80 (m, 1H), 9.85 (s, 1H), 8.63 (d, *J* = 10.8 Hz, 1H), 8.44 (d, *J* = 3.2 Hz, 1H), 8.36-7.90 (m, 1H), 7.67 (d, *J* = 12.0 Hz, 1H), 6.54 (s, 1H), 4.59 (br s, 4H), 3.80 (s, 3H), 2.54 (s, 3H), 2.08 (s, 3H).

**Example 1-37 Preparation of Compound A49**

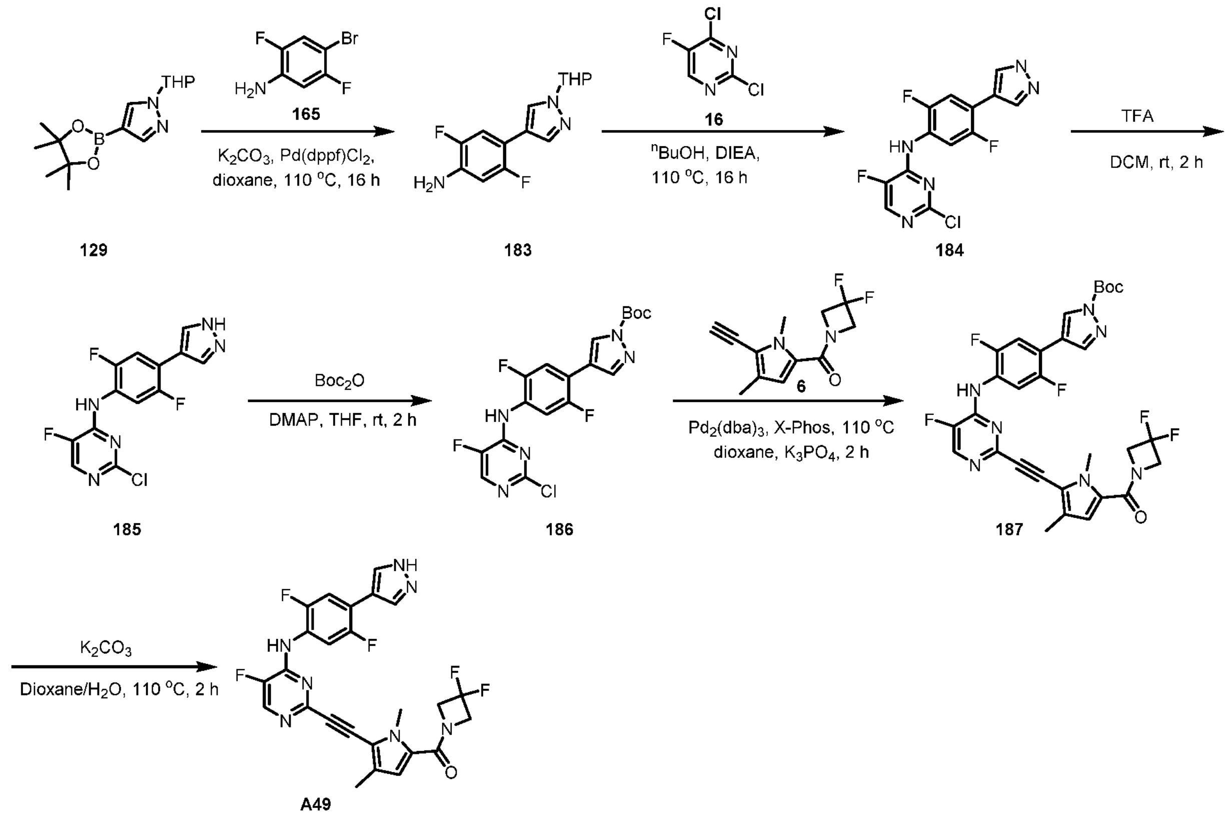


Step 1: Compound 183

Compound 129 (2.01 g, 7.3 mmol, 1.5 eq), Compound 165 (1 g, 4.8 mmol, 1 eq), Pd(dppf)$Cl_2$ (349 mg, 0.48 mmol, 0.1 eq), and Potassium Carbonate (1.99 mg, 14.4 mmol, 3.0 eq) were added to dioxane/water (20 mL/5 mL). After the addition was completed, nitrogen was displaced and the reaction mixture was reacted under nitrogen atmosphere at 90 °C for 16 h. After LC-MS detection showed that the reaction was completed, the reaction mixture was filtered through diatomaceous earth and the solids were washed with ethyl acetate. The filtrate was directly distilled under reduced pressure to remove the solvent and the residue was purified by silica gel column chromatography (petroleum ether/ethyl acetate=5/1) to obtain compound 183 (1.3 g, 97%) as a pale yellow solid.
LCMS: $[M+H]^+$ = 280.1

Step 2: Compound 184

Compound 183 (600 mg, 2.15 mmol, 1.0 eq) and Compound 16 (3.59 mg, 21.48 mmol, 10 eq) were dissolved in n-butanol (10 mL) followed by addition of diisopropylethylamine (2.78 mg, 21.48 mmol, 10.0 eq). After addition, the reaction solution was reacted under nitrogen atmosphere at 110 °C for 16 hours. The reaction solution was distilled under reduced pressure to remove the solvent and the residue was purified by silica gel column chromatography (petroleum ether/ethyl acetate) to obtain compound 184 (280 mg, 32%) as a pale yellow solid.
LCMS: $[M+H]^+$ = 410.1

Step 3: Compound 185

Compound 184 (278 mg, 0.682 mmol, 1.0 eq) was dissolved in dichloromethane (4 mL) followed by addition of trifluoroacetic acid (3 mL) to the solution. After the addition, the reaction solution was reacted at room temperature for 2 hours. The reaction solution was concentrated by distillation under reduced pressure. The residue was dissolved in dichloromethane, pH (8-9) was adjusted with saturated sodium carbonate solution, and the aqueous phase was extracted with dichloromethane (10 mL × 3). The organic phases were combined, dried over anhydrous sodium sulfate, concentrated by distillation under reduced pressure, and dried under vacuum to obtain the crude compound 185 (222 mg) as a pale yellow solid.
LCMS: $[M+H]^+$ = 326.0

Step 4: Compound 186

The crude compound 185 was dissolved in tetrahydrofuran (4 mL), followed by the addition of di-tert-butyl dicarbonate (118 mg, 0.541 mmol, 0.8 eq) and 4-dimethylaminopyridine (8 mg, 0.068 mmol, 0.1 eq). After the completion of addition, the reaction solution was reacted at room temperature for two hours. After LC-MS detection showed that the reaction was completed, the solvent was removed by distillation of the reaction solution under reduced pressure and the residue was purified by fast column chromatography (silica gel, petroleum ether/ethyl acetate 0-10%) to obtain compound 186 (180 mg, 62%) as a yellow solid.
LCMS: $[M+H]^+$ = 426.1

Step 4: Compound 187

Compound 186 (200 mg , 0.47 mmol, 1.0 eq) was dissolved in dioxane (8 mL), followed by the sequential addition of compound 6 (112 mg, 0.47 mmol, 1.0 eq), potassium phosphate monohydrate (324 mg, 1.41 mmol, 3.0 eq), X-Phos (45 mg, 0.09 mmol, 0.2 eq) and $Pd_2(dba)_3$ (46 mg, 0.05 mmol, 0.1 eq). Nitrogen was replaced and the reaction solution was stirred at 100 °C for 2 h under nitrogen atmosphere. The reaction solution was cooled to room temperature and concentrated. The crude product 187 was used directly in the next step of the reaction.
LCMS: $[M+H]^+$ = 628.2

Step 5: Compound A49

Crude compound 187 was dissolved in a solvent mixture of dioxane ( 5 mL) and water (2 mL) followed by addition of potassium carbonate (195 mg, 1.41 mmol, 3.0 eq). The reaction solution was stirred at 110 °C for 3 hours. The reaction solution was cooled to room temperature and concentrated. The residue was purified by prep-HPLC (mobile phase: 0.1% formic acid/acetonitrile/water) to obtain compound A49 (41.5 mg, two-step yield: 16%) as a white solid.

LCMS: $[M+H]^+$ = 528.1
$^1$H NMR (400 MHz, DMSO-$d_6$) δ 13.15 (br s, 1H), 9.73 (s, 1H), 8.45 (d, *J* = 3.2 Hz, 1H), 8.28-7.98 (m, 2H), 7.77 (dd, *J* = 11.6, 7.2 Hz, 1H), 7.61 (dd, *J* = 12.0, 6.8 Hz, 1H), 6.55 (s, 1H), 4.60 (br s, 4H), 3.82 (s, 3H), 2.11 (s, 3H).

**Example 1-38 Preparation of Compound A50**

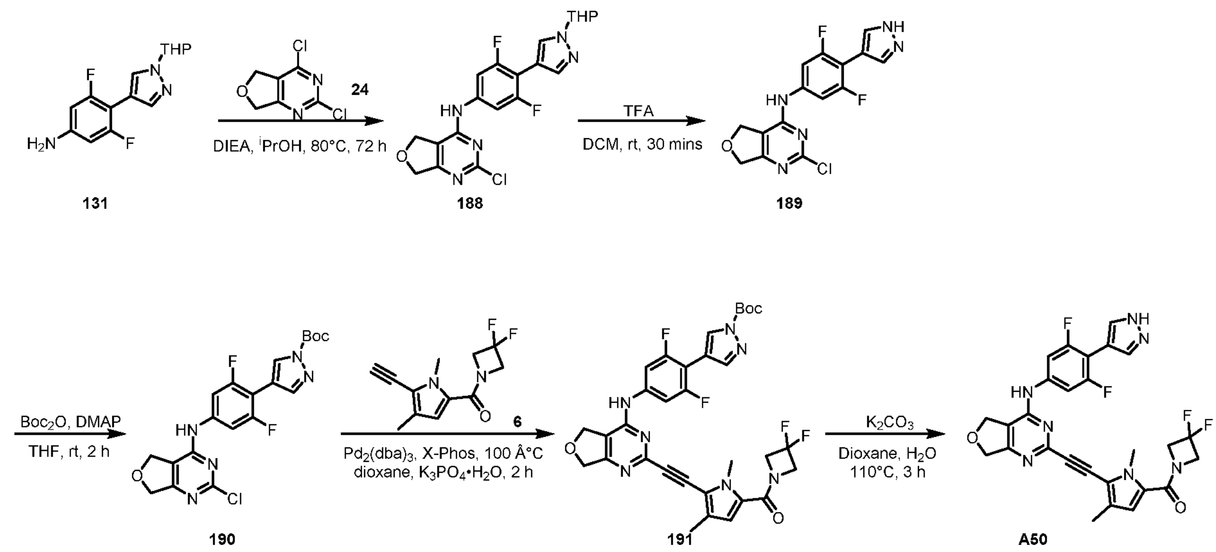


131 188 189

190 191 A50

Step 1: Compound 188

Compound 131 (400 mg, 1.43 mmol, 1.0 eq), compound 24 (383 mg, 2.01 mmol, 1.4 eq) and diisopropylethylamine (740 mg, 5.73 mmol, 4.0 eq) were dissolved in isopropanol (8 mL). The reaction solution was stirred at 80 °C for 72 h. The reaction solution was cooled to room temperature and the solvent was removed under reduced pressure. The

residue was purified by fast silica gel column chromatography (PE/EA = 1/1) to obtain compound 188 (443 mg, 71%) as a yellow solid.
LCMS: [M+H] + = 434.1

Step 2: Compound 189

Compound 188 (440 mg, 1.01 mmol, 1.0 eq) was dissolved in dichloromethane (5 mL) and then trifluoroacetic acid (10 mL) was added. The reaction solution was stirred at 25 °C for 30 min. After the reaction solution was concentrated in vacuum, water (15 mL) was added to the residue, the pH was adjusted to 8 with saturated aqueous sodium carbonate solution, and extracted with ethyl acetate (20 mL × 3). The combined organic phases were washed with saturated saline, dried over anhydrous sodium sulfate, and concentrated to obtain compound 189 (350 mg, 98%) as a yellow solid.
LCMS: $[M+H]^+$ = 350.1

Step 3: Compound 190

Compound 189 (350 mg, 1.00 mmol, 1.0 eq) was dissolved in tetrahydrofuran (10 mL), then 4-dimethylaminopyridine (12 mg, 0.100 mmol, 0.1 eq) and di-tert-butyl dicarbonate (218 mg, 1.00 mmol, 1.0 eq) were added. The reaction solution was stirred at 25°C for 2 hours. After the reaction was completed, the reaction solution was concentrated. The residue was purified by fast silica gel column chromatography (PE/EA = 3/2) to obtain compound 190 (315 mg, 69%) as a yellow solid.
LCMS: [M+H] + = 450.1

Step 4: Compound 191

Compound 190 (300 mg, 0.689 mmol, 1.0 eq), Compound 6 (164 mg, 0.689 mmol, 1.0 eq), and Potassium phosphate monohydrate (476 mg, 2.07 mmol, 3.0 eq) were dissolved in dioxane (5 mL), followed by a rapid addition of X-phos (66 mg, 0.138 mmol, 0.2 eq) and $Pd_2(dba)_3$ (63 mg, 0.069 mmol, 0.1 eq). After displacing nitrogen three times, the reaction solution was stirred at 100 °C for 2 h under nitrogen atmosphere. The reaction solution was concentrated and the residue was purified by fast silica gel column chromatography (PE/EA = 1/1) to obtain compound 191 (200 mg, 44%) as a brown solid.
LCMS: $[M+H]^+$ = 652.2

Step 5: Compound A50

Compound 191 (200 mg, 0.307 mmol, 1.0 eq) and potassium carbonate (127 mg, 0.921 mmol, 3.0 eq) were dissolved in a mixture of solvents (dioxane/water = 2/1, 12 mL). The reaction solution was stirred at 110 °C for 3 hours. The reaction solution was cooled to room temperature and concentrated. The residue was purified by pre-HPLC (mobile phase: 0.1% formic acid/acetonitrile/water) to obtain A50 (16.4 mg, 9% ) as a yellow solid.

LCMS: $[M+H]^+$ = 552.2
$^1$H NMR (400 MHz, DMSO-$d_6$) δ 13.19 (br. s, 1H), 9.60 (s, 1H), 8.20-7.82 (m, 2H), 7.78-7.69 (m, 2H), 6.60 (s, 1H), 5.08 (s, 2H), 4.91 (s, 2H), 4.63 (br. s, 4H), 3.92 (s, 3H), 2.20 (s, 3H).

**Example 1-39 Preparation of Compound A51**

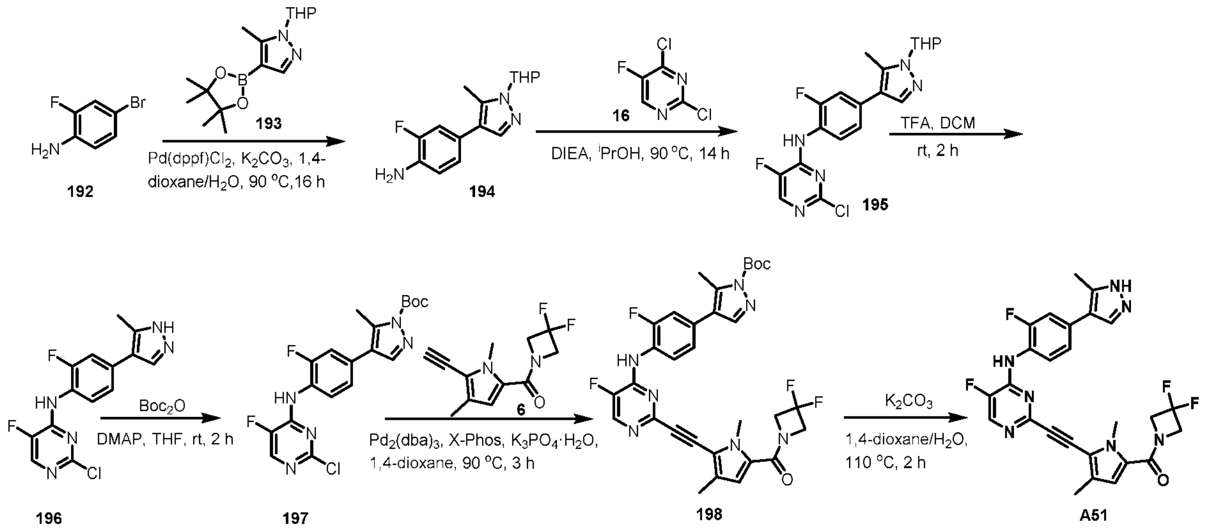


Step 1: Compound 194

Compound 192 (4.6 g, 24.21 mmol, 1.0 eq) and compound 193 (8.4 g, 29.05 mmol, 1.2 eq) were dissolved in a mixture of 1,4-dioxane (40 mL) and water (8 mL), followed by addition of potassium carbonate (10.0 g, 72.63 mmol, 3.0 eq) and 1,1 '-bis(diphenylphosphino)ferrocene palladium dichloride (526 mg, 0.72 mmol, 0.03 eq). Nitrogen was replaced and the reaction solution was stirred under nitrogen atmosphere at 90 °C for 16 h. The reaction solution was cooled to room temperature, passed through diatomaceous earth and the filtrate was concentrated to obtain the residue. The residue was purified by silica gel column chromatography (PE/EA=4/1) to obtain compound 194 (5.69 g, 85%) as a yellow solid.
LCMS: $[M+H]^+$ = 276.1

Step 2: Compound 195

Compound 194 (1.6 g, 5.81 mmol, 1.0 eq) was dissolved in isopropanol (20 mL), compound 16 (1.46 mg, 8.72 mmol, 1.5 eq) was added together with diisopropylethylamine (3.74 g, 29.05 mmol, 5.0 eq), and the reaction mixture was stirred at 90 °C for 14 h. LC-MS detection showed that the reaction was completed, the reaction mixture was concentrated to obtain the residue, the residue was purified by rapid column chromatography on silica gel (petroleum ether/ethyl acetate=80/20) to obtain compound 195 (2.27 g, 96%) as a yellow solid.
LCMS: $[M+H]^+$ = 406.1

Step 3: Compound 196

Compound 195 (2.2 g, 5.43 mmol, 1.0 eq) was dissolved in dichloromethane (18 mL) and then trifluoroacetic acid (30 mL) was added. The reaction solution was stirred at room temperature for 2 hours. The reaction solution was concentrated to obtain the residue, the residue was adjusted to be alkaline pH with saturated aqueous sodium carbonate solution, a large amount of solid was precipitated, the solid was collected by filtration and dried to obtain the crude compound 196 (1.34 g) as a yellow solid.
LCMS: $[M+H]^+$ = 322.2

Step 4: Compound 197

Compound 196 (1.34 g, 4.2 mmol, 1.0 eq) was added to tetrahydrofuran (10 mL) followed by DMAP (102 mg, 0.84 mmol, 0.2 eq) and $Boc_2O$ (916 mg, 4.2 mmol, 1.0 eq) sequentially. The reaction solution was stirred at room temperature for 2 hours. The reaction solution was diluted with ethyl acetate and then washed sequentially with water and saturated saline, dried over anhydrous sodium sulfate and concentrated. The residue was purified by silica gel column chromatography (petroleum ether/ethyl acetate=4/1) to obtain compound 197 (1.4 g, 79%) as a yellow solid.
LCMS: $[M+H]^+$ = 422.2

Step 5: Compound 198

Compound 197 (500 mg, 1.18 mmol, 1.0 eq) was dissolved in the solvent dioxane (12 mL), then compound 6 (338

mg, 1.42 mmol, 1.2 eq), potassium phosphate monohydrate (819 mg, 3.56 mmol, 3.0 eq), X-Phos (109 mg, 0.23 mmol, 0.2 eq) and $Pd_2(dba)_3$ (155 mg, 0.17 mmol, 0.15 eq) were added. The reaction solution was stirred at 90 °C for 3 h under nitrogen atmosphere after dosing. Cooled to room temperature, the reaction solution was diluted with ethyl acetate, passed through the diatomaceous earth, the filtrate was concentrated to obtain the residue, the residue crude 198 was directly put into the next step.
LCMS: $[M+H]^+$ =624.5

Step 6: Compound A51

Crude compound 198 (150 mg, 0.24 mmol, 1.0 eq) was dissolved in a solvent mixture of dioxane (6 mL) and water (2 mL), then potassium carbonate (331 mg, 2.4 mmol, 10.0 eq) was added. The reaction solution was stirred at 110 °C for 2 hours. The reaction solution was diluted with ethyl acetate and then washed sequentially with water and saturated saline, dried over anhydrous sodium sulfate and concentrated to obtain the residue. The residue was purified by silica gel column chromatography (petroleum ether/ethyl acetate=4/1) to obtain the compound as a crude product, the crude product was purified by pulping with a mixed solvent (petroleum ether/ethyl acetate=4/1) to obtain compound A51 (100.5 mg, 80%) as a yellow solid.

LCMS: $[M+H]^+$ =524.1
$^1H$ NMR (400 MHz, DMSO-$d_6$) δ 12.73 (s, 1H), 9.66 (s, 1H), 8.40 (d, *J* = 3.6 Hz, 1H), 7.75 (br. s, 1H), 7.51 (t, *J* = 8.4 Hz, 1H), 7.39 (d, *J* = 12.0 Hz, 1H), 7.32 (d, *J* = 8.0 Hz, 1H), 6.54 (s, 1H), 4.59 (br. s, 4H), 3.81 (s, 3H), 2.41 (s, 3H), 2.09 (s, 3H).

**Example 1-40 Preparation of Compound A52**

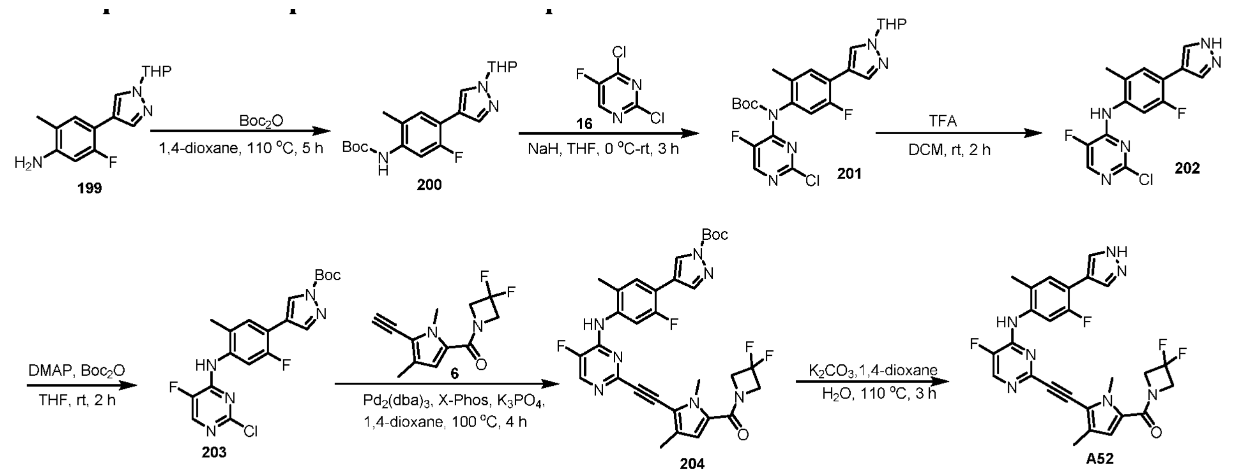


Step 1: Compound 200

Compound 199 (500 mg, 1.82 mmol, 1.0 eq) was dissolved in dioxane (10 mL) and then $Boc_2O$ (1.98 g, 9.1 mmol, 5.0 eq) was added. The reaction solution was stirred at 110 °C for 5 hours. The reaction solution was cooled to room temperature and concentrated. The residue was purified by silica gel column chromatography (PE/EA=10/1) to obtain compound 200 (650 mg, 95%) as a yellow solid.
LCMS: $[M+H]^+$ = 376.2

Step 2: Compound 201

Compound 200 (650 mg, 1.58 mmol, 1.0 eq) was dissolved in tetrahydrofuran ( 10 mL), and the solution was stirred for five minutes in an ice-water bath under the protection of $N_2$, NaH (277 mg, 60% content in mineral oil, 6.93 mmol, 4.0 eq) was added. The mixture was continued to be stirred in an ice-water bath for one hour and compound 3 (1.45 g, 8.7 mmol, 5.0 eq) was added. The reaction solution was stirred at room temperature for 2 hours. The reaction solution was poured into water and extracted with ethyl acetate. The ethyl acetate phases were combined, washed with saturated brine, dried over anhydrous sodium sulfate and concentrated. The residue was purified by silica gel column chromatography

(PE/EA=4/1) to obtain compound 201 (850 mg, 97%) as a yellow solid.
LCMS: $[M+H]^+$ = 506.1

Step 3: Compound 202

Compound 201 (850 mg, 1.68 mmol, 1.0 eq) was dissolved in dichloromethane (5 mL) and then trifluoroacetic acid (5 mL) was added. The reaction solution was stirred at room temperature for 2 hours. The reaction solution was concentrated and the residue was diluted with dichloromethane and the pH was adjusted to be alkaline with saturated aqueous sodium carbonate solution. The resulting reaction mixture was filtered and the solid was collected. The solid was lyophilized to obtain compound 202 (440 mg, 81%) as a yellow solid.
LCMS: $[M+H]^+$ = 321.9

Step 4: Compound 203

Compound 202 (440 mg, 1.37 mmol, 1.0 eq) was added to tetrahydrofuran (10 mL), followed by DMAP ( 8 mg, 0.07 mmol, 0.05eq) and $Boc_2O$ (299 mg, 1.37 mmol, 1.0 eq) sequentially. The reaction solution was stirred at room temperature for 2 hours. The reaction solution was concentrated and the residue was purified by silica gel column chromatography (PE/EA=5/1) to obtain compound 203 (550 mg, 95%) as a white solid.
LCMS: $[M+H]^+$ = 422.1

Step 5: Compound 204

Compound 203 ( 500 mg , 1.18 mmol, 1.0 eq) was dissolved in dioxane (8 mL) and then compound 7 (282 mg, 1.18 mmol, 1.0 eq), potassium phosphate (750 mg, 3.54 mmol, 3.0 eq), X-Phos (115 mg, 0.24 mmol, 0.2 eq) and $Pd_2(dba)_3$ (108 mg, 0.12 mmol, 0.1 eq) were added in order. Nitrogen was replaced and the reaction solution was stirred at 100 °C for 4 h under nitrogen atmosphere. The reaction solution was cooled to room temperature, filtered, and the filtrate was concentrated to obtain the crude product 204 which was used directly in the next step of the reaction.
LCMS: $[M+H]^+$ = 624.2

Step 6: Compound A52

Crude compound 204 was dissolved in a solvent mixture of dioxane ( 5 mL) and water (2 mL), then potassium carbonate (489 mg, 3.54 mmol, 3.0 eq) was added. The reaction solution was stirred at 110 °C for 3 hours. The reaction solution was cooled to room temperature, the reaction solution was diluted with ethyl acetate, washed with water and the aqueous phase was extracted with ethyl acetate. The ethyl acetate phases were combined, washed with saturated brine, dried over anhydrous sodium sulfate and concentrated. The residue was purified by silica gel column chromatography (PE/EA=3/1) to obtain the crude product. The crude was dissolved in ethyl acetate (30 mL) and stirred for 14 h. The obtained was filtered and the filter cake was collected. The filter cake was dissolved in acetonitrile/water (150 mL/350 mL) and lyophilized to obtain compound A52 (237.5 mg, two-step yield: 38%) as a yellow solid.

LCMS: $[M+H]^+$ = 524.2
$^1H$ NMR (400 MHz, DMSO-$d_6$) $\delta$ 13.09 (s, 1H), 9.41 (s, 1H), 8.38 (d, *J* = 3.6 Hz, 1H), 8.14 (br. s, 1H), 7.96 (br. s, 1H), 7.66 (d, *J* = 8.8 Hz, 1H), 7.35 (d, *J* = 12.4 Hz, 1H), 6.54 (s, 1H), 4.59 (br. s, 4H), 3.81 (s, 3H), 2.23 (s, 3H), 2.09 (s, 3H).

**Example 1-41 Preparation of Compound A53**

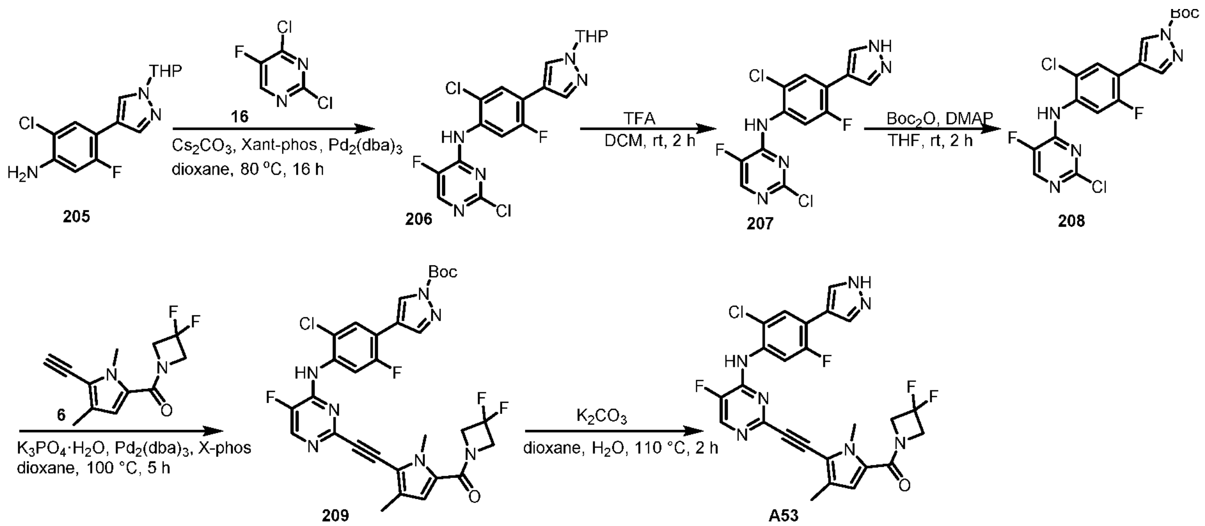


205 206 207 208 209 A53

Step 1: Compound 206

Compound 205 (500 mg, 1.69 mmol, 1.0 eq) was dissolved in dioxane (10 mL), followed by rapid addition of Compound 16 (565 mg, 3.38 mmol, 2.0 eq), cesium carbonate (1.9 g, 3.38 mmol, 2.0 eq) , Xant-Phos (196 mg, 0.338 mmol, 0.2 eq) and $Pd_2(dba)_3$ (155 mg, 0.169 mmol, 0.1 eq). After three air exchanges with nitrogen, the reaction solution was stirred under nitrogen atmosphere at 80 °C for 16 h. The reaction solution was cooled to room temperature and concentrated, and the residue was purified by silica gel column chromatography (petroleum ether/ethyl acetate=4/1) to obtain compound 206 (600 mg, 83 %) as a yellow solid.
LCMS: $[M+H]^+$ = 426.0

Step 2: Compound 207

Compound 206 (300 mg, 0.66 mmol, 1.0 eq) was dissolved in dichloromethane (5 mL) and then TFA (5 mL) was added. The reaction solution was stirred at room temperature for 2 hours. The reaction solution was concentrated, the residue was dissolved in ethyl acetate, the resulting solution was adjusted to pH 7 with aqueous sodium carbonate solution, and the aqueous phase was extracted with ethyl acetate (50 mL × 3). The combined organic phases were washed with saturated brine, dried over anhydrous sodium sulfate and concentrated to obtain compound 207 (230 mg, 95%) as a yellow solid.
LCMS: $[M+H]^+$ = 342.1

Step 3: Compound 208

Compound 207 (230 mg, 0.67 mmol, 1.0 eq) was dissolved in tetrahydrofuran (5 mL), then $Boc_2O$ (147 mg, 0.67 mmol, 1.0 eq) and DMAP (4 mg. 0.034 mmol, 0.05 eq) were added. The reaction solution was stirred at room temperature for 2 hours. The reaction solution was diluted with ethyl acetate, then washed with water, washed with saturated brine, dried over anhydrous sodium sulfate and concentrated, and the residue was purified by silica gel column chromatography (Petroleum Ether/Ethyl Acetate=4/1) to obtain compound 208 (280 mg, 95 %) as a yellow solid.
LCMS: $[M+H]^+$= 442.0

Step 4: Compound 209

Compound 208 (200 mg , 0.45 mmol, 1.0 eq) and Compound 6 (107 mg, 0.45 mmol, 1.0 eq) were dissolved in dioxane (15 mL), followed by the sequential addition of potassium phosphate monohydrate (364 mg, 1.6 mmol, 3.5 eq), X-Phos (43 mg, 0.09 mmol, 0.2 eq) and $Pd_2(dba)_3$ (41 mg, 0.045 mmol, 0.1 eq). Nitrogen was replaced and the reaction solution was stirred at 100 °C for 5 h under nitrogen atmosphere. The reaction solution was cooled to room temperature and concentrated. The crude product 209 was used directly in the next step of the reaction.
LCMS: $[M+H]^+$ = 644.1

Step 5: Compound A53

Crude compound 209 was dissolved in a solvent mixture of dioxane (15 mL) and water (3 mL), then potassium carbonate (187 mg, 1.4 mmol, 3.0 eq) was added. The reaction solution was stirred at 110 °C for 2 hours. The reaction solution was cooled to room temperature and concentrated. The residue was purified by silica gel column chromatography (petroleum ether/ethyl acetate=1/5) to obtain the crude product. The crude was purified by prep-HPLC (mobile phase: 0.1% formic acid/acetonitrile/water) to obtain compound A53 (10.1 mg, two-step yield: 4%) as a yellow solid.

LCMS: $[M+H]^+$ = 544.1
$^1H$ NMR (400 MHz, DMSO-$d_6$) δ 13.19 (br. s, 1H), 9.59 (s, 1H), 8.44 (d, *J* = 3.2 Hz, 1H), 8.27 (br. s, 1H), 8.03 (br. s, 1H), 7.99 (d, *J* = 8.0 Hz, 1H), 7.72 (d, *J* = 12.0 Hz, 1H), 6.54 (s, 1H), 4.59 (br. s, 4H), 3.81 (s, 3H), 2.10 (s, 3H).

**Example 1-42 Preparation of Compound A54**

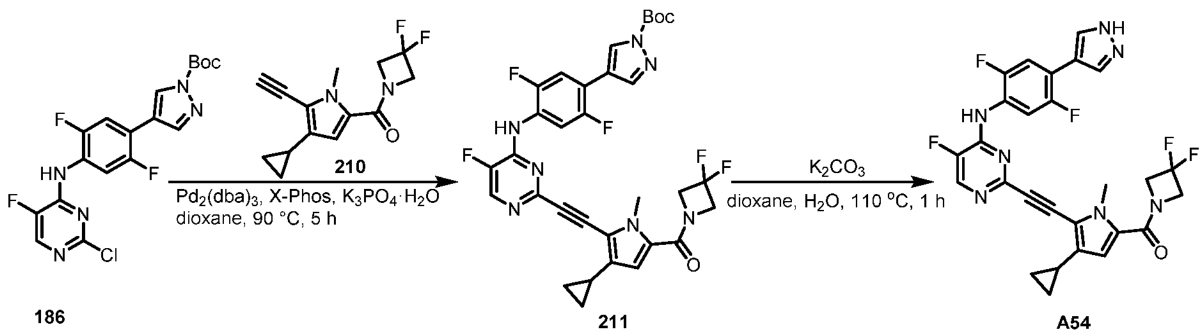


186 211 A54

Step 1: Compound 211

Compound 186 (200 mg , 0.47 mmol, 1.0 eq) was dissolved in dioxane (8 mL) and then compound 210 (124 mg, 0.47 mmol, 1.0 eq), potassium phosphate monohydrate (379 mg, 1.64 mmol, 3.5 eq), X-Phos (45 mg, 0.094 mmol, 0.2 eq) and $Pd_2(dba)_3$ (43 mg, 0.047 mmol, 0.1 eq) were added in order. Nitrogen was replaced and the reaction solution was stirred at 90 °C for 5 h under nitrogen atmosphere. The reaction solution was cooled to room temperature and concentrated. The crude product 211 was used directly in the next step of the reaction.
LCMS: $[M+H]^+$ = 654.0

Step 2: Compound A54

Crude compound 211 was dissolved in a solvent mixture of dioxane (5 mL) and water (1 mL), then potassium carbonate (195 mg, 1.4 mmol, 3.0 eq) was added. The reaction solution was stirred at 110 °C for 1 hour. The reaction solution was cooled to room temperature and concentrated. The residue was purified by prep-HPLC (mobile phase: 0.1% formic acid/acetonitrile/water) to obtain compound A54 (56.2 mg, two-step yield: 22%) as a yellow solid.

LCMS: $[M+H]^+$ = 554.0
$^1H$ NMR (400 MHz, DMSO-$d_6$) δ 13.16 (br. s, 1H), 9.72 (s, 1H), 8.44 (d, *J* = 3.6 Hz, 1H), 8.22 (br. s, 1H), 7.99 (br. s, 1H), 7.79-7.72 (m, 1H), 7.65-7.57 (m, 1H), 6.31 (s, 1H), 4.58 (br. s, 4H), 3.80 (s, 3H), 1.87-1.71 (m, 1H), 0.95-0.77 (m, 2H), 0.72-0.62 (m, 2H).

**Example 1-43 Preparation of Compound A55**

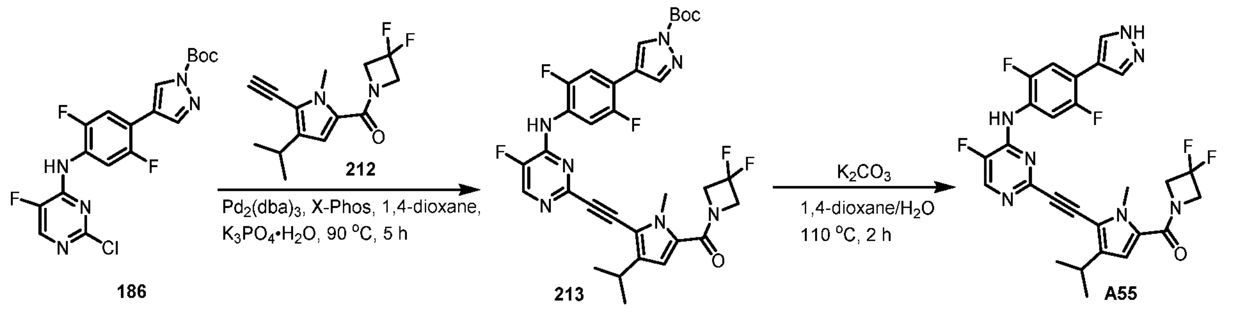


Step 1: Compound 213

Compound 186 (200 mg , 0.47 mmol, 1.0 eq) was dissolved in dioxane (8 mL) and then compound 212 (156 mg, 0.47 mmol, 1.0 eq), potassium phosphate monohydrate (299 mg, 1.41 mmol, 3.0 eq), X-Phos (48 mg, 0.1 mmol, 0.2 eq) and $Pd_2(dba)_3$ (46 mg, 0.05 mmol, 0.1 eq) were added in order. Nitrogen was replaced and the reaction solution was stirred at 90 °C for 5 h under nitrogen atmosphere. The reaction solution was cooled to room temperature and concentrated. The crude product 213 was used directly in the next step of the reaction.
LCMS: [M+H] + = 656.2

Step 2: Compound A55

Crude compound 213 was dissolved in a solvent mixture of dioxane (5 mL) and water (2 mL) followed by addition of potassium carbonate (195 mg, 1.41 mmol, 3.0 eq). The reaction solution was stirred at 110 °C for 2 hours. The reaction solution was cooled to room temperature and concentrated. The residue was purified by prep-HPLC (mobile phase: 0.1% formic acid/acetonitrile/water) to obtain compound A55 (104.4 mg, two-step yield: 40%) as a white solid.

LCMS: [M+H] + = 556.2
$^1$H NMR (400 MHz, DMSO-$d_6$) δ 13.18 (br. s, 1H), 9.73 (s, 1H), 8.47 (d, *J* = 3.2 Hz, 1H), 8.24 (br. s, 1H), 8.04 (br. s, 1H), 7.78 (dd, *J* = 11.6, 6.8 Hz, 1H), 7.66 (dd, *J* = 12.0, 6.8 Hz, 1H), 6.59 (s, 1H), 4.63 (br. s, 4H), 3.84 (s, 3H), 3.00-2.88 (m, 1H), 1.21 (d, *J* = 6.8 Hz, 6H).

**Example 1-44 Preparation of Compound A56**

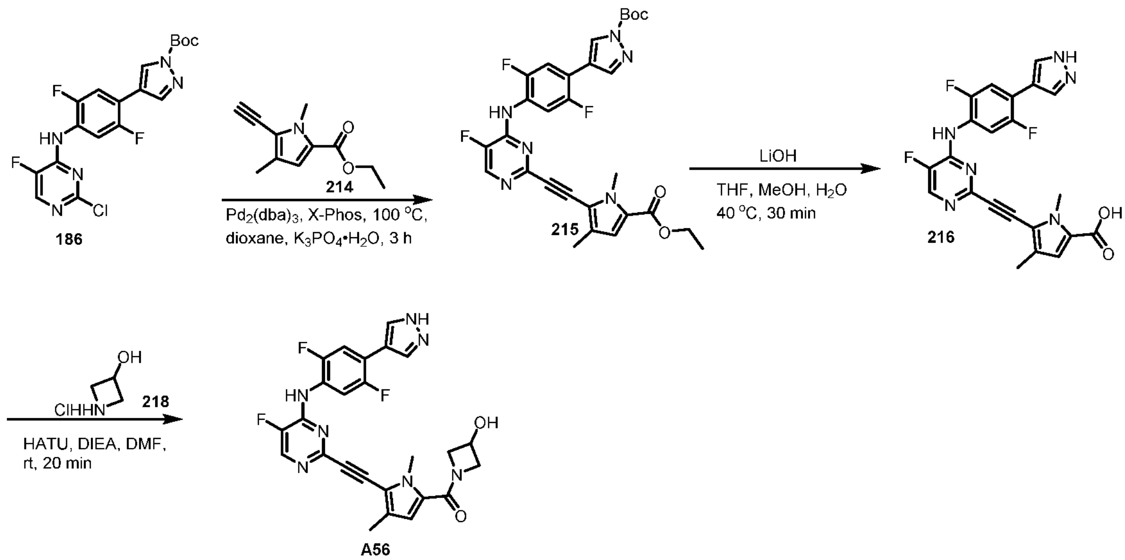


Step 1: Compound 215

Compound 186 (1.0 g, 2.35 mmol, 1.0 eq), Compound 214 (384 mg, 2.35 mmol, 1.0 eq), and Potassium

Phosphate (1.49 g, 7.1 mmol, 3.0 eq) were added to dioxane (15 mL), followed by rapid addition of X-phos (42 mg, 0.24 mmol, 0.1 eq) and $Pd_2(dba)_3$ (114 mg, 0.24 mmol, 0.1 eq). After three nitrogen replacements, the reaction mixture was stirred at 100 °C for 3 h under nitrogen atmosphere. The reaction solution was cooled to room temperature and concentrated. The residue was purified by fast silica gel column chromatography (petroleum ether/ethyl acetate = 2/1) to obtain compound 215 (900 mg, 66%) as a tan solid.
LCMS: $[M+H]^+$ = 581.2

Step 2: Compound 216

Compound 215 (900 mg, 1.55 mmol, 1.0 eq) was dissolved in a mixed solvent (tetrahydrofuran/methanol/water = 1/1/2, 20 mL) and then lithium hydroxide (372 mg, 15.5 mmol, 10.0 eq) was added. The reaction solution was allowed to react at 40 °C for 30 min. After the reaction was completed, the reaction solution was adjusted to pH 3 with saturated aqueous citric acid solution and extracted with ethyl acetate (100 mL × 3) . The combined organic phases were washed with saturated brine, dried over anhydrous sodium sulfate, and concentrated to obtain the crude compound 216 (520 mg, 74%) as a yellow solid.
LCMS: $[M+H]^+$ = 453.1

Step 3: Compound A56

Compound 216 (150 mg, 0.33 mmol, 1.0 eq), Compound 218 (44 mg, 0.40 mmol, 1.2 eq), and HATU (152 mg, 0.40 mmol, 1.2 eq) were dissolved in anhydrous DMF (10 mL), followed by addition of diisopropylethylamine (170 mg, 1.32 mmol, 4.0 eq) at 0°C. The reaction solution was stirred at room temperature for 20 min. The reaction solution was diluted with ethyl acetate, washed with water, and the aqueous phase was extracted with ethyl acetate (20 mL × 3). The combined organic phases were washed with saturated saline, dried over anhydrous sodium sulfate, and concentrated. The residue was purified by prep-HPLC (mobile phase: 0.1% formic acid/acetonitrile/water) to obtain A56 (63.8 mg, 38%) as a yellow solid.

LCMS: $[M+H]^+$ = 508.1
$^1H$ NMR (400 MHz, DMSO-$d_6$) δ 13.19 (br. s, 1H), 9.70 (s, 1H), 8.44 (d, $J$ = 3.6 Hz, 1H), 8.14 (br. s, 2H), 7.76 (dd, $J$ = 11.6, 6.8 Hz, 1H), 7.61 (dd, $J$ = 12.0, 6.8 Hz, 1H), 6.41 (s, 1H), 5.73 (br. s, 1H), 4.47 (br. s, 2H), 4.18 (br. s, 1H), 4.00 (br. s, 1H), 3.81 (s, 3H), 3.71 (br. s, 1H),2.10 (s, 3H).

**Example 1-45 Preparation of Compound A57**

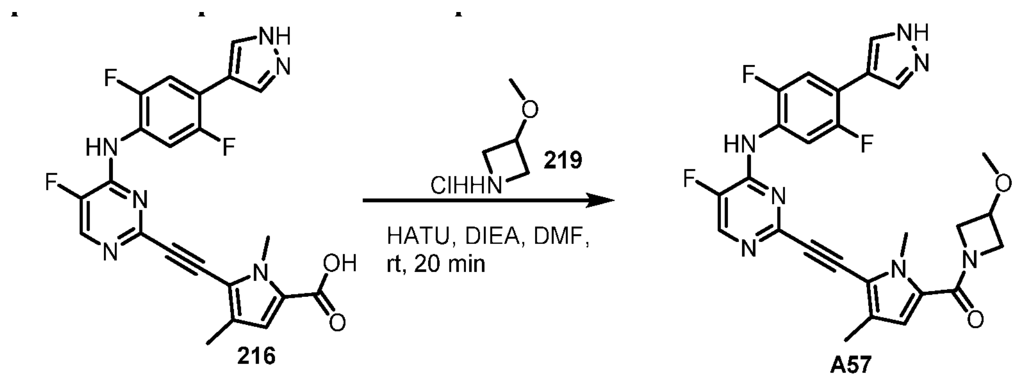

216
A57

Step 1: Compound A57

Compound 216 (150 mg, 0.33 mmol, 1.0 eq), Compound 219 (49 mg, 0.40 mmol, 1.2 eq) and HATU (152 mg, 0.40 mmol, 1.2 eq) were dissolved in anhydrous DMF (10 mL) followed by addition of diisopropylethylamine (170 mg, 1.32 mmol, 4.0 eq) at 0°C. The reaction solution was stirred at 25 °C for 20 min. The reaction solution was diluted with ethyl acetate, washed with water, and the aqueous phase was extracted with ethyl acetate (20 mL × 3). The combined organic phases were washed with saturated saline, dried over anhydrous sodium sulfate, and concentrated. The residue was purified by prep-HPLC (mobile phase: 0.1% formic acid/acetonitrile/water) to obtain A57 (50.1 mg, 29%) as a yellow solid.

LCMS: $[M+H]^+$ = 522.1
$^1$H NMR (400 MHz, DMSO-$d_6$) δ 9.70 (s, 1H), 8.44 (d, *J* = 3.6 Hz, 1H), 8.12 (br. s, 2H), 7.76 (dd, *J* = 11.6, 7.2 Hz, 1H), 7.61 (dd, *J* = 12.0, 6.8 Hz, 1H), 6.45 (s, 1H), 4.44 (br. s, 1H), 4.27-4.15 (m, 4H), 3.81 (s, 3H), 3.22 (s, 3H), 2.10 (s, 3H).

**Example 1-46 Preparation of Compound A58**

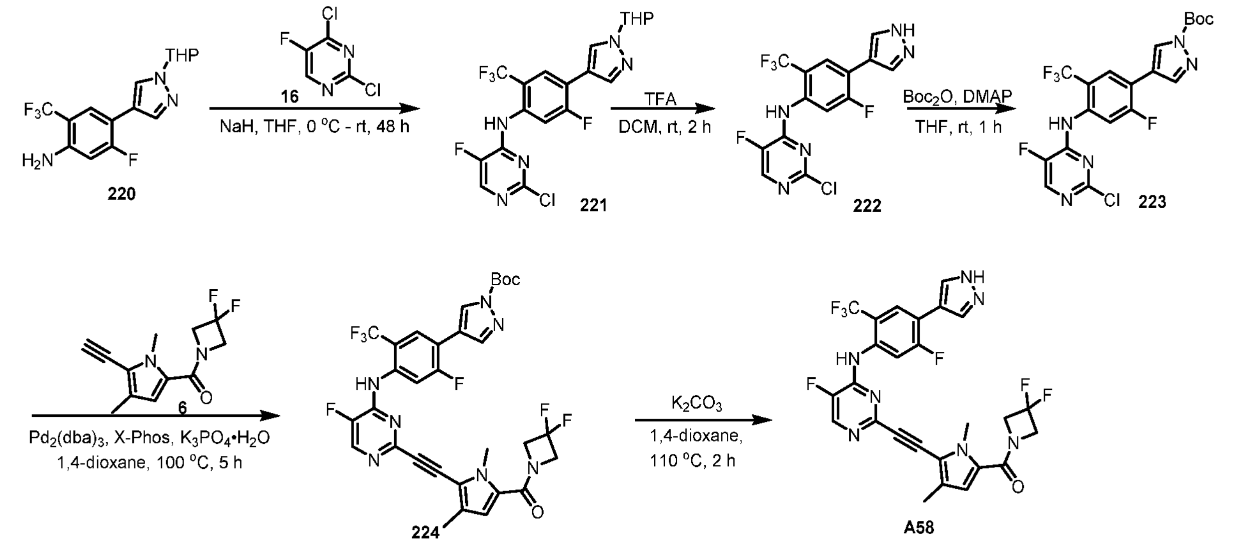


Step 1: Compound 221

Compound 220 (350 mg, 1.06 mmol, 1.0 eq) was dissolved in tetrahydrofuran (5 mL), then cooled to 0 °C under nitrogen protection and NaH (255 mg, 60% in mineral oil, 6.38 mmol, 6.0 eq) was added at 0 °C. The reaction solution was warmed to room temperature and stirred at room temperature for 15 min. Compound 2 (1.77 g, 10.63 mmol, 10.0 eq) was added to the reaction solution. The reaction solution was stirred at room temperature for 48 hours. The reaction solution was diluted with ethyl acetate and then washed sequentially with water and saturated saline, dried over anhydrous sodium sulfate and concentrated. The crude product was purified by silica gel column chromatography (PE/EA=3/1) to obtain compound 221 (300 mg, 61%) as a yellow solid.
LCMS: [M+H] = 460.0

Step 2: Compound 222

Compound 221 (300 mg, 0.65 mmol, 1.0 eq) was dissolved in dichloromethane (5 mL) and then trifluoroacetic acid (5 mL) was added. The reaction solution was stirred at 25°C for 2 hours. The reaction solution was concentrated and the residue was diluted with ethyl acetate and extracted with ethyl acetate after adjusting the pH to be alkaline with saturated aqueous sodium carbonate solution. The combined organic phases were dried over anhydrous sodium sulfate and concentrated to obtain compound 222 (230 mg, 94%) as a reddish brown solid.
LCMS: $[M+H]^+$ = 376.1

Step 3: Compound 223

Compound 222 (230 mg, 0.61 mmol, 1.0 eq) was added to tetrahydrofuran (5 mL) followed by DMAP (7 mg, 0.06 mmol, 0.1 eq) and $Boc_2O$ (134 mg, 0.61 mmol, 1.0 eq) sequentially. The reaction solution was stirred at 25 °C for 1 hour. The reaction solution was concentrated and the residue was purified by silica gel column chromatography (PE/EA=3/1) to obtain compound 223 (210 mg, 72%) as a white solid.
LCMS: $[M+H]^+$ = 475.2

Step 4: Compound 224

Compound 223 (200 mg , 0.42 mmol, 1.0 eq) was dissolved in dioxane (8 mL) and then compound 6 (100 mg, 0.42

mmol, 1.0 eq), potassium phosphate monohydrate (290 mg, 1.26 mmol, 3.0 eq), X-Phos (40 mg, 0.08 mmol, 0.2 eq), and $Pd_2(dba)_3$ (38 mg, 0.04 mmol, 0.1 eq) were added sequentially. Nitrogen was replaced and the reaction solution was stirred under nitrogen atmosphere at 100 °C for 5 hours. After completion of the reaction, the crude product 224 was used directly in the next step.
LCMS: [M+H] = 678.0

Step 5: Compound A58

Water (2.5 mL) was added to the reaction solution of 224 from the previous step followed by potassium carbonate (580 mg, 4.2 mmol, 10.0 eq). The reaction solution was stirred at 110 °C for 2 hours. The reaction solution was cooled to room temperature and concentrated. The residue was initially purified by silica gel column chromatography (PE/EA=1/1) to obtain the crude product (110 mg). The crude was pulped with a solvent mixture (PE/EA=1/1, 10 mL) for 16 hours. The mixed solution was filtered to obtain compound A58 (62 mg, 26%) as a yellow solid.

LCMS: $[M+H]^+$ = 578.1
$^1$H NMR (400 MHz, DMSO-$d_6$) δ 13.25 (br. s, 1H), 9.69 (s, 1H), 8.42 (d, $J$ = 3.6 Hz, 1H), 8.36 (br. s, 1H), 8.14 (d, $J$ = 7.6 Hz, 1H), 8.10 (br. s, 1H), 7.67 (d, $J$ = 11.6 Hz, 1H), 6.53 (s, 1H), 4.58 (br. s, 4H), 3.78 (s, 3H), 2.07 (s, 3H).

**Example 1-47 Preparation of Compound A59**

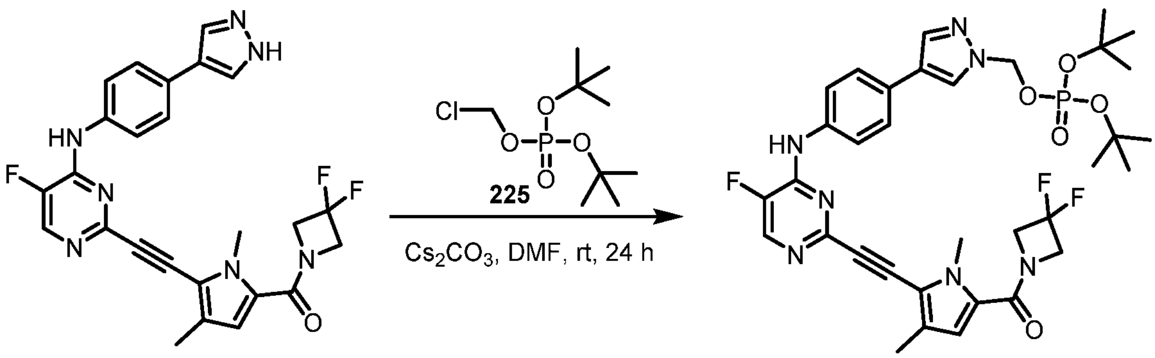


A42 A59

Step 1: Compound A59

Compound A42 (250 mg, 0.508 mmol, 1.0 eq) and compound 225 (210 mg, 0.814 mmol, 1.6 eq) were dissolved in DMF (6 mL), then cesium carbonate (496 mg, 1.524 mmol, 3.0 eq) was added, and then the reaction solution was stirred under nitrogen atmosphere at 25°C for 24 h. After LC-MS showed that the reaction was completed, the reaction mixture was diluted with water and extracted with ethyl acetate, the organic phases were combined, washed with saturated brine, dried over anhydrous sodium sulfate, and concentrated, and the residue was purified by silica gel column chromatography (petroleum ether/ethyl acetate=1:2) to obtain the crude compound, the crude was purified by pre-HPLC (mobile phase: 0.1% $NH_4HCO_3$/water/acetonitrile) to obtain compound A59 (50.4 mg, 14%) as a white solid.

LCMS: $[M+H]^+$ = 714.2
$^1$H NMR (400 MHz, DMSO-$d_6$) δ 9.80 (s, 1H), 8.40 (d, $J$ = 3.6 Hz, 1H), 8.31 (s, 1H), 8.06 (s, 1H), 7.82 (d, $J$ = 8.8 Hz, 2H), 7.61 (d, $J$ = 8.8 Hz, 2H), 6.58 (s, 1H), 5.84 (d, $J$= 10.8 Hz, 2H), 4.62 (br. s, 4H), 3.89 (s, 3H), 2.17 (s, 3H), 1.39 (s, 18H).

**Example 1-48 Preparation of Compound A60**

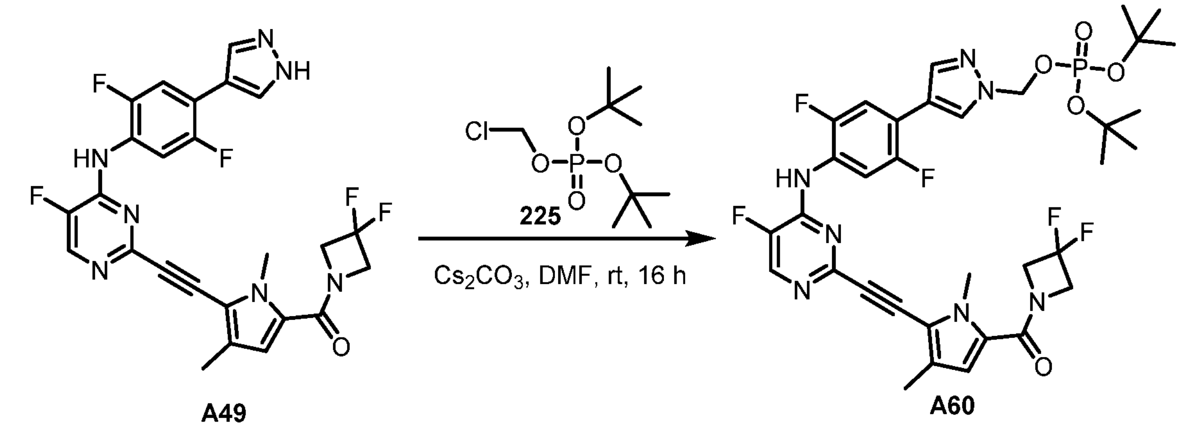


A49 A60

Step 1: Compound A60

Compound A49 (300 mg, 0.568 mmol, 1.0 eq) and compound 225 (235 mg, 0.909 mmol, 1.6 eq) were dissolved in DMF (7 mL), and then cesium carbonate (554 mg, 1.704 mmol, 3.0 eq) was added, and then the reaction solution was stirred under nitrogen atmosphere at 25°C for 16 h. After LC-MS showed that the reaction was completed, the reaction mixture was diluted with water and extracted with ethyl acetate, the organic phases were combined, washed with saturated brine, dried over anhydrous sodium sulfate, and concentrated, and the residue was purified by silica gel column chromatography (petroleum ether/ethyl acetate=1:2) to obtain the crude compound, the crude was purified by pre-HPLC (mobile phase: 0.1% $NH_4HCO_3$/water/acetonitrile) to obtain compound A60 ( 13.0 mg, 3%) as a white solid.

LCMS: $[M+H]^+$ = 750.2

$^1$H NMR (400 MHz, DMSO-$d_6$) δ 9.75 (s, 1H), 8.46 (d, *J* = 3.6 Hz, 1H), 8.37 (s, 1H), 8.17 (s, 1H), 7.82 (dd, *J* = 11.6, 7.2 Hz, 1H), 7.67 (dd, *J* = 11.6, 6.4 Hz, 1H), 6.55 (s, 1H), 5.89 (d, *J* = 11.0 Hz, 2H), 4.59 (br. s, 4H), 3.82 (s, 3H), 2.11 (s, 3H), 1.39 (s, 18H).

**Example 1-49 Preparation of Compound A61 and Compound A62**

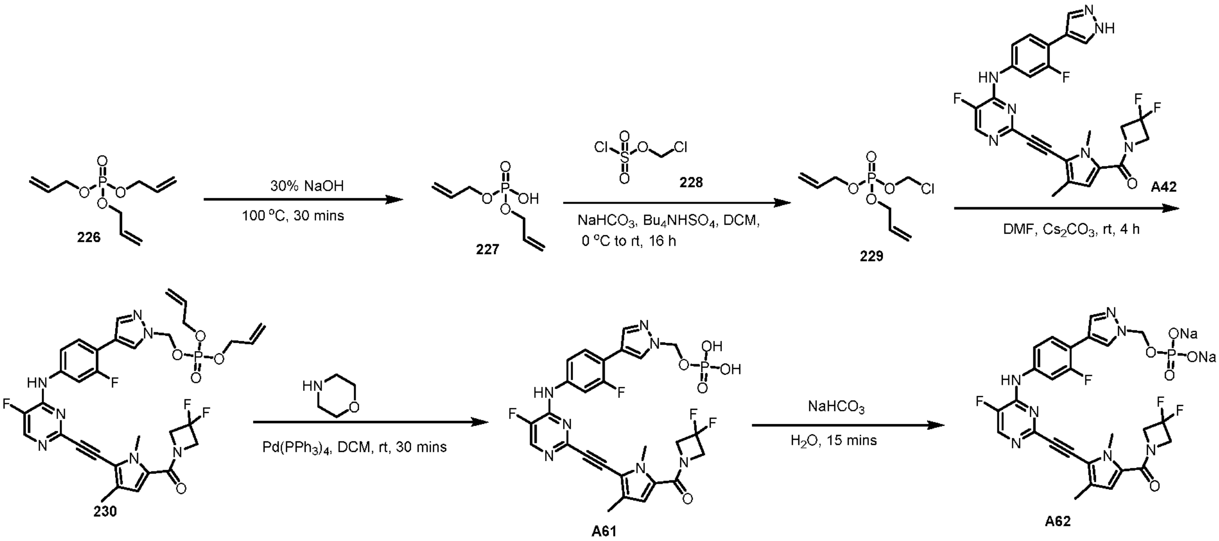


226 227 229 230 A61 A62

Step 1: Compound 227

Compound 226 (5.0 g, 22.916 mmol, 1.0 eq) was dissolved in 30% sodium hydroxide solution (25 mL), replaced with nitrogen, and the reaction solution was stirred at 100 °C for half an hour under nitrogen atmosphere. The reaction solution was cooled to room temperature, neutralized with concentrated hydrochloric acid to about pH = 2. The mixture was

extracted three times with ether, and the combined organic phases were dried over anhydrous sodium sulfate and concentrated to obtain compound 227 (3.8 g, 93%) as a yellow oil.

Step 2: Compound 229

Compound 227 (2.0 g, 11.236 mmol, 1.0 eq), sodium bicarbonate (3.8 g, 44.944 mmol, 1.0 eq) and tetrabutylammonium hydrogensulfate (381 mg, 1.124 mmol, 0.1 eq) were dissolved in dichloromethane/water (30 mL/30 mL), and the reaction mixture was cooled down to 0 °C and stirred for 10 min. Then compound 228 (2.78 g, 16.854 mmol, 1.5 eq) was added. The reaction solution was stirred at 25°C for 16 hours. The reaction solution was washed with saturated brine and the separated organic phase was dried over anhydrous sodium sulfate and concentrated to obtain compound 229 (900 mg, 36%) as a pale yellow oil.

Step 3: Compound 230

Compound A42 (900 mg, 01.765 mmol, 1.0 eq) was dissolved in DMF (10 mL), then compound 229 (800 mg, 3.530 mmol, 2.0 eq) and cesium carbonate (1.7 g, 5.295 mmol, 3.0 eq) were added to the reaction solution. The reaction solution was stirred at 25 °C for 4 hours. The reaction solution was diluted with a large amount of ethyl acetate, then washed sequentially with water and saturated saline, dried over anhydrous sodium sulfate and concentrated. The crude was purified by silica gel column chromatography (DCM/MeOH=15/1) to obtain compound 230 (1.2 g, 100%, containing a small amount of DMF) as a brown oil.
LCMS: $[M+H]^+$ = 700.2

Step 4: Compound A61

Compound 230 ( 1.2 g , 1.714 mmol, 1.0 eq) was dissolved in DCM (60 mL) and then morpholine (1.49 g, 17.143 mmol, 10.0 eq) and $Pd(PPh_3)_4$ (396 mg, 0.343 mmol, 0.2 eq) were added sequentially. Nitrogen was replaced and the reaction solution was stirred under nitrogen atmosphere at 25 °C for half an hour. Upon completion of the reaction, the reaction solution was concentrated and the residue was purified by prep-HPLC (mobile phase: 0.1% ammonia/acetonitrile/water) to obtain A61 (210 mg, 20%) as a yellow solid.

LCMS: $[M+H]^+$ = 620.5
$^1H$ NMR (400 MHz, DMSO-$d_6$) δ 8.43 (d, *J* = 3.6 Hz, 1H), 8.26 (s, 1H), 8.00 (dd, *J* = 14.0, 2.0 Hz, 1H), 7.89 (s, 1H), 7.68 (t, *J* = 8.8 Hz, 1H), 7.61 (dd, *J* = 8.8, 2.0 Hz, 1H), 6.57 (s, 1H), 5.59 (d, *J* = 9.5 Hz, 2H), 4.62 (s, 4H), 3.90 (s, 3H), 2.19 (s, 3H).

Step 5: Compound A62

Compound A61 ( 31.0 mg, 0.05 mmol, 1.0 eq) was added to distilled water (4 mL) followed by sodium bicarbonate (8.4 mg, 0.10 mmol, 2.0 eq). The reaction solution was stirred at 25 °C for 15 min, after the reaction solution was completely clarified, the reaction solution was lyophilized directly to obtain compound A62 (31 mg, 94%) as a yellow solid.

LCMS: $[M+H]^+$ = 620.1
$^1H$ NMR (400 MHz, CD3OD-$d_4$) δ 8.33 (s, 1H), 8.24 (d, *J* = 4.0 Hz, 1H), 7.98 (dd, *J* = 13.6, 2.0 Hz, 1H), 7.90 (d, *J* = 1.0 Hz, 1H), 7.64 (t, *J* = 8.6 Hz, 1H), 7.51 (dd, *J* = 8.6, 2.0 Hz, 1H), 6.50 (s, 1H), 5.81 (d, *J* = 8.8 Hz, 2H), 4.60 (s, 4H), 3.99 (s, 3H), 2.25 (s, 3H).
$^{31}P$ NMR (162 MHz, CD3OD-$d_4$) δ 1.18 ppm.

**Example 1-50 Preparation of Compound A64**

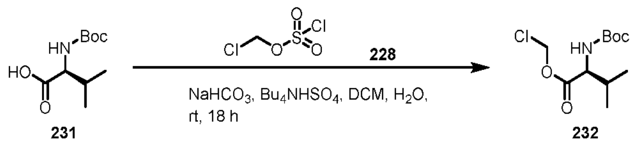


231 232

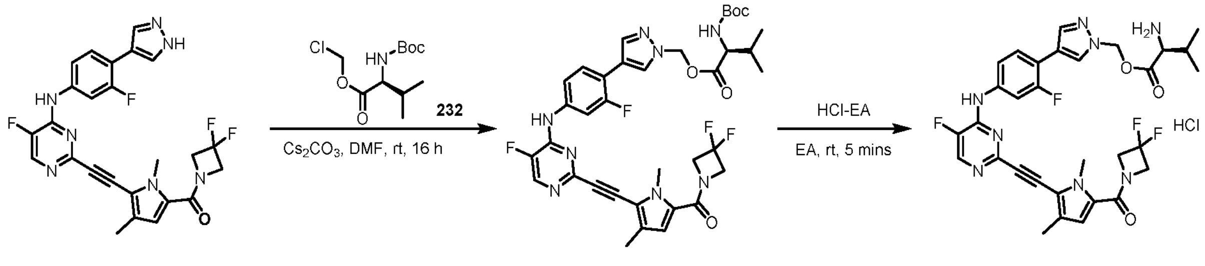


A42 233 A64

Step 1: Compound 232

Compound 231 (2.0 g, 9.21 mmol, 1.0 eq) was dissolved in DCM/$H_2O$ (80 mL, 1/1). The reaction solution was cooled to 0 °C. $NaHCO_3$ (3.09 g, 36.84 mmol, 4.0 eq) and $Bu_4NHSO_4$ (313 mg, 0.921 mmol, 0.1 eq) were added to the reaction solution. The reaction solution was stirred at 0 °C for 10 min. Compound 2 (3.04 g, 18.42 mmol, 2.0 eq) was added dropwise to the reaction solution at 0 °C and the temperature was controlled at 0-5 °C. The reaction solution was stirred at room temperature for 18 h. After LCMS detection showed that the reaction was completed, it was quenched with ice water and the reaction solution was extracted with DCM (50 mL × 3). The combined organic phases were washed with saturated saline, dried over anhydrous sodium sulfate and concentrated. The residue was purified by silica gel column chromatography (DCM/EA = 1/1) to obtain compound 232 (2.4 g, 97%) as a colorless oil.
LCMS: $[M+Na]^+$ = 288.2

Step 2: Compound 233

Compound A42 (1.0 g, 1.96 mmol, 1.0 eq) and compound 232 (1.04 g, 3.92 mmol, 2.0 eq) were dissolved in anhydrous DMF (10 mL) and $Cs_2CO_3$ (1.28 g, 3.92 mmol, 2.0 eq) was added. The reaction solution was stirred at 25 °C for 16 h. The reaction mixture was filtered and the solid was washed with ethyl acetate. The filtrate was diluted with ethyl acetate (30 mL), water (30 mL) was added to the reaction solution and the aqueous phase was extracted with ethyl acetate (30 mL × 3). The combined organic phases were washed with saturated saline, dried over anhydrous sodium sulfate and concentrated. The residue was purified by fast silica gel column chromatography (DCM/EA = 3/1) to obtain compound 233 (1.4 g, 96%) as a yellow solid.
LCMS: $[M+H]^+$ = 739.2

Step 3: Compound A64

Compound 233 (500 mg, 0.676 mmol, 1.0 eq) was dissolved in EA (15 mL) and HCl (2.0 M in EA, 15 mL) was added. The reaction solution was stirred at room temperature for 5 min while a solid was precipitated. The reaction mixture was diluted by rapid addition of petroleum ether (1 L) and filtered, and the solid was washed with petroleum ether. The residual solid was purified by pre-HPLC (mobile phase: 0.1% trifluoroacetic acid/acetonitrile/water) to obtain A64 (111.3 mg, 24%) as a yellow solid.

LCMS: $[M+H]^+$ = 639.2
$^1$H NMR (400 MHz, DMSO-$d_6$) δ 10.03 (s, 1H), 8.47 (d, *J* = 3.6 Hz, 1H), 8.39 (br. s, 3H), 8.35 (d, *J* = 2.0 Hz, 1H), 8.12 (s, 1H), 8.05 (dd, *J* = 14.4, 2.0 Hz, 1H), 7.76 (t, *J* = 8.8 Hz, 1H), 7.64 (dd, *J* = 8.4, 2.0 Hz, 1H), 6.59 (s, 1H), 6.36 (d, *J* = 10.8 Hz, 1H), 6.25 (d, *J* = 10.8 Hz, 1H), 4.62 (br. s, 4H), 4.03 (s, 1H), 3.91 (s, 3H), 2.19 (s, 3H), 2.15-2.06 (m, 1H), 0.91 (d, *J* = 6.8 Hz, 3H), 0.87 (d, *J* = 6.8 Hz, 3H).

**Example 1-51 Preparation of Compound A65**

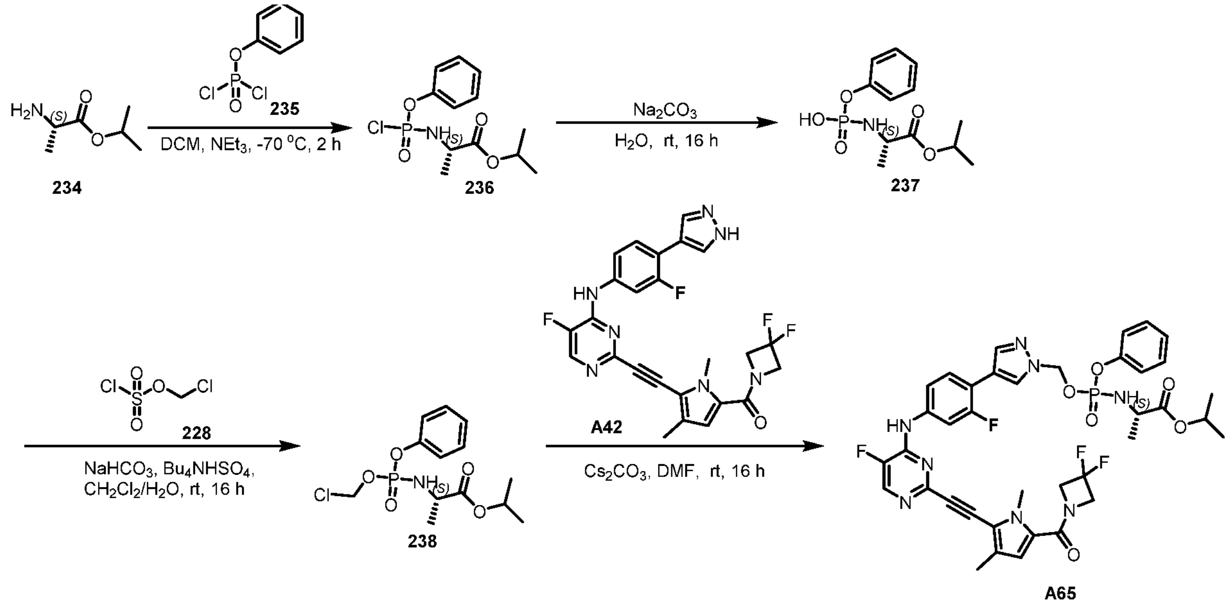

234 236 237 238 A65

Step 1: Compound 236

Compound 234 (5.0 g, 38.12 mmol, 1.0 eq) and compound 235 (8.0 g, 38.12 mmol, 1.0 eq) were dissolved in dichloromethane (50 mL), the solution was cooled to -70 °C, and then a solution of triethylamine (4.2 g, 41.93 mmol, 1.1 eq) dissolved in dichloromethane (30 mL) was added dropwise. After the addition was completed, the reaction solution was reacted at -70 °C for 2 h. After LC-MS detection showed that the reaction was completed, the reaction solution was warmed up to room temperature, and the crude product 236 was carried out directly to the next reaction.
LCMS: $[M+H]^+$ = 306.0

Step 2: Compound 237

An aqueous solution (30 mL) of $Na_2CO_3$ (12.1 g, 114.3 mmol, 3.0 eq) dissolved in water was added to the above reaction solution containing compound 236. After the addition was completed, the reaction solution was reacted at 25 °C for 16 h. After LC-MS detection showed that the reaction was completed, the reaction solution was diluted with dichloromethane, the reaction solution was separated, and the aqueous phase was extracted with dichloromethane (100 mL × 3). The dichloromethane phases were combined, dried over anhydrous sodium sulfate, and concentrated under reduced pressure to obtain the crude compound 237 (3.6 g, 28%).
LCMS: $[M+H]^+$ = 288.1

Step 3: Compound 238

Crude compound 237 (3.6 g, 12.53 mmol, 1.0 eq) was dissolved in dichloromethane/water (70 mL/70 mL), sodium bicarbonate (4.2 g, 50.13 mmol, 4.0 eq) and $Bu_4NHSO_4$ (426 mg, 1.25 mmol, 0.1 eq) were added, the reaction solution was cooled down to 0 °C, and compound 5 ( 3.1 g, 18.80 mmol, 1.5 eq) was added. The reaction solution was stirred at 25 °C for 16 h. After LC-MS detection showed the reaction was completed, the reaction solution was diluted with dichloromethane, the reaction solution was separated, and the aqueous phase was extracted with dichloromethane (100 mL × 3). The dichloromethane phases were combined, dried over anhydrous sodium sulfate, concentrated under reduced pressure to remove the solvent, and the residue was purified by silica gel column chromatography (dichloromethane/ethyl acetate = 15/1) to obtain compound 238 (1.3 g, 31%) as a colorless oil.
LCMS: $[M+H]^+$ = 336.0

Step 4: Compound A65

Compound A42 (400 mg, 0.79 mmol, 1.0 eq) was dissolved in DMF (10 mL) and then compound 238 (527 mg, 1.57 mmol, 2.0 eq) was added. The reaction solution was stirred at 25 °C for 16 hours. After the end of the reaction, the reaction solution was diluted with ethyl acetate, the reaction solution was separated and the aqueous phase was extracted with ethyl acetate (100 mL x 3). The ethyl acetate phases were combined and concentrated under reduced pressure to

remove the solvent. The residue was purified by prep-HPLC (mobile phase: 0.1% formic acid/acetonitrile/water) to obtain compound A65 (61.5 mg, 10%) as a yellow solid.

LCMS: $[M+H]^+$ =809.2

$^1H$ NMR (400 MHz, DMSO-$d_6$) δ 10.01 (s, 1H), 8.46 (d, *J* = 4.0 Hz, 1H), 8.31 (dd, *J* = 12.4, 1.6 Hz, 1H), 8.10-7.99 (m, 2H), 7.74 (t, *J* = 8.8 Hz, 1H), 7.63 (dd, *J* = 8.4, 1.6 Hz, 1H), 7.35 (t, *J* = 8.0 Hz, 1H), 7.22-7.12 (m, 3H), 6.59 (s, 1H), 6.24-6.12 (m, 1H), 6.10-5.88 (m, 2H), 4.91-4.80 (m, 1H), 4.62 (br. s, 4H), 3.91 (s, 3H), 3.85-3.71 (m, 1H), 2.19 (s, 3H), 1.26-1.16 (m, 3H), 1.17 - 1.11 (m, 6H).

**Example 1-52 Preparation of Compound A68**

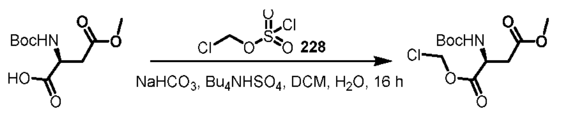


239 240

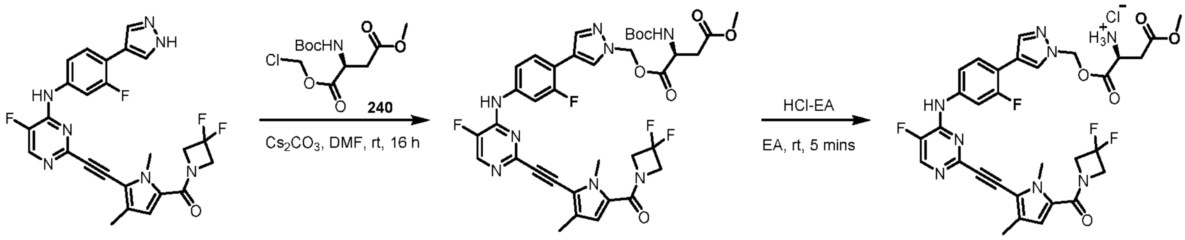


A42 241 A68

Step 1: Compound 240

Compound 239 (2.0 g, 8.1 mmol, 1.0 eq) was dissolved in a solvent mixture of dichloromethane (30 mL) and water (30 mL). Tetrabutylammonium hydrogensulfate (275 mg, 0.8 mmol, 0.1 eq) was added at 0 °C followed by sodium bicarbonate (2.72 g, 32.4 mmol, 4.0 eq). The reaction solution was stirred at 0 °C for 20 min. Compound 228 (2.67 g, 16.2 mmol, 2.0 eq) was slowly added to the reaction solution. After the addition was completed, the reaction solution was reacted at 25°C for 16 hours. The reaction solution was diluted with dichloromethane (50 mL), washed with water, and the organic phase was dried over anhydrous sodium sulfate and concentrated. The residue was purified by silica gel column chromatography (petroleum ether/ethyl acetate=4:1) to obtain compound 240 (2.1 g, 88%) as a white solid.
LCMS: $[M+H]^+$ = 296.1

Step 2: Compound 241

Compound A42 (400 mg, 0.79 mmol, 1.0 eq) and $Cs_2CO_3$ (767 mg, 2.36 mmol, 3.0 eq) were dissolved in DMF (7 mL) and compound 240 (697 mg, 2.36 mmol, 3.0 eq) was added. The reaction solution was stirred at 25 °C for 16 h. After LC-MS detection showed that the reaction was completed, the reaction solution was diluted with ethyl acetate (50 mL), then washed sequentially with water (20 mL × 3) and saturated saline, dried over anhydrous sodium sulfate and concentrated. The residue was purified by silica gel column chromatography (dichloromethane/methanol=20/1) to obtain compound 241 (550 mg, 91%) as a yellow solid.
LCMS: $[M+H]^+$ = 769.1

Step 3: Compound A68

Compound 241 (300 mg, 0.39 mmol, 1.0 eq) was dissolved in ethyl acetate ( 5 mL) and hydrochloric acid (4.0 M in EA) solution ( 5 mL) was added. The reaction solution was stirred at 25 °C for 5 min. A large amount of yellow solid was precipitated from the reaction solution, 30 mL of petroleum ether was added to the reaction solution to dilute the reaction solution and more yellow solid was precipitated. The mixture was filtered and the yellow solid was washed with petroleum ether (10 mL x 3). The yellow solid was purified by prep-HPLC (mobile phase: 0.1% formic acid/acetonitrile/water) to obtain compound A68 (43.8 mg, 17%) as a yellow solid.

LCMS: [M+H] + =669.2

$^1$H NMR (400 MHz, DMSO-$d_6$) δ 10.01 (s, 1H), 8.46 (d, *J* = 3.6 Hz, 1H), 8.28 (d, *J* = 2.0 Hz, 1H), 8.12-7.98 (m, 2H), 7.75 (t, *J* = 8.8 Hz, 1H), 7.63 (dd, *J* = 8.4, 2.0 Hz, 1H), 6.59 (s, 1H), 6.19 - 6.11 (m, 2H), 4.62 (s, 4H), 3.91 (s, 3H), 3.89-3.82 (m, 1H), 3.54 (s, 3H), 2.71-2.64 (m, 2H), 2.19 (s, 3H).

**Example 1-53 Preparation of Compound A69**

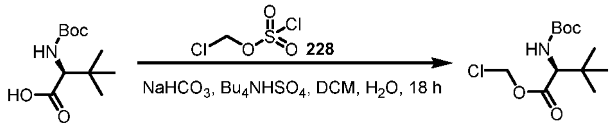


242 243

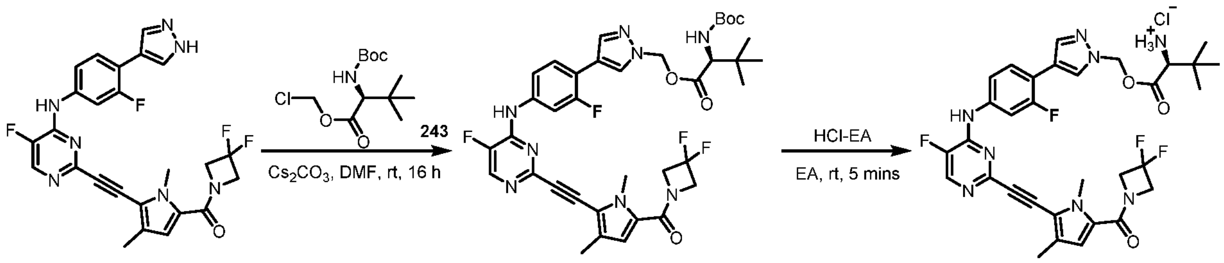


A42 244 A69

Step 1: Compound 243

Compound 242 (150 mg, 0.648 mmol, 1.0 eq) was dissolved in DCM/$H_2O$ (4 mL, 1/1). The reaction solution was cooled to 0 °C. $NaHCO_3$ (218 mg, 2.592 mmol, 4.0 eq) and $Bu_4NHSO_4$ (22 mg, 0.065 mmol, 0.1 eq) were added to the reaction solution. The reaction solution was stirred at 0 °C for 10 min. Compound 228 (214 mg, 1.296 mmol, 2.0 eq) was added dropwise to the reaction solution at 0 °C and the temperature was controlled to be 0-5 °C. The reaction solution was stirred at 25 °C for 18 h. After LCMS detection showed that the reaction was completed, the reaction solution was quenched with ice water and the aqueous phase was extracted with dichloromethane (20 mL × 3). The combined organic phases were washed with saturated saline, dried over anhydrous sodium sulfate and concentrated. The colorless oily residue crude 243 (180 mg, 99%) obtained was used directly in the next step of the reaction.
LCMS: [M+Na] + = 302.1

Step 2: Compound 244

Compound A42 (150 mg, 0.294 mmol, 1.0 eq) and compound 243 (165 mg, 0.588 mmol, 2.0 eq) were dissolved in anhydrous DMF (4 mL) and $Cs_2CO_3$ (192 mg, 0.588 mmol, 2.0 eq) was added. The reaction solution was stirred at 25 °C for 16 hours. The reaction mixture was filtered and the solid was washed with ethyl acetate. The filtrate was washed with water (20 mL) and the aqueous phase was extracted with ethyl acetate (20 mL × 3). The combined organic phases were washed with saturated saline, dried over anhydrous sodium sulfate and concentrated. The residue was purified by fast silica gel column chromatography (DCM/MeOH = 93/7) to obtain compound 244 (210 mg, 94% ) as a yellow solid.
LCMS: [M+H] + = 753.2

Step 3: Compound A69

Compound 244 (200 mg, 0.265 mmol, 1.0 eq) was dissolved in ethyl acetate (3 mL) and HCl (2.0M ethyl acetate solution, 3 mL) was added. The reaction solution was stirred at 25 °C for 5 min and a solid was precipitated. Petroleum ether (100 mL) was added rapidly to the reaction mixture to precipitate more solids. The reaction mixture was filtered and the solids were washed with a large amount of petroleum ether. The solid was purified by pre-HPLC (mobile phase: 0.1% trifluoroacetic acid/acetonitrile/water) to obtain A69 (73.2 mg, 42%) as a yellow solid.

LCMS: [M+H] + = 653.3

$^1H$ NMR (400 MHz, DMSO-$d_6$) δ 10.03 (s, 1H), 8.47 (d, *J*= 4.0 Hz, 1H), 8.39-8.29 (m, 4H), 8.13 (s, 1H), 8.05 (dd, *J* = 14.0, 2.0 Hz, 1H), 7.75 (t, *J* = 8.8 Hz, 1H), 7.63 (dd, *J* = 8.8, 2.0 Hz, 1H), 6.59 (s, 1H), 6.36 (d, *J* = 10.8 Hz, 1H), 6.26 (d, *J* = 10.8 Hz, 1H), 4.62 (br. s, 4H), 3.96-3.86 (m, 4H), 2.19 (s, 3H), 0.93 (s, 9H)

**Example 1-54 Preparation of Compound A70**

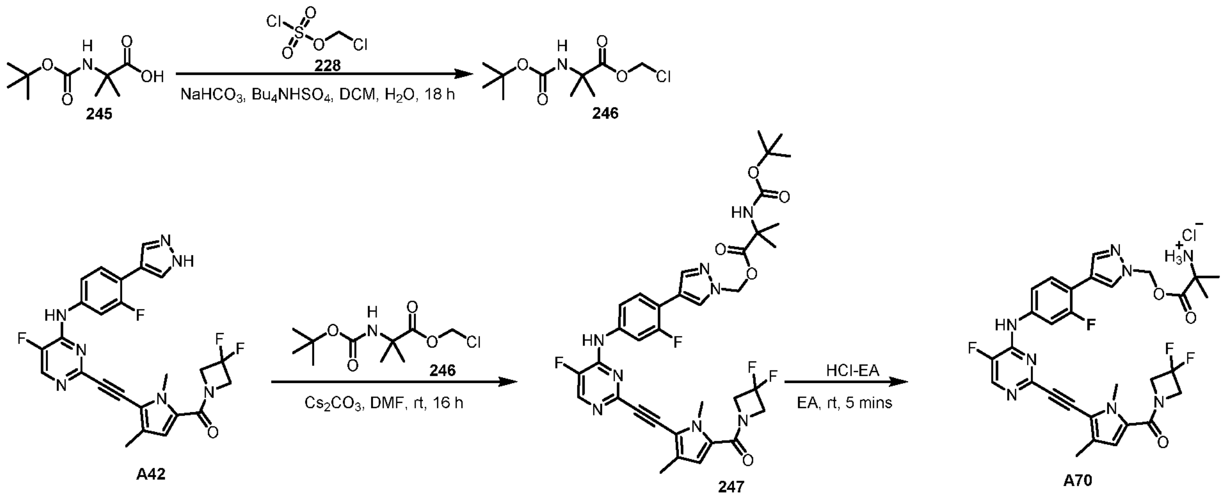


Step 1: Compound 246

Compound 245 (2.0 g, 9.8 mmol, 1.0 eq) was dissolved in DCM/$H_2O$ (70 mL, 1/1). The reaction solution was cooled to 0 °C. $NaHCO_3$ (3.3 g, 39.4 mmol, 4.0 eq) and $Bu_4NHSO_4$ (334 mg, 0.98 mmol, 0.1 eq) were added to the reaction solution. The reaction solution was stirred at 0 °C for 10 min. Compound 228 (3.2 g, 19.7 mmol, 2.0 eq) was added dropwise to the reaction solution at 0 °C and the temperature was controlled at 0-5 °C. The reaction solution was stirred at 25 °C for 18 h. After LCMS detection showed that the reaction was completed, the reaction solution was quenched with ice water and extracted with dichloromethane (60 mL × 3). The combined organic phases were washed with saturated saline, dried over anhydrous sodium sulfate and concentrated to obtain compound 246 (2.0 g, 81% ) as a white solid.
LCMS: $[M+H]^+$ = 252.1

Step 2: Compound 247

Compound A42 (304 mg, 0.597 mmol, 1.0 eq) and compound 246 (600 mg, 2.4 mmol, 4.0 eq) were dissolved in DMF (10 mL) and $Cs_2CO_3$ (583 mg, 1.8 mmol, 3.0 eq) was added. The reaction solution was stirred at 25 °C for 16 h. The reaction solution was diluted with ethyl acetate (100 mL) and then sequentially washed with water, washed with saturated saline, dried over anhydrous sodium sulfate and concentrated. The residue was purified by fast silica gel column chromatography (DCM/MeOH = 12/1) to obtain compound 247 (400 mg, 92% ) as a yellow solid.
LCMS: $[M+H]^+$ = 725.3

Step 3: Compound A70

Compound 247 (260 mg, 0.36 mmol, 1.0 eq) was dissolved in ethyl acetate (20 mL) and HCl (2.0 M ethyl acetate solution, 3 mL) was added. The reaction solution was stirred at 25 °C for 5 min and a solid was precipitated. Petroleum ether (50 mL) was added to the reaction mixture to precipitate more solids. The reaction mixture was filtered and the solids were washed with petroleum ether. The solid was purified by pre-HPLC (mobile phase: 0.1% trifluoroacetic acid/acetonitrile/-water) to obtain A70 (95.8 mg, 35%) as a yellow solid.

LCMS: $[M+H]^+$ = 625.2
$^1H$ NMR (400 MHz, DMSO-$d_6$) δ 10.04 (s, 1H), 8.54 (s, 3H), 8.47 (d, *J* = 4.0 Hz, 1H), 8.36 (d, *J* = 2.0 Hz, 1H), 8.12 (s, 1H), 8.07 (dd, *J* = 14.4, 2.0 Hz, 1H), 7.79-7.74 (m, 1H), 7.64 (dd, *J* = 8.8, 2.0 Hz, 1H), 6.59 (s, 1H), 6.28 (s, 2H), 4.62 (s, 4H), 3.91 (s, 3H), 2.19 (s, 3H), 1.44 (s, 6H).

**Example 1-55 Preparation of Compound A71**

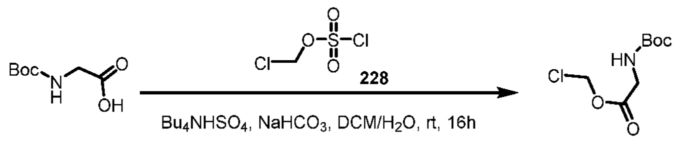


248 249

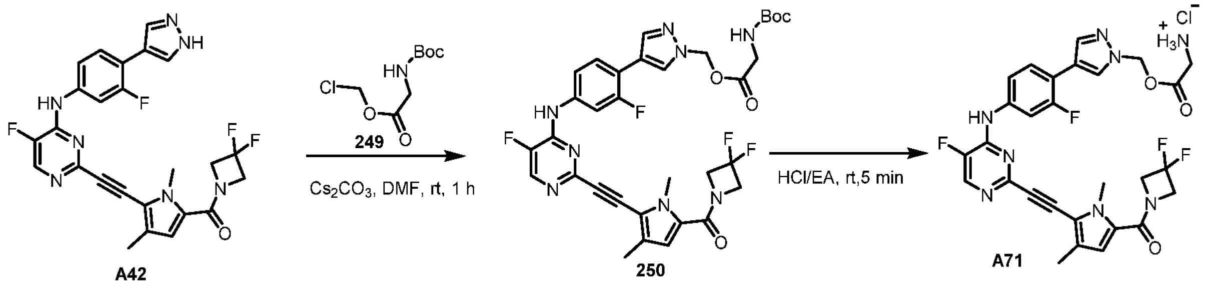


A42 250 A71

Step 1: Compound 249

Compound 248 (2.0 g, 11.42 mmol, 1.0 eq) was dissolved in DCM/$H_2O$ (45 mL/45 mL). The reaction solution was cooled down to 0 °C under $N_2$ protection and $Bu_4NHSO_4$ (387.63 mg, 1.14 mmol, 0.1 eq) and $NaHCO_3$ (5.75 g, 68.50 mmol, 6.0 eq) were added to the reaction solution, the reaction solution was stirred at 0 °C for 5 min. Compound 228 (5.65 g, 34.25 mmol, 3.0 eq) was slowly added to the reaction solution and stirred at 25 °C for 16 h. After LCMS detection showed that the reaction was completed, the reaction solution was quenched with ice water and extracted with ethyl acetate. The ethyl acetate phases were combined, washed with saturated brine, dried over anhydrous sodium sulfate and concentrated to obtain compound 249 (2.3 g, 90%) as a colorless oily solid.

$^1H$ NMR (400 MHz, CDCl3) $\delta$ 5.75 (s, 2H), 5.09 (br. s, 2H), 3.94-4.09 (m, 2H), 1.44 (s, 9H).

Step 2: Compound 250

Compound A42 (300 mg, 0.58 mmol, 1.0 eq) and $Cs_2CO_3$ (575.57 mg, 1.77 mmol ,3.0 eq) were dissolved in DMF (5 mL), the reaction solution was stirred at room temperature for 5 min, then compound 249 (329.24 mg, 1.47 mmol, 2.5 eq) was added to the reaction solution. The reaction solution was stirred at 25 °C for 1 h. After LCMS detection showed that the reaction was completed, the reaction solution was diluted with ethyl acetate, washed with saturated saline, dried over anhydrous sodium sulfate and concentrated. The residue was purified by silica gel column chromatography (DCM/MeOH=7/93) to obtain compound 250 (350 mg, 85%) as a pale yellow solid.
LCMS: $[M+H]^+$ = 697.3

Step 3: Compound A71

Compound 250 (200 mg, 0.23 mmol, 1.0 eq) was dissolved in ethyl acetate (3 mL), and ethyl acetate hydrochloride (3 mL, 4 M) was added to the reaction solution, and the reaction mixture was stirred at 25 °C for 5 min. After LCMS detection showed that the reaction was completed, the reaction solution was filtered to obtain a solid, the solid was washed with petroleum ether. The resulting solid was purified by prep-HPLC (mobile phase: 0.1% $CF_3COOH$/acetonitrile/water) to obtain compound A71 (96 mg, 52 %) as a yellow solid.

LCMS: $[M+H]^+$ = 597.2
$^1H$ NMR (400 MHz, $CDCl_3$) $\delta$ 10.49 (s, 1H), 8.94 (d, *J* = 3.6 Hz, 1H), 8.81 (s, 1H), 8.71 (s, 3H), 8.60-8.48 (m, 2H), 8.22 (d, *J* = 8.8 Hz, 1H), 8.10 (d, *J* = 8.8 Hz, 1H), 7.06 (s, 1H), 6.73 (s, 2H), 5.08 (s, 4H), 4.37-4.26 (m, 5H), 2.65 (s, 3H).

**Example 1-56 Preparation of Compound A72**

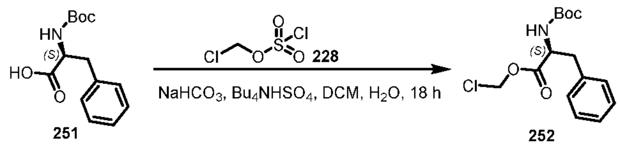


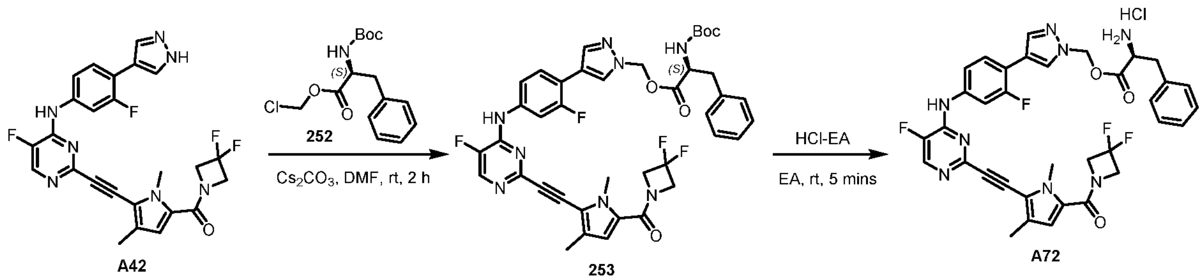


Step 1: Compound 252

Compound 251 (1.0 g, 3.76 mmol, 1.0 eq) was dissolved in a solvent mixture of dichloromethane (15 mL) and water (15 mL) , and tetrabutylammonium hydrogensulfate (1.29 g, 34.05 mmol, 3.0 eq) and sodium bicarbonate (1.89 g, 22.56 mmol, 6.0 eq) were added to the reaction solution at 0 °C. The reaction solution was stirred at 0°C for 20 min. Compound 2 (1.24 g, 7.52 mmol, 2.0 eq) was slowly added to the reaction solution, the reaction solution was then transferred to room temperature and reacted for 18 h. After LC-MS detection showed that the reaction was completed, the reaction solution was diluted with dichloromethane (25 mL), then washed sequentially with water and saturated saline, dried over anhydrous sodium sulfate and concentrated. The residue was purified by silica gel column chromatography (petroleum ether/ethyl acetate = 20:1-15:1) to obtain compound 252 (960 mg, 81%).
LCMS: $[M-Boc+H]^+$ =214.0

Step 2: Compound 253

Compound A42 (330 mg, 0.64 mmol, 1.0 eq) and compound 252 (502 mg, 1.6 mmol, 2.5 eq) were dissolved in DMF (14 mL), and then $Cs_2CO_3$ (625 mg, 1.92 mmol, 3.0 eq) was added. The reaction solution was stirred for 2 hours at 25 °C under nitrogen atmosphere. After LC-MS detection showed that the reaction was completed, the reaction solution was diluted with ethyl acetate (30 mL), then washed sequentially with water (20 mL × 3) and saturated saline, dried over anhydrous sodium sulfate and concentrated. The residue was purified by rapid column chromatography on silica gel (petroleum ether/ethyl acetate=95/5) to obtain compound 253 (490 mg, 97%).
LCMS: $[M+H]^+$ = 787.2

Step 3: Compound A72

Compound 253 (280 mg, 0.35 mmol, 1.0 eq) was dissolved in ethyl acetate ( 8 mL) and then hydrochloric acid in ethyl acetate solution (4M, 2 mL) was added. The reaction solution was stirred at 25 °C for 5 min. A large amount of yellow solid was precipitated from the reaction solution which was filtered and the yellow solid was washed with petroleum ether (30 mL x 3). The resulting yellow solid was purified by prep-HPLC (mobile phase: 0.1% formic acid/acetonitrile/water) to obtain compound A72 (38.7 mg, 15%) as a yellow solid.

LCMS: $[M+H]^+$ =687.2
$^1$H NMR (400 MHz, DMSO-$d_6$) δ 10.03 (s, 1H), 8.50-8.39 (m, 4H), 8.24 (d, $J$ = 2.0 Hz, 1H), 8.14 (s, 1H), 8.07 (dd, $J$ = 14.4, 2.0 Hz, 1H), 7.78 (t, $J$ = 8.8 Hz, 1H), 7.66-7.63 (m, 1H), 7.22-7.20 (m, 3H), 7.08 (dd, $J$ = 3.6, 2.0 Hz, 2H), 6.59 (s, 1H), 6.24 (q, $J$ = 10.8 Hz, 2H), 4.61 (s, 4H), 4.44 (s, 1H), 3.91 (s, 3H), 3.12-3.00 (m, 2H), 2.19 (s, 3H).

**Example 1-57 Preparation of Compound A73**

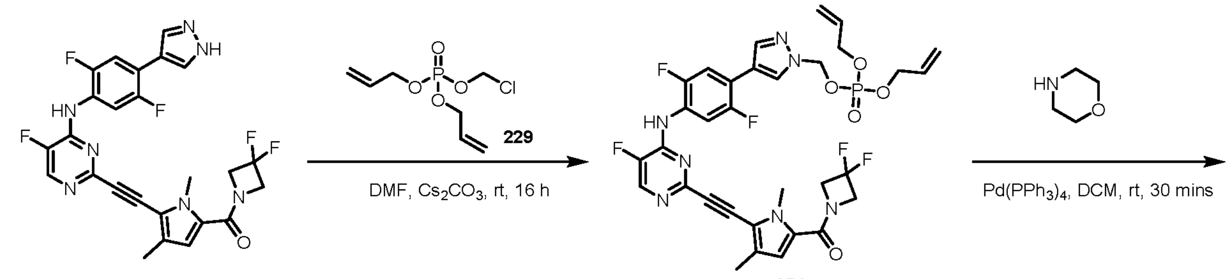


A49

254

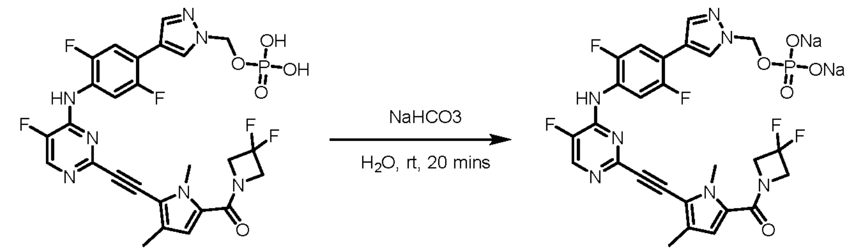


255

A73

Step 1: Compound 254

Compound A49 (500 mg, 0.948 mmol, 1.0 eq) was dissolved in DMF (30 mL), then compound 2 (537 mg, 2.370 mmol, 2.0 eq) and cesium carbonate (772 mg, 2.370 mmol, 2.5 eq) were added to the reaction solution. The reaction solution was stirred at 25°C for 16 hours. The reaction solution was diluted with a large amount of ethyl acetate, then washed sequentially with water and saturated saline, dried over anhydrous sodium sulfate and concentrated. The crude was purified by silica gel column chromatography (DCM/MeOH=19/1) to obtain compound 254 (497 mg, 73%) as a yellow solid.
LCMS: $[M+H]^+$ = 718.2

Step 2: Compound 255

Compound 254 ( 300 mg , 0.418 mmol, 1.0 eq) was dissolved in DCM (5 mL), then morpholine (109 mg, 1.254 mmol, 3.0 eq) and $Pd(PPh_3)_4$ (48 mg, 0.042 mmol, 0.1 eq) were added sequentially. Nitrogen was replaced and the reaction solution was stirred under nitrogen atmosphere at 25 °C for 30 min. After completion of the reaction, the reaction solution was concentrated and the residue was purified by prep-HPLC (mobile phase: 0.1% ammonia/acetonitrile/water) to obtain compound 255 (145 mg, 54%) as a white solid.

LCMS: [M+H] + = 638.2
$^1$H NMR (400 MHz, DMSO-$d_6$) δ 8.42 (d, $J$ = 3.2 Hz, 1H), 8.35 (d, $J$ = 2.0 Hz, 1H), 7.97 (s, 1H), 7.72 (dd, $J$ = 11.6, 6.8 Hz, 1H), 7.64 (dd, $J$ = 12.0, 6.8 Hz, 1H), 6.54 (s, 1H), 5.59 (d, $J$ = 9.2 Hz, 2H), 4.59 (br. s, 4H), 3.82 (s, 3H), 2.11 (s, 3H).

Step 3: Compound A73

Compound 255 ( 50.6 mg , 0.079 mmol, 1.0 eq) was added to distilled water (20 mL) followed by sodium bicarbonate (13.4 mg, 0.158 mmol, 2.0 eq). The reaction solution was stirred at 25 °C for 20 min and after the reaction was completely clarified, the reaction solution was lyophilized directly to obtain compound A73 (51.6 mg, 95%) as a white solid.

LCMS: $[M+H]^+$ = 638.2
$^1$H NMR (400 MHz, MeOD-$d_4$) δ 8.46 (s, 1H), 8.27 (d, $J$ = 3.6 Hz, 1H), 7.95-7.85 (m, 2H), 7.58 (dd, $J$ = 11.6, 6.8 Hz, 1H), 6.48 (s, 1H), 5.80 (d, $J$ = 8.0 Hz, 2H), 4.59 (br. s, 4H), 3.95 (s, 3H), 2.21 (s, 3H).
$^{31}$P NMR (162 MHz, CD3OD-$d_4$) δ 4.19 ppm.

**Example 2-1 Evaluation of kinase activity in vitro**

Kinase $IC_{50}$ assay was performed using the kinase assay kit HTRF kinEASE STK S2 kit (Cat # 62ST2PEC,

Cisbio). Kinases ROCK1 (Cat # PV3691) and ROCK2 (Cat # PV3759) were purchased from Invitrogen.

The working solution required for the assay was prepared with the appropriate reagents as described in the kinase assay kit instructions:

Table A Table of components and concentration of ROCK's reaction system

| Reagent | | ROCK1 | ROCK2 |
|---|---|---|---|
| concentration of enzyme | final concentration in enzyme reaction step | 2.5 ng/well | 2.5 ng/well |
| concentration of ATP | | 1.5 μM | 1 μM |
| concentration of STK-biotin substrate | | 2 μM | 2 μM |
| enzyme reaction time | | 120 min | 120 min |
| concentration of Streptavidin-XL665 | final concentration in the detection step | 125 nM | 125 nM |
| concentration of STK Antibody-Cryptate | | 1:100 dilution | 1:100 dilution |

1X Kinase Buffer

1mL of 1X Kinase Buffer contains 200μL 5X Enzyme buffer, 5μL 1M $MgCl_2$ (Cat.M8266, Sigma), 1μL 1M DTT (Cat.D5545, Sigma), 794μL $ddH_2O$.

2.5X Substrate and ATP working solution

The specific concentrations of STK-biotin substrate and ATP are shown in Table A. STK-biotin substrate and ATP were diluted with 1X kinase buffer to 2.5 times of the reaction concentration.

5X ROCK1/ ROCK2 kinase working solution

Concentrations of ROCK1/ ROCK2 are shown in Table A. 5X ROCK1/ ROCK2 Kinase working solution was prepared with 1X Kinase Buffer.

2X Streptavidin-XL665 and STK Antibody-Cryptate Working Solution

See Table A for the concentrations of Streptavidin-XL665 and STK Antibody-Cryptate in the reaction. 2X Streptavidin-XL665 and STK Antibody-Cryptate Working Solutions were prepared 5 minutes prior to the end of the kinase reaction using Detection Buffer from the kit.

A 100 μM compound mother solution was prepared using 1X kinase buffer containing 2.5% DMSO, and the mother solution was diluted 4-fold stepwise with the above DMSO-containing kinase buffer to obtain nine different concentrations of the compound solutions to be tested. In addition to the test compound wells, positive control wells (containing all reagents except the compounds) and negative control wells (containing all reagents except the compounds and kinase) were set up. 4 μL of the test compound solution was added to all reaction wells except the control wells and 4 μL of 2.5% DMSO solution was added to the control wells. Then 4 μL of previously prepared substrate and ATP working solution were added to all wells and mix well. Then, 2μL/well of 5X ROCK1/ ROCK2 kinase working solution (containing 2.5ng ROCK1/ ROCK2 kinase) was added to all reaction wells except negative control wells, and the negative control wells were made up to volume with 2μL of 1X kinase buffer. Mix well, seal with plastic film and incubate at room temperature for 2 hours. Start preparing the assay solution 5 minutes before the end of the kinase reaction. After the end of the kinase reaction, 10 μL of prepared Streptavidin-XL665 and STK Antibody-Cryptate working solution were added to all reaction wells, the plates were sealed and mixed well, and then incubated at room temperature for 1 hour. Finally, the fluorescence signal was detected by SpectraMax i3x Multi-Mode Reader (Molecular Devices) (excitation wavelength is 320nm, emission wavelength is 665nm and 615nm).

The inhibition rate in each well was calculated from the fluorescence intensity values of each well:

$$\text{Inhibition} = (ER_{vehicle} - ER_{compound}) / (ER_{vehicle} - ER_{blank}) \times 100\%$$

ER (Emission Ratio, Emission light ratio) = fluorescence intensity at 665 nm / fluorescence intensity at 615 nm; $ER_{compound}$, ER of test compound wells; $ER_{vehicle}$, ER of solvent (positive) control wells, $ER_{blank}$, ER of blank

(negative) control wells.

The graphs were plotted using the software GraphPad Prism 6.0, while the half inhibitory concentration ($IC_{50}$) of each compound to be tested was obtained by fitting with Prism 6.0.

Based on the above test methods, the inhibitory activities of the compounds of the present invention against ROCK2 and ROCK1 are shown in the table below:

Table 1. Inhibitory activity of compounds of the present invention against ROCK2 and ROCK1

| Compound | ROCK2 ($IC_{50}$:μM) | ROCK1 ($IC_{50}$:μM) |
|---|---|---|
| **A1** | 0.104 | >100 |
| **A2** | 0.303 | 3.90 |
| **A4** | 0.186 | 80.0 |
| **A5** | 0.091 | >100 |
| **A6** | 0.2024 | 35.2 |
| **A7** | 0.0544 | >100 |
| **A8** | 3.49 | >100 |
| **A9** | 0.702 | >100 |
| **A10** | 0.0778 | >100 |
| **A11** | 0.0867 | >100 |
| **A12** | 0.199 | 32.3 |
| **A14** | 0.585 | >100 |
| **A15** | 0.421 | >100 |
| **A16** | 0.0349 | 29.6 |
| **A17** | 1.06 | 50.3 |
| **A18** | 2.35 | 53.8 |
| **A19** | 50.5 | >100 |
| **A20** | 0.206 | >100 |
| **A21** | 0.754 | >100 |
| **A22** | 3.59 | 40.9 |
| **A27** | 0.274 | >100 |
| **A28** | 1.10 | >100 |
| **A29** | 2.00 | 57.1 |
| **A30** | 0.118 | 41.9 |
| **A32** | 0.232 | 30.7 |
| **A37** | 0.420 | >100 |
| **A38** | 0.445 | >100 |
| **A40** | 0.0172 | >100 |
| **A41** | 0.0724 | >100 |
| **A42** | 0.005 | 4.93 |
| **A43** | 0.0515 | 20.6 |
| **A44** | 0.0127 | >100 |
| **A45** | 0.0352 | 8.80 |
| **A46** | 0.161 | >100 |

(continued)

| Table 1. Inhibitory activity of compounds of the present invention against ROCK2 and ROCK1 | | |
|---|---|---|
| **Compound** | **ROCK2 ($IC_{50}$:μM)** | **ROCK1 ($IC_{50}$:μM)** |
| **A47** | 0.476 | >100 |
| **A48** | 0.0377 | 18.4 |
| **A49** | 0.0177 | 37.4 |
| **A50** | 0.0089 | 0.073 |
| **A51** | 0.131 | >100 |
| **A54** | 0.0192 | 64.0 |
| **A55** | 0.0573 | >100 |
| **A56** | 0.0193 | >100 |
| **A57** | 0.0324 | >100 |

From the above results, it can be seen that the compounds of the present invention all have good inhibitory activity against ROCK2 kinase. In addition, the compounds of the present invention have good selectivity for the inhibition of ROCK2 and ROCK1.

**Example 3-1: Pharmacokinetic Study**

1) Purpose of the study: obtain the pharmacokinetic characterization of the compounds of the present invention in male SD rats
2) Experimental content

For each compound, 6 healthy male SD rats (weight range 200-250 g) was taken and divided into 2 groups of 3 each; Group 1 was administered intravenously in a single dose (see Table 2 for dosage) and Group 2 was administered orally in a single gavage (see Table 2 for dosage, fasted overnight before administration). Blood was collected by jugular vein or cardiac puncture at 0.083 (group 1 only), 0.25, 0.5, 1, 2, 4, 6 (group 2 only), 8 and 24 h after drug administration, respectively, and at least 0.2 mL of whole blood was collected from each sample into EDTA-K2 anticoagulant tubes, and the samples were mixed and placed on wet ice after sample collection, and then centrifuged to separate plasma (6,800 g, 6 min, 2-8 °C) at 1 h. The plasma samples were stored in ultra-low temperature refrigerators before analysis.

Experimental results: The pharmacokinetic parameters after administration were calculated from the blood concentration data obtained by LC-MS/MS assay using the non-compartmental model of WinNonlin® 7.0 software (Pharsight, Inc., USA) and are shown in the table below:

| Table 2. Pharmacokinetic parameters of the com pounds of the invention (male SD rats) | | | | | | |
|---|---|---|---|---|---|---|
| Compound | Mode of administration (Dose) | $T_{1/2}$ (h) | Tmax (h) | Cmax (ng/mL) | $AUC_{last}$ (hr*ng/mL) | F (%) |
| A1 | intravenous injection (1 mg/kg) | 2.05 | 0.08 | 765.27 | 1214.25 | / |
| | Gavage (10 mg/kg) | 3.47 | 1.33 | 251.19 | 1398.12 | 11.51 |

All literatures mentioned in the present invention are incorporated by reference herein, as though individually incorporated by reference. Additionally, it should be understood that after reading the above teaching, many variations and modifications may be made by the skilled in the art, and these equivalents also fall within the scope as defined by the appended claims.

## Claims

1. A compound of formula I or a pharmaceutically acceptable salt, stereoisomer, polymorph, solvate, N-oxide, isotope labeled compound, metabolite or prodrug thereof:

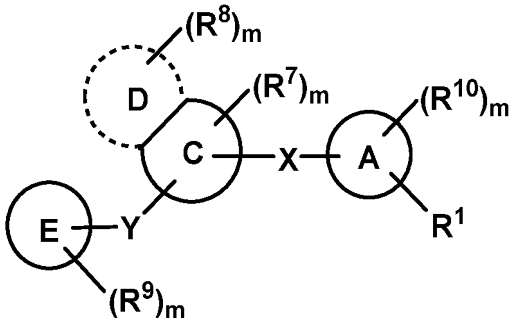

Formula I

wherein

X is selected from the group consisting of

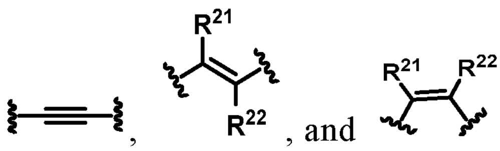

$R^{21}$ and $R^{22}$ are each independently selected from the group consisting of H, $C_{1-6}$ alkyl, and $C_{3-10}$ cycloalkyl;
Y is selected from the group consisting of none, C(=O), O, $S(=O)_i$, $CR^{23}R^{24}$, and NR;
R is selected from the group consisting of H, $C_{1-6}$ alkyl, $C_{2-6}$ alkenyl, $C_{2-6}$ alkynyl, saturated or partially unsaturated $C_{3-10}$ cycloalkyl, saturated or unsaturated 3-10 membered heterocyclyl containing 1, 2, or 3 heteroatoms selected from N, O or S, $C_{6-10}$ aryl, 5-14 membered heteroaryl containing 1, 2, or 3 heteroatoms selected from N, O or S, and $C_{6-12}$ aralkyl, and at most two ring members in the cycloalkyl and heterocyclyl are C(=O);
$R^{23}$, $R^{24}$ are each independently selected from the group consisting of H, halogen, C $_{1-6}$ alkyl, and $C_{3-10}$ cycloalkyl;
Ring A is selected from the group consisting of saturated or partially unsaturated $C_{3-10}$ cycloalkyl, saturated or partially unsaturated 3-10 membered heterocyclyl containing 1, 2 or 3 heteroatoms selected from N, O or S, $C_{6-10}$ aryl, and 5-14 membered heteroaryl containing 1, 2 or 3 heteroatoms selected from N, O or S, and at most two ring members in the cycloalkyl and heterocyclyl are C(=O);
Ring C is selected from the group consisting of saturated or partially unsaturated $C_{3-10}$ cycloalkyl, saturated or partially unsaturated 3-10 membered heterocyclyl containing 1, 2 or 3 heteroatoms selected from N, O or S, $C_{6-10}$ aryl, and 5-14 membered heteroaryl containing 1, 2 or 3 heteroatoms selected from N, O or S; and at most two ring members in the cycloalkyl and heterocyclyl are C(=O);
Ring D is absent or is selected from the group consisting of saturated or partially unsaturated $C_{3-10}$ cycloalkyl, saturated or partially unsaturated 3-10 membered heterocyclyl containing 1, 2 or 3 heteroatoms selected from N, O or S, $C_{6-10}$ aryl, and 5-14 membered heteroaryl containing 1, 2 or 3 heteroatoms selected from N, O or S, and at most two ring members in the cycloalkyl and heterocyclyl are C(=O);
Ring E is selected from the group consisting of

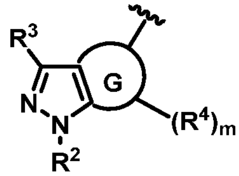

,

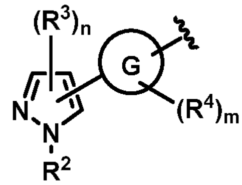 , 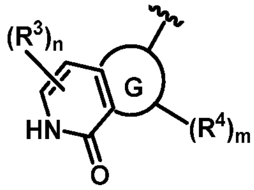 , and 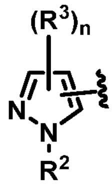 ;

Ring G is selected from the group consisting of saturated or partially unsaturated $C_{3-10}$ cycloalkyl, saturated or partially unsaturated 3-10 membered heterocyclyl containing 1, 2 or 3 heteroatoms selected from N, O or S, $C_{6-10}$ aryl, and 5-14 membered heteroaryl containing 1, 2 or 3 heteroatoms selected from N, O or S, and at most two ring members in the cycloalkyl and heterocyclyl are C(=O);

$R^1$ is selected from the group consisting of H, halogen, -CN, $-NH_2$ , $C_{1-6}$ alkyl, $C_{6-10}$ aryl, 5-14 membered heteroaryl containing 1, 2 or 3 heteroatoms selected from N, O or S, N-methyltetrahydropyrrolyl, N-methylpiperidinyl, acetyl, -C(=O)-($C_{1-6}$ alkylene)$_n$-$CF_3$, -($C_{1-6}$ alkylene)$_n$-$CF_3$, -C(=O)-($C_{1-6}$ alkylene)$_n$-CN, -C(=O)-(saturated or partially unsaturated $C_{3-10}$ cycloalkyl)$_n$, -NH-C(=O)-(saturated or partially unsaturated $C_{3-10}$ cycloalkyl), -C(=O)-(saturated or partially unsaturated 3-10 membered heterocyclyl containing 1, 2 or 3 heteroatoms selected from N, O or S), -C(=O)-$C_{1-6}$ alkylene-(saturated or partially unsaturated 3-10 membered heterocyclyl containing 1, 2 or 3 heteroatoms selected from N, O or S), -C(=O)-(5-14 membered heteroaryl containing 1, 2 or 3 heteroatoms selected from N, O or S), -C(=O)-$C_{1-6}$ alkylene-NH-($C_{1-6}$ alkyl), -C(=O)-$C_{1-6}$ alkylene-N($C_{1-6}$ alkyl)$_2$, N-methylpiperazine-substituted acetyl, $-S(=O)_2R^{1a}$, $-P(=O)R^{1a}R^{1b}$,

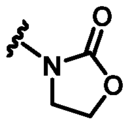 ,

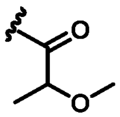 , 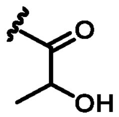 , 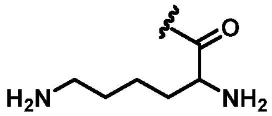 , 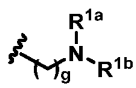 , 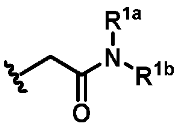 , 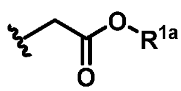 ,

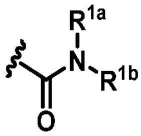 , 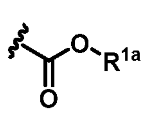 , 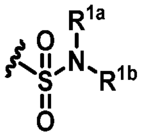 , 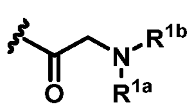 , and 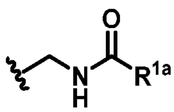 ;

$R^{1a}$ and $R^{1b}$ are each independently selected from the group consisting of H, halogen, amino, cyano, nitro, $C_{1-6}$ alkyl, $C_{2-6}$ alkenyl, $C_{2-6}$ alkynyl, $C_{3-10}$ cycloalkyl, 3-10 membered heterocyclyl containing 1, 2 or 3 heteroatoms selected from N, O or S, $C_{6-10}$ aryl, 5-14 membered heteroaryl containing 1, 2 or 3 heteroatoms selected from N, O or S, $C_{6-12}$ aralkyl, $-C(=O)R^5$, $-OC(=O)R^5$, $-C(=O)OR^5$, $-OR^5$, $-SR^5$, $-S(=O)R^5$, $-S(=O)_2R^5$, $-S(=O)_2NR^5R^6$, $-NR^5R^6$, $-C(=O)NR^5R^6$, $-NR^5-C(=O)R^6$, $-NR^5-C(=O)OR^6$, $-NR^5-S(=O)_2-R^6$, $-NR^5-C(=O)-NR^5R^6$, -$C_{1-6}$ alkylene-$NR^5R^6$, -$C_{1-6}$ alkylene-$OR^5$, and -O-$C_{1-6}$ alkylene -$NR^5R^6$; or $R^{1a}$ and $R^{1b}$ together with the atoms to which they are attached constitute a 3-12 membered heterocyclyl or heteroaryl containing 1, 2 or 3 heteroatoms selected from N, O or S;

$R^3$, $R^4$, $R^7$, $R^8$, $R^9$ and $R^{10}$ are each independently at each occurrence selected from the group consisting of H, halogen, amino, cyano, nitro, $C_{1-6}$ alkyl, deuterated $C_{1-6}$ alkyl, halogenated $C_{1-6}$ alkyl, $C_{2-6}$ alkenyl, $C_{2-6}$ alkynyl, $C_{3-10}$ cycloalkyl, 3-10 membered heterocyclyl containing 1, 2 or 3 heteroatoms selected from N, O or S, $C_{6-10}$ aryl, 5-14 membered heteroaryl containing 1, 2 or 3 heteroatoms selected from N, O or S, $C_{6-12}$ aralkyl, $-C(=O)R^5$, $-OC(=O)R^5$, $-C(=O)OR^5$, $-OR^5$, $-SR^5$, $-S(=O)R^5$, $-S(=O)_2R^5$, - $S(=O)_2NR^5R^6$, $-NR^5R^6$, $-C(=O)NR^5R^6$, $-NR^5-C(=O)R^6$, $-NR^5-C(=O)OR^6$, $-NR^5-S(=O)_2-R^6$, $-NR^5-C(=O)-NR^5R^6$, -$C_{1-6}$ alkylene-$NR^5R^6$, -$C_{1-6}$ alkylene-$OR^5$, -O-$C_{1-6}$ alkylene-$NR^5R^6$, -$C_{1-6}$ alkylene-O-(P=O)$(OH)_2$;

$R^2$ is selected from the group consisting of H, halogen, amino, cyano, nitro, $C_{1-6}$ alkyl, deuterated $C_{1-6}$ alkyl, halogenated $C_{1-6}$ alkyl, $C_{2-6}$ alkenyl, $C_{2-6}$ alkynyl, $C_{3-10}$ cycloalkyl, 3-10 membered heterocyclyl containing 1, 2 or 3 heteroatoms selected from N, O or S, $C_{6-10}$ aryl, 5-14 membered heteroaryl containing 1, 2 or 3 heteroatoms selected from N, O or S, $C_{6-12}$ aralkyl, $-C(=O)R^5$, $-OC(=O)R^5$, $-C(=O)OR^5$, $-OR^5$, $-SR^5$, $-S(=O)R^5$, $-S(=O)_2R^5$,

$-S(=O)_2NR^5R^6$, $-NR^5R^6$, $-C(=O)NR^5R^6$, $-NR^5-C(=O)R^6$, $-NR^5-C(=O)OR^6$, - $NR^5-S(=O)_2-R^6$, $-NR^5-C(=O)-NR^5R^6$, $-C_{1-6}$ alkylene-$NR^5R^6$, $-C_{1-6}$ alkylene-$OR^5$, $-O-C_{1-6}$alkylene-$NR^5R^6$, $-C_{1-6}$ alkylene-$O-(P=O)(OH)_2$, $-C_{1-6}$ alkylene-$O(C=O)R^{11}$, $-C_{1-6}$ alkylene-$O(P=O)(OR^{12})(NR^{13}R^{14})$, and $-C_{1-6}$ alkylene-$O(P=O)(OR^{12})(OR^{13})$;

$R^{11}$, $R^{12}$, $R^{13}$, $R^{14}$ are each independently at each occurrence selected from the group consisting of H, $C_{1-6}$ alkyl, $C_{3-6}$ cycloalkyl, 3-10 membered heterocyclyl containing 1, 2 or 3 heteroatoms selected from N, O or S, $C_{6-10}$ aryl, 3-10 membered heteroaryl containing 1, 2 or 3 heteroatoms selected from N, O or S, and cation;

the cation is selected from the group consisting of $Na^+$, $K^+$, $Ca^{2+}$, $Mg^{2+}$, and $NH^{4+}$; the alkyl, alkylene, alkenyl, alkynyl, cycloalkyl, heterocyclyl, aryl, heteroaryl, and aralkyl are each optionally at each occurrence substituted with one or more substituents independently selected from the group consisting of halogen, hydroxyl, oxo, amino, cyano, nitro, $C_{1-6}$ alkyl, $C_{2-6}$ alkenyl, $C_{2-6}$ alkynyl, $C_{3-10}$ cycloalkyl, 3-10 membered heterocyclyl containing 1, 2 or 3 heteroatoms selected from N, O or S, $C_{6-10}$ aryl, 5-14 membered heteroaryl containing 1, 2 or 3 heteroatoms selected from N, O or S, $C_{6-12}$ aralkyl, $=N-OR^5$, $-C(=NH)NH_2$, $-C(=O)R^5$, $-OC(=O)R^5$, $-C(=O)OR^5$, $-OR^5$, $-SR^5$, $-S(=O)R^5$, $-S(=O)_2R^5$, $-S(=O)_2NR^5R^6$, $-NR^5R^6$, $-C(=O)NR^5R^6$, $-NR^5-C(=O)R^6$, $-NR^5-C(=O)OR^6$, $-NR^5-S(=O)_2-R^6$, $-NR^5-C(=O)-NR^5R^6$, $-C_{1-6}$ alkylene-$NR^5R^6$, $-C_{1-6}$ alkylene-$OR^5$, and $-O-C_{1-6}$ alkylene-$NR^5R^6$;

$R^5$ and $R^6$ are each independently at each occurrence selected from the group consisting of H, $C_{1-6}$ alkyl, $C_{3-10}$ cycloalkyl, 3-10 membered heterocyclyl containing 1, 2, or 3 heteroatoms selected from N, O, or S, $C_{6-10}$ aryl, 5-14 membered heteroaryl containing 1, 2, or 3 heteroatoms selected from N, O, or S, and $C_{6-12}$ aralkyl;

each m is independently at each occurrence selected from the group consisting of 0, 1, 2, and 3;

each n is independently at each occurrence selected from the group consisting of 0, 1, and 2;

i is selected from the group consisting of 1, and 2;

g is selected from the group consisting of 0, 1, 2, 3, and 4.

**2.** The compound or the pharmaceutically acceptable salt, stereoisomer, polymorph, solvate, N-oxide, isotope labeled compound, metabolite or prodrug thereof according to claim 1, wherein,

X is selected from the group consisting of

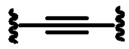, 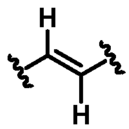, 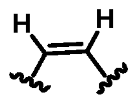,

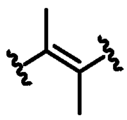, and 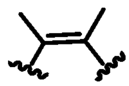;

Y is selected from the group consisting of NH, and $NCH_3$.

**3.** The compound or the pharmaceutically acceptable salt, stereoisomer, polymorph, solvate, N-oxide, isotope labeled compound, metabolite or prodrug thereof according to claim 1, wherein,

Ring C is

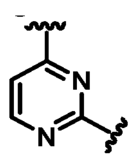;

Ring E is selected from the group consisting of

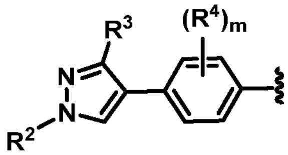

,

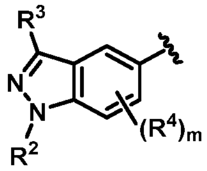

, and

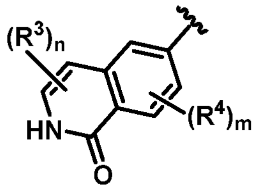

.

**4.** The compound or a pharmaceutically acceptable salt, stereoisomer, polymorph, solvate, N-oxide, isotope labeled compound, metabolite or prodrug thereof according to claim 1, wherein,

Ring A is selected from the group consisting of saturated or partially unsaturated $C_{3-10}$ cycloalkyl, saturated or partially unsaturated 3-10 membered heterocyclyl containing 1, 2 or 3 heteroatoms selected from N, O or S, $C_{6-10}$ aryl, and 5-14 membered heteroaryl containing 1, 2 or 3 heteroatoms selected from N, O or S, and at most two ring members in the cycloalkyl and heterocyclyl are C(=O);

Ring D is absent or is selected from the group consisting of: saturated or partially unsaturated $C_{3-10}$ cycloalkyl, and saturated or partially unsaturated 3-10 membered heterocyclyl containing 1, 2 or 3 heteroatoms selected from N, O or S.

**5.** The compound or the pharmaceutically acceptable salt, stereoisomer, polymorph, solvate, N-oxide, isotope labeled compound, metabolite or prodrug thereof according to claim 1, wherein the compound has a structure of any of the following formula:

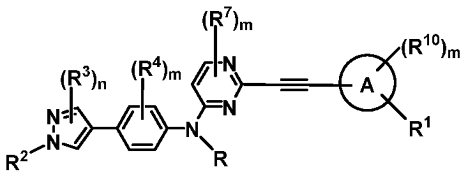

Formula Va

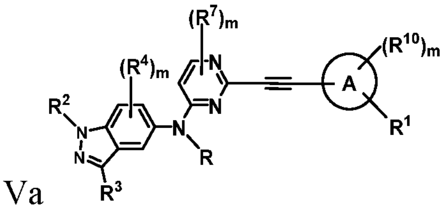

Formula Vb

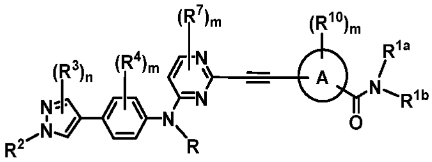

Formula Vc

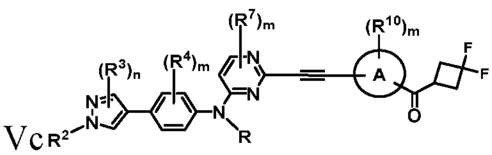

Formula Vd

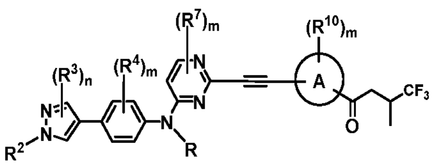

Formula Ve

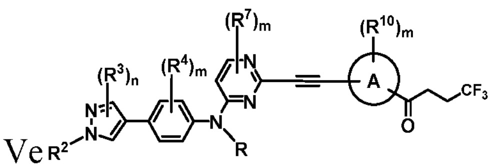

Formula Vf

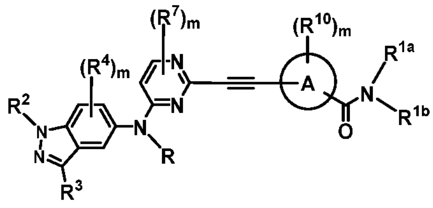
Formula Vg

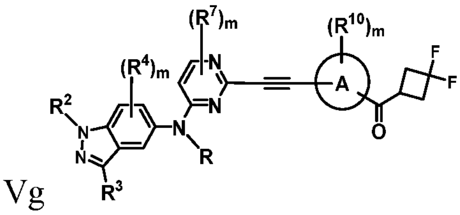
Formula Vh

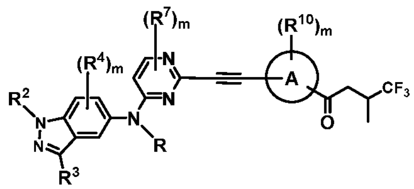
Formula Vi

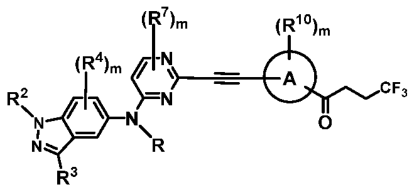
Formula Vj

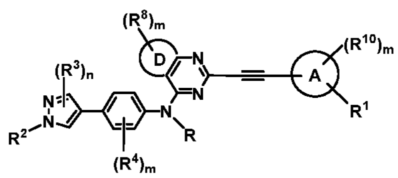
Formula Vk

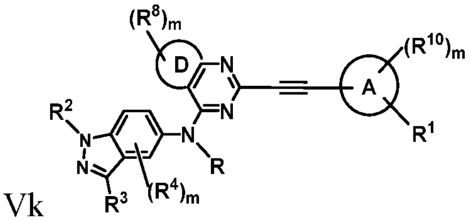
Formula Vl

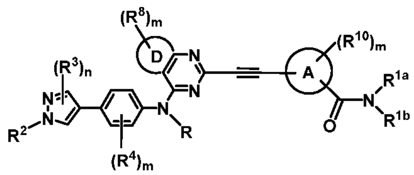
Formula Vm

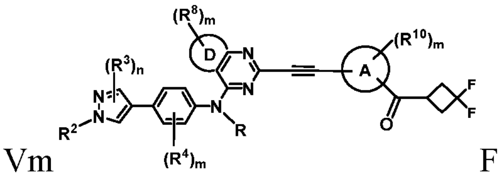
Formula Vn

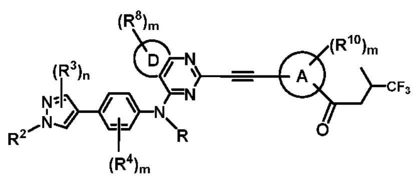
Formula Vp

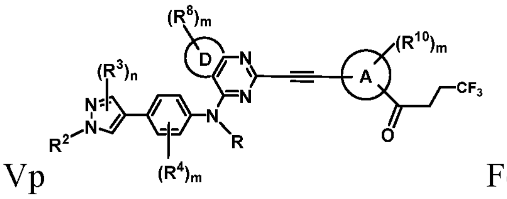
Formula Vq

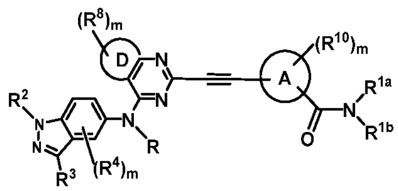
Formula Vr

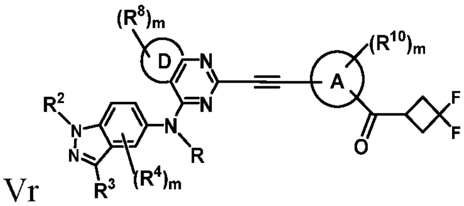
Formula Vs

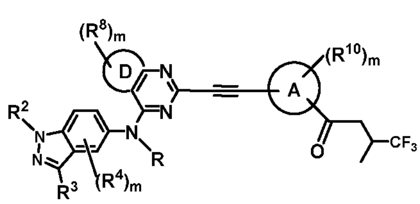
Formula Vt

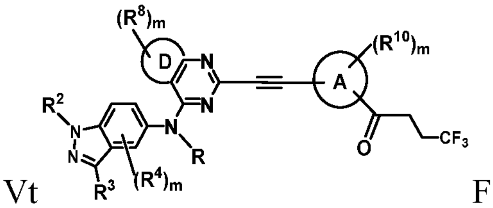
Formula Vu

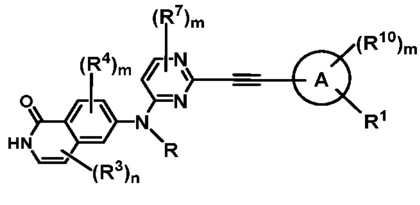
Formula Vw

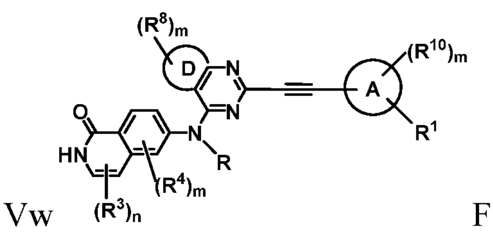
Formula Vx

wherein, each group is as defined in claim 1.

**6.** The compound or the pharmaceutically acceptable salt, stereoisomer, polymorph, solvate, N-oxide, isotope labeled compound, metabolite or prodrug thereof according to claim 1, wherein the compound is selected from the group consisting of:

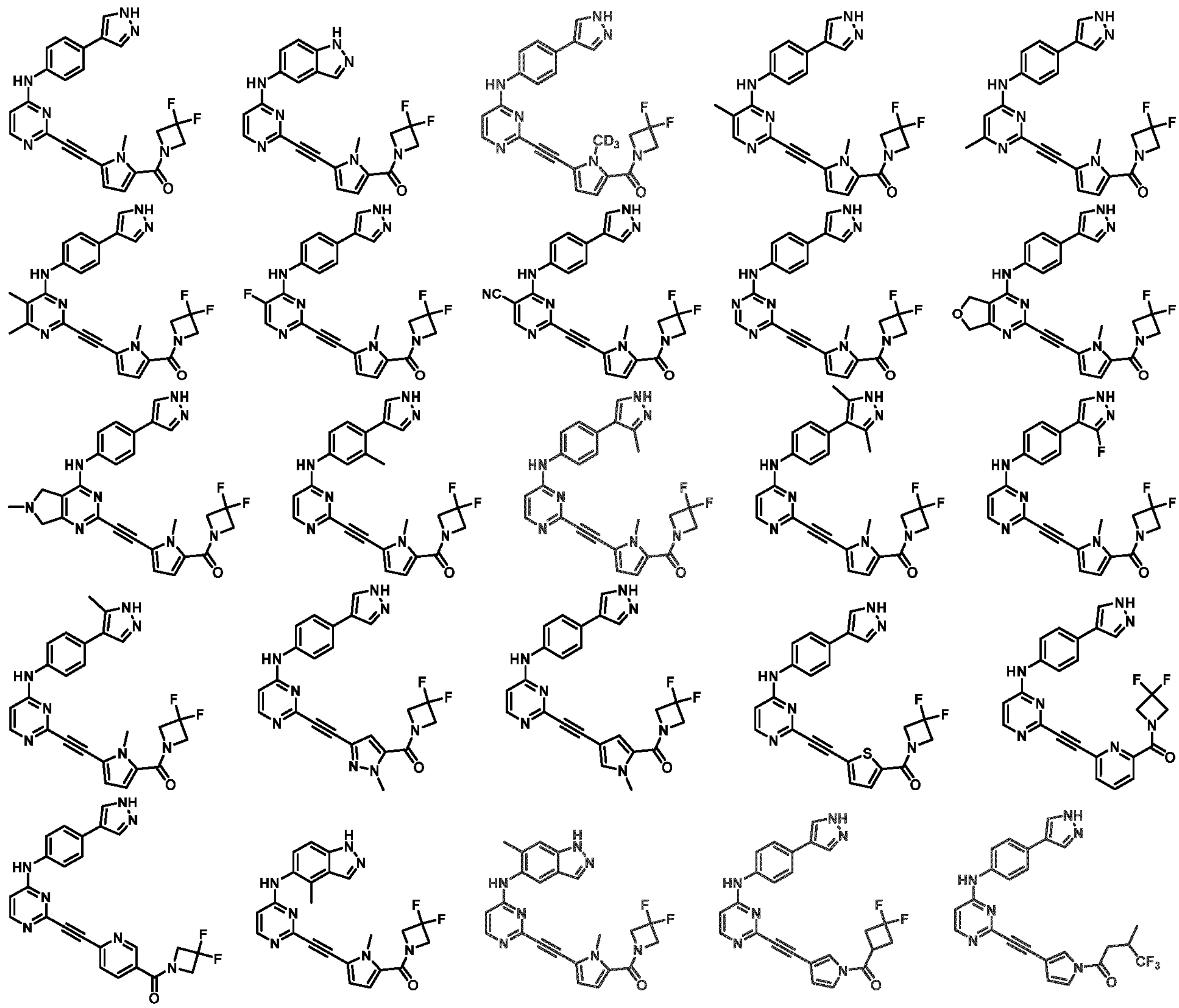

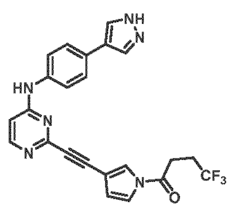
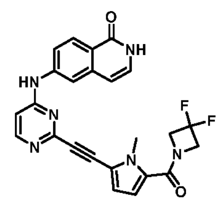
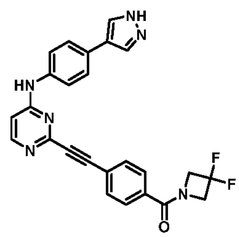
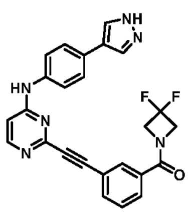
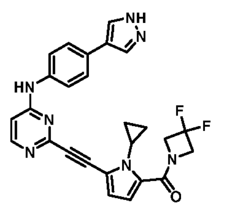

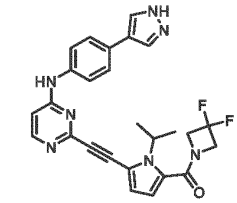
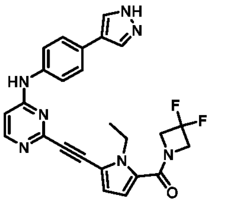
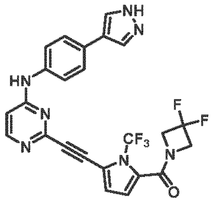
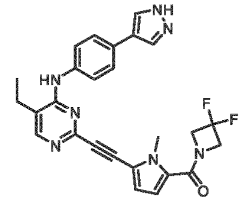
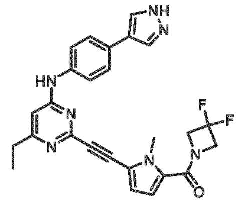
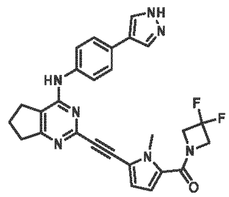
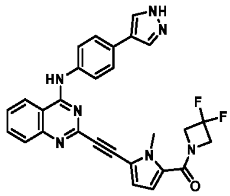
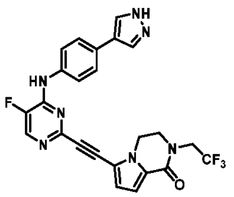
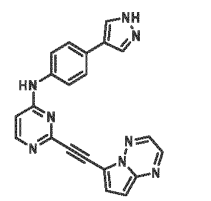
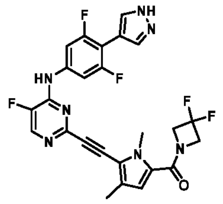
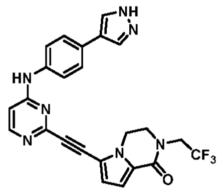
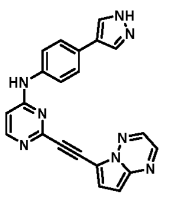
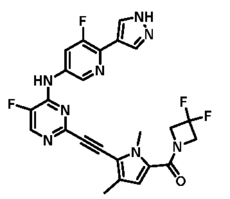
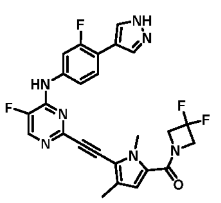
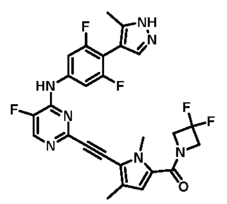
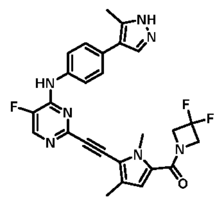
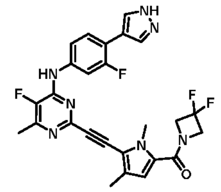
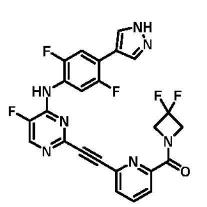
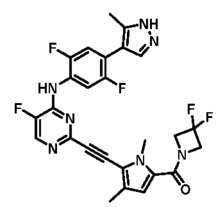
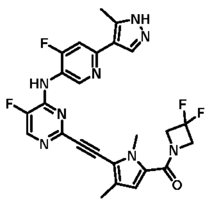
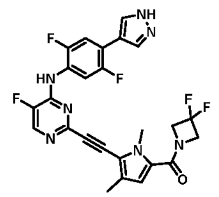
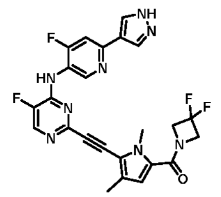
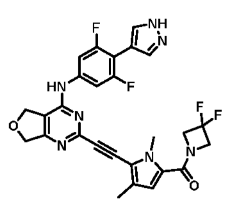
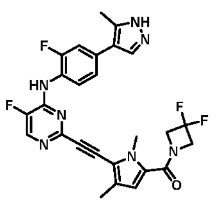
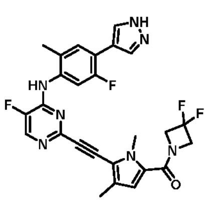
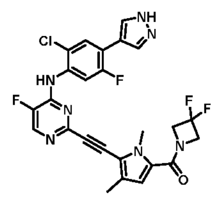
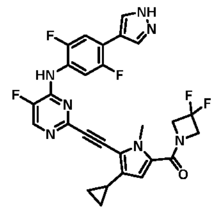
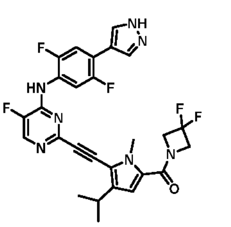
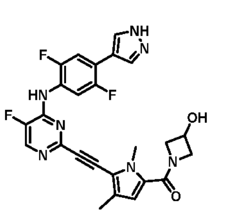
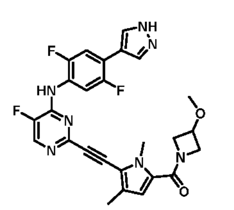
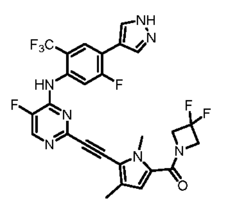

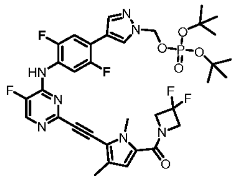
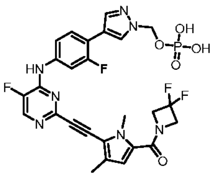
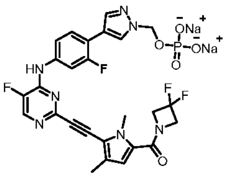
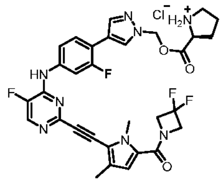
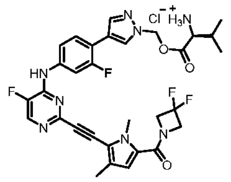
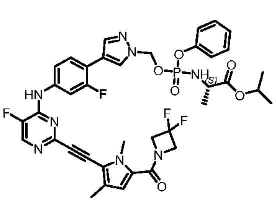
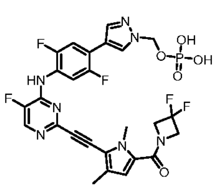
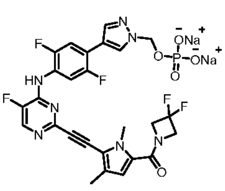
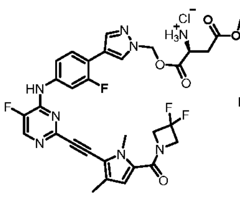
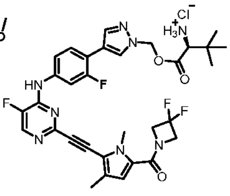
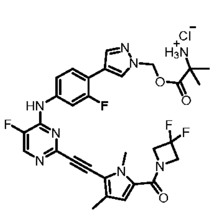
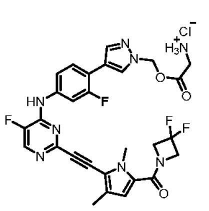
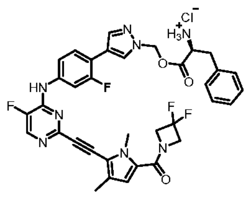
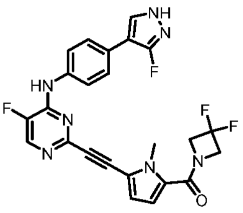
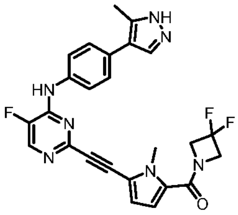
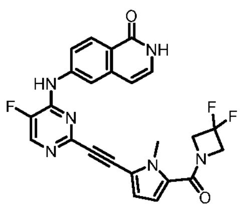
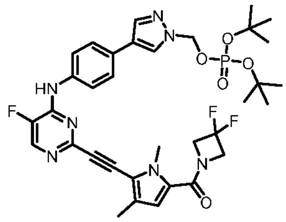

**7.** A pharmaceutical composition comprising one or more pharmaceutically acceptable carriers and a prophylactically and/or therapeutically effective amount of one or more of the compounds or the pharmaceutically acceptable salt, stereoisomer, polymorph, solvate, N-oxide, isotope labeled compound, metabolite or prodrug thereof of claim 1.

**8.** Use of the compound or the pharmaceutically acceptable salt, stereoisomer, polymorph, solvate, N-oxide, isotope labeled compound, metabolite or prodrug thereof of claim 1 for the preparation of a drug, the drug is used for the prevention and/or treatment of Rho-associated protein kinase-mediated diseases.

**9.** The use according to claim 8, wherein the Rho-associated protein kinase-mediated disease is selected from the group consisting of autoimmune disease, cardiovascular disease, inflammation, central nervous system disease, arterial thrombotic disease, fibrotic disease, neoplastic disease, metabolic syndrome, insulin resistance, hyperinsulinemia, type 2 diabetes mellitus, glucose intolerance, osteoporosis, and ocular disease.

**10.** The use according to claim 8, wherein the Rho-associated protein kinase-mediated disease is selected from the group consisting of lupus nephritis, atherosclerosis, rheumatoid arthritis (RA), hemangioma, angiofibroma, pulmonary fibrosis, hepatic fibrosis, psoriasis, corneal transplant rejection, insulin-dependent diabetes mellitus, multiple sclerosis, myasthenia gravis, Crohn's disease, autoimmune nephritis, Primary biliary cirrhosis, acute pancreatitis, allograft rejection, allergic inflammation, contact dermatitis, delayed hypersensitivity reaction, inflammatory bowel disease, infectious shock, osteoporosis, osteoarthritis, neuronal inflammation, Osler-Weber syndrome, restenosis, fungal infections, parasitic infections, and viral infections.

## INTERNATIONAL SEARCH REPORT

International application No.
**PCT/CN2023/089314**

**A. CLASSIFICATION OF SUBJECT MATTER**

C07D403/14(2006.01)i； A61K31/506(2006.01)i； A61P19/02(2006.01)i； A61P17/00(2006.01)i

According to International Patent Classification (IPC) or to both national classification and IPC

**B. FIELDS SEARCHED**

Minimum documentation searched (classification system followed by classification symbols)

C07D; A61K; A61P

Documentation searched other than minimum documentation to the extent that such documents are included in the fields searched

Electronic data base consulted during the international search (name of data base and, where practicable, search terms used)

VCN, DWPI, ENTXT, CNKI, STN(REGISTRY, CAPLUS): 上海挚盟医药, 炔, ROH, ROCK, ALKYN+, structural formula search

**C. DOCUMENTS CONSIDERED TO BE RELEVANT**

| Category* | Citation of document, with indication, where appropriate, of the relevant passages | Relevant to claim No. |
|---|---|---|
| A | CN 111217797 A (BEIJING TIDE PHARMACEUTICAL CO., LTD.) 02 June 2020 (2020-06-02)<br>entire document | 1-10 |
| A | CN 110582489 A (BEIJING TIDE PHARMACEUTICAL CO., LTD.) 17 December 2019 (2019-12-17)<br>entire document | 1-10 |
| A | CN 110582491 A (BEIJING TIDE PHARMACEUTICAL CO., LTD.) 17 December 2019 (2019-12-17)<br>entire document | 1-10 |
| A | WO 2019000683 A1 (BEIJING TIDE PHARMACEUTICAL CO., LTD.) 03 January 2019 (2019-01-03)<br>entire document | 1-10 |
| A | WO 2019001572 A1 (BEIJING TIDE PHARMACEUTICAL CO., LTD.) 03 January 2019 (2019-01-03)<br>entire document | 1-10 |

☑ Further documents are listed in the continuation of Box C.  ☑ See patent family annex.

* Special categories of cited documents:
- "A" document defining the general state of the art which is not considered to be of particular relevance
- "D" document cited by the applicant in the international application
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **29 June 2023** | **10 July 2023** |
| Name and mailing address of the ISA/CN<br>**China National Intellectual Property Administration (ISA/CN)**<br>**China No. 6, Xitucheng Road, Jimenqiao, Haidian District, Beijing 100088** | Authorized officer<br><br>Telephone No. |

Form PCT/ISA/210 (second sheet) (July 2022)

**INTERNATIONAL SEARCH REPORT**

International application No.
**PCT/CN2023/089314**

**C. DOCUMENTS CONSIDERED TO BE RELEVANT**

| Category* | Citation of document, with indication, where appropriate, of the relevant passages | Relevant to claim No. |
|---|---|---|
| A | WO 2022012409 A1 (WUHAN LL SCIENCE AND TECHNOLOGY DEVELOPMENT CO., LTD.) 20 January 2022 (2022-01-20) entire document | 1-10 |

Form PCT/ISA/210 (second sheet) (July 2022)

**INTERNATIONAL SEARCH REPORT**
**Information on patent family members**

International application No.
**PCT/CN2023/089314**

| Patent document cited in search report | Publication date (day/month/year) | Patent family member(s) | Publication date (day/month/year) |
|---|---|---|---|
| CN 111217797 A | 02 June 2020 | None | |
| CN 110582489 A | 17 December 2019 | AU 2018294054 A1 | 02 January 2020 |
| | | AU 2018294054 B2 | 26 May 2022 |
| | | KR 20200016297 A | 14 February 2020 |
| | | EP 3647311 A1 | 06 May 2020 |
| | | EP 3647311 A4 | 06 January 2021 |
| | | US 2023030115 A1 | 02 February 2023 |
| | | US 2019276440 A1 | 12 September 2019 |
| | | US 11390609 B2 | 19 July 2022 |
| | | JP 2020525522 A | 27 August 2020 |
| | | CA 3063616 A1 | 06 December 2019 |
| | | WO 2019001572 A1 | 03 January 2019 |
| CN 110582491 A | 17 December 2019 | KR 20200024777 A | 09 March 2020 |
| | | KR 102469161 B1 | 23 November 2022 |
| | | JP 2020525523 A | 27 August 2020 |
| | | JP 7039802 B2 | 23 March 2022 |
| | | WO 2019000683 A1 | 03 January 2019 |
| | | US 2019002442 A1 | 03 January 2019 |
| | | US 10323023 B2 | 18 June 2019 |
| | | EP 3421464 A1 | 02 January 2019 |
| | | EP 3421464 B1 | 24 November 2021 |
| WO 2019000683 A1 | 03 January 2019 | None | |
| WO 2019001572 A1 | 03 January 2019 | None | |
| WO 2022012409 A1 | 20 January 2022 | EP 4155308 A1 | 29 March 2023 |
| | | KR 20230038697 A | 21 March 2023 |
| | | TW 202214628 A | 16 April 2022 |
| | | AU 2021309876 A1 | 23 February 2023 |
| | | CA 3184616 A1 | 20 January 2022 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **SAMBROOK et al.** Molecular Cloning: Laboratory Manual. Cold Spring Harbor Laboratory Press, 1989